# FASCICULE DE BREVET EUROPEEN

(11) **EP 1 776 347 B1**
(45) Date de publication et mention de la délivrance du brevet: **26.05.2010**
(21) Numéro de dépôt: 05796062.7
(22) Date de dépôt: 02.08.2005
(51) Int. Cl.: C07D 233/66

(54) **DERIVES DE SULFONAMIDES, LEUR PREPARATION ET LEUR APPLICATION EN THERAPEUTIQUE**
SULFONAMIDDERIVATE, IHRE HERSTELLUNG UND IHRE THERAPEUTISCHE ANWENDUNG
SULPHONAMIDE DERIVATIVES, THEIR PREPARATION AND THEIR THERAPEUTIC APPLICATION

(30) Priorité: 03.08.2004 FR 0408546
(43) Date de publication de la demande: 25.04.2007
(73) Titulaire: Sanofi-Aventis, 75013 Paris (FR)
(72) Inventeur: COURTEMANCHE, Gilles, F-31600 Saubens (FR); DESPEYROUX, Pierre, F-31860 Labarthe sur Leze (FR); FONTAINE, Evelyne, F-31750 Escalquens (FR); ROCHARD, Pierrick, F-31120 Roques-sur-Garonne (FR); SERRADEIL LE GAL, Claudine, F-31750 Escalquens (FR)
(74) Mandataire: Kugel, Dominique
(86) Numéro de dépôt international: PCT/FR2005/002017
(87) Numéro de publication internationale: WO 2006/024779

(56) Documents cités:
- EP-A- 0 358 571
- EP-A- 1 144 409
- WO-A-99/12916
- WO-A-03/032991
- SAKURAI T ET AL: "Oxerins and oxerin receptors: A family of hypothalamic neuropeptides and G Protein-coupled receptors that regulate feeding behaviour" CELL, CELL PRESS, CAMBRIDGE, NA, US, vol. 92, 20 février 1998 (1998-02-20), pages 573-585, XP002105412 ISSN: 0092-8674 cité dans la demande

## Description

La présente invention a pour objet des dérivés de sulfonamides, leur procédé de préparation et leur utilisation en thérapeutique.

Les orexines A et B (ou hypocrétines 1 et 2) sont des neuropeptides hypothalamiques de 33 et de 28 acides aminés respectivement, récemment identifiés comme les ligands endogènes de deux récepteurs à sept domaines transmembranaires, nommés récepteurs orexine 1 et orexine 2 (Sakurai T., Cell, Vol 92, 573-585, 1998; De Lecea L., Proc. Natl. Acad. Sci., Vol. 95, 322-327, 1998).

Le récepteur d'orexine 2 a la propriété de reconnaître les deux formes d'orexine A et B de façon équivalente. Par contre, le récepteur orexine 1, qui présente 64% d'homologie avec le récepteur orexine 2, est plus sélectif et lie dix fois mieux l'orexine A que l'orexine B (Sakurai T., Cell, Vol 92, 573-585, 1998).

Via ces récepteurs, les orexines controlent diverses fonctions centrales et périphériques, notamment la prise de nourriture et de boisson, certaines fonctions endocrines cardiovasculaires et le cycle d'éveil/sommeil (Sakurai T., Regulatory Peptides, Vol 85, 25-30, 1999). Des derivés thiourées et sulfonyles sont connus de l'art antérieur comme ayant une affinité pour les récepteurs de l'orexine (WO 03/032991; EP 1 144 409).

Il a maintenant été trouvé que certains dérivés de sulfonamides présentent une grande affinité vis-à-vis des récepteurs de l'orexine 2 et sont de puissants antagonistes de ces récepteurs.

Ainsi, la présente invention a pour objet des composés répondant à la formule générale (I) 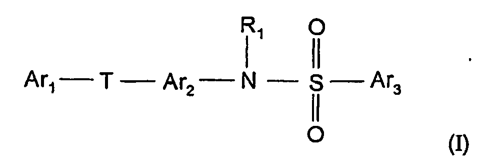 dans laquelle
- **Ar₁** représente
   - un groupe aryle tel qu'un groupe phényle éventuellement substitué par un ou plusieurs groupes choisis indépendamment les uns des autres parmi les groupes suivants un atome d'halogène, un groupe (C₁-C₄) alkyle, un groupe (C₁-C₄) alcoxy, un groupe fluoro(C₁-C₄)alkyle, un groupe cyano, un groupe -CO-NRₐR_{b}, un groupe -NRₐR_{b},
      avec Rₐ et R_{b} étant indépendamment l'un de l'autre un atome d'hydrogène, un groupe (C₁-C₄)alkyle,
   - un groupe hétérocyclyle choisi parmi le pyridinyle, le pyrimidinyle, le thiazolyle, le thiényle, lesdits groupes hétérocyclyles étant éventuellement substitués par un atome d'halogène, un groupe (C₁-C₄) alkyle, un groupe (C₁-C₄) alcoxy,
   - un groupe (C₃-C₆) cycloalkyle ;
- **T** représente
   - un groupe -(CH₂)ₙ- avec n = 0,1,2,
   - un groupe 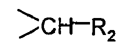 avec R₂ étant un groupe hydroxyle, un groupe (C₁-C₄) alkyle,
   - un groupe 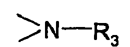 avec R₃ étant un groupe (C₁-C₄) alkyle,
   - un groupe 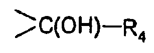 avec R₄ étant un groupe (C₁-C₄) alkyle ;
- **Ar₂** représente
   - un groupe aryle tel que le phényle éventuellement substitué par un ou plusieurs groupes choisis indépendamment les uns des autres parmi les groupes suivants : un atome d'halogène, un groupe (C₁-C₄) alkyle, un groupe (C₁-C₄) alcoxy, un groupe fluoro(C₁-C₄)alkyle, un groupe fluoro(C₁-C₄)alcoxy,
      ou bien par un groupe -NR_{c}R_{d} avec R_{c} et R_{d} étant indépendamment l'un de l'autre un atome d'hydrogène, un groupe (C₁-C₄)alkyle,
   - un groupe hétérocyclyle tel que le pyridinyle éventuellement substitué par un groupe (C₁-C₄)alkyle, un groupe (C₁-C₄) alcoxy ;
- **Ar₃** représente
   - un groupe aryle choisi parmi le phényle et le naphtyle, lesdits groupes aryles étant éventuellement substitués par un ou plusieurs groupes choisis indépendamment les uns des autres parmi les groupes suivants : un atome d'halogène, un groupe (C₁-C₄) alkyle, un groupe (C₁-C₄) alcoxy, un groupe fluoro(C₁-C₄)alkyle, un groupe fluoro(C₁-C₄)alcoxy, un groupe nitro, un groupe hydroxy,
      ou bien par un groupe -NR₅R₆ avec R₅ et R₆ étant, indépendamment l'un de l'autre, un atome d'hydrogène, un groupe (C₁-C₄) alkyle,
   - un groupe hétérocyclyle tel que le pyridinyle éventuellement substitué par un groupe (C₁-C₄) alkyle ou un (C₁-C₄) alcoxy,
      ou bien par un groupe -NRₓR_{y} avec Rₓ et R_{y} étant indépendemment l'un de l'autre un atome d'hydrogène, un groupe (C₁-C₄) alkyle, un groupe (C₁-C₄) alcoxy,
   - un groupe choisi parmi 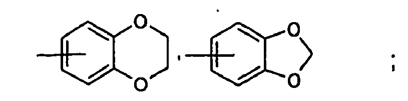
- R₁ représente
   - un groupe -C(O)-CF₃,
   - un groupe de formule 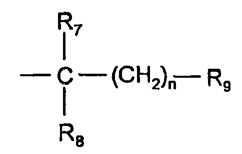 dans laquelle
      n=0,1,2,3
      o R₇ représente
         - un atome d'hydrogène, un groupe (C₁-C₄) alkyle, un atome de fluor, un groupe (C₁-C₄) alcoxy,
         - un groupe -(CH₂)ₘ-aryle, avec m=1, 2, et le groupe aryle étant un groupe phényle éventuellement substitué par un atome d'halogène, un groupe (C₁-C₄) alkyle, un groupe (C₁-C₄)alcoxy,
      o R₈ repésente
         - un atome d'hydrogène, un atome de fluor, un groupe (C₁-C₄) alkyle,
      o R₉ représente
         - un atome d'hydrogène, un groupe (C₁-C₄) alkyle, un groupe (C₃-C₆) cycloalkyle, un groupe hydroxyle, un groupe (C₁-C₄) alcoxy, un groupe fluoro(C₁-C₄)alkyle, un groupe -C≡CH, un groupe -C≡N, un groupe phénoxy, un groupe (C₁-C₄)alcényle,
         - un groupe -NR₁₀R₁₁ avec R₁₀ et R₁₁ étant indépendamment l'un de l'autre un atome d'hydrogène, un groupe (C₁-C₄) alkyle, un groupe (C₁-C₄) alkylcarbonyle, un groupe (C₁-C₄) alkylsulfonyle, un benzyle, un groupe -(CH₂)₂-N(CH₃)₂,
            ou bien R₁₀ et R₁₁ forment ensemble avec l'atome d'azote qui les porte une pyrrolidine, une pyrrolidinone, une morpholine ou une pipéridine,
         - un groupe -CO-NR₁₂R₁₃ avec R₁₂ et R₁₃ étant indépendamment l'un de l'autre
      o un atome d'hydrogène,
      o un groupe (C₁-C₄) alkyle éventuellement substitué par un groupe -C≡N, un groupe aryle tel que le phényle, un groupe hétérocyclyle tel que le pyridinyle, lesdits groupes aryle et hétérocyclyle étant éventuellement substitués par un groupe (C₁-C₄) alkyle, un groupe hydroxyle,
         ou bien R₁₂ et R₁₃ forment ensemble avec l'atome d'azote qui les porte un groupe 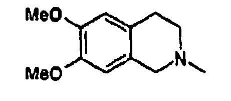
         - un groupe -COOR₁₄ avec R₁₄ étant un groupe (C₁-C₄) alkyle,
         - un groupe -NH-CO-NR₁₅R₁₈ avec R₁₅ et R₁₆ étant indépendamment l'un de l'autre un atome d'hydrogène, un groupe (C₁-C₄) alkyle, un groupe phényle, un groupe benzyle, lesdits groupes phényle et benzyle étant éventuellement substitués par un ou plusieurs substituants choisis parmi un atome d'halogène, un groupe (C₁-C₄)alkyle, un groupe (C₁-C₄) alcoxy,
         - un groupe -SO₂-NR₁₇R₁₈ avec R₁₇ et R₁₈ étant indépendamment l'un de l'autre un atome d'hydrogène, un groupe (C₁-C₄) alkyle,
         - un groupe -SO₂-R₁₉ avec R₁₉ étant un groupe (C₁-C₄) alkyle, un groupe aryle tel que le phényle,
         - un groupe hétérocyclyle choisi parmi le 1,3-dioxolanyle, l'imidazolyle, le tétrazolyle, le triazolyle éventuellement substitué par un groupe (C₁-C₄) alkyle, le thiazolyle, le pyrimidinyle, l'oxadiazolyle, le pyridinyle, l'imidazolyle étant éventuellement substitué sur l'atome d'azote par un groupe (C₁-C₄) alkyle ou un benzyle, le tétrazolyle étant éventuellement substitué par un groupe (C₁-C₄) alkyle ou un benzyle, l'oxadiazolyle étant éventuellement substitué par un (C₁-C₄) alkyle,
         - un groupe -NH-CO-OR₁₅, R₁₅ répondant à la définition ci-dessus,
         le composé suivant étant exclu - N-méthyl benzyl-2' chloro-4' tosanilide,
         à l'état de base, de sel d'addition à un acide, d'hydrate ou de solvat, sous forme d'énantiomères, de diastéréoisomères, de rotamères, d'atropoisomères ou de leurs mélanges.

Parmi les composés objets de l'invention, on peut citer un premier groupe de composés de formule générale (I), dans laquelle
- Ar₁ représente
   - un groupe aryle tel qu'un groupe phényle éventuellement substitué par un ou plusieurs groupes choisis indépendamment les uns des autres parmi les groupes suivants : un atome d'halogène, un groupe (C₁-C₄) alkyle, un groupe (C₁-C₄) alcoxy, un groupe fluoro(C₁-C₄)alkyle,
   - un groupe hétérocyclyle choisi parmi le pyridinyle, le thiazolyle, le thiényle, le thiényle étant éventuellement substitué par un groupe (C₁-C₄) alkyle,
   - un groupe (C₃-C₆) cycloalkyle ;
- **T** représente
   - un groupe -(CH₂)ₙ- avec n = 1
   - un groupe 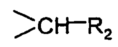 avec R₂ étant un groupe hydroxyle, un groupe (C₁-C₄) alkyle,
   - un groupe 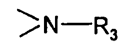 avec R₃ étant un groupe (C₁-C₄) alkyle,
   - un groupe 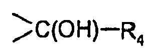 avec R₄ étant un groupe (C₁-C₄) alkyle ;
- **Ar₂** représente
   - un groupe aryle tel que le phényle éventuellement substitué par un ou plusieurs groupes choisis indépendamment les uns des autres parmi les groupes suivants : un atome d'halogène, un groupe (C₁-C₄) alkyle, un groupe (C₁-C₄) alcoxy, un groupe fluoro(C₁-C₄)alcoxy, un groupe -NR_{c}R_{d} avec R_{c} et R_{d} étant, indépendamment l'un de l'autre, un atome d'hydrogène, un groupe (C₁-C₄) alkyle ;
- **Ar₃** représente
   - un groupe aryle choisi parmi le phényle et le naphtyle, le groupe phényle étant éventuellement substitué par un ou plusieurs groupes choisis indépendamment les uns des autres parmi les groupes suivants : un atome d'halogène, un groupe (C₁-C₄) alkyle, un groupe (C₁-C₄) alcoxy, un groupe fluoro(C₁-C₄)alkyle, groupe fluoro(C₁-C₄)alcoxy, un groupe nitro,
      ou bien un groupe -NR₅R₆ avec R₅ et R₆ étant, indépendamment l'un de l'autre, un atome d'hydrogène, un groupe (C₁-C₄) alkyle,
   - un groupe choisi parmi 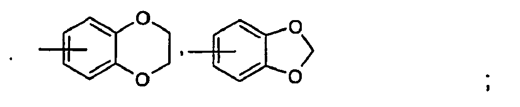
- **R₁** représente
   - un groupe -C(O)-CF₃,
   - un groupe de formule 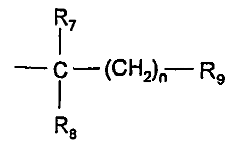 dans laquelle
      n = 0, 1, 2,
      o R₇ représente
         - un atome d'hydrogène, un groupe (C₁-C₄) alkyle
      o R₈ repésente
         - un atome d'hydrogène,
      o R₉ représente
         - un atome d'hydrogène, un groupe (C₁-C₄) alkyle, un groupe hydroxyle, un groupe (C₁-C₄) alcoxy, un groupe fluoro(C₁-C₄) alkyle, un groupe -C≡CH, un groupe -C≡N,
         - un groupe -NR₁₀R₁₁ avec R₁₀ et R₁₁ étant indépendamment l'un de l'autre un atome d'hydrogène, un groupe (C₁-C₄) alkyle, un groupe (C₁-C₄) alkylcarbonyle, un benzyle,
            ou bien R₁₀ et R₁₁ forment ensemble avec l'atome d'azote qui les porte une pyrrolidine une pipéridine ou une pyrrolidinone,
         - un groupe -CO-NR₁₂R₁₃ avec R₁₂ et R₁₃ étant indépendamment l'un de l'autre un atome d'hydrogène, un groupe (C₁-C₄) alkyle,
            ou bien R₁₂ et R₁₃ forment ensemble avec l'atome d'azote qui les porte un groupe 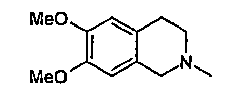
         - un groupe -COOR₁₄ avec R₁₄ étant un groupe (C₁-C₄) alkyle,
         - un groupe -NH-CO-NR₁₅R₁₆ avec R₁₅ et R₁₆ étant indépendamment l'un de l'autre un atome d'hydrogène, un groupe (C₁-C₄) alkyle, un phényle, un benzyle,
         - un groupe hétérocyclyle choisi parmi l'imidazolyle éventuellement substitué sur l'atome d'azote par un groupe (C₁-C₄) alkyle, le tétrazolyle éventuellement substitué par un groupe (C₁-C₄) alkyle, le triazolyle,
         à l'état de base, de sel d'addition à un acide, d'hydrate ou de solvat, sous forme d'énantiomères, de diastéréoisomères, de rotamères, d'atropoisoméres ou de leurs mélanges.

Parmi les composés objets de l'invention, on peut citer un second groupe de composés de formule générale (I), dans laquelle
- **Ar₁** représente
   - un groupe aryle tel que le phényle éventuellement substitué par un ou plusieurs groupes choisis indépendamment les uns des autres parmi les groupes suivants : un atome d'halogène, un groupe (C₁-C₄) alkyle, un groupe (C₁-C₄) alcoxy, un groupe fluoro(C₁-C₄)alkyle,
   - un groupe hétérocyclyle choisi parmi le pyridinyle, le thiazolyle, le thiényle, le thiényle étant éventuellement substitué par un groupe (C₁-C₄) alkyle ;
- **T** représente
   - un groupe -(CH₂)ₙ- avec n = 1
   - un groupe 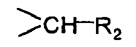 avec R₂ étant un groupe hydroxyle ;
- **Ar₂** représente
   - un groupe aryle tel que le phényle éventuellement substitué par un ou plusieurs groupes choisis indépendamment les uns des autres parmi les groupes suivants : un atome d'halogène, un groupe (C₁-C₄) alkyle, un groupe (C₁-C₄) alcoxy ; un groupe
   - NR_{c}R_{d} avec Rₑ et R_{d} étant, indépendamment l'un de l'autre, un atome d'hydrogène, un groupe (C₁-C₄) alkyle ;
- **Ar**₃ représente
   - un groupe aryle tel que le phényle, le groupe phényle étant éventuellement substitué par.un ou plusieurs groupes choisis indépendamment les uns des autres parmi les groupes suivants : un atome d'halogène, un groupe (C₁-C₄) alkyle, un groupe (C₁-C₄) alcoxy,
   ou bien un groupe -NR₅R₈ avec R₅ et R₆ étant, indépendamment l'un de l'autre, un atome d'hydrogène, un groupe (C₁-C₄) alkyle ;
- **R₁** représente
   - un groupe de formule 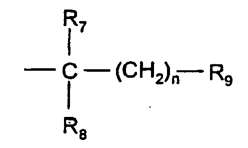 dans laquelle
      n = 0, 1, 2,
      o R₇ représente
         - un atome d'hydrogène, un groupe (C₁-C₄) alkyle,
      o R₈ repésente
         - un atome d'hydrogène,
      o R₉ représente
         - un atome d'hydrogène, un groupe -C≡CH, un groupe -C≡N,
         - un groupe -CO-NR₁₂R₁₃ avec R₁₂ et R₁₃ étant indépendamment l'un de l'autre un atome d'hydrogène, un groupe (C₁-C₄) alkyle
         - un hétérocyclyle choisi parmi l'imidazolyle éventuellement substitué sur l'atome d'azote par un groupe (C₁-C₄) alkyle, le tétrazolyle éventuellement substitué par un groupe (C₁-C₄) alkyle, le triazolyle,
         à l'état de base, de sel d'addition à un acide, d'hydrate ou de solvat, sous forme d'énantiomères, de diastéréoisomères, de rotamères, d'atropoisoméres ou de leurs mélanges.

Parmi les composés objets de l'invention, on peut citer un troisième groupe de composés de formule générale (I), dans laquelle
- Ar₁ représente
   - un groupe aryle tel que le phényle éventuellement substitué par un ou plusieurs groupes choisis indépendamment les uns des autres parmi les groupes suivants : un atome d'halogène, un groupe (C₁-C₄) alkyle, un groupe (C₁-C₄) alcoxy,
   - un groupe hétérocyclyle tel que le thiényle éventuellement substitué par un groupe (C₁-C₄) alkyle,
- T représente
   - un groupe -(CH₂)ₙ- avec n = 1
- Ar₂ représente
   - un groupe phényle éventuellement substitué par un ou plusieurs groupes choisis indépendamment les uns des autres parmi les groupes suivants : un atome d'halogène, un groupe (C₁-C₄) alkyle, un groupe (C₁-C₄) alcoxy, un groupe -NR_{c}R_{d} avec R_{c} et R_{d} étant, indépendamment l'un de l'autre, un atome d'hydrogène, un groupe (C₁-C₄) alkyle;
- Ar₃ représente
   - un groupe phényle éventuellement substitué par un ou plusieurs groupes choisis indépendamment les uns des autres parmi les groupes suivants : un atome d'halogène, un groupe (C₁-C₄) alkyle, un groupe (C₁-C₄) alcoxy,
      ou bien un groupe -NR₅R₆ avec R₅ et R₆ étant, indépendamment l'un de l'autre, un atome d'hydrogène, un groupe (C₁-C₄) alkyle;
- R₁ représente
   - un groupe de formule 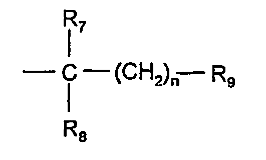 dans lequel
      n = 0, 1, 2,
      o R₇ représente
         - un atome d'hydrogène, un groupe (C₁-C₄) alkyle ;
      o R₈ repésente
         - un atome d'hydrogène ;
      o R₉ représente
         - un groupe -CO-NR₁₂R₁₃ avec R₁₂ et R₁₃ étant indépendamment l'un de l'autre un atome d'hydrogène, un groupe (C₁-C₄) alkyle,
         - un hétérocycle choisi parmi l'imidazolyle substitué sur l'atome d'azote par un groupe (C₁-C₄) alkyle, le tétrazolyle substitué par un groupe (C₁-C₄) alkyle, le triazolyle,
         à l'état de base, de sel d'addition à un acide, d'hydrate ou de solvat, sous forme d'énantiomères, de diastéréoisomères, de rotamères, d'atropoisoméres ou de leurs mélanges.

Lorsque Ar₂ est un groupe phényle éventuellement substitué, les liaisons T-Ar₂ d'une part et Ar₂-N d'autre part sont en position ortho. Autrement dit, l'atome d'azote et le substituant T sont sur deux atomes de carbone adjacents.

A titre d'exemple de composés selon l'invention, on peut citer les composés suivants :

| | |
|---|---|
| Composé 1 : Ethyl N-{4-chloro-2-[(2-chlorophényl)(hydroxy)méthyl]phényl}-N-[(3,4-diméthoxyphényl)sulfonyl]glycinate | 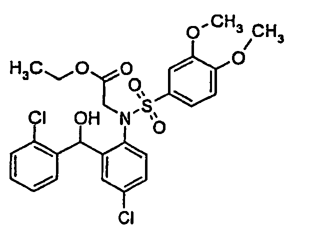 |
| Composé 2 : Ethyl N-(2-benzyl-4-chlorophényl)-N-[(3,4-diméthoxyphényl)sulfonyl]glycinate | 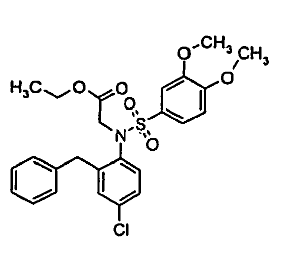 |
| Composé 3 : Ethyl N-[4-chloro-2-(2-chlorobenzyl)phényl]-N-[(3-méthoxyphényl)sulfonyl]glycinate | 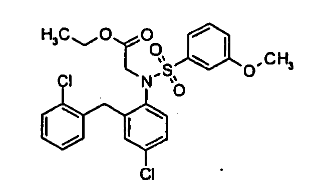 |
| Composé 4 : Ethyl N-[4-chloro-2-(2-chlorobenzyl)phényl]-N-[(2-méthoxyphényl)sulfonyl]glycinate | 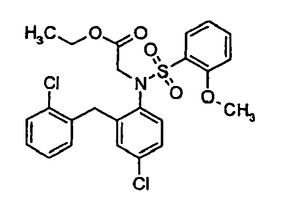 |
| Composé 5 : Ethyl N-[4-chloro-2-(2-chlorobenzyl)phényl]-N-[(2,5-diméthoxyphényl)sulfonyl]glycinate | 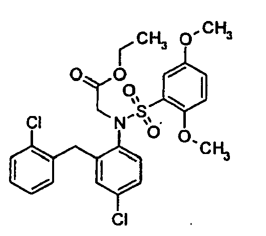 |
| Composé 6 : Ethyl N-[4-chloro-2-(2-chlorobenzyl)phényl]-N-[(2,4-diméthoxyphényl)sulfonyl]glycinate | 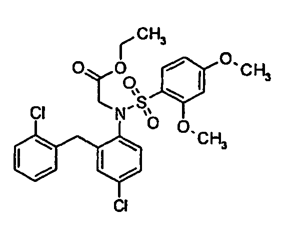 |
| Composé 7 : Ethyl N-{4-chloro-2-[hydroxy(phényl)méthyl]phényl}-N-[(3,4-diméthoxyphényl)sulfonyl]glycinate | 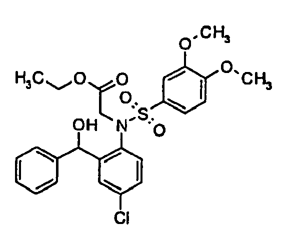 |
| Composé 8 : Ethyl N-[(3,4-diméthoxyphényl)sulfonyl]-N-{2-[hydroxy(phényl)méthyl]phényl}glycinate | 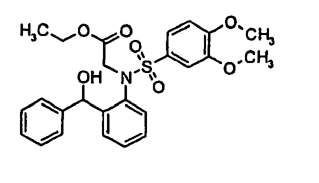 |
| Composé 9 : Ethyl N-{4-chloro-2-[(2-chlorophényl)(hydroxy)méthyl]phényl}-N-[(2,4-diméthoxyphényl)sulfonyl]glycinate | 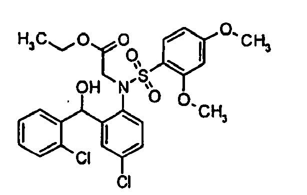 |
| Composé 10 : Ethyl N-{4-chloro-2-[(2-chlorophényl)(hydroxy)méthyl]phényl}-N-(phénylsulfonyl)glycinate | 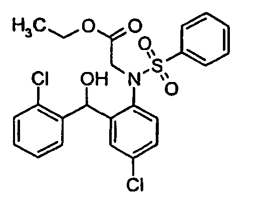 |
| Composé 11 : N-{4-chloro-2-[(2-chlorophényl)(hydroxy)méthyl]phényl}-N-[(2-éthyl-2H-tétrazol-5-yl)méthyl]-3,4-diméthoxybenzènesulfonamide | 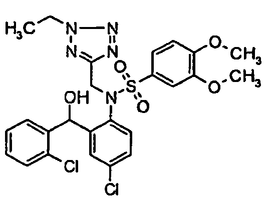 |
| Composé 12 : N-{4-chloro-2-[(2-chlorophényl)(hydroxy)méthyl]phényl}-N-[(1-éthyl-1H-tétrazol-5-yl)méthyl]-3,4-diméthoxybenzènesulfonamide | 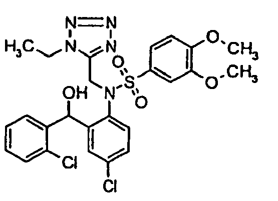 |
| Composé 13 : Ethyl N-{4-chloro-2-[(2-chlorophényl)(hydroxy)méthyl]phényl}-N-[(4-méthoxyphényl)sulfonyl]glycinate | 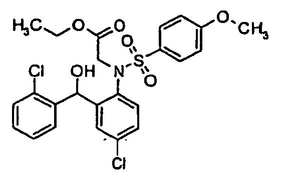 |
| Composé 14 : N-{4-chloro-2-[(2-chlorophényl)(hydroxy)méthyl]phényl}-3,4-diméthoxy-N-[(2-méthyl-2H-tétrazol-5-yl)méthyl]benzènesulfonamide | 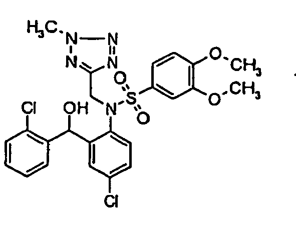 |
| Composé 15 : N-{4-chloro-2-[(2-chlorophényl)(hydroxy)méthyl]phényl}-3,4-diméthoxy-N-[(1-méthyl-1H-tétrazol-5-yl)méthyl]benzènesulfonamide | 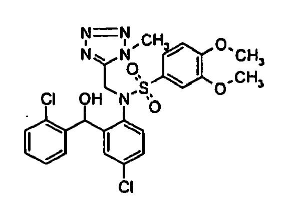 |
| Composé 16 : Ethyl N-{4-chloro-2-[(3-chlorophényl)(hydroxy)méthyl]phényl}-N-[(3,4-diméthoxyphényl)sulfonyl]glycinate | 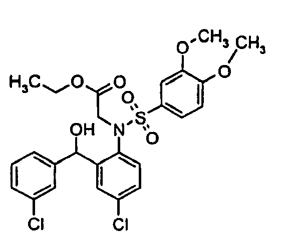 |
| Composé 17 : Ethyl N-{4-chloro-2-[hydroxy(2-méthoxyphényl)méthyl]phényl}-N-[(3,4-diméthoxyphényl)sulfonyl]glycinate | 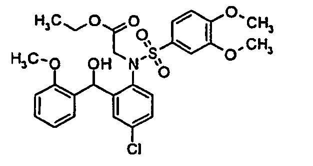 |
| Composé 18 : Ethyl N-{4-chloro-2-[(2-chlorophényl)(hydroxy)méthyl]phényl}-N-[(3,4-dichlorophényl)sulfonyl]glycinate | 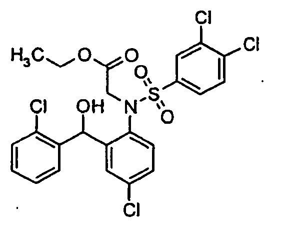 |
| Composé 19 : N²-{4-chloro-2-[(2-chlorophényl)(hydroxy)méthyl]phényl}-N~2~-[(3,4-diméthoxyphényl)sulfonyl]glycinamide | 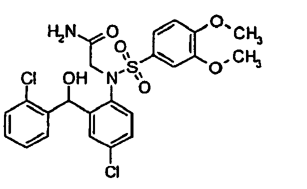 |
| Composé 20 : N-{4-chloro-2-[(2-chlorophényl)(hydroxy)méthyl]phényl}-N-éthyl-3,4-diméthoxybenzènesulfonamide | 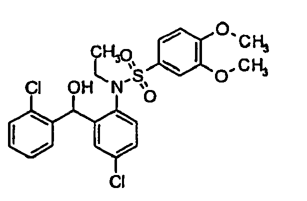 |
| Composé 21 : Méthyl N-{4-chloro-2-[(2-chlorophényl)(hydroxy)méthyl]phényl}-N-[(3,4-diméthoxyphényl)sulfonyl]glycinate | 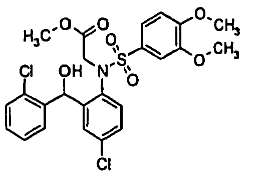 |
| Composé 22 : N-{4-chloro-2-[(2-chlorophényl)(hydroxy)méthyl]phényl}-3,4-diméthoxy-N-[(1-méthyl-1H-imidazol-2-yl)méthyl]benzènesulfonamide | 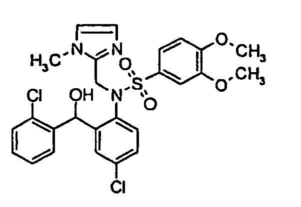 |
| Composé 23 : N-{4-chloro-2-[(2-chlorophényl)(hydroxy)méthyl]phényl}-3,4-diméthoxy-N-(2-méthoxyéthyl)benzènesulfonamide | 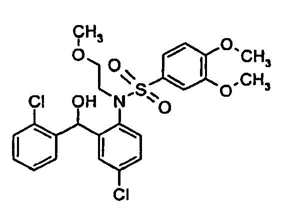 |
| Composé 24: N-{4-chloro-2-[(2-chlorophényl)(hydroxy)méthyl]phényl}-N-(2-hydroxyéthyl)-3,4-diméthoxybenzènesulfonamide | 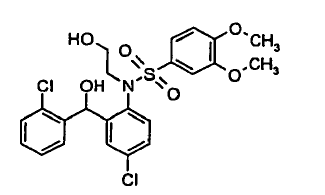 |
| Composé 25 : N-{4-chloro-2-[(2-chlorophényl)(hydroxy)méthyl]phényl}-3,4-diméthoxy-N-méthylbenzènesulfonamide | 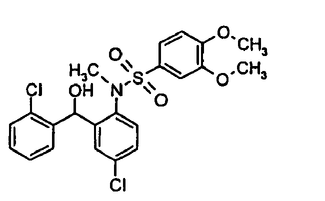 |
| Composé 26 : Ethyl N-{4-chloro-2-[hydroxy(2-méthylphényl)méthyl]phényl}-N-[(3,4-diméthoxyphényl)sulfonyl]glycinate | 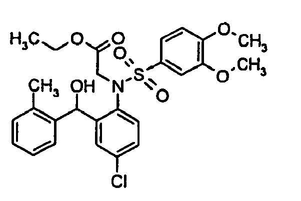 |
| Composé 27 : *N²-*[4-chloro-2-(2-chlorobenzyl)phényl]-*N²*-[(3-méthoxyphényl)sulfonyl]glycinamide | 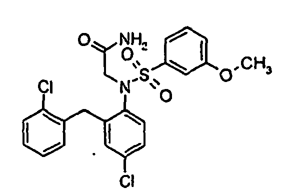 |
| Composé 28 : *N²*-{4-chloro-2-[(2-chlorophényl)(hydroxy)méthyl]phényl}-*N²*-[(3,4-diméthylphényl)sulfonyl]glycinamide | 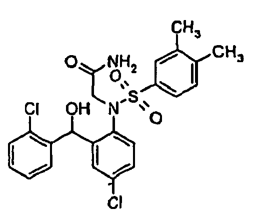 |
| Composé 29 : *N²*-{4-chloro-2-[(2-chlorophényl)(hydroxy)méthyl]phényl}-*N²*-(2-naphthylsulfonyl)glycinamide | 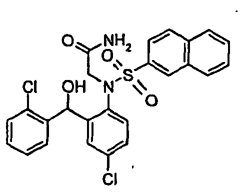 |
| Composé 30 : *N²*-{4-chloro-2-[(2-chlorophényl)(hydroxy)méthyl]phényl}-*N²*-[(3-méthoxyphényl)sulfonyl]glycinamide | 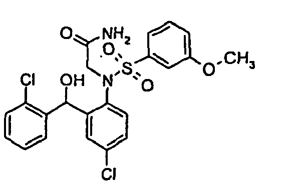 |
| Composé 31 : *N²*-{4-chloro-2-[(2-chlorophényl)(hydroxy)méthyl]phényl}-*N²*-[(3-chlorophényl)sulfonyl]glycinamide | 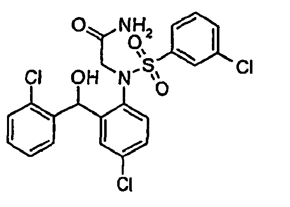 |
| Composé 32: *N²*-{4-chloro-2-[(2-chlorophényl)(hydroxy)méthyl]phényl}-*N²*-[(4-méthoxyphényl)sulfonyl]glycinamide | 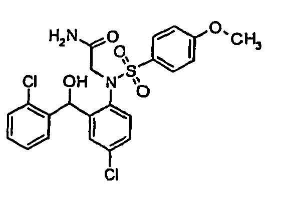 |
| Composé 33 : *N²*-{4-chloro-2-[(2-chlorophényl)(hydroxy)méthyl]phényl}-*N²-*[(2,4-diméthoxyphényl)sulfonyl]glycinamide | 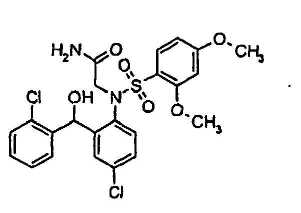 |
| Composé 34 : *N²*-{4-chloro-2-[(2-chlorophényl)(hydroxy)méthyl]phényl}-*N²*-[(3,4-dichlorophényl)sulfonyl]glycinamide | 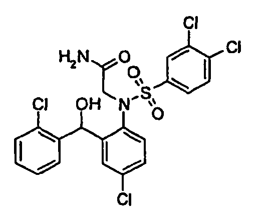 |
| Composé 35: *N²*-{4-chloro-2-[(2-chlorophényl)(hydroxy)méthyl]phényl}-N *N²*-[(4-chlorophényl)sulfonyl]glycinamide | 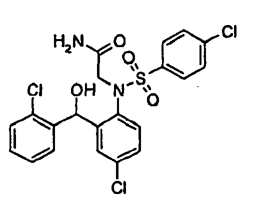 |
| Composé 36 : *N²*{4-chloro-2-[(2-chlorophényl)(hydroxy)méthyl]phényl}-*N²*-[(2,5-diméthoxyphényl)sulfonyl]glycinamide | 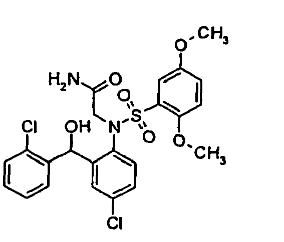 |
| Composé 37 : *N²*-{4-chloro-2-[(2-chlorophényl)(hydroxy)méthyl]phényl}-*N²*-[(2-chlorophényl)sulfonyl]glycinamide | 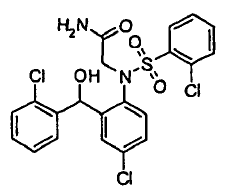 |
| Composé 38 : *N²*-{4-chloro-2-[(2-chlorophényl)(hydroxy)méthyl]phényl}-*N²*-[(4-chloro-2,5-diméthylphényl)sulfonyl]glycinamide | 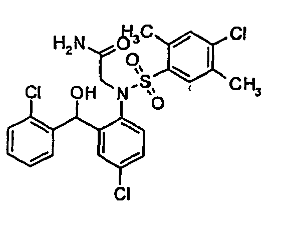 |
| Composé 39 : *N²*-[4-chloro-2-(2-chlorobenzyl)phényl]-*N*²-[(4-méthoxyphényl)sulfonyl]glycinamide | 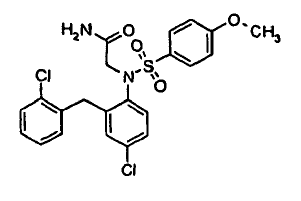 |
| Composé 40 : *N²*-[4-chloro-2-(2-chlorobenzyl)phényl]-*N²*-[(2,5-diméthoxyphényl)sulfonyl]glycinamide | 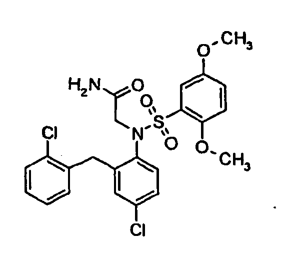 |
| Composé 41 : *N²*-{4-chloro-2-[(2-chlorophényl)(hydroxy)méthyl]phényl}-*N²*-[(2,3-diméthoxyphényl)sulfonyl]glycinamide | 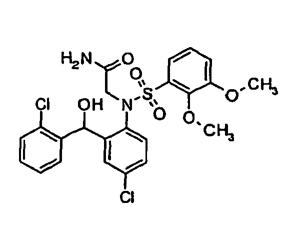 |
| Composé 42 : *N²*-[4-chloro-2-(2-chlorobenzyl)phényl]-*N²*-{[3-(trifluorométhyl)phényl]sulfonyl}glycinamide | 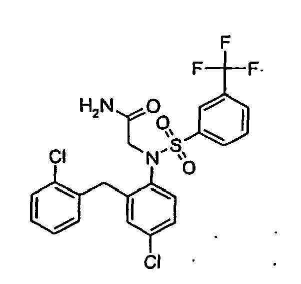 |
| Composé 43 : *N²*-[4-chloro-2-(2-chlorobenzyl)phényl]-*N²*-{[3-(trifluorométhoxy)phényl]sulfonyl}glycinamide | 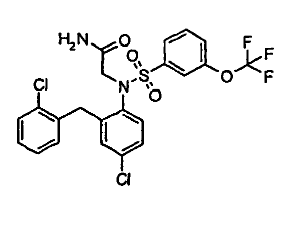 |
| Composé 44: *N²*-{4-chloro-2-[(3-chlorophényl)(hydroxy)méthyl]phényl}- *N²*-[(3,4-diméthoxyphényl)sulfonyl]glycinamide | 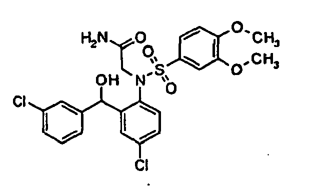 |
| Composé 45 : *N²*-[4-chloro-2-(2-chlorobenzyl)phényl]-*N²*-[(2-méthoxyphényl)sulfonyl]glycinamide | 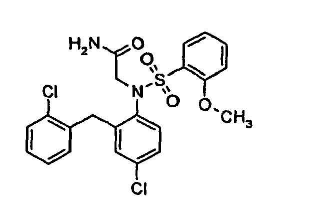 |
| Composé 46 : *N²*-[4-chloro-2-(2-chlorobenzyl)phény]-*N²*-[(3,4-diméthoxyphényl)sulfonyl]-*N¹*-méthylglycinamide | 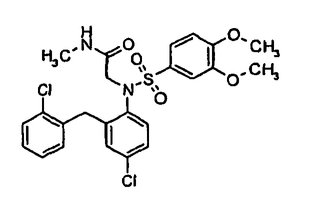 |
| Composé 47 : *N²*-[4-chloro-2-(2-chlorobenzyl)phényl]-*N²*-[(4-chlorophényl)sulfonyl]glycinamide | 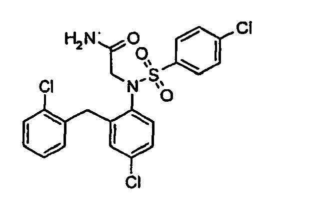 |
| Composé 48 : *N²*-[4-chloro-2-(2-chlorobenzyl)phényl]-*N²*-[(3,4-dichlorophényl)sulfonyl]glycinamide | 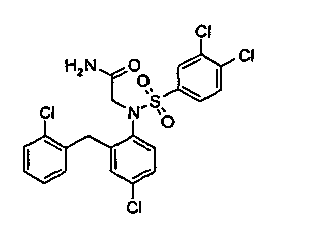 |
| Composé 49 : *N²*-[4-chloro-2-(2-chlorobenzyl)phényl]-*N²*-[(4-chloro-2,5-diméthylphényl)sulfonyl]glycinamide | 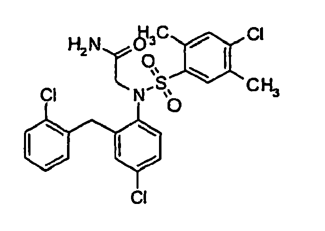 |
| Composé 50 : *N²*-[4-chloro-2-(2-chlorobenzyl)phényl]-*N²*-[(2-chlorophényl)sulfonyl]glycinamide | 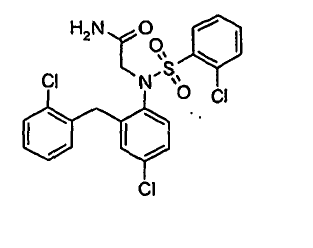 |
| Composé 51 : *N²*-{4-chloro-2-[hydroxy(3-méthoxyphényl)méthyl]phényl}-*N²*-[(3,4-diméthoxyphényl)sulfonyl]glycinamide | 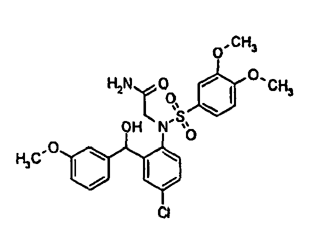 |
| Composé 52 : *N²*-{4-chloro-2-[(2-chlorophényl)(hydroxy)méthyl]phényl}-*N²*-(phénylsulfonyl)glycinamide | 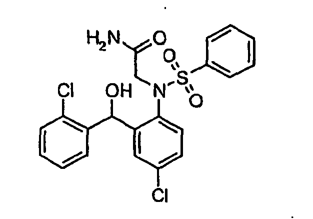 |
| Composé 53 : *N²*-[4-chloro-2-(2-chlorobenzyl)phényl]-*N²*-(phénylsulfonyl)glycinamide | 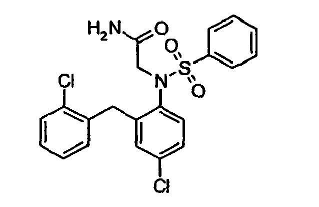 |
| Composé 54 : N-[4-chloro-2-(2-chlorobenzyl)phényl]-3,4-diméthoxy-N-méthylbenzènesulfonamide | 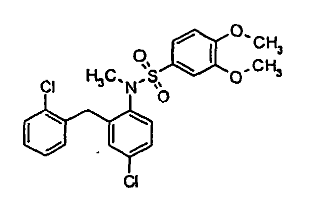 |
| Composé 55 : *N²*-[4-chloro-2-(2-fluorobenzyl)phényl]-*N²*-[(3,4-diméthoxyphényl)sulfonyl]glycinamide | 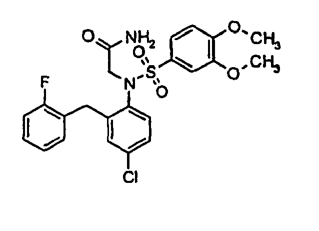 |
| Composé 56 : *N²*-[4-chloro-2-(2-méthoxybenzyl)phényl]-*N²*-[(3,4-diméthoxyphényl)sulfonyl]glycinamide | 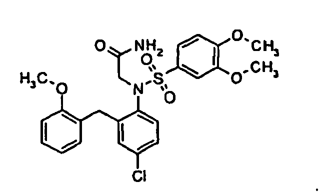 |
| Composé 57 : *N²*-{4-chloro-2-[(2-fluorophényl)(hydroxy)méthyl]phényl}-*N²*-[(3,4-diméthoxyphényl)sulfonyl]glycinamide | 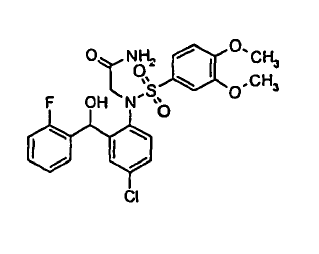 |
| Composé 58 : N~2~-[4-chloro-2-(3-méthoxybenzyl)phènyl]-N~2~-[(3,4-diméthoxyphényl)sulfonyl]glycinamide | 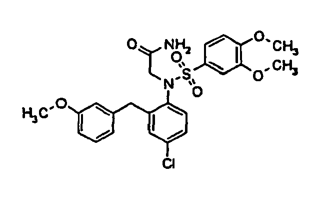 |
| Composé 59 : *N²*-{4-chloro-2-[hydroxy(4-méthoxyphényl)méthyl]phényl}-*N²*-[(3,4-diméthoxyphényl)sulfonyl]glycinamide | 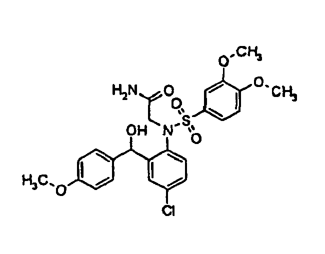 |
| Composé 60 : *N²*-[4-chloro-2-(4-méthoxybenzyl)phényl]-*N²*-[(3,4-diméthoxyphényl)sulfonyl]glycinamide | 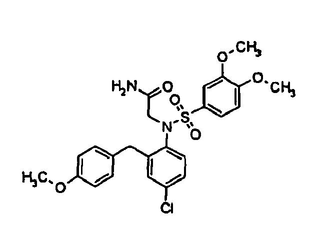 |
| Composé 61 : *N²*-{2-[(2-chlorophényl)(hydroxy)méthyl]-4-fluorophényl}-*N²*-[(3,4-diméthoxyphényl)sulfonyl]glycinamide | 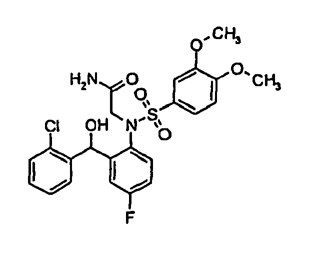 |
| Composé 62 : *N²*-[4-chloro-2-(2-chlorobenzyl)phényl]-*N²*-[(3-méthylphényl)sulfonyl]glycinamide | 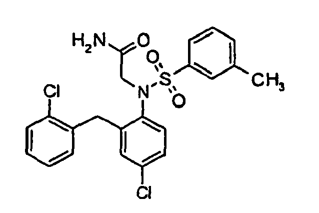 |
| Composé 63 : *N²*-[4-chloro-2-(2-chlorobenzyl)phényl]-*N²*-(2,3-dihydro-1,4-benzodioxin-6-ylsulfonyl)glycinamide | 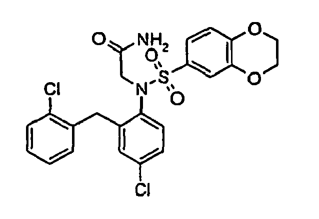 |
| Composé 64 : *N²*-{4-chloro-2-[(2-chlorophényl)(hydroxy)méthyl]phényl}- *N²*-(2,3-dihydro-1,4-benzodioxin-6-ylsulfonyl)glycinamide | 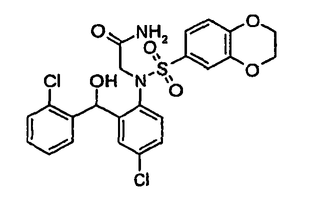 |
| Composé 65 : *N²*-(2-benzyl-4-chlorophényl)-*N²*-[(3,4-diméthoxyphényl)sulfonyl]glycinamide | 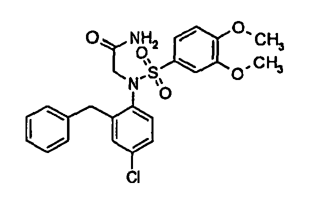 |
| Composé 66 : *N²*-{4-chloro-2-[hydroxy(phényl)méthyl]phényl}-*N²*-[(3,4-diméthoxyphényl)sulfonyl]glycinamide | 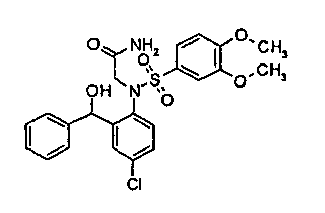 |
| Composé 67 : *N²*-[2-(2-chlorobenzyl)phényl]-*N²*-[(3,4-diméthoxyphényl)sulfonyl]glycinamide | 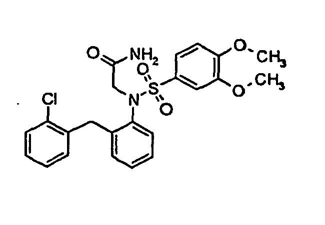 |
| Composé 68 : *N²*-{4-chloro-2-[hydroxy(2-méthoxyphényl)méthyl]phényl}-*N²*-[(3,4-diméthoxyphényl)sulfonyl]glycinamide | 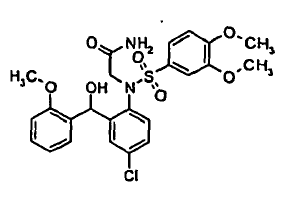 |
| Composé 69 : *N²*-{2-[(2-chlorophényl)(hydroxy)méthyl]-4-méthoxyphényl}-*N²*-[(3,4-diméthoxyphényl)sulfonyl]glycinamide | 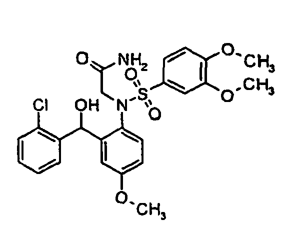 |
| Composé 70 : *N²*-[4-chloro-2-(2-chlorobenzyl)phényl]-*N²*-[(3,4-diméthoxyphényl)sulfonyl]-*N¹*,*N¹*-diméthylglycinamide | 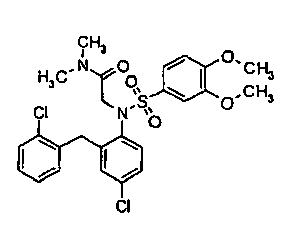 |
| Composé 71 : *N²*-(1,3-benzodioxol-5-ylsulfonyl)-*N²*-[4-chloro-2-(2-chlorobenzyl)phényl]glycinamide | 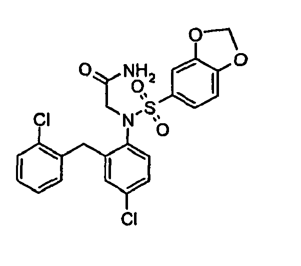 |
| Composé 72 : *N²*-{4-chloro-2-[(2-chlorophényl)(hydroxy)méthyl]phényl}-*N²*-[(3,4-diméthoxyphényl)sulfonyl]-N-méthylglycinamide | 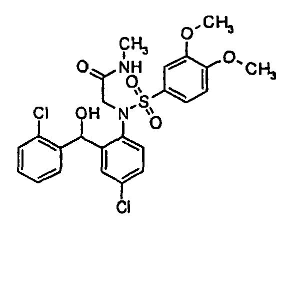 |
| Composé 73 : *N²*-[4-chloro-2-(2-méthylbenzyl)phényl]-*N²*-[(3,4-diméthoxyphényl)sulfonyl]glycinamide | 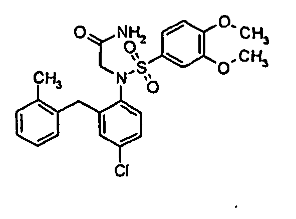 |
| Composé 74 : *N²*-{4-chloro-2-[(2,5-dichlorophényl)(hydroxy)méthyl]phényl}-*N²*-[(3,4-diméthoxyphényl)sulfonyl]glycinamide | 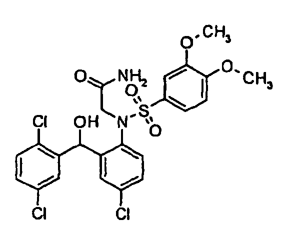 |
| Composé 75 : *N²*-[4-chloro-2-(2-chlorobenzyl)phényl]-*N²*-[(3,4-diméthoxyphényl)sulfonyl]-N-éthylglycinamide | 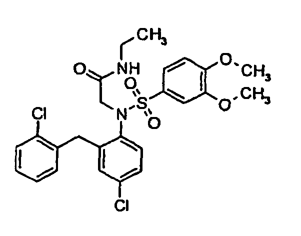 |
| Composé 76 : *N²*-(1,3-benzodioxol-5-ylsulfonyl)-*N²*-{4-chloro-2-[(2-chlorophényl)(hydroxy)méthyl]phényl}glycinamide | 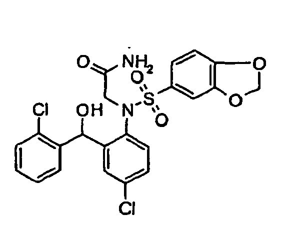 |
| Composé 77 : *N²*-[2-[(2-chlorophényl)(hydroxy)méthyl]-4-(trifluorométhoxy)phényl]-*N²*-[(3,4-diméthoxyphényl)sulfonyl]glycinamide | 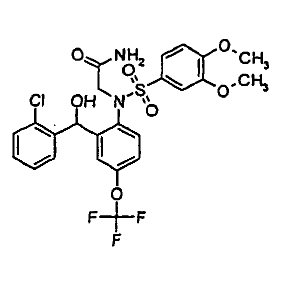 |
| Composé 78 : *N²*-[4-chloro-2-(2,6-dichlorobenzyl)phényl]-*N²*-[(3,4-diméthoxyphényl)sulfonyl]glycinamide | 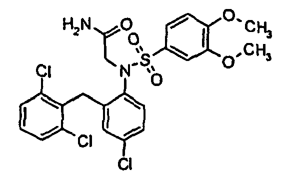 |
| Composé 79 : *N²*-{4-chloro-2-[(2-chlorophényl)(hydroxy)méthyl]phényl}-*N²*-[(2-méthylphényl)sulfonyl]glycinamide | 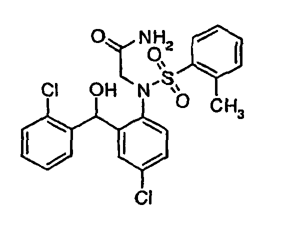 |
| Composé 80 : *N²*-[4-chloro-2-(2,5-dichlorobenzyl)phényl]-*N²*-[(3,4-diméthoxyphényl)sulfonyl]glycinamide | 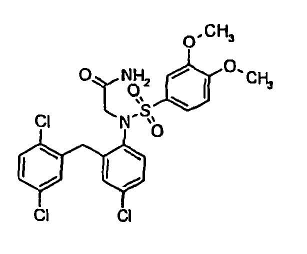 |
| Composé 81 : *N²*-[4-chloro-2-(2-chlorobenzyl)phényl]-*N²*-[(2-méthylphényl)sulfonyl]glycinamide | 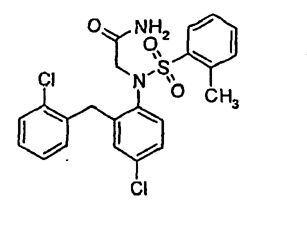 |
| Composé 82 : *N²*-[4-chloro-2-(2-chlorobenzyl)phényl]-*N²*-[(3,4-diméthylphényl)sulfonyl]glycinamide | 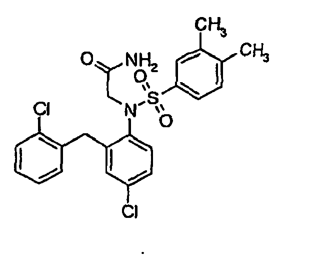 |
| Composé 83: N-{4-chloro-2-[(2-chlorophényl)(hydroxy)méthyl]phényl}-3,4-diméthoxy-N-[(3-méthyl-1,2,4-oxadiazol-5-yl)méthyl]benzènesulfonamide | 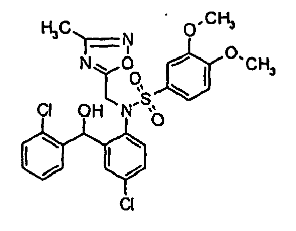 |
| Composé 84 : N-[4-chloro-2-(2-chlorobenzyl)phényl]-3,4-diméthoxy-N-[(3-méthyl-1,2,4-oxadiazol-5-yl)méthyl]benzénesulfonamide | 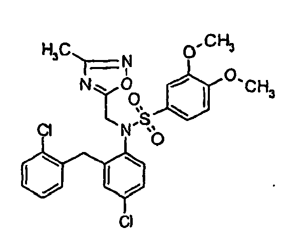 |
| Composé 85 : *N²*-{4-chloro-2-[(2-chlorophényl)(hydroxy)méthyl]phényl}-*N²*-[(2,6-dichlorophényl)sulfonyl]glycinamide | 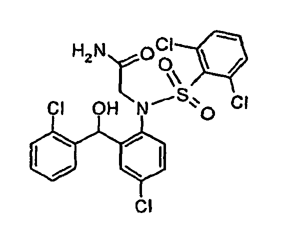 |
| Composé 86 : *N²*-{4-chloro-2-[(2-chlorophényl)(hydroxy)méthyl]phényl}-*N²*-[(3,5-diméthylphényl)sulfonyl]glycinamide | 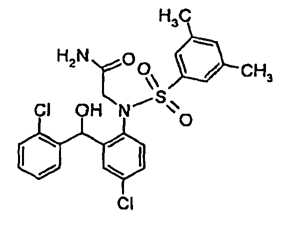 |
| Composé 87 : *N²*-[4-chloro-2-(2-chlorobenzyl)phényl]-*N²*-[(2,6-dichlorophényl)sulfonyl]glycinamide | 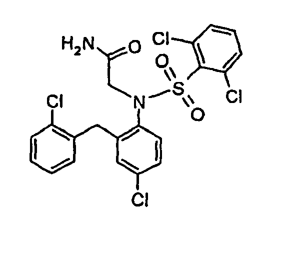 |
| Composé 88 : N-[4-chloro-2-(2-chlorobenzyl)phényl]-3,4-diméthoxy-N-[(1-méthyl-1H-tétrazol-5-yl)méthyl]benzènesulfonamide | 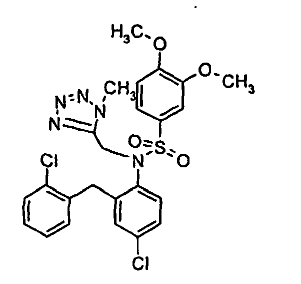 |
| Composé 89 : *N²*-[4-chloro-2-(2-chlorobenzyl)phényl]-*N²*-[(3,5-diméthylphényl)sulfonyl]glycinamide | 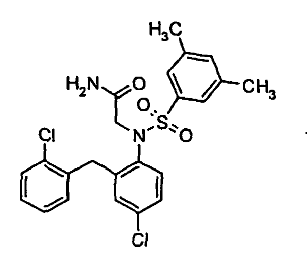 |
| Composé 90 : N-[4-chloro-2-(2-chlorobenzyl)phényl]-N-[(1-éthyl-1H-tétrazol-5-yl)méthyl]-3,4-diméthoxybenzènesulfonamide | 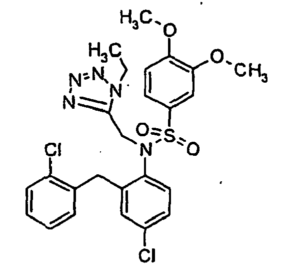 |
| Composé 91 : *N²*-[4-chloro-2-(2,6-difluorobenzyl)phényl]-*N²*-[(3,4-diméthoxyphényl)sulfonyl]glycinamide | 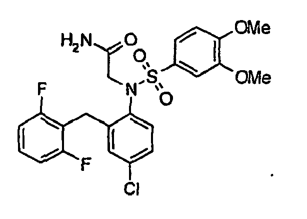 |
| Composé 92 : *N²*-{4-chloro-2-[méthyl(phényl)amino]phényl}- *N²*-[(3,4-diméthoxyphényl)sulfonyl]glycinamide | 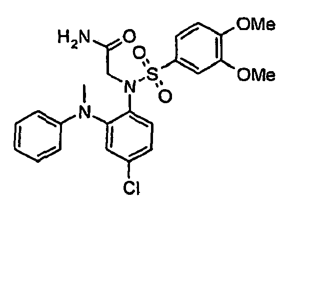 |
| Composé 93 : *N²*-[2-(2-chlorobenzyl)-4-éthylphényl]-*N²*-[(3,4-diméthoxyphényl)sulfonyl]glycinamide | 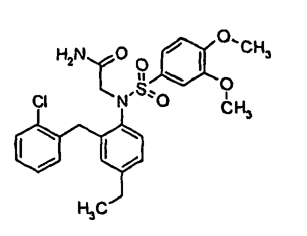 |
| Composé 94 : *N²*-{4-chloro-2-[(2-chlorophényl)(hydroxy)méthyl]phényl}-*N²*-[(2,5-diméthoxy-4-méthylphényl)sulfonyl]glycinamide | 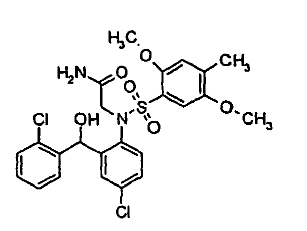 |
| Composé 95 : *N²*-(2-benzyl-4-chlorophényl)-*N²*-[(3,4-diméthoxyphényl)sulfonyl]-*N¹,N¹*-diméthylglycinamide | 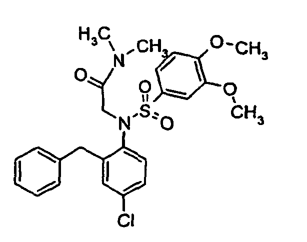 |
| Composé 96 : *N²*-(2-benzyl-4-chlorophényl)-*N²*-[(3,4-diméthoxyphényl)sulfonyl]-*N¹*-méthylglycinamide | 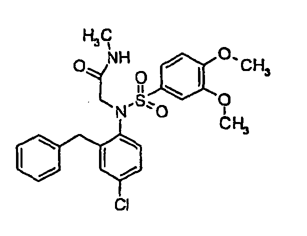 |
| Composé 97 : *N²*-[4-chloro-2-(2-fluorobenzyl)phényl]-*N²*-[(3,4-diméthoxyphényl)sulfonyl]-*N¹,N¹*-diméthylglycinamide | 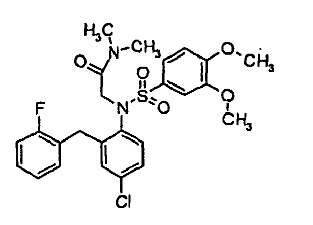 |
| Composé 98 : *N²*-[4-chloro-2-(2-fluorobenzyl)phényl]-*N*²-[(3,4-diméthoxyphényl)sulfonyl]-*N¹*-méthylglycinamide | 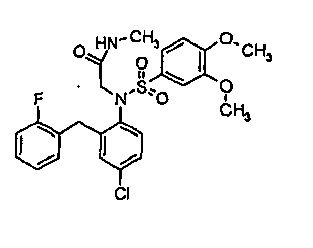 |
| Composé 99 : *N²*-[4-chloro-2-(2-chlorobenzyl)phényl]-*N²*-[(2,5-diméthoxy-4-méthylphényl)sulfonyl]glycinamide | 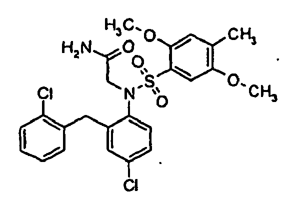 |
| Composé 100 : *N²*-[4-chloro-2-(2-chlorobenzyl)phényl]-*N²*-[(2,4-dichloro-5-méthylphényl)sulfonyl]glycinamide | 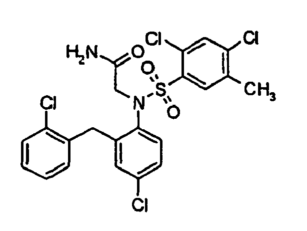 |
| Composé 101 : *N²*-{4-chloro-2-[(2,6-difluorophényl)(hydroxy)méthyl]phényl}-*N²*-[(3,4-diméthoxyphényl)sulfonyl]glycinamide | 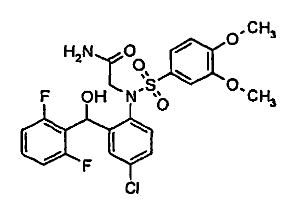 |
| Composé 102 : *N²*-{4-chloro-2-[(2-chlorophényl)(hydroxy)méthyl]phényl}-*N²*-[(2,4,5-triméthoxyphényl)sulfonyl]glycinamide | 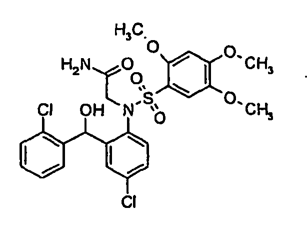 |
| Composé 103 : *N²*-[2-(2-chlorobenzyl)-4-éthylphényl]-*N²*-[(3,4-diméthoxyphényl)sulfonyl]-*N¹*,*N¹*-diméthylglycinamide | 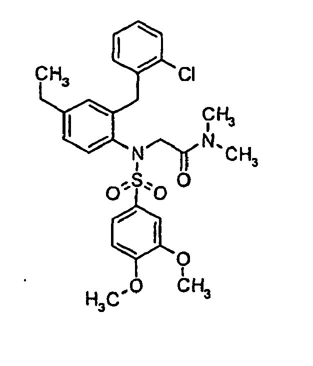 |
| Composé 104 : *N²*-[4-chloro-2-(2-chlorobenzyl)phényl]-*N²*-[(2,4,5-triméthoxyphényl)sulfonyl]glycinamide | 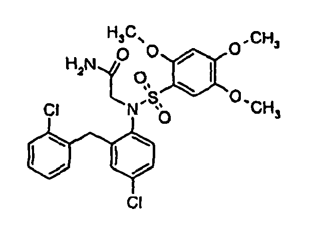 |
| Composé 105: *N²*-[4-chloro-2-(2-chlorobenzyl)phényl]-*N²*-[(4-chloro-2,5-diméthylphényl)sulfonyl]-*N¹*-méthylglycinamide | 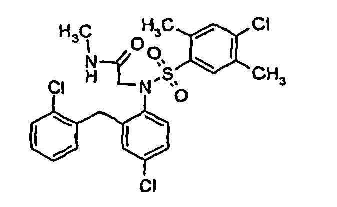 |
| Composé 106 : *N²*-{4-chloro-2-[(2-chlorophènyl)(hydroxy)méthyl]phényl}-*N²*-[(2,4,5-triméthylphényl)sulfonyl]glycinamide | 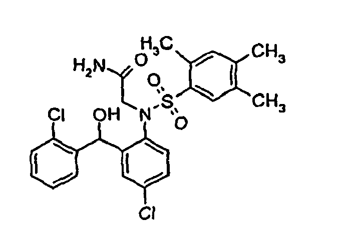 |
| Composé 107 : N-[4-chloro-2-(2-chlorobenzyl)phényl]-3,4-diméthoxy-N-[(1-méthyl-1H-imidazol-2-yl)méthyl]benzènesulfonamide | 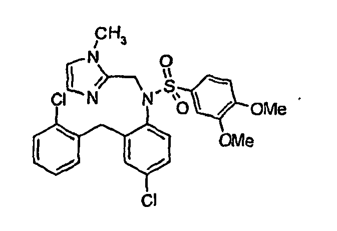 |
| Composé 108 : N-[4-chloro-2-(2-chlorobenzyl)phényl]-N-[(1-isopropyl-1H-tétrazol-5-yl)méthyl]-3,4-diméthoxybenzènesulfonamide | 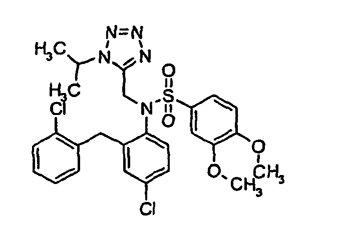 |
| Composé 109 : *N²*-[4-chloro-2-(2-chlorobenzyl)phényl]-N'-méthyl-*N²*-[(2,4,5-triméthoxyphényl)sulfonyl]glycinamide | 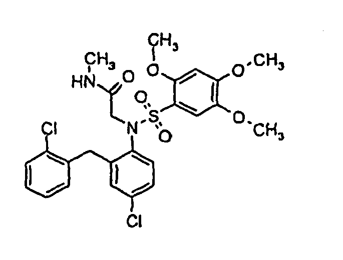 |
| Composé110 : *N²*-{4-chloro-2-[(2-chlorophényl)(hydroxy)méthyl]phényl}-*N¹*-méthyl- *N²*-[(2,4,5-triméthylphényl)sulfonyl]glycinamide | 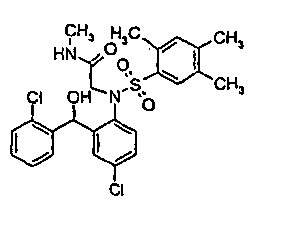 |
| Composé 111 : N-[(1-benzyl-1H-tétrazol-5-yl)méthyl]-N-[4-chloro-2-(2-chlorobenzyl)phényl]-3,4-diméthoxybenzènesulfonamide | 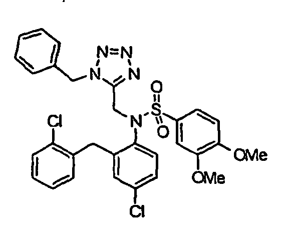 |
| Composé 112 : N-[4-chloro-2-(2-chlorobenzyl)phényl]-3,5-diméthyl-N-[(1-méthyl-1H-imidazol-2-yl)méthyl]benzènesulfonamide | 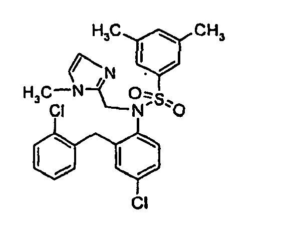 |
| Composé 113 : *N²*-{4-chloro-2-[(2-chlorophényl)(hydroxy)méthyl]phényl}-*N¹*-méthyl- *N²*-[(2,4,5-triméthoxyphényl)sulfonyl]glycinamide | 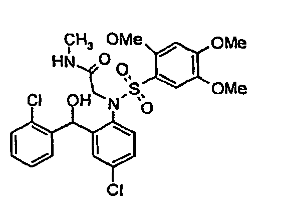 |
| Composé 114 : *N²*-[4-chloro-2-(2-chlorobenzyl)phényl]-*N²*-[(2,4,5-triméthylphényl)sulfonyl]glycinamide | 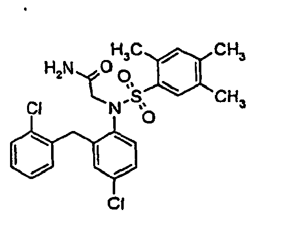 |
| Composé 115 : *N²*-[4-chloro-2-(2-chlorobenzyl)phényl]-*N¹*-méthyl-*N²*-[(2,4,5-triméthylphényl)sulfonyl]glycinamide | 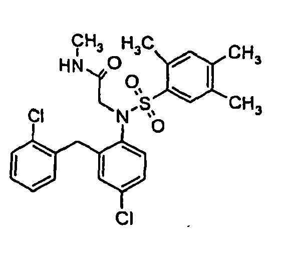 |
| Composé-116 : *N²*-[4-chloro-2-(3-chlorobenzyl)phényl]-*N²*-[(3,4-diméthoxyphényl)sulfonyl]glycinamide | 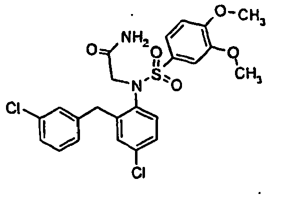 |
| Composé 117 : *N²*-{4-chloro-2-[(2,6-dichlorophényl)(hydroxy)méthyl]phényl}-*N²*-[(3,4-diméthoxyphényl)sulfonyl]glycinamide | 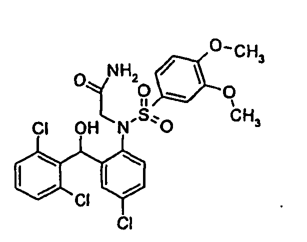 |
| Composé 118 : *N²*-[4-chloro-2-(2-chlorobenzyl)-phényl]-*N²*-[(4-chloro-2,5-diméthylphényl)sulfonyl]-*N¹*,*N¹*-diméthylglycinamide | 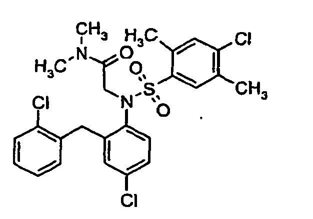 |
| Composé 119 : *N²*-[4-chloro-2-(2-chlorobenzyl)phényl]-*N²*-[(3,5-diméthoxyphényl)sulfonyl]glycinamide | 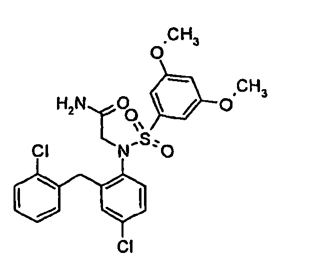 |
| Composé 120 : *N²*-{4-chloro-2-[hydroxy(1,3-thiazol-2-yl)méthyl]phényl}-*N²*-[(3,4-diméthoxyphényl)sulfonyl]glycinamide | 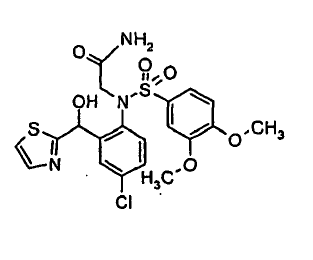 |
| Composé 121 : *N²*-{4-chloro-2-[hydroxy(1,3-thiazol-2-yl)méthyl]phényl}-*N²*-[(3,4-diméthoxyphényl)sulfonyl]-*N¹*-méthylglycinamide | 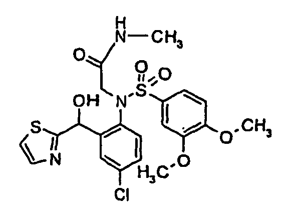 |
| Composé 122 : N-[4-chloro-2-(2-chlorobenzyl)phényl]-N-[(2-éthyl-2H-tétrazol-5-yl)méthyl]-3,5-diméthylbenzènesulfonamide | 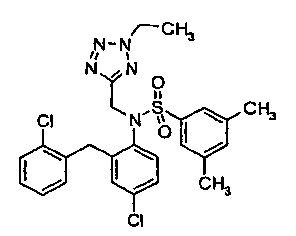 |
| Composé 123 : N-[4-chloro-2-(2-chlorobenzyl)phényl]-N-[(1-éthyl-1H-tètrazol-5-yl)méthyl]-3,5-diméthylbenzènesulfonamide | 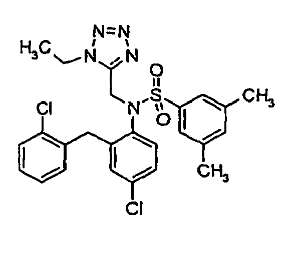 |
| Composé 124 : *N²*-{4-chloro-2-[méthyl(phényl)amino]phényl}-*N²*-[(3,4-diméthoxyphényl)sulfonyl]-*N¹*-méthylglycinamide | 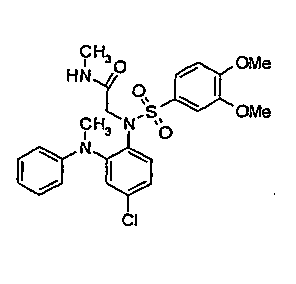 |
| Composé 125 : *N²*-(4-chloro-2-{hydroxy[2-(trifluorométhyl)phényl]méthyl}phényl)-*N²*-[(3,4-diméthoxyphényl)sulfonyl]glycinamide | 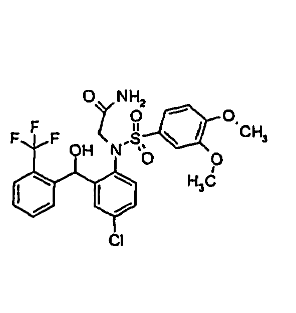 |
| Composé 126: *N²*-{4-chloro-2-[(2,3-difluorophényl)(hydroxy)méthyl]phényl}-*N²*-[(3,4-diméthoxyphényl)sulfonyl]glycinamide | 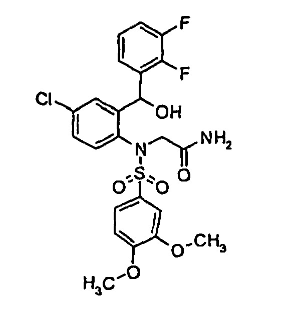 |
| Composé 127 : *N²*-{4-chloro-2-[(2,3-difluorophényl)(hydroxy)méthyl]phényl}-*N²*-[(3,4-diméthoxyphényl)sulfonyl]-*N¹*-éthylglycinamide | 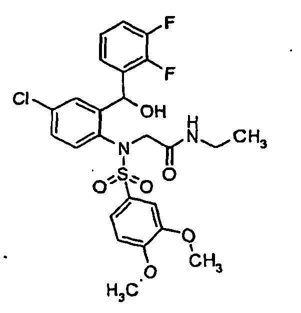 |
| Composé 128 : *N²*-{4-chloro-2-[(2,3-difluorophényl)(hydroxy)méthyl]phényl}-*N²*-[(3,4-diméthoxyphényl)sulfonyl]-*N¹*,*N¹*-diméthylglycinamide | 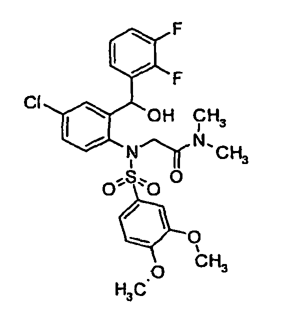 |
| Composé 129 : *N²*-{4-chloro-2-[(2-chlorophényl)(hydroxy)méthyl]phényl}-*N²*-[(2,4-dichloro-5-méthylphényl)sulfonyl]-*N¹*,*N¹*-diméthylglycinamide | 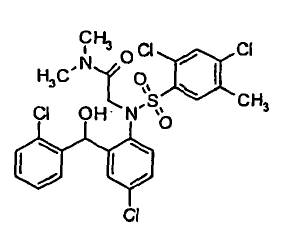 |
| Composé 130 : *N²*-[4-chloro-2-(2,6-dichlorobenzyl)phényl]-*N²*-[(3,4-diméthoxyphényl)sulfonyl]-*N¹*-méthylglycinamide | 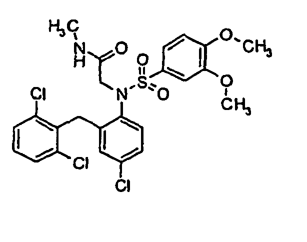 |
| Composé 131 : *N²*-{4-chloro-2-[hydroxy(2-méthylphényl)méthyl]phényl}-*N²*-[(3,4-diméthoxyphényl)sulfonyl]glycinamide | 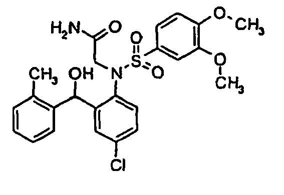 |
| Composé 132 : *N²*-{4-chloro-2-[(2-chlorophényl)(hydroxy)méthyl]phényl}-*N²*-[(3,5-diméthylphényl)sulfonyl]-*N¹*-méthylglycinamide | 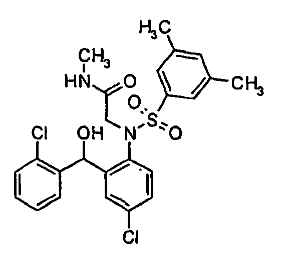 |
| Composé 133 : *N²*-{4-chloro-2-[(2-chlorophényl)(hydroxy)méthyl]phényl}-*N²*-[(3,5-diméthylphényl)sulfonyl]-*N¹*,*N¹*-diméthylglycinamide | 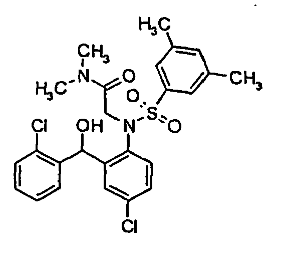 |
| Composé 134 : *N²*-[4-chloro-2-(2-chlorobenzyl)phényl]- *N²*-[(3-nitrophényl)sulfonyl]glycinamide | 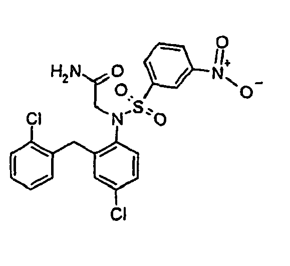 |
| Composé 135 : *N²*-[(3-aminophényl)sulfonyl]-*N²*-[4-chloro-2-(2-chlorobenzyl)phényl]glycinamide | 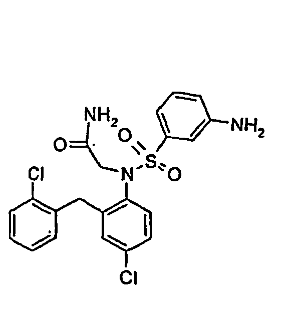 |
| Composé 136 : *N²*-{2-[(2-chlorophényl)(hydroxy)méthyl]-4-méthylphényl}-*N²*-[(3,4-diméthoxyphényl)sulfonyl]glycinamide | 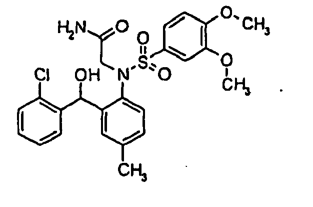 |
| Composé 137 : *N²*-{4-chloro-2-[hydroxy(phényl)méthyl]phényl}-*N²*-[(3,4-diméthoxyphényl)sulfonyl]-*N¹*-méthylglycinamide | 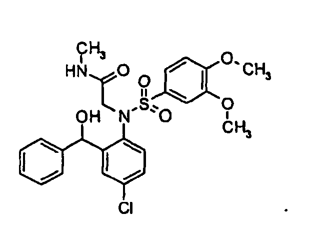 |
| Composé 138 : *N²*-[2-(2-chlorobenzyl)-4-méthoxyphényl]-*N²*-[(3,4-diméthoxyphényl)sulfonyl]glycinamide | 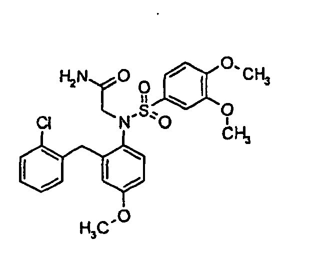 |
| Composé 139 : *N²*-[4-chloro-2-(2,6-difluorobenzyl)phényl]-*N²*-[(3,4-diméthoxyphényl)sulfonyl]-*N¹*-méthylglycinamide | 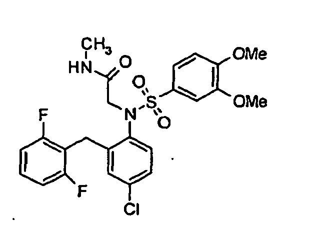 |
| Composé 140 : N-[4-chloro-2-(2,6-difluorobenzyl)phényl]-3,4-diméthoxy-N-propylbenzènesulfonamide | 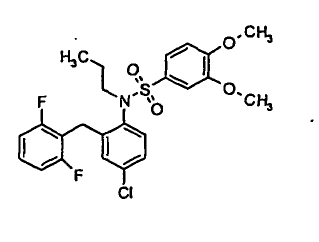 |
| Composé 141 : *N²*-[4-chloro-2-(2,3-difluorobenzyl)phényl]-*N²*-[(3,4-diméthoxyphényl)sulfonyl]-*N¹*-méthylglycinamide | 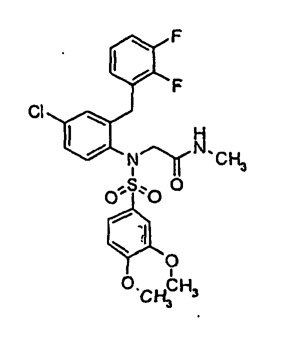 |
| Composé 142 : *N²*-[4-chloro-2-(2,3-difluorobenzyl)phényl]-*N²*-[(3,4-diméthoxyphényl)sulfonyl]glycinamide | 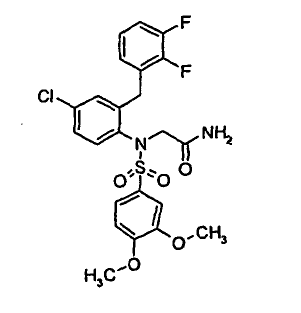 |
| Composé 143 : *N²*-[4-chloro-2-(2,3-difluorobenzyl)phényl]-*N²*-[(3,4-diméthoxyphényl)sulfonyl]-*N¹*,*N¹*-diméthylglycinamide | 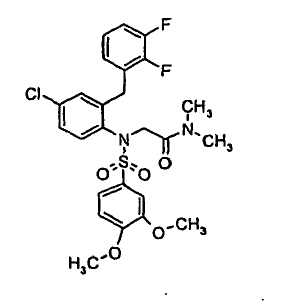 |
| Composé 144 : N-[4-chloro-2-(2,6-difluorobenzyl)phényl]-3,4-diméthoxy-N-méthylbenzènesulfonamide | 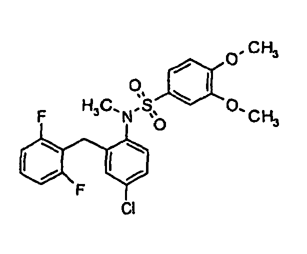 |
| Composé 145 : N-[4-chloro-2-(2,6-difluorobenzyl)phényl]-N-[2-(diméthylamino)éthyl]-3,4-diméthoxybenzènesulfonamide | 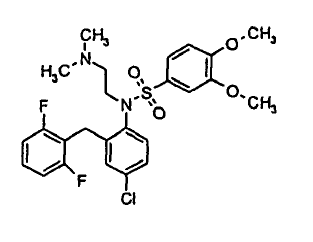 |
| Composé 146 : *N²*-{4-chloro-2-[hydroxy(2-thiènyl)méthyl]phényl}-*N²*-[(3,4-diméthoxyphényl)sulfonyl]-*N¹*-méthylglycinamide | 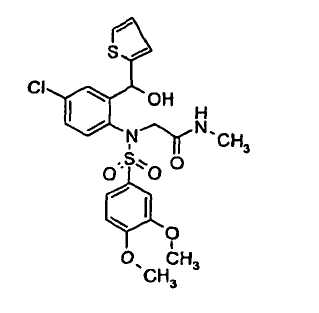 |
| Composé 147 : *N²*-{4-chloro-2-[(2,6-difluorophényl)(hydroxy)méthyl]phényl}-*N²*-[(3,5-diméthylphényl)sulfonyl]glycinamide | 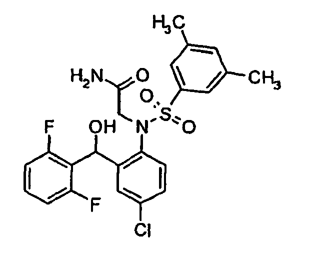 |
| Composé 148 : *N²*-[(3-aminophényl)sulfonyl]-*N²*-{4-chloro-2-[(2-chlorophényl)(hydroxy)méthyl]phényl}glycinamide | 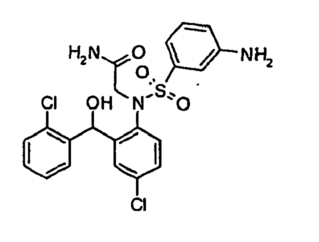 |
| Composé 149 : *N²*-{4-chloro-2-[(2-fluoro-6-méthoxyphényl)(hydroxy)méthyl]phényl}-*N²*-[(3,4-diméthoxyphényl)sulfonyl]glycinamide | 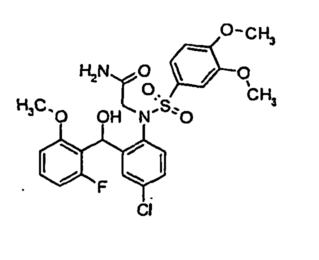 |
| Composé 150 : *N²*-[4-chloro-2-(2,6-difluorobenzyl)phényl]-*N²*-[(3,4-diméthoxyphényl)sulfonyl]-*N¹*,*N¹*-diméthylglycinamide | 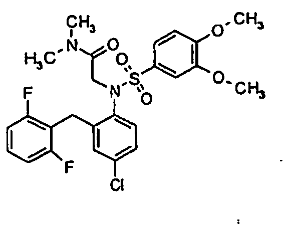 |
| Composé 151 : N-[4-chloro-2-(2,6-difluorobenzyl)phényl]-3,4-diméthoxy-N-[(2-méthyl-2H-tétrazol-5-yl)méthyl]benzénesulfonamide | 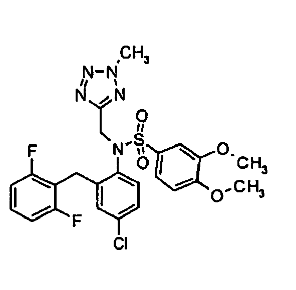 |
| Composé 152 : N-[4-chloro-2-(2,6-difluorobenzyl)phényl]-3,4-diméthoxy-N-[(1-méthyl-1H-tétrazol-5-yl)méthyl]benzènesulfonamide | 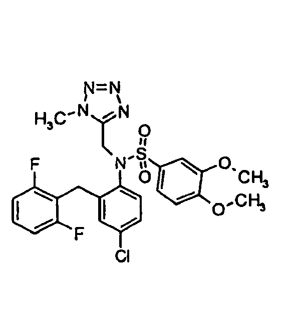 |
| Composé 153 : N-[4-chloro-2-(2,6-difluorobenzyl)phényl]-N-[(2-éthyl-2H-tétrazol-5-yl)méthyl]-3,4-diméthoxybenzènesulfonamide | 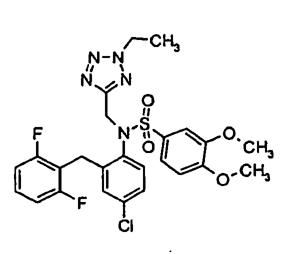 |
| Composé 154 : N-[4-chloro-2-(2,6-difluorobenzyl)phényl]-N-[(1-éthyl-1H-tétrazol-5-yl)méthyl]-3,4-diméthoxybenzénesulfonamide | 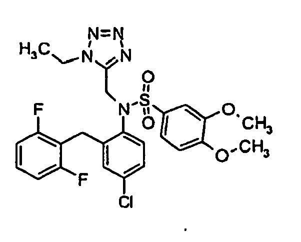 |
| Composé 155 : *N²*-{4-chloro-2-[hydroxy(2-thiènyl)méthyl]phényl}-*N²*-[(3,4-diméthoxyphényl)sulfonyl]glycinamide | 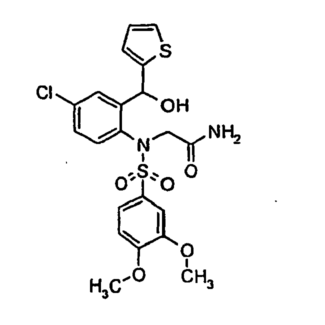 |
| Composés 156 : N-[4-chloro-2-(2,6-difluorobenzyl)phényl]-N-[2-(6,7-diméthoxy-3,4-dihydroisoquinolin-2(1H)-yl)-2-oxoéthyl]-3,4-diméthoxybenzènesulfonamide | 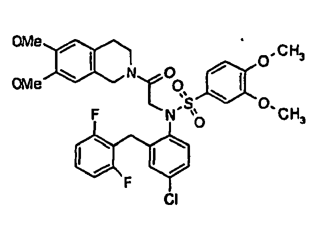 |
| Composé 157 : N-[4-chloro-2-(2,6-difluorobenzyl)phényl]-3,5-diméthyl-N-[(2-méthyl-2H-tétrazol-5-yl)méthyl]benzènesulfonamide | 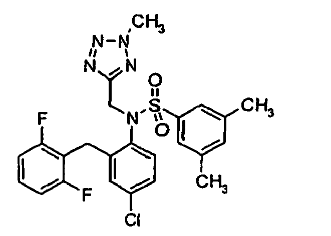 |
| Composé 158 : N-[4-chloro-2-(2,6-difluorobenzyl)phényl]-3,5-diméthyl-N-[(1-méthyl-1H-tétrazol-5-yl)méthyl]benzènesulfonamide | 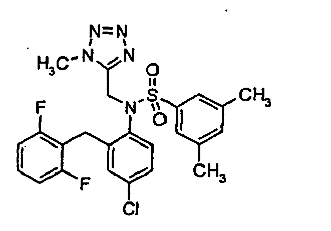 |
| Composé 159 : N-[4-chloro-2-(2,6-difluorobenzyl)phényl]-N-[(2-éthyl-2H-tétrazol-5-yl)méthyl]-3,5-diméthylbenzènesulfonamide | 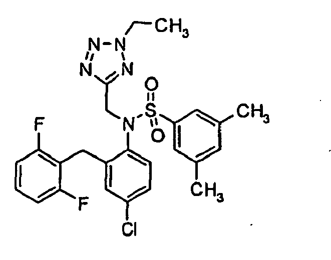 |
| Composé 160 : N-[4-chloro-2-(2,6-difluorobenzyl)phényl]-N-[(1-éthyl-1H-tétrazol-5-yl)méthyl]-3,5-diméthylbenzènesulfonamide | 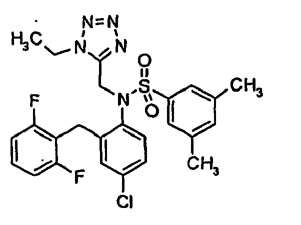 |
| Composé 161 : *N²*-[4,5-dichloro-2-(2-chlorobenzyl)phényl]-*N²*-[(3,4-diméthoxyphényl)sulfonyl]glycinamide | 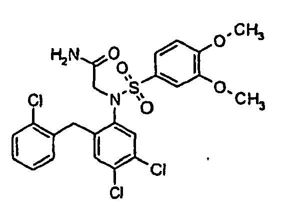 |
| Composé 162: *N²*-{4-chloro-2-[hydroxy(2,4,6-trifluorophényl)méthyl]phényl}-*N²*-[(3,4-diméthoxyphényl)sulfonyl]glycinamide | 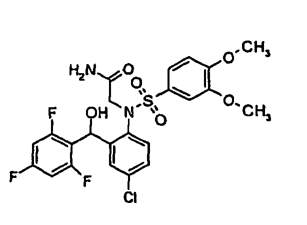 |
| Composé 163 : *N²*-{4-chloro-2-[(2,4-difluorophényl)(hydroxy)méthyl]phényl}-*N²*-[(3,4-diméthoxyphényl)sulfonyl]glycinamide | 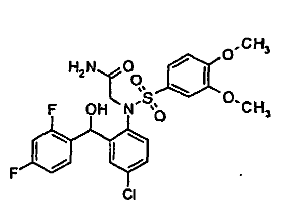 |
| Composé 164 : *N²*-{4,5-dichloro-2-[(2-chlorophényl)(hydroxy)méthyl]phényl}-*N²*-[(3,4-diméthoxyphényl)sulfonyl]glycinamide | 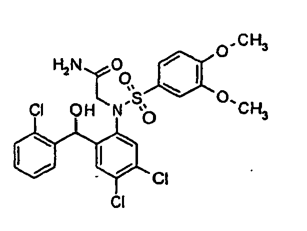 |
| Composé 165: *N²*-[2-(2-chlorobenzyl)-4-méthylphényl]-*N²*-[(3,4-diméthoxyphényl)sulfonyl]glycinamide | 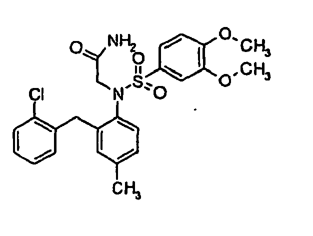 |
| Composé 166: *N²*-[4-chloro-2-(2,6-dichlorobenzyl)phényl]-*N²*-[(3,4-diméthoxyphényl)sulfonyl]-*N¹*,*N¹*-diméthylglycinamide | 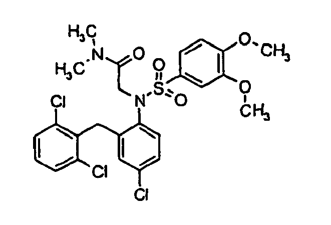 |
| Composé 167 : *N²*-{4-chloro-2-[(4-chloro-2-fluorophényl)(hydroxy)méthyl]phényl}-*N²*-[(3,4-diméthoxyphényl)sulfonyl]glycinamide | 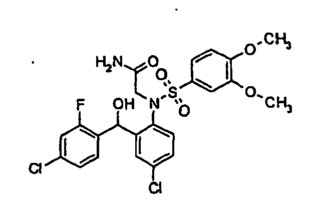 |
| Composé 168 : *N²*-[4-chloro-2-(4-chloro-2-fluorobenzyl)phényl]-*N²*-[(3,4-diméthoxyphényl)sulfonyl]glycinamide | 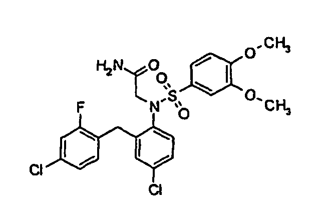 |
| Composé 169 : *N²*-[4-chloro-2-(2,6-difluorobenzyl)phényl]-*N²*-[(3,5-diméthylphényl)sulfonyl]glycinamide | 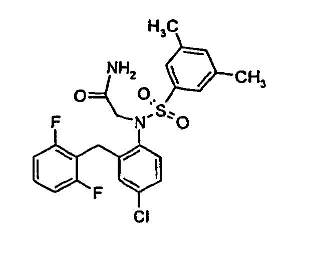 |
| Composé 170 : *N²*-[4-chloro-2-(2-fluoro-6-méthoxybenzyl)phényl]-*N²*-[(3,4-diméthoxyphényl)sulfonyl]glycinamide | 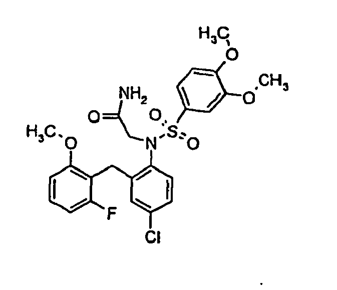 |
| Composé 171 : *N²*-[4-chloro-2-(2,6-difluorobenzyl)phényl]-*N²*-[(3,5-diméthylphényl)sulfonyl]-*N¹*-méthylglycinamide | 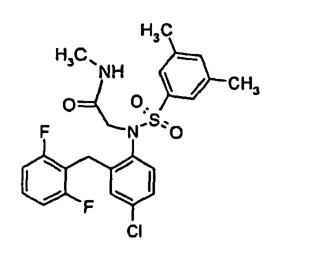 |
| Composé 172 : *N²*-{4-chloro-2-[1-(2-chlorophényl)-1-hydroxyéthyl]phényl}-*N²*-[(3,4-diméthoxyphényl)sulfonyl]glycinamide | 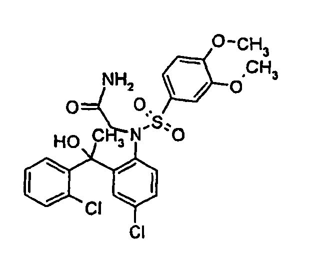 |
| Composé 173 : *N²*-[4-chloro-2-(2-chlorobenzyl)phényl]-*N²*-[(2,4-dichloro-5-méthylphényl)sulfonyl]-*N¹*-méthylglycinamide | 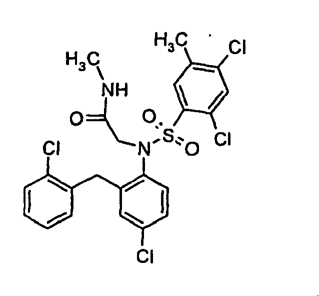 |
| Composé 174 : *N²*-[4-chloro-2-(2-chlorobenzyl)phényl]-*N²*-[(2,4-dichloro-5-méthylphényl)sulfonyl]-N∼1∼,N∼1∼-diméthylglycinamide | 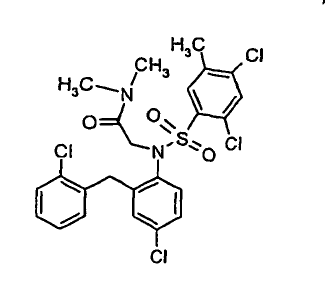 |
| Composé 175 : *N²*-{5-chloro-2-[(2-chlorophényl)(hydroxy)méthyl]phényl}-N∼2∼-[(3,4-diméthoxyphényl)sulfonyl]glycinamide | 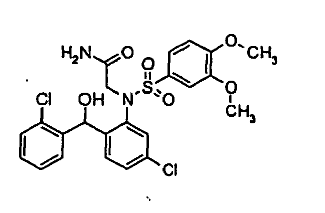 |
| Composé 176: *N²*-{4-chloro-2-[(5-chloro-2-fluorophényl)(hydroxy)méthyl]phényl}-*N²*-[(3,4-diméthoxyphényl)sulfonyl]glycinamide | 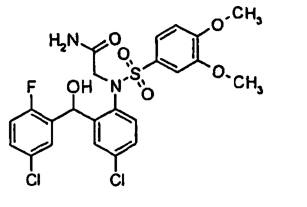 |
| Composé 177 : *N²*-[4-chloro-2-(2,5-difluorobenzyl)phényl]-*N²*-[(3,4-diméthoxyphényl)sulfonyl]glycinamide | 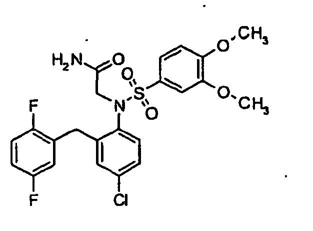 |
| Composé 178 : *N²*-{4-chloro-2-[(3,5-difluorophényl)(hydroxy)méthyl]phenyl}-*N²*-[(3,4-diméthoxyphényl)sulfonyl]glycinamide | 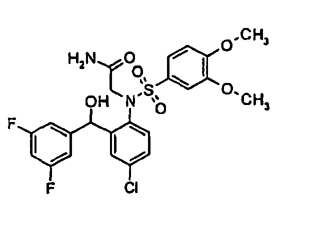 |
| Composé 179 : *N²*-[5-chloro-2-(2-chlorobenzyl)phényl]-*N²*-[(3,4-diméthoxyphényl)sulfonyl]glycinamide | 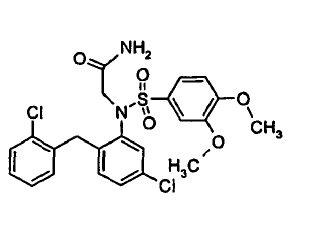 |
| Composé 180: *N²*-[4-chloro-2-(2-thiènylméthyl)phényl]-*N²*-[(3,4-diméthoxyphényl)sulfonyl]glycinamide | 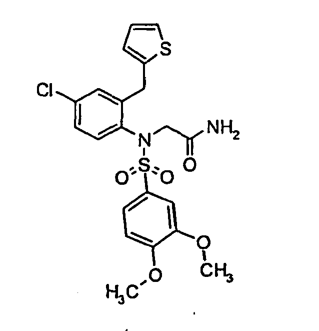 |
| Composé 181 : N-[4-chloro-2-(2,6-difluorobenzyl)phényl]-3,4-diméthoxy-N-[(1-méthyl-1H-imidazol-2-yl)méthyl]benzénesulfonamide | 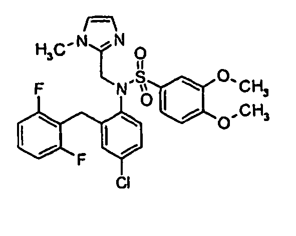 |
| Composé 182 : *N²*-[4-chloro-2-(5-chloro-2-fluorobenzyl)phényl]-*N²*-[(3,4-diméthoxyphényl)sulfonyl]glycinamide | 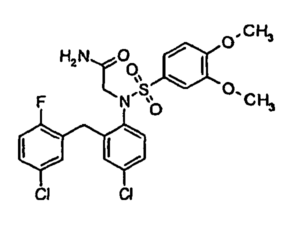 |
| Composé 183 : *N²*-[4-chloro-2-(3,5-difluorobenzyl)phényl]-*N²*-[(3,4-diméthoxyphényl)sulfonyl]glycinamide | 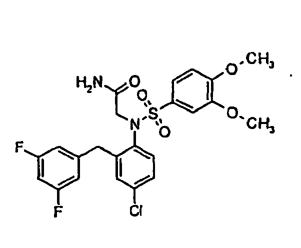 |
| Composé 184 : *N²*-{4-chloro-2-[hydroxy(pyridin-4-yl)méthyl]phényl}-*N²*-[(3,4-diméthoxyphényl)sulfonyl]glycinamide | 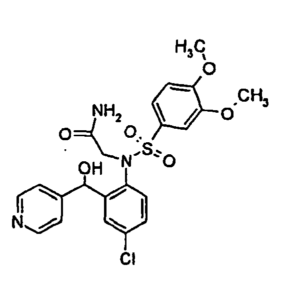 |
| Composé 185 : N-[4-chloro-2-(2,6-difluorobenzyl)phényl]-3,4-diméthoxy-N-prop-2-yn-1-ylbenzènesulfonamide | 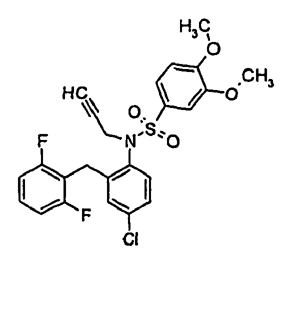 |
| Composé 186 : *N²*-{4-chloro-2-[1-(2-chlorophényl)éthyl]phényl}-*N²*-[(3,4-diméthoxyphényl)sulfonyl]glycinamide | 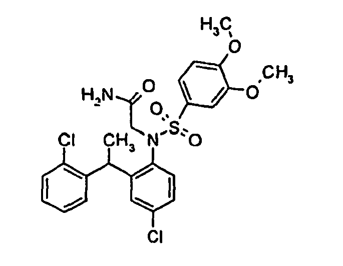 |
| Composé 187 : *N²*-{4-chloro-2-[1-(2-chlorophényl)éthyl]phényl}-*N²*-[(3,4-diméthoxyphényl)sulfonyl]-*N¹*-méthylglycinamide | 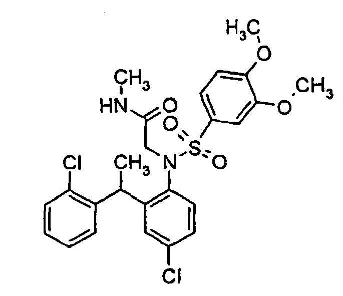 |
| Composé 188 : *N²*-[4-chloro-2-(2,6-difluorobenzyl)phényl]-*N²*-{[3-(diméthylamino)phényl]sulfonyl}glycinamide | 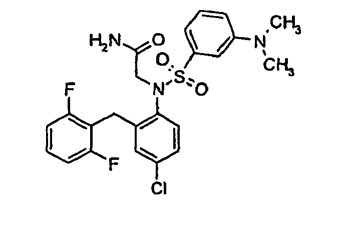 |
| Composé 189 : *N²*-[4-chloro-2-(2,6-difluorobenzyl)phényl]-*N²*-{[3-(méthylamino)phényl]sulfonyl}glycinamide | 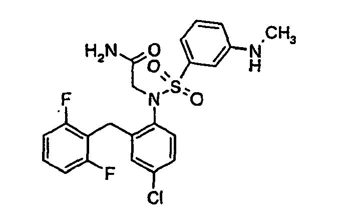 |
| Composé 190 : N-[4-chloro-2-(2,6-difluorobenzyl)phényl]-3,4-diméthoxy-N-(2,2,2-trifluoroéthyl)benzènesulfonamide | 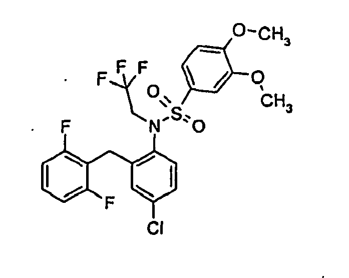 |
| Composé 191 : *N*²-{4-chloro-2-[(2-chloro-6-méthoxyphényl)(hydroxy)méthyl]phényl}- *N*²-[(3,4-diméthoxyphényl)sulfonyl]glycinamide | 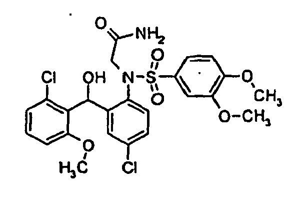 |
| Composé 192 : *N*²-[4-chloro-2-(2-chloro-6-méthoxybenzyl)phényl]-*N*²-[(3,4-diméthoxyphényl)sulfonyl]glycinamide | 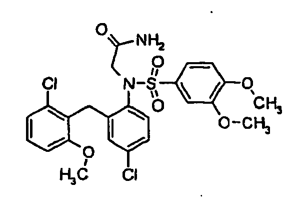 |
| Composé 193 : *N*²-[2-(2,6-difluorobenzyl)-4-méthoxyphényl]-*N*²-[(3,4-diméthoxyphényl)sulfonyl]glycinamide | 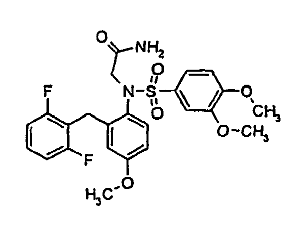 |
| Composé 194 : *N*²-{4-chloro-2-[(2-fluoro-5-méthylphényl)(hydroxy)méthyl]phényl}-*N*²-[(3,4-diméthoxyphényl)sulfonyl]glycinamide | 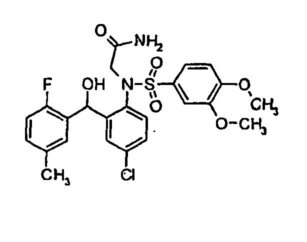 |
| Composé 195 : *N*²-[3-chloro-2-(2,6-difluorobenzyl)phényl]-*N*²-[(3,4-diméthoxyphényl)sulfonyl]glycinamide | 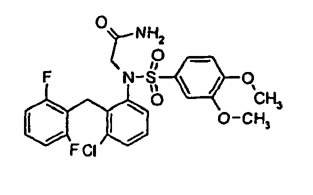 |
| Composé 196 : *N*²-[4-chloro-2-(2-fluoro-5-méthylbenzyl)phényl]-*N*²-[(3,4-diméthoxyphényl)sulfonyl]glycinamide | 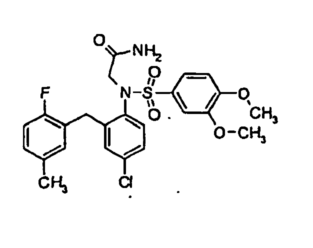 |
| Composé 197 : N-[4-chloro-2-(2,6-difluorobenzyl)phényl]-N-[3-(diméthylamino)propyl]-3,4-diméthoxybenzènesulfonamide | 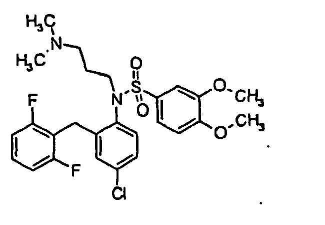 |
| Composé 198 : *N*²-{2-[(2,6-difluorophényl)(hydroxy)méthyl]-4-méthylphényl}- *N*²-[(3,4-diméthoxyphényl)sulfonyl]glycinamide | 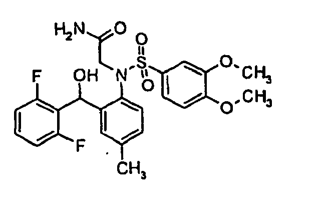 |
| Composé 199 : N-[4-chloro-2-(2,6-difluorobenzyl)phényl]-3,4-diméthoxy-N-(2-pyrrolidin-1-yléthyl)benzènesulfonamide | 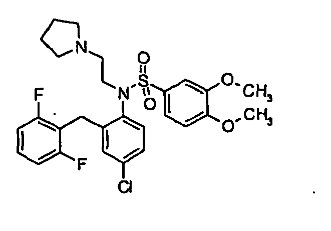 |
| Composé 200 : *N*²-[2-(2,6-difluorobenzyl)-4-méthylphényl]-*N*²-[(3,4-diméthoxyphényl)sulfonyl]glycinamide | 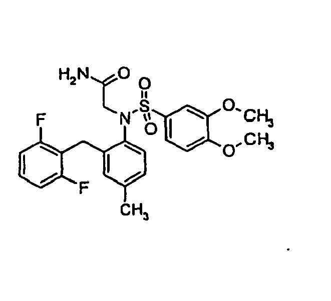 |
| Composé 201 : N-[4-chloro-2-(2,6-difluorobenzyl)phényl]-N-[2-(diméthylamino)-1-méthyléthyl]-3,4-diméthoxybenzènesulfonamide | 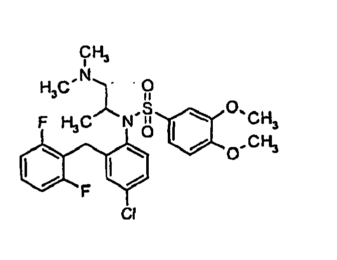 |
| Composé 202 : *N*²-[2-(2,6-difluorobenzyl)-5-(diméthylamino)phényl]-*N*²-[(3,4-diméthoxyphényl)sulfonyl]glycinamide | 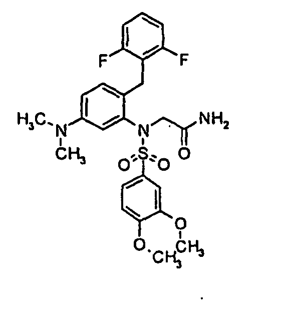 |
| Composé 203 : N-{2-[benzyl(méthyl)amino]éthyl}-N-[4-chloro-2-(2,6-difluorobenzyl)phényl]-3,4-diméthoxybenzènesulfonamide | 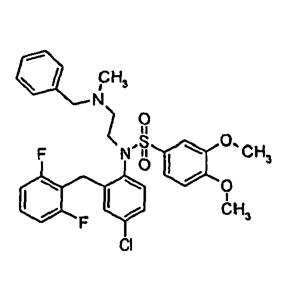 |
| Composé 204 : 4-{[4-chloro-2-(2,6-difluorobenzyl)phényl][(3,4-diméthoxyphényl)sulfonyl]amino}butanamide | 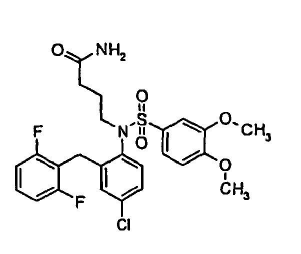 |
| Composé 205 : 2-{[2-(2,6-difluorobenzyl)-4-méthylphényl)[(3,4-diméthoxyphényl)sulfonyl]amino}butanamide | 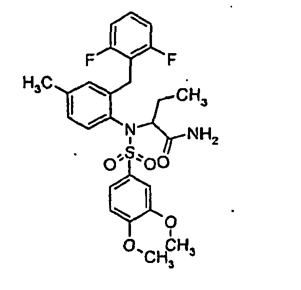 |
| Composé 206 : *N*²-{2-[(2,6-difluorophényl)(hydroxy)méthyl]-4-fluorophényl}- *N*²-[(3,4-diméthoxyphényl)sulfonyl]glycinamide | 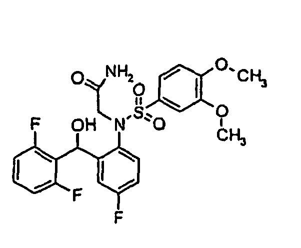 |
| Composé 207 : *N²*-{4-chloro-2-[2-(trifluorométhyl)benzyl]phényl}-*N²*-[(3,4-diméthoxyphényl)sulfonyl]glycinamide | 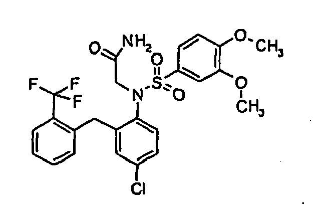 |
| Composé 208 : *N²*-[2-(2,6-difluorobenzyl)-4,6-diméthoxyphényl]-*N²*-[(3,4-diméthoxyphényl)sulfonyl]glycinamide | 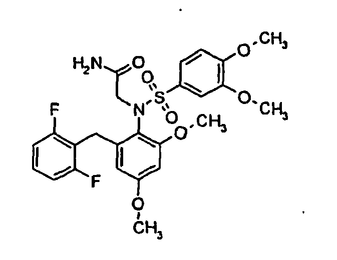 |
| Composé 209 : *N²*-{2-[(2,6-difluorophényl)(hydroxy)méthyl]-4-méthoxyphényl}-*N²*-[(3,4-diméthoxyphényl)sulfonyl]glycinamide | 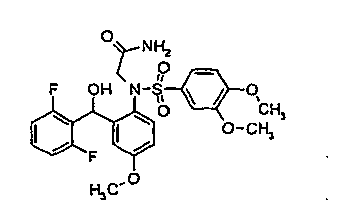 |
| Composé 210 : *N²*-[2-(2,6-difluorabenzyl)-3,6-diméthoxyphényl]-*N²*-[(3,4-diméthoxyphényl)sulfonyl]glycinamide | 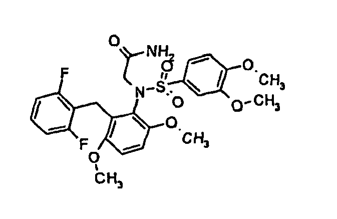 |
| Composé 211 : N-[4-chloro-2-(2,6-difluorobenzyl)phényl]-N-(cyanométhyl)-3,4-diméthoxybenzènesulfonamide | 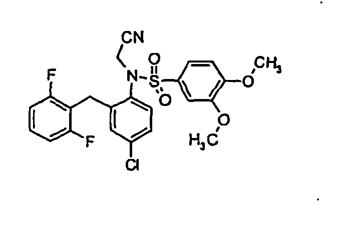 |
| Composé 212 : *N²*-{4-chloro-2-[(5-méthyl-2-thiènyl)méthyl]phényl}-*N²*-[(3,4-diméthoxyphényl)sulfonyl]glycinamide | 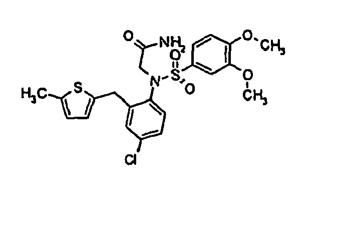 |
| Composé 213 : *N²*-{4-chloro-2-[(2-chloro-6-méthylphényl)(hydroxy)méthyl]phényl}-*N²*-[(3,4-diméthoxyphényl)sulfonyl]glycinamide | 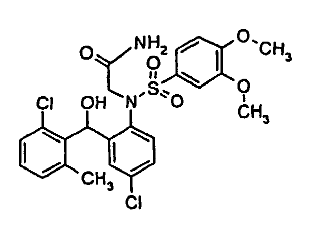 |
| Composé 214 : *N²*-{2,4-dichloro-6-[(2,6-difluorophényl)(hydroxy)méthyl]phényl}-*N²*-[(3,4-diméthoxyphényl)sulfonyl]glycinamide | 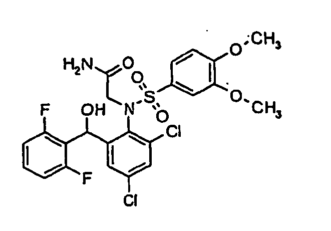 |
| Composé 215 : N-(2-aminoéthyl)-N-[2-(2,6-difluorobenzyl)-4-méthylphényl]-3,4-diméthoxybenzénesulfonamide | 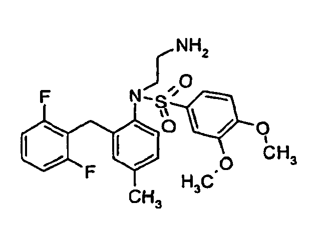 |
| Composé 216: *N²*-{2,4-dichloro-6-[(2,6-difluorophényl)(hydroxy)méthyl]phényl}-*N²*-[(3,4-diméthoxyphényl)sulfonyl]glycinamide | 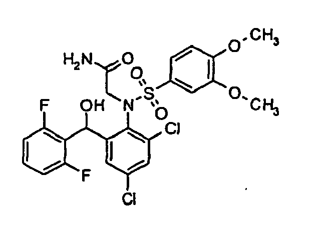 |
| Composé 217 : N-{2-[(anilinocarbonyl)amino]éthyl}-N-[2-(2,6-difluorobenzyl)-4-méthylphényl]-3,4-diméthoxybenzènesulfonamide | 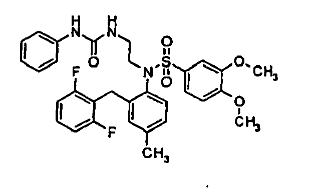 |
| Composé 218 : N-(2-{[(benzylamino)carbonyl]amino}éthyl)-N-[2-(2,6-difluorobenzyl)-4-méthylphényl]-3,4-diméthoxybenzènesulfonamide | 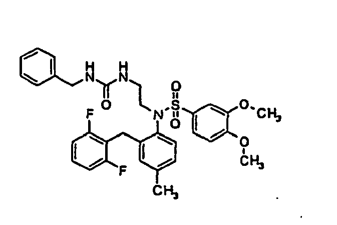 |
| Composé 219 : N-(2-{[2-(2,6-difluorobenzyl)-4-méthylphényl][(3,4-diméthoxyphényl)sulfonyl]amino}éthyl)acétamide | 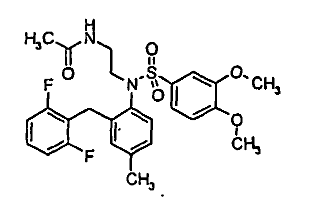 |
| Composé 220 : *N²*-[2-(2,6-difluorobenzyl)-4-fluorophényl]-*N²*-[(3,4-diméthoxyphényl)sulfonyl]glycinamide | 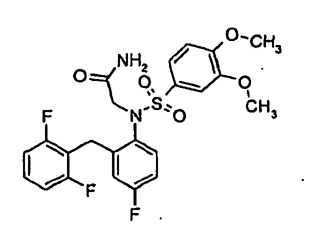 |
| Composé 221 : *N²*-{4,5-dichloro-2-[(2,6-difluorophényl)(hydroxy)méthyl]phényl}-*N²*-[(3,4-diméthoxyphényl)sulfonyl]glycinamide | 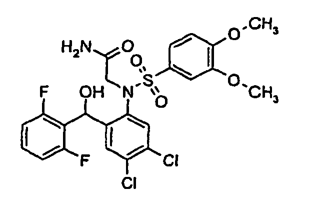 |
| Composé 222 : *N²*-{4-chloro-2-[(2-chloro-6-fluorophényl)(hydroxy)méthyl]phényl}-*N²*-[(3,4-diméthoxyphényl)sulfonyl]glycinamide | 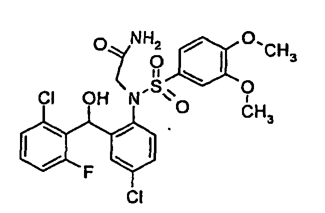 |
| Composé 223 : N-[4-chloro-2-(2,6-difluorobenzyl)phényl]-N-[(3,4-diméthoxyphényl)sulfonyl]-2,2,2-trifluoroacétamide | 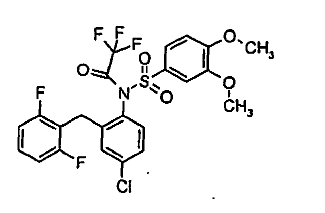 |
| Composé 224 : *N²*-[4-chloro-2-(2-chloro-6-fluorobenzyl)phényl]-*N²*-[(3,4-diméthoxyphényl)sulfonyl]glycinamide | 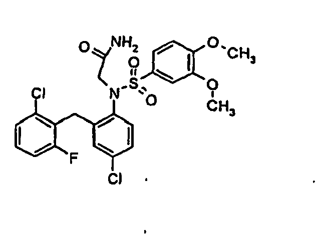 |
| Composé 225 : *N²*-(2-[(2,6-difluorophényl)(hydroxy)méthyl]-6-méthoxyphényl)-*N²*-[(3,4-diméthoxyphényl)sulfonyl]glycinamide | 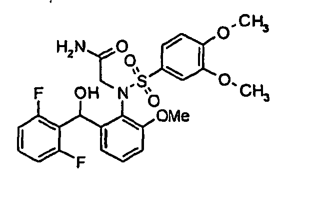 |
| Composé 226 : *N²*-{2-[(2,6-difluorophényl)(hydroxy)méthyl]-6-méthoxyphényl}-*N²*-[(3,4-diméthoxyphényl)sulfonyl]glycinamide | 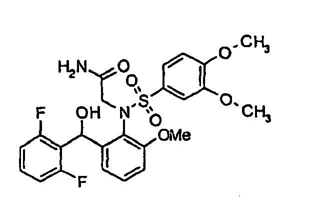 |
| Composé 227 : *N²*-{2-[(2,6-difluorophényl)(hydroxy)méthyl]-5-méthoxy-4-méthylphényl}-*N²*-[(3,4-diméthoxyphényl)sulfonyl]glycinamide | 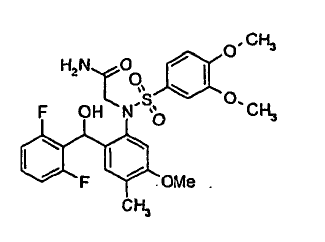 |
| Composé 228 : *N²*-[4,5-dichloro-2-(2,6-difluorobenzyl)phényl]-*N²*-[(3,4-diméthoxyphényl)sulfonyl]glycinamide | 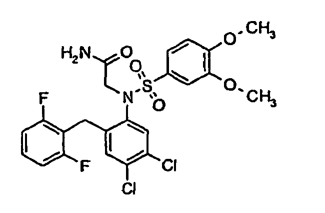 |
| Composé 229 : N-[4-chloro-2-(2,6-difluorobenzyl)phényl]-N-{3-[(diéthylamino)sulfonyl]propyl}-3,4-diméthoxybenzènesulfonamide | 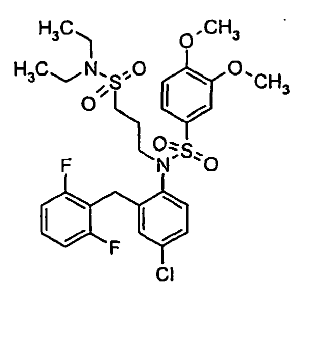 |
| Composé 230 : N-[4-chloro-2-(2,6-difluorobenzyl)phényl]-N-{3-[(diméthylamino)sulfonyl]propyl}-3,4-diméthoxybenzènesulfonamide | 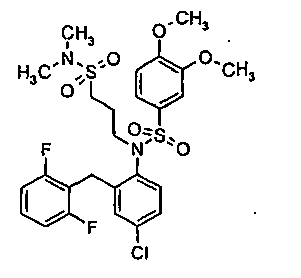 |
| Composé 231 : N-{2-[(aminocarbonyl)amino]éthyl}-N-[4-chloro-2-(2,6-difluorobenzyl)phényl]-3,4-diméthoxybenzènesulfonamide | 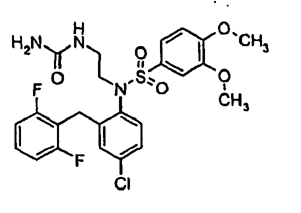 |
| Composé 232 : N-[4-chloro-2-(2,6-difluorobenzyl)phényl]-3,4-diméthoxy-N-(3-pyridin-3-ylpropyl)benzènesulfonamide | 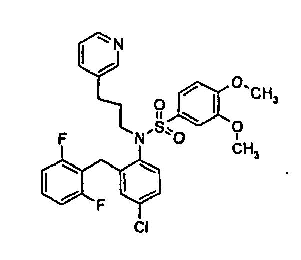 |
| Composé 233 : *N²*-[4-chloro-2-(2-chlorobenzyl)phényl]-*N²*-[(3,4-diméthoxyphényl)sulfonyl]glycinamide | 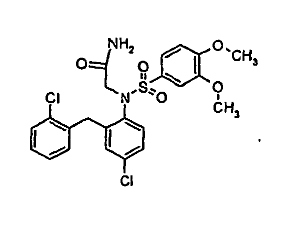 |
| Composé 234 : N-[4-chloro-2-(2,6-difluorobenzyl)phényl]-3,4-diméthoxy-N-[2-(2-oxopyrrolidin-1-yl)éthyl]benzènesulfonamide | 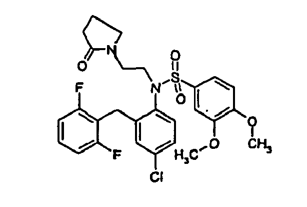 |
| Composé 235 : 4-{[4-chloro-2-(2,6-difluorobenzyl)phényl][(4-chloro-2,5-diméthylphényl)sulfonyl]amino}butanamide | 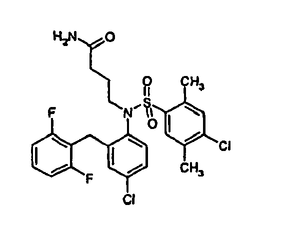 |
| Composé 236 : N-[4-chloro-2-(2,6-difluorobenzyl)phényl]-3,4-diméthoxy-N-(2-méthoxyéthyl)benzènesulfonamide | 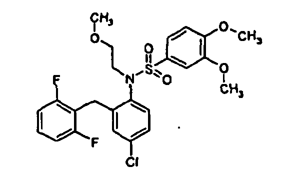 |
| Composé 237 : N-[4-chloro-2-(2,6-difluorobenzyl)phényl]-N-(2-hydroxyéthyl)-3,4-diméthoxybenzènesulfonamide | 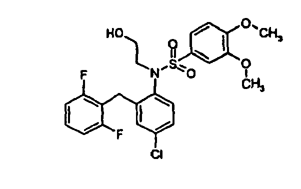 |
| Composé 238 : N-[4-chloro-2-(2,6-difluorobenzyl)phényl]-N-[2-({[(3-chlorophényl)amino]carbonyl}amino)éthyl]-3,4-diméthoxybenzènesulfonamide | 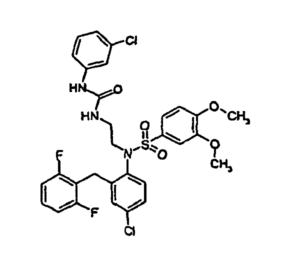 |
| Composé 239 : N-[4-chloro-2-(2,6-difluorobenzyl)phényl]-N-(2-{[(diméthylamino)carbonyl]amino}éthyl)-3,4-diméthoxybenzènesulfonamide | 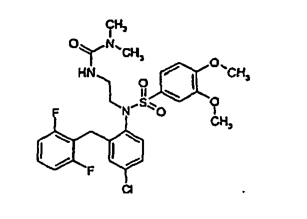 |
| Composé 240 : N-[4-chloro-2-(2,6-difluorobenzyl)phényl]-N-[2-({[(2-chlorophényl)amino]carbonyl}amino)éthyl]-3,4-diméthoxybenzènesulfonamide | 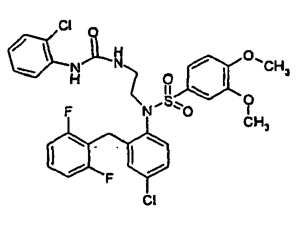 |
| Composé 241 : N∼2∼-[5-bromo-2-(2,6-difluorobenzyl)phényl]-N∼2∼-[(3,4-diméthoxyphényl)sulfonyl]glycinamide | 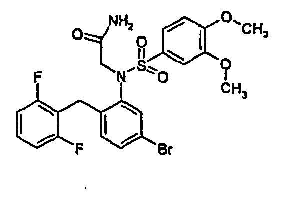 |
| Composé 242 : N∼2∼-[4-bromo-2-(2,6-difluorobenzyl)phényl]-N∼-2∼-[(3,4-diméthoxyphényl)sulfonyl]glycinamide | 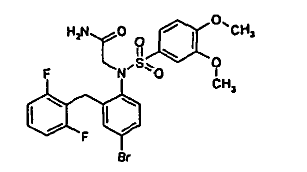 |
| Composé 243 : N-[4-chloro-2-(2,6-difluorobenzyl)phényl]-3,4-diméthoxy-N-[1-(2-méthyl-2H-tétrazol-5-yl)éthyl]benzènesulfonamide | 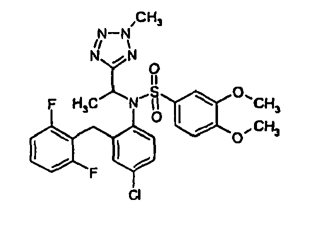 |
| Composé 244 : N-[4-chloro-2-(2,6-difluorobenzyl)phényl]-3,4-diméthoxy-N-[1-(1-méthyl-1H-tétrazol-5-yl)éthyl]benzènesulfonamide | 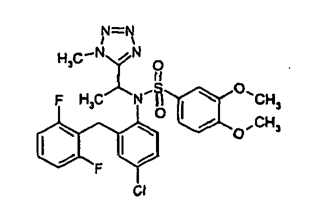 |
| Composé 245 : N-[4-chloro-2-(2,6-difluorobenzyl)phényl]-3,4-diméthoxy-N-(3-pyridin-3-ylpropyl)benzènesulfonamide | 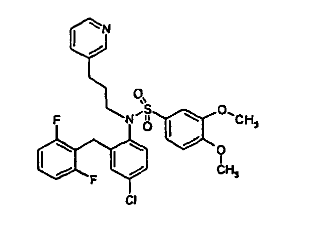 |
| Composé 246 : N-[4-chloro-2-(2,6-difluorobenzyl)phényl]-N-(1-cyanoéthyl)-3,4-diméthoxybenzènesulfonamide | 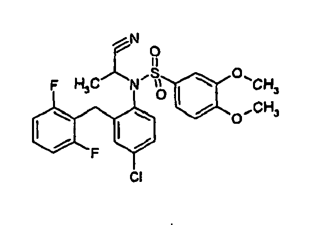 |
| Composé 247 : N~2~-[2-(2,6-difluorobenzyl)-6-méthoxyphényl]-N~2~-[(3,4-diméthoxyphényl)sulfonyl]glycinamide | 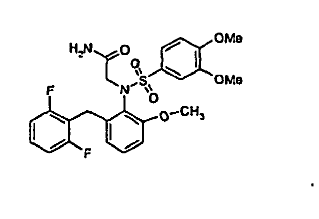 |
| Composé 248 : N-[4-chloro-2-(2,6-difluorobenzyl)phényl]-N-(cyclopropylméthyl)-3,4-diméthoxybenzènesulfonamide | 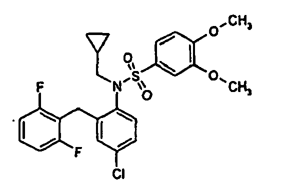 |
| Composé 249 : N∼2∼-[4-chloro-2-(2,6-difluorobenzyl)phényl]-N∼2∼-[(4-chloro-2,5-diméthylphényl)sulfonyl]glycinamide | 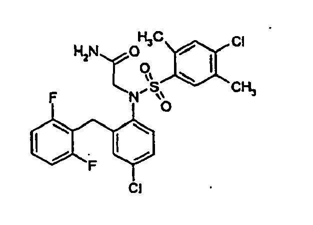 |
| Composé 250 : N-[4-chloro-2-(2,6-difluorobenzyl)phényl]-3,4-diméthoxy-N-(2-pyridin-2-yléthyl)benzènesulfonamide | 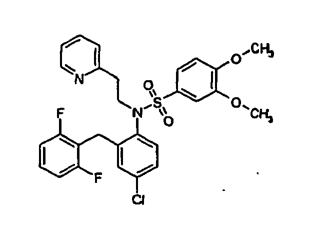 |
| Composé 251 : N∼2∼-{4-chloro-2-[(2,6-difluorophényl)(hydroxy)méthyl]phényl}-N∼2∼-[(3,4-diméthoxyphényl)sulfonyl]glycinamide | 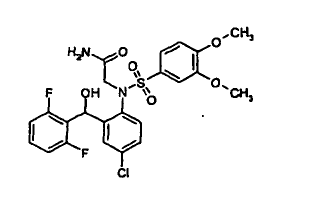 |
| Composé 252 : N-[4-chloro-2-(2,6-difluorobenzyl)phényl]-N-[2-({[(3,4-diméthoxyphényl)amino]carbonyl}amino)éthyl]-3,4-diméthoxybenzènesulfonamide | 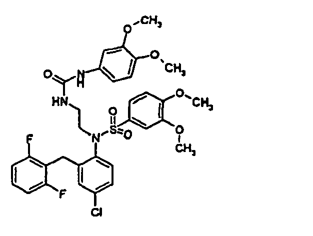 |
| Composé 253 : N-[4-chloro-2-(2,6-difluorobenzyl)phényl]-3,4-diméthoxy-N-(2-{[(méthylamino)carbonyl]amino}éthyl)benzènesulfonamide | 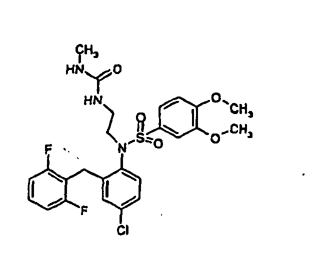 |
| Composé 254 : N-[4-chloro-2-(2,6-difluorobenzyl)phényl]-3,4-diméthoxy-N-(2-pyridin-3-yléthyl)benzénesulfonamide | 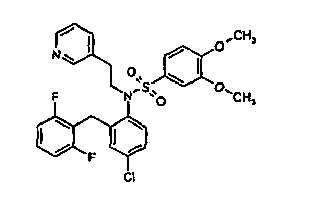 |
| Composé 255 : N-[4-chloro-2-(2,6-difluorobenzyl)phényl]-3,4-diméthoxy-N-(2-pyridin-4-yléthyl)benzènesulfonamide | 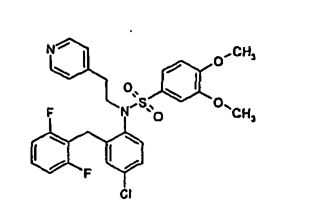 |
| Composé 256 : N-(2-aminoéthyl)-N-[4-chloro-2-(2,6-difluorobenzyl)phényl]-3,4-diméthoxybenzènesulfonamide | 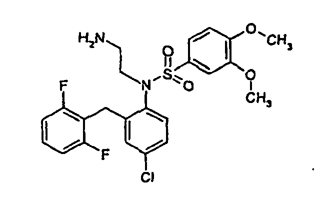 |
| Composé 257 : N-(2-aminoéthyl)-N-[2-(2,5-difluorobenzyl)-4-méthylphényl]-3,4-diméthoxybenzènesulfonamide | 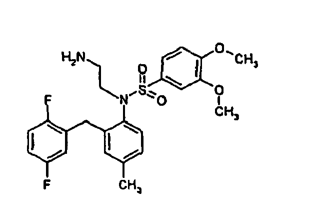 |
| Composé 258 : N-[2-(2,6-difluorobenzyl)-4-méthylphényl]-3,4-diméthoxy-N-[2-(méthylamino)éthyl]benzénesulfonamide | 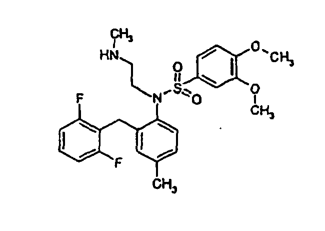 |
| Composé 259 : N∼2∼-[4-chloro-2-(2,6-difluorobenzyl)phényl]-N∼2∼-[(4-méthoxy-3-méthylphényl)sulfonyl]glycinamide | 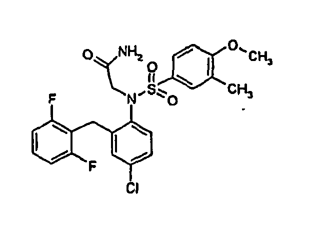 |
| Composé 260 : N-[4-chloro-2-(2,6-difluorobenzyl)phényl]-3,4-diméthoxy-N-(2-phénoxyéthyl)benzènesutfonamide | 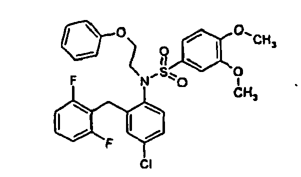 |
| Composé 261 : N∼2∼-[4-chloro-2-(2,6-difluorobenzyl)phényl]-N∼2∼-[(3,4-diméthoxyphényl)sulfonylJalaninamide | 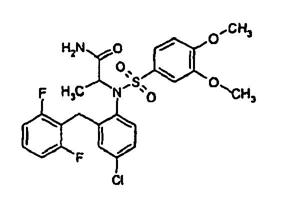 |
| Composé 262 : N-(2-aminoéthyl)-N-[4-chloro-2-(2,5-difluorobenzyl)phényl]-3,4-diméthoxybenzènesulfonamide | 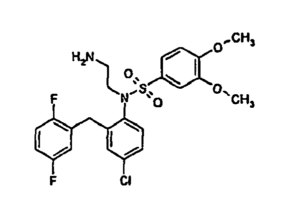 |
| Composé 263 : N~2~-[(4-*tert*-butylphényl)sulfonyl]-N~2~-[4-chloro-2-(2,6-difluorobenzyl)phényl]glycinamide | 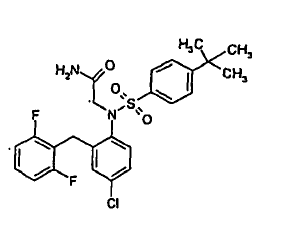 |
| Composé 264 : N-[4-chloro-2-(2,6-difluorobenzyl)phényl]-N-(2-hydroxy-1-méthyléthyl)-3,4-diméthoxybenzènesulfonamide | 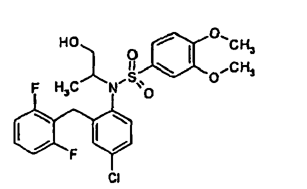 |
| Composé 265 : N∼2∼-[2-(2-chlorobenzyl)-4-(diméthylamino)phényl]-N∼2∼-[(3,4-diméthoxyphényl)sulfonyl]glycinamide | 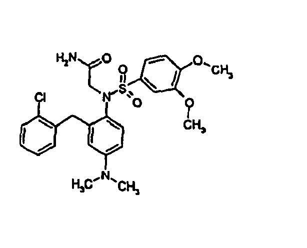 |
| Composé 266 : 4-{[4-chloro-2-(2,5-difluorobenzyl)phényl][(3,4-diméthoxyphényl)sulfonyl]amino}butanamide | 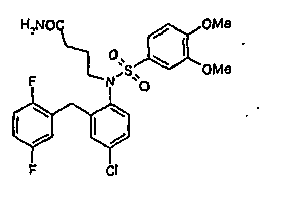 |
| Composé 267 : N∼2∼-[4-chloro-2-(2,5-difluorobenzyl)phényl]-N∼2∼-[(4-chloro-2,5-diméthylphényl)sulfonyl]glycinamide | 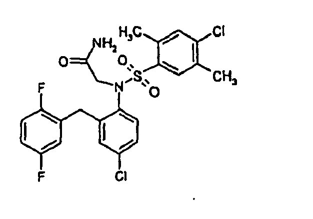 |
| Composé 268 : N-[4-chloro-2-(2,6-difluorobenzyl)phényl]-N-[2-(1,3-dioxolan-2-yl)éthyl]-3,4-diméthoxybenzènesutfonamide | 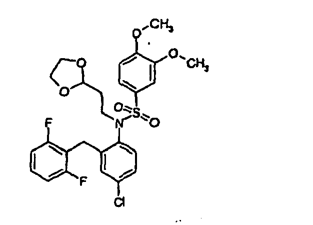 |
| Composé 269 : phényl (2-{[4-chloro-2-(2,6-difluorobenzyl)phényl][(3,4-diméthoxyphényl)sulfonyl]amino}éthyl)carbamate | 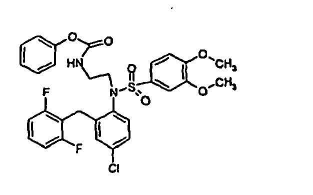 |
| Composé 270 : N-[4-chloro-2-(2,6-difluorobenzyl)phényl]-N-(3-hydroxypropyl)-3,4-diméthoxybenzènesulfonamide | 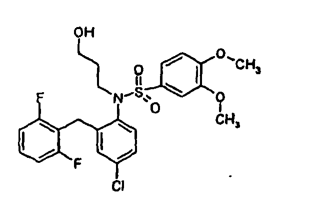 |
| Composé 271 : N-[4-chloro-2-(2,6-difluorobenzyl)phényl]-N-[2-(1H-imidazol-1-yl)éthyl]-3,4-diméthoxybenzènesulfonamide | 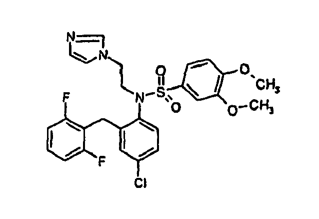 |
| Composé 272 : 4-{[2-(2,6-difluorobenzyl)-6-méthoxyphényl][(3,4-diméthoxyphényl)sulfonyl]amino}butanamide | 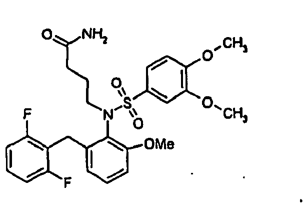 |
| Composé 273 : N∼3∼-[4-chloro-2-(2,6-difluorobenzyl)phényl]-N∼3∼-[(3,4-diméthoxyphényl)sulfonyl]-béta-alaninamide | 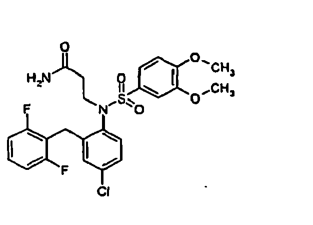 |
| Composé 274 : N-[4-chloro-2-(2,6-difluorobenzyl)phényl]-3,4-diméthoxy-N-[(2R)-pyrrolidin-2-ylméthyl]benzénesulfonamide | 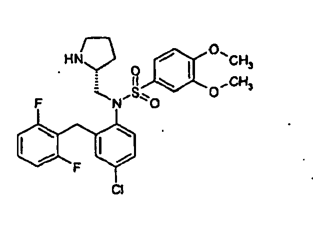 |
| Composé 275 : N-[4-chloro-2-(2,6-difluorobenzyl)phényl]-3,4-diméthoxy-N-[(2S)-pyrrolidin-2-ylméthyl]benzènesulfonamide | 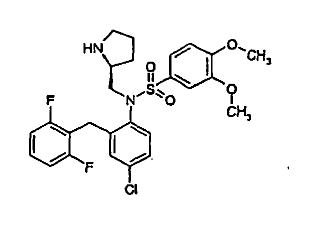 |
| Composé 276 : 2-{[4-chloro-2-(2,6-difluorobenzyl)phényl][(3,4-diméthoxyphényl)sulfonyl]amino}-2-éthoxyacétamide | 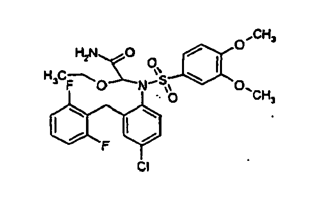 |
| Composé 277 : N-[4-chloro-2-(2,6-difluorobenzyl)phényl]-3,4-diméthoxy-N-[2-(1H-1,2,4-triazol-1-yl)éthyl]benzènesulfonamide | 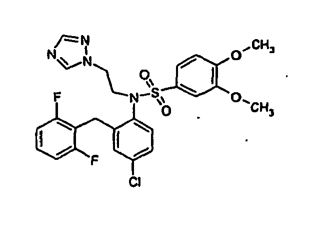 |
| Composé 278 : N-[4-chloro-2-(2,6-difluorobenzyl)phényl]-N-(2-{[2-(diméthylamino)éthyl]amino}éthyl)-3,4-diméthoxybenzènesulfonamide | 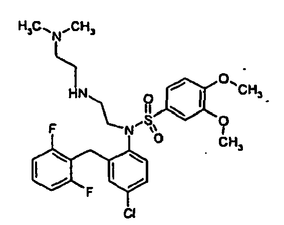 |
| Composé 279 : N∼2∼-[(4-aminophényl)sulfonyl]-N∼2∼-[4-chloro-2-(2,6-difluorobenzyl)phényl]glycinamide | 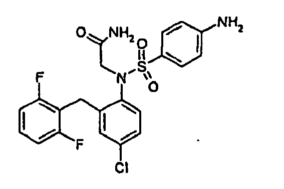 |
| Composé 280 : N∼2∼--[2-(2,5-difluorobenzyl)-4-méthylphényl]-N∼2∼-[(3,4-diméthoxyphényl)sulfonyl]glycinamide | 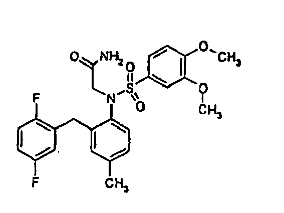 |
| Composé 281 : N-(2-aminoéthyl)-N-[2-(2,6-difluorobenzyl)-6-méthoxyphényl]-3,4-diméthoxybenzènesulfonamide | 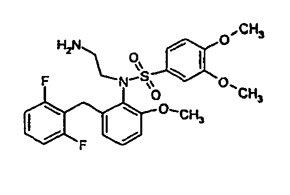 |
| Composé 282 : N-[2-(2,6-difluorobenzyl)-6-méthoxyphényl]-3,4-diméthoxy-N-[2-(méthylamino)éthyl]benzènesulfonamide | 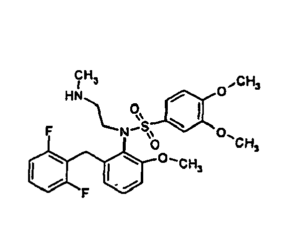 |
| Composé 283 : N∼2∼-[4-chloro-2-(2,6-difluorobenzyl)phényl]-N∼2∼-[(6-méthoxypyridin-3-yl)sulfonyl]glycinamide | 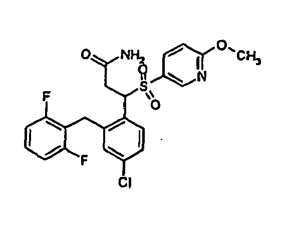 |
| Composé 284 : N-[4-chloro-2-(2,6-difluorobenzyl)phényl]-3,4-diméthoxy-N-[2-(m éthylamino)éthyl]benzènesulfonam ide | 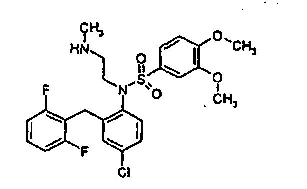 |
| Composé 285 : N∼2∼-[2-(2,5-difluorobenzyl)-6-méthoxyphényl]-N∼2~-[(3,4-diméthoxyphényl)sulfonyl]glycinamide | 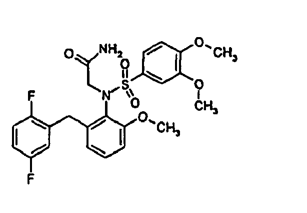 |
| Composé 286 : N∼2∼-[5-chloro-2-(2,6-difluorobenzyl)phényl]-N∼2∼-[(3,4-diméthoxyphényl)sulfonyl]glycinamide | 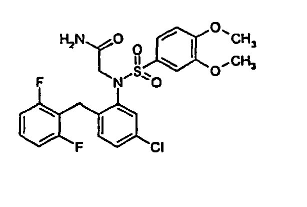 |
| Composé 287 : N-(3-aminopropyl)-N-[4-chloro-2-(2,6-difluorobenzyl)phényl]-3,4-diméthoxybenzènesulfonamide | 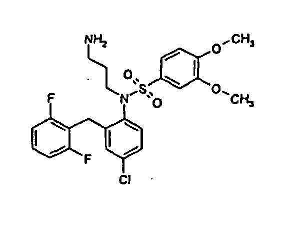 |
| Composé 288 : N∼2∼-[2-(2,6-difluorobenzyl)-6-méthylphényl]-N∼2∼-[(3,4-diméthoxyphényl)sulfonyl]glycinamide | 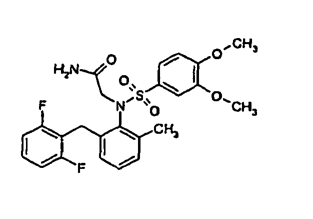 |
| Composé 289 : N-[4-chloro-2-(2,6-difluorobenzyl)phényl]-3,4-diméthoxy-N-vinylbenzènesulfonamide | 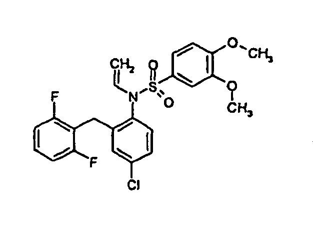 |
| Composé 290 : N-[4-chloro-2-(2,6-difluorobenzyl)phényl]-3,4-diméthoxy-N-(1,3-thiazol-2-ylméthyl)benzènesulfonamide | 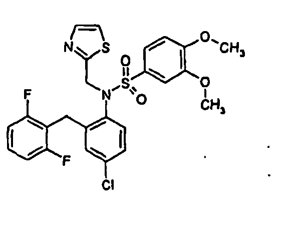 |
| Composé 291 : N-[4-chloro-2-(2,6-difluorobenzyl)phényl]-3,4-diméthoxy-N-(pyrimidin-2-ylméthyl)benzènesulfonamide | 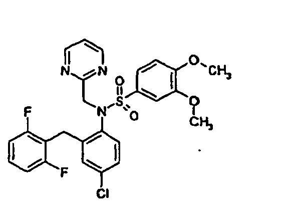 |
| Composé 292 : N-[4-chloro-2-(2,6-difluorobenzyl)phényl]-3,4-diméthoxy-N-(2-morpholin-4-yléthyl)benzènesulfonamide | 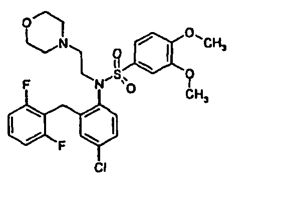 |
| Composé 293 : N∼2∼-[2-chloro-6-(2,6-difluorobenzyl)phényl]-N∼2∼-[(3,4-diméthoxyphényl)sulfonyl]glycinamide | 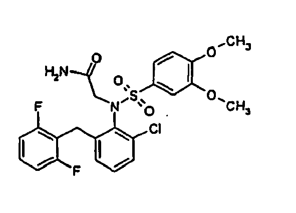 |
| Composé 294 : N-(2-aminoéthyl)-N-[2-(2,5-difluorobenzyl)-6-méthoxyphényl]-3,4-diméthoxybenzènesulfonamide | 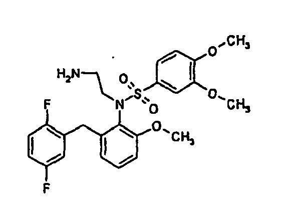 |
| Composé 295 : N-[4-chloro-2-(2,6-difluorobenzyl)phényl]-3,4-diméthoxy-N-(pyridin-2-ylméthyl)benzènesulfonamide | 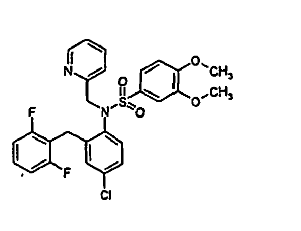 |
| Composé 296 : N∼2∼-[2-(2,6-difluorobenzyl)-5-méthoxyphényl]-N∼2∼-[(3,4-diméthoxyphényl)sulfonyl]glycinamide | 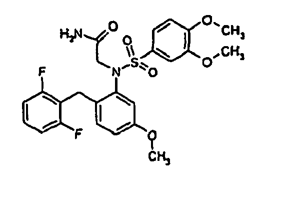 |
| Composé 297 : N-[2-(2,6-difluorobenzyl)-6-méthoxyphényl]-3,4-diméthoxy-N-méthylbenzènesulfonamide | 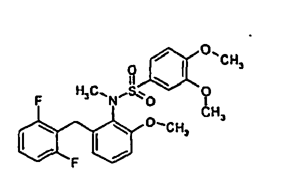 |
| Composé 298 : 3-{[4-chloro-2-(2,6-difluorobenzyl)phényl][(3,4-diméthoxyphényl)sulfonyl]amino}butanamide | 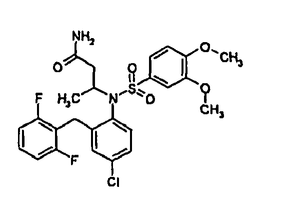 |
| Composé 299 : 4-{[2-(2,5-difluorobenzyl)-6-méthoxyphényl][(3,4-diméthoxyphényl)sulfonyl]amino}butanamide | 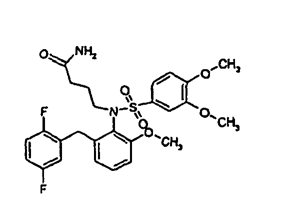 |
| Composé 300 : N-(2-aminoéthyl)-N-[4-chloro-2-(2,6-difluorobenzyl)-6-méthoxyphényl]-3,4-diméthoxybenzènesulfonamide | 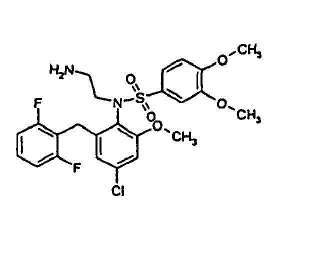 |
| Composé 301 : N-[2-(2,6-difluorobenzyl)-6-méthoxyphényl]-N-[2-(1H-imidazol-1-yl)éthyl]-3,4-diméthoxybenzènesulfonamide | 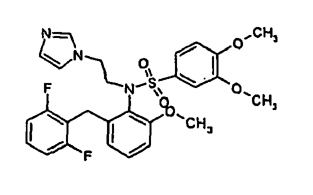 |
| Composé 302 : N∼2∼-[4-chloro-2-(2,6-difluorobenzyl)-6-méthoxyphényl]-N∼2∼-[(3,4-diméthoxyphényl)sulfonyl]glycinamide | 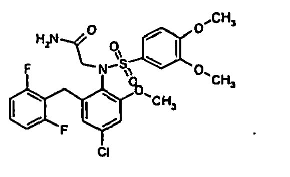 |
| Composé 303 : N∼2∼-[2-(2,6-difluorobenzyl)-6-méthoxy-4-méthylphényl]-N∼2∼-[(3,4-diméthoxyphényl)sulfonyl]glycinamide | 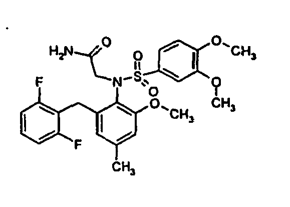 |
| Composé 304 : (+)N∼2∼-[4-chloro-2-(2,6-difluorobenzyl)phényl]-N∼2∼[(3,4-diméthoxyphényl)sulfonyl]alaninamide | 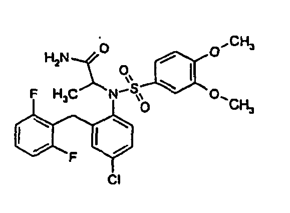 |
| Composé 305 : (-)N∼2∼-[4-chloro-2-(2,6-difluorobenzyl)phényl]-N∼2∼-[(3,4-diméthoxyphényl)sulfonyl]alaninamide | 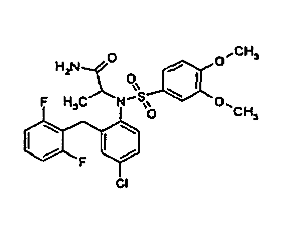 |
| Composé 306 : N-[2-(2,6-difluorobenzyl)-6-méthoxyphényl]-3,4-diméthoxy-N-[2-(1H-1,2,4-triazol-1-yl)éthyl]benzènesulfonamide | 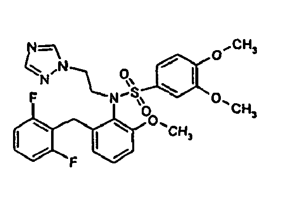 |
| Composé 307 : N∼2∼-[4-chloro-2-(2,6-difluorobenzyl)phényl]-N∼2∼-[(3,5-diméthoxyphényl)sulfonyl]glycinamide | 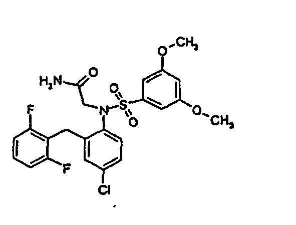 |
| Composé 308 : N~2~-[4-chloro-2-(2,6-difluorobenzyl)phényl]-N~2~-[(3-méthoxy-4-méthylphényl)sulfonyl]glycinamide | 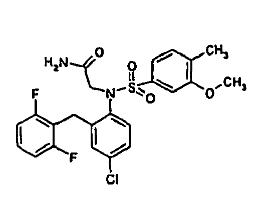 |
| Composé 309 : N∼2∼-[2-(2,6-difluorobenzyl)-6-méthoxyphényl]-N∼2∼-[(3,4-diméthoxyphényl)sulfonyl]-N-éthylglycinamide | 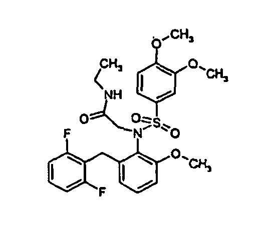 |
| Composé 310 : N-[2-(2,6-difluorobenzyl)-6-méthoxyphényl]-N-[(2-éthyl-2H-tétrazol-5-yl)méthyl]-3,4-diméthoxybenzènesulfonamide | 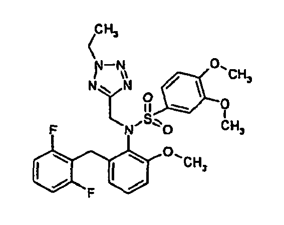 |
| Composé 311 : N-[2-(2,6-difluorobenzyl)-6-méthoxyphényl]-N-[(1-éthyl-1H-tétrazol-5-yl)méthyl]-3,4-diméthoxybenzènesulfonamide | 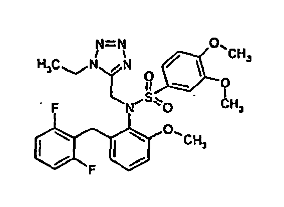 |
| Composé 312 : N∼2∼-[(3,4-diméthoxyphényl)sulfonyl]-N-2--(3-méthoxybiphényl-2-yl)glycinamide | 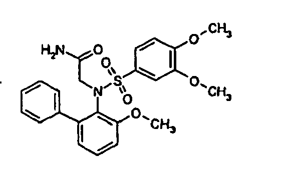 |
| Composé 313 : N-[4-chloro-2-(2,6-difluorobenzyl)phényl]-3,4-diméthoxy-N-{2-[(méthylsulfonyl)amino]éthyl}benzènesulfonamide | 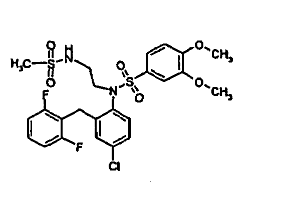 |
| Composé 314 : N∼2∼-[4-chloro-2-(2,6-difluorobenzyl)phényl]-N∼2∼-[(2-fluoro-4,5-diméthoxyphényl)sulfonyljglycinamide | 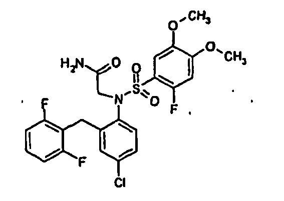 |
| Composé 315 : méthyl N-[2-(2,6-difluorobenzyl)-6-méthoxyphényl]-N-[(3,4-diméthoxyphényl)sulfonyl]-L-alaninate | 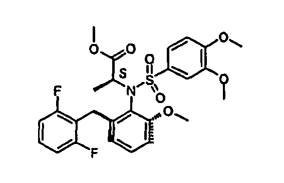 |
| Composé 316 : méthyl N-[2-(2,6-difluorobenzyl)-6-méthoxyphényl]-N-[(3,4 diméthoxyphényl)sulfonyl]-L-alaninate | 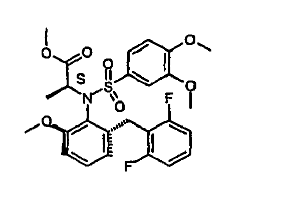 |
| Composé 317 : méthyl N-[2-(2,6-difluorobenzyl)-6-méthoxyphényl]-N-[(3,4-diméthoxyphényl)sulfonyl]-D-alaninate | 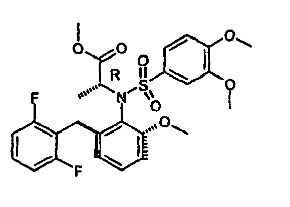 |
| Composé 318 : méthyl N-[2-(2,6-difluorobenzyl)-6-méthoxyphényl]-N-[(3,4-diméthoxyphényl)sulfonyl]-D-alaninate | 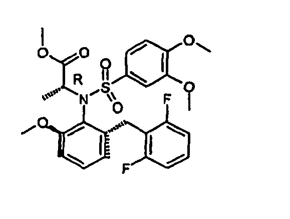 |
| Composé 319 : N∼2∼-[(4-*tert*-butylphényl)sulfonyl]-N∼2∼-[2-(2,6-difluorobenzyl)-6-méthoxyphényl]glycinamide | 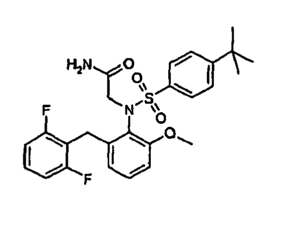 |
| Composé 320 : N∼2∼-[2-(2,6-difluorobenzyl)-6-méthoxyphényl]-N∼2∼-[(3,4-difluorophényl)sulfonyl]glycinamide | 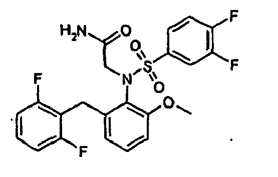 |
| Com posé 321 : N²-[2-(2,6-difluorobenzyl)-6-méthoxyphényl]-N²-[(3,4-diméthoxy phényl)sulfonyl]-R-alaninamide | 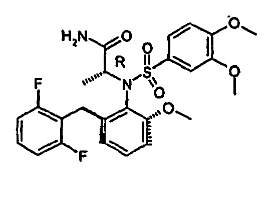 |
| Composé 322 : N²-[2-(2,6-difluorobenzyl)-6-méthoxyphényl]-N²-[(3,4-diméthoxyphényl)sulfonyl]-S-alaninamide | 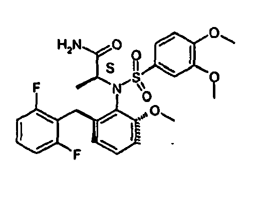 |
| Composé 323 : N²-[2-méthoxy-6-(2-phényléthyl)phényl]-N²-[(3,4-diméthoxy phényl)sulfonyl]glycinamide | 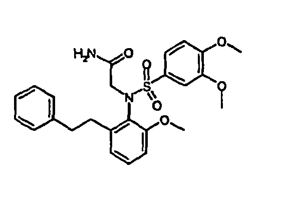 |

Dans le cadre de l'invention, on entend par :
- un groupe (C₁-C₄) alkyle : un groupe aliphatique saturé, linéaire ou ramifié comprenant de 1 à 4 atomes de carbone, tel que le méthyle, l'éthyle, le propyle, l'isopropyle, le butyle, l'isobutyle, le sec-butyle, le *tert*-butyle ;
- un groupe (C₁-C₄) alkyle éventuellement substitué : un groupe alkyle tel que défini ci-dessus dans lequel un ou plusieurs atomes d'hydrogène ont été substitués par un substituant ;
- un groupe (C₃-C₈) cycloalkyle : un groupe alkyle cyclique saturé, comprenant de 3 à 6 atomes de carbone, par exemple le cyclopropyle, le cyclobutyle, le cyclopentyle, le cyclohexyle. Le groupe cycloalkyle peut éventuellement être substitué par un groupe (C₁-C₄) alkyle, par exemple le méthylcyclopropyl, diméthylcyclopropyl, méthylcyclobutyl, méthyl-cyclopentyl, méthyl-cyclohexyl, diméthyl-cyclohexyl ;
- un groupe (C₁-C₄) alcoxy : un radical (C₁-C₄)alkyl-O- où le groupe (C₁-C₄) alkyle est tel que défini précédemment, par exemple le méthoxy, l'éthoxy, le propoxy, l'isopropoxy, le butoxy, l'isobutoxy, le sec-butoxy, le *tert*-butoxy ;
- un groupe (C₁-C₄) alkylcarbonyle : un groupe (C₁-C₄)alkyl-C(O)- où le groupe (C₁-C₄) alkyle est tel que défini précédemment ;
- un groupe fluoro(C₁-C₄)alkyle : un groupe (C₁-C₄)alkyle tel que défini précédemment dont un ou plusieurs atomes d'hydrogène ont été substitués par un ou plusieurs atomes de fluor. A titre d'exemples, on peut citer les groupes -CF₃, -CH₂-CF₃ ;
- un groupe (C₁-C₄)alcényle : un groupe aliphatique mono- ou poly-insaturé, linéaire ou ramifié comprenant de 1 à 4 atomes de carbone, présentant par exemple une ou deux insaturations éthyléniques ;
- un groupe fluoro(C₁-C₄)alcoxy : un groupe (C₁-C₄)alcoxy tel que défini précédemment dont un ou plusieurs atomes d'hydrogène ont été substitués par un ou plusieus atomes de fluor. A titre d'exemples, on peut citer les groupes -O-CF₃, -O-CH₂-CF₃ ;
- un atome d'halogène : un atome de fluor, un atome de chlore, un atome de brome ou un atome d'iode ;
- un groupe aryle : un groupe aromatique monocyclique ou bicyclique comprenant entre 6 et 10 atomes de carbone, par exemple le phényle, le naphtyle. Le groupe aryle peut éventuellement être substitué par 1, 2, 3 ou 4 substituants. A titre de substituants on peut citer par exemple un atome d'halogène, un groupe (C₁-C₄) alkyle, un groupe (C₁-C₄) alcoxy, un groupe fluoro(C₁-C₄)alkyle, un groupe fluoro(C₁-C₄)alcoxy, un groupe hydroxyle, un groupe -CN, un groupe nitro, un groupe -CO-NRₐR_{b} avec Rₐ et R_{b} étant, indépendamment l'un de l'autre, un atome d'hydrogène, un groupe (C₁-C₄) alkyle ;
- un groupe hétérocyclyle : un groupe monocyclique ou bicyclique saturé, insaturé ou aromatique comprenant entre 5 et 10 atomes et comprenant de 1 à 4 hétéroatomes choisis parmi l'azote, l'oxygène ou le soufre. A titre d'exemple on peut citer le 1,3-dioxolanyle, l'imidazolyle, le tétrazolyle, l'oxadiazolyle, le pyridinyle, le thiazolyle, le thiényle, le pyrimidinyle, le triazolyle. Le groupe hétérocyclyle peut éventuellement être substitués par 1, 2, 3, 4 ou 5 substituants. A titre de substituants on peut citer par exemple, un atome d'halogène, un groupe (C₁-C₄) alkyle, un groupe (C₁-C₄) alcoxy, un groupe hydroxyle, un groupe -CN.

Comme déjà indiqué, les composés de formule générale (I) peuvent comporter un ou plusieurs carbones asymétriques. Ils peuvent donc exister sous forme d'énantiomères ou de diastéréoisomères. Ces énantiomères, diastéréoisomères, ainsi que leurs mélanges, y compris les mélanges racémiques, font partie de l'invention.

De par leur structure, les composés de formule générale (I) peuvent également exister sous forme de rotamères. Dans le cadre de l'invention, on entend par rotamères des composés qui ont des formules dévéloppées identiques mais des conformations spatiales figées différentes. Ces différences dans les conformations spatiales figées de ces composés peuvent leur conférer des propriétés physicochimiques différentes et, même dans certains cas, des activités biologiques différentes.

Les composés de formule générale (I) peuvent encore exister sous forme d'atropoisomères. Les atropoisomères sont des composés de formules développées identiques, mais qui présentent une configuration spatiale particulière, résultant d'une rotation restreinte autour d'une liaison simple, due à un encombrement stérique important de part et d'autre de cette liaison simple. L'atropioisomérie est indépendante de la présence d'éléments stéréogènes, tel qu'un carbone asymétrique.

Les composés de formule (I) peuvent exister à l'état de bases ou de sels d'addition à des acides. De tels sels d'addition font partie de l'invention.

Ces sels sont avantageusement préparés avec des acides pharmaceutiquement acceptable, mais les sels d'autres acides utiles, par exemple, pour la purification ou la séparation des composés de formule générale (I) font également partie de l'invention.

Les composés de formule générale (I) peuvent, en outre, se trouver sous forme d'hydrates ou de solvats, à savoir sous forme d'associations ou de combinaisons avec une ou plusieurs molécules d'eau ou avec un solvant. De tels hydrates et solvats font également partie de l'invention. Dans ce qui suit, on entend par groupe partant, un groupe pouvant être facilement clivé d'une molécule, avec départ d'une paire électronique, par rupture d'une liaison hétérolytique. Ce groupe peut ainsi être remplacé facilement par un autre groupe lors d'une réaction de substitution par exemple. De tels groupes partants sont, par exemple, les halogènes, ou un groupe hydroxy activé tel qu'un mésylate, tosylate, triflate, acétyle, etc. Des exemples de groupes partants ainsi que des références pour leur préparation sont donnés dans « Advanced Organic Chemistry », J. March, 3rd Edition, Wiley Interscience, p 310-316.

La présente invention a également pour objet le procédé de préparation des composés de formule générale (I).

Ainsi, les composés de formule générale (I) peuvent être préparés par les procédés illustrés dans le schéma 1. Selon ce schéma, les composés de formule (I), peuvent être obtenus :
- soit par l'alkylation des composés de formule générale (II) avec des composés de formule générale (IV), en présence d'une base, selon une adaptation du procédé décrit par Levin et al., Bioorg. Med. Chem., 2001, EN 11 ; 22 ; 2975-2978 ;
- soit par une réaction de Mitsunobu entre les alcools de formule (X) et les composés de formule générale (II).

Dans les composés de formule (II), (IV) et (X), Ar₁, Ar₂, Ar₃, T et R₁ sont tels que définis dans la formule (I) et Z est un groupe partant tel qu'un atome d'halogène choisi parmi le brome, le chlore et l'iode ; ou encore un mésylate, un tosylate, un triflate.

La base peut être une base organique comme par exemple le *tert*-butylate de potassium ou inorganique comme par exemple le carbonate de potassium ou encore un agent de transfert de phase comme par exemple le bromure de tétrabutylammonium.

Dans la réaction de Mitsunobu, le diisopropylazodicarboxylate (DIAD) peut être remplacé par ses analogues comme le diéthylazodicarboxylate et le diterbutylazodicarboxylate, et la triphénylphosphine peut être greffée sur une résine (R. G. Gentles et al., J. Comb. Chem. 2002, 4, 442-456).

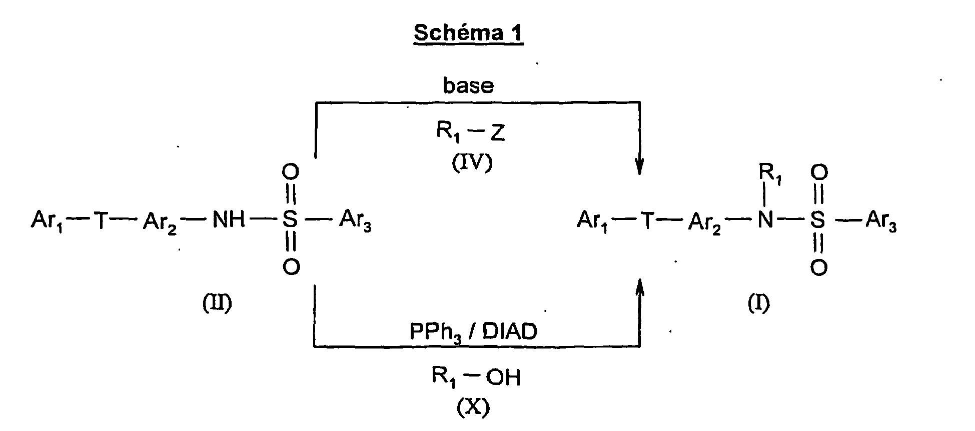

Les composés de structure (I) pour lesquels R₁ est un enchaînement de type -CR₇R₈-(CH₂)ₙ-R₉, avec R₉ = -CONR₁₂R₁₃, peuvent être obtenus à partir des ester correspondants de formule -CR₇R₈-(CH₂)ₙCOOR₁₄, selon la séquence de réaction suivante : saponification de la fonction carboxylate -COOR₁₄ ; activation de la fonction acide carboxylique générée, par exemple par des chloroformiates pour former des anhydrides mixtes ; réaction d'amidation avec des amines de types NHR₁₂R₁₃ avec n, R₇, R₈, R₁₂, R₁₃, R₁₄ comme définis précédemment.

Les composés de structure (I) pour lesquels R₁ est un enchaînement de type -CR₇R₈-(CH₂)ₙ-R₉, avec R₉ = -NH-CO-NR₁₅R₁₆, peuvent être obtenus à partir des composés (I) pour lesquels R₉ = NH₂ par action d'isocyanates de types R₁₅NCO ou R₁₆NCO avec n, R₇, R₈, R₁₅, R₁₆ comme définis précédemment.

Les composés de structure (I) pour lesquels R₁ est un enchaînement de type -CR₇R₈-(CH₂)ₙ-R₉, R₉ = -NR₁₀R₁₁ dans lequel R₁₀ et R₁₁, indépendamment l'un de l'autre, représentent un atome d'hydrogène et un groupe (C₁-C₄) alkylcarbonyle, peuvent être obtenus à partir des composés (I) pour lesquels R₉ = NH₂ par action d'acides carboxyliques de formule (C₁-C₄)alkyl-COOH ou de leurs chlorures d'acides de formule (C₁-C₄)alkyl-COCl avec n, R₇, R₈, R₁₀, R₁₁, comme définis précédemment.

Les composés (I) pour lesquels R₉ est un groupe hétérocyclyle tel que le tétrazolyle substitué sont obtenus par réactions chimiques classiques, connues de l'homme du métier, à partir des composés (I) pour lesquels R₉ est un groupement cyano.

Les composés de structure (I), pour lesquels T = -(CH₂)ₙ avec n = 1, peuvent dans certains cas être obtenus à partir des composés de structure (I), pour lesquels T = -CHOH, par action d'hydrure, par exemple le triéthylsilane, en présence d'éthérate de trifluorure de bore.

Les composés de structure (I), pour lesquels 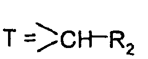 avec R₂ étant un groupe hydroxyle, peuvent dans certains cas être obtenus à partir des composés correspondants pour lesquels 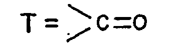 par action d'hydrure, par exemple le borohydrure de sodium.

Les composés de structure (II) sont obtenus au préalable selon le schéma 2, par la sulfonylation du composé de formule (III) avec des chlorures de sulfonyle de formule (V) en présence d'une base choisie parmi les amines tertiaires telle que la pyridine selon le procédé décrit par Stauffer et al., Bioorg. Med. Chem., 2000, EN 8, 6, 1293-1316. Comme amines tertiaires on peut également utiliser la triéthylamine ou la diisopropyléthylamine.

Dans certains cas, on peut même envisager d'utiliser un mélange d'amines tertiaires. Les composés de formule (V) sont commerciaux ou peuvent être obtenus par adaptation des procédés décrits, par exemple, par A. J. Prinsen et al, Recl. Trav. Chim. Pays-Bas 1965, EN 84, 24.

Dans les composés de formule (III) et (V), Ar₁, Ar₂, Ar₃, et T sont tels que définis dans la formule (I).

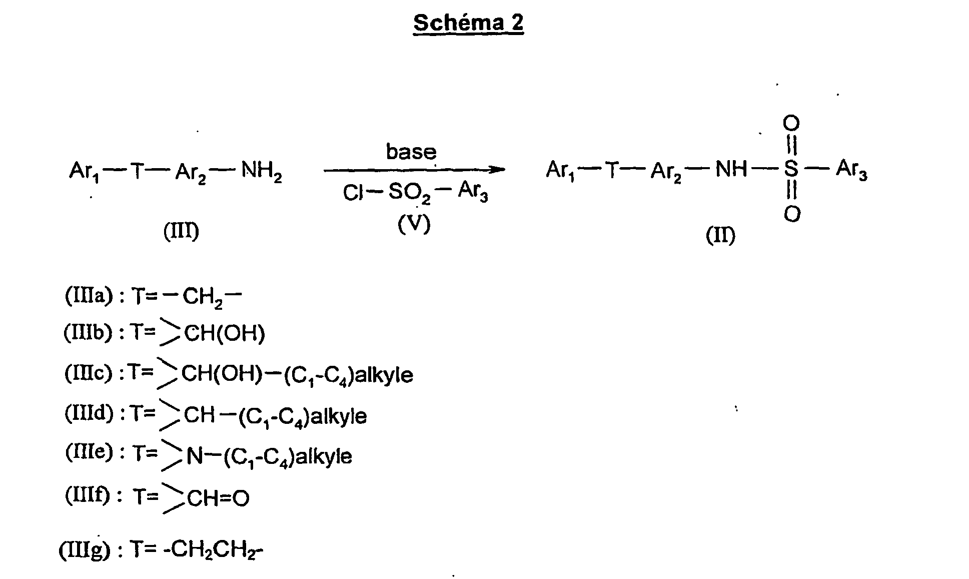

Les composés de formule (IIIa), (IIIb) et (IIIf) sont préparés selon les schémas 3 à 5. Les dérivés 2-nitro aldéhydes de formule (VI) réagissent avec des composés organométalliques de formule (VII) dans laquelle M représente un groupe MgBr, Mgl, Znl ou Li pour conduire aux composés de structure (VIII). Les composés organométalliques de formule (VII) sont commerciaux, ou formés selon les procédés classiques décrits dans la littérature. Les nitro alcools de formule (VIII) sont réduits par hydrogénation, par exemple sous l'action d'étain métallique et d'acide chlorhydrique concentré dans l'éthanol, pour donner les composés de structure (IIIb). Les dérivés de formule (IIIb) sont réduits par l'action d'hydrures, par exemple par un mélange de triéthylsilane et d'acide trifluoroacétique dans le dichlorométhane pour aboutir aux dérivés de formule (IIIa).

Les nitroaldéhydes de formule (VI) sont commerciaux ou peuvent être préparés, par exemple, selon une adaptation du procédé décrit par J. Kenneth Horner et al., J. Med. Chem., 1968, 11; 5; 946.

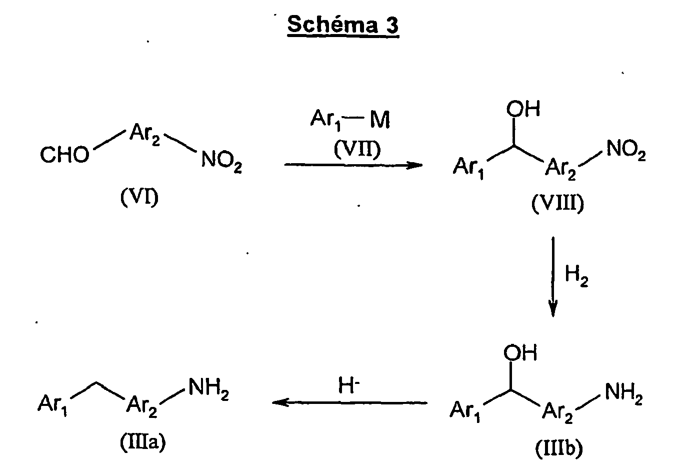

D'autres possibilités pour synthétiser les composés de formules générales (IIIb) et (IIIf) sont présentées dans le schéma 4.

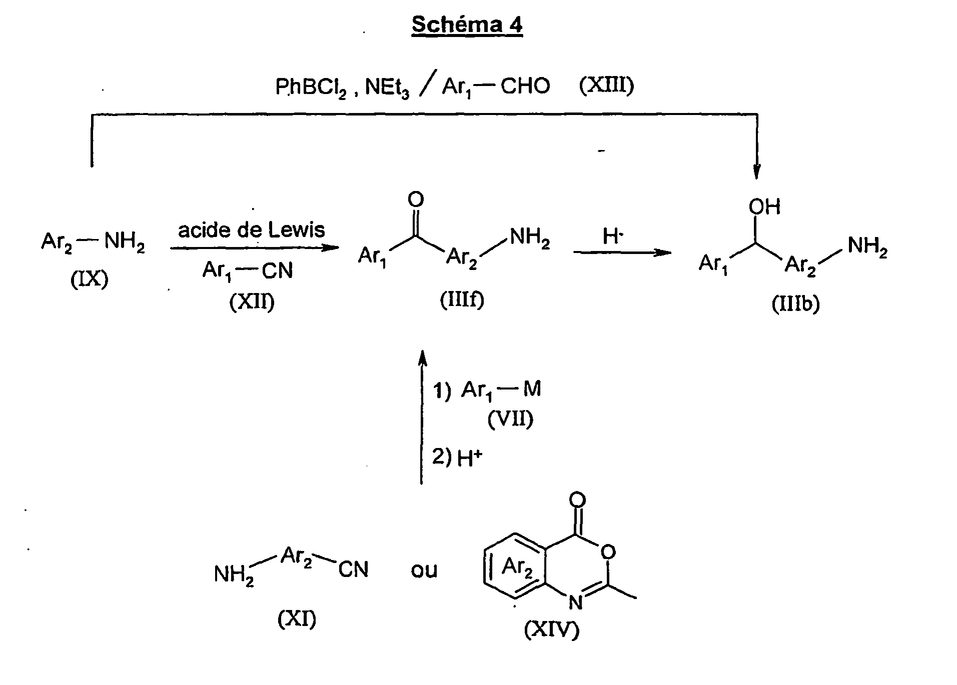

Les anilines de formule (IX) sont condensées avec des nitriles de formule (XII), en présence d'acide de Lewis comme par exemple le trichlorure de bore avec le trichlorure d'aluminium ou avec le trichlorure de gallium pour donner les composés de formule (IIIf), selon le procédé décrit par T. Sugasawa et al J.A.C.S.1978; 100; 4842. Les composés de formule (IIIf) peuvent être obtenus par condensation d'aminonitriles (XI) avec les dérivés organométalliques (VII), selon le procédé décrit par R. Fryer et al., J. Heterocycl. Chem, 1991, EN 28; 7, 1661. Les composés de formule (IIIf) peuvent aussi être obtenus à partir de l'intermédiaire (XIV) selon une adaptation du procédé décrit par D. Lednicer, J. Heterocyclic. Chem, 1971; 903.

La fonction carbonyle des composés (IIIf) est réduite par l'action d'un hydrure, par exemple le borohydrure de sodium dans l'éthanol, pour conduire aux composés de structure (IIIb).

Une autre méthode de préparation des composés de formule (IIIb) consiste à condenser des anilines de formule (IX) sur des dérivés benzaldéhydes de formule (XIII) en présence de phényl-dichloro-borane et de triéthylamine selon le procédé décrit par T. Toyoda et al., Tet. Lett, 1980, 21, 173.

Il est à noter que les composés de formule (IIIf) sous l'action de triéthylsilane et d'acide trifluoroacétique par exemple, peuvent conduire aux composés de formule (IIIa).

Une autre possibilité pour synthétiser les composés de formules générales (IIIa), dans lequel Ar₁ représente un héteroaryle, est présentée dans le schéma 5.

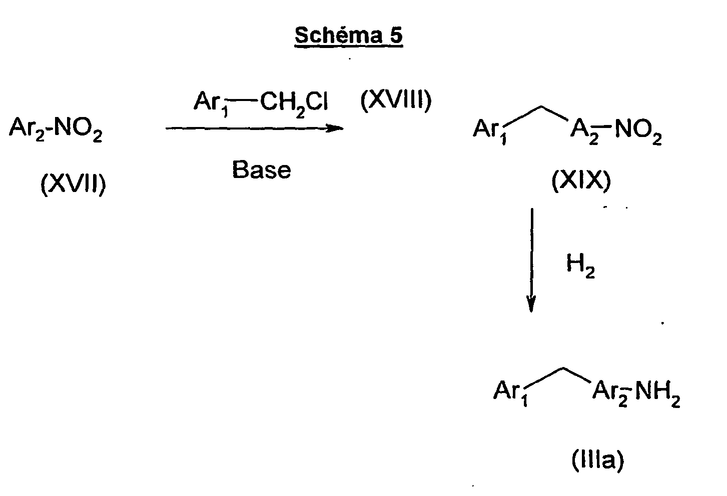

Les nitrophényles de formule (XVII) sont condensés sur des chloro méthyl hétéroaryles de formule (XVIII) en présence d'une base, par exemple le *tert*-butylate de potassium, pour conduire aux dérivés (XIX) selon le procédé décrit par Florio.S et al., Eur.J.Org.Chem.2004, 2118, qui sont réduit par exemple par l'action de l'étain métallique en présence d'acide chlorhydrique 12M, pour conduire aux dérivés de formule (IIIa).

Les composés de formule (IIIc) et (IIId) sont préparés selon le schéma 6.

Les composés de formule (IIIf), sous l'action d'un (C₁-C₄)alkylmagnésien conduisent aux dérivés de formule (IIIc). Ces composés sont déhydroxylés sous l'action de trichlorure d'aluminium et d'hydrure mixte de lithium et d'aluminium pour conduire aux composés (IIId).

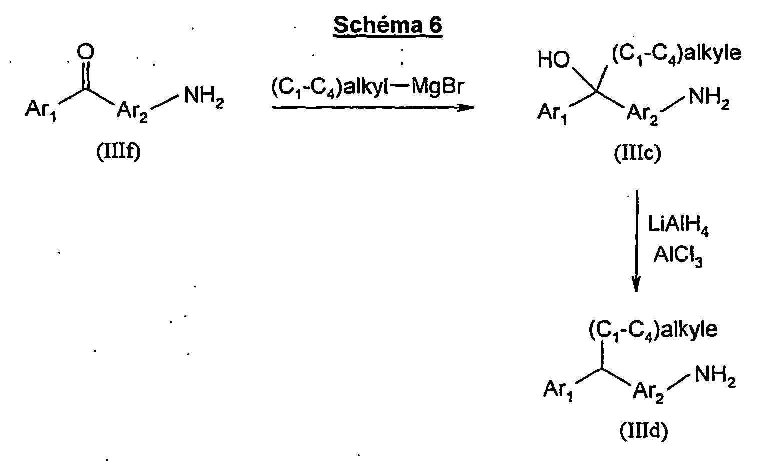

Les composés de formule (IIIe) sont préparés selon le schéma 7. Les dérivés (XV), sous l'action d'une base, par exemple le carbonate de césium et d'un halogenure d'alkyle en C₁-C₄, conduisent aux dérivés (XVI). Le groupement nitro de ces dérivés peut être réduit, par exemple, en présence d'étain métallique et d'acide chlorhydrique dans l'éthanol pour conduire aux composés de formule (IIIe)..

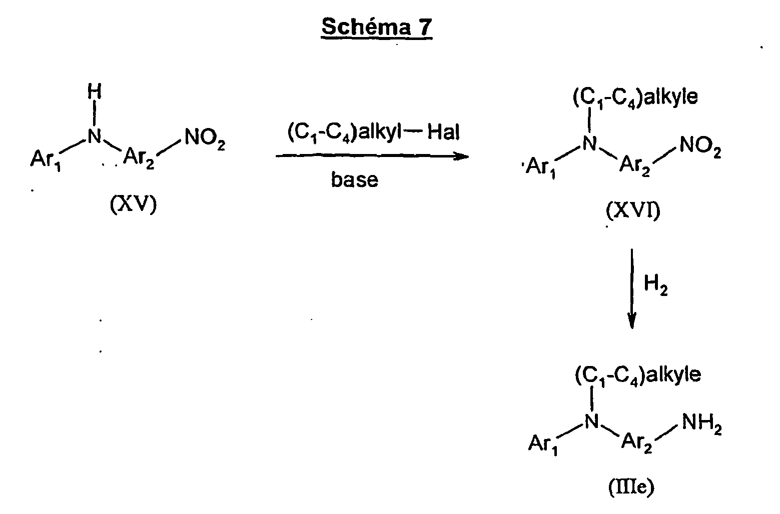

Les composés de formule (IIIg) sont préparés selon le schéma 8. Les nitroaldehydes (VI), par condensation avec les dérivés (XX) selon une réaction de wittig conduisent aux composés (XXI). Ces dérivés sont réduits par exemple par hydrogénation catalytique au palladium pour donner les composés de formule (IIIg).

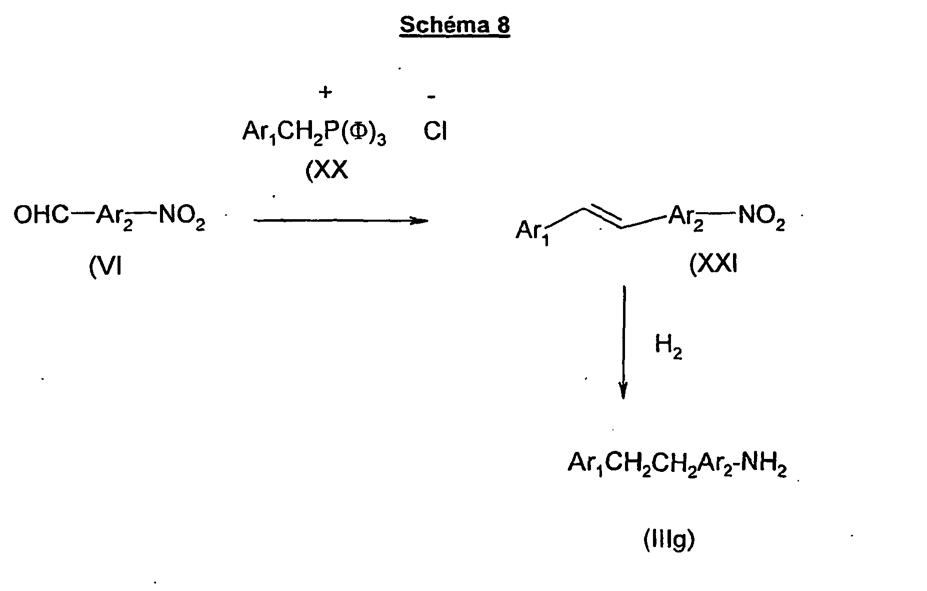

Dans tous les schémas et pour tous les composés de formules (II) à (XXI), les significations de Ar₁, T, Ar₂, Ar₃, R₁ sont telles que définies pour les composés de formule générale (I).

Dans les schémas 1 à 8, les composés de départ et les réactifs, lorsque leur mode de préparation n'est pas décrit, sont disponibles dans le commerce ou décrits dans la littérature, ou bien peuvent être préparés par des méthodes qui y sont décrites ou qui sont connues de l'homme du métier.

Lorsqu'un composé comporte une fonction réactive, par exemple un groupe hydroxyle, elle peut nécessiter une protection préalable avant réaction. L'homme du métier pourra déterminer la nécessité d'une protection préalable.

Les composés de formule (II) à (XXI) sont utiles en tant qu'intermédiaires de synthèse pour la préparation des composés de formule générale (I) et font partie intégrante de la présente invention.

Les exemples suivants décrivent la préparation des composés conformes à l'invention. Ces exemples ne sont pas limitatifs et ne font qu'illustrer l'invention.

Les numéros des composés exemplifiés renvoient à ceux donnés dans le tableau ci-avant. Les micro-analyses élémentaires, les spectres de masse, et les spectres RANZ confirment les structures des composés obtenus.

Les conditions d'analyse par chromatographie liquide couplée à une spectrométrie de masse LC/MS sont les suivantes:
- pour la partie chromatographie liquide : Colonne symetry C18 (2,1x 50mm) 3-5µm. Eluant A= H₂O +0,005% de TFA, pH=3,14; Eluant B=CH₃CN +0,005% de TFA, avec un gradient de 100% de A à 90% de B en 10 minutes, puis 5 minutes à 90% de B
- pour la partie spectrométrie de masse : mode d'ionisation électrospray positif. Lorsque le spectre de RMN¹H met en évidence des rotamères, seule l'interprétation correspondant au rotamère majoritaire est décrite.

Dans les tableaux suivants:
- F(°C) représente le point de fusion du composé en degré Celsius
- MH⁺ représente le pic de masse du produit ionisé
- Le temps de rétention est exprimé en minutes
- n.d. signifie « non déterminé ».

### Exemple 1 : Ethyl N-{4-chloro-2-[(2-chlorophényl)(hydroxy)méthyl]phényl}-N-[(3,4-diméthoxyphényl)sulfonyl]glycinate (composé 1)

### • Exemple 1.1 : N-[4-chloro-2-(2-chlorobenzoyl)phényl]-3,4-diméthoxybenzène sulfonamide

A 5,3g de (2-amino-5-chlorophényl)(2-chlorophényl)méthanone en solution dans 50ml de pyridine on additionne 5,29g de 3,4-diméthoxybenzène sulfonylchlorure et laisse 3 heures à température ambiante. On concentre le milieu réactionnel, le résidu est repris à l'éther diisopropylique, le précipité formé est filtré pour obtenir après séchage 5,2g de produit attendu
RMN ¹H δ en ppm (DMSO d 6): 3,75(s, 3H); 3,83(s, 3H); 7,06-7,69(massif, 11H).

### • Exemple 1.2: N-{4-chloro-2-[(2-chlorophényl)(hydroxy)méthyl]phényl}-3,4-diméthoxybenzènesulfonamide

A 16g de N-[4-chloro-2-(2-chlorobenzoyl)phényl]-3,4-diméthoxybenzènesulfonamide en solution dans 900ml d'éthanol on additionne par portions 3,93g de borohydrure de sodium et laisse 18 heures à température ambiante. Le milieu réactionnel est concentré, Le résidu est repris par de l'acétate d'éthyle et lavé à l'eau. La phase organique est séchée sur sulfate de sodium anhydre et concentrée. Le résidu est concrété à l'éther diisopropylique; après filtration et séchage du précipité on obtient 14,66g de produit attendu.
RMN ¹H δ en ppm (DMSO d 6): 3,77(s, 3H); 3,84(s, 3H); 6,27(s, 1H); 6,96-7,44(massif, 10H).

### • Exemple 1.3 : Ethyl N-{4-chloro-2-[(2-chlorophényl)(hydroxy)méthyl]phényl}-N-[(3,4-diméthoxyphényl)sulfonyl]glycinate

A 6g de N-{4-chloro-2-[(2-chlorophényl)(hydroxy)méthyl]phényl}-3,4-diméthoxybenzène sulfonamide en solution dans 50ml de DMF à 0°C on additionne par portions 0,674g d'hydrure de sodium. Après 1 heure à 0 °C on intoduit 1,4ml de 2-bromoacétate d'éthyle et laisse 18 heures à température ambiante. Le milieu réactionnel est repris à l'acétate d'éthyle et lavé à l'eau. La phase organique est séchée sur sulfate de sodium anhydre et concentrée. Le résidu est chromatographié sur une colonne de gel de silice en éluant au dichlorométhane pour obtenir 1,3g de produit attendu.
RMN ¹H δ en ppm (DMSO d6): 1,03(t, 3H); 2,90(d, 1H); 3,77(s, 3H); 3,87(t, 3H); 4,11 (d, 1H); 4,73(q, 2H); 5,94(d, 1H); 6,47(s, 1H); 7,10 - 7,77(massif, 10H). F= 86°C.

Le tableau I illustre les structures chimiques et les propriétés physiques de quelques composés de l'invention obtenus selon cet exemple

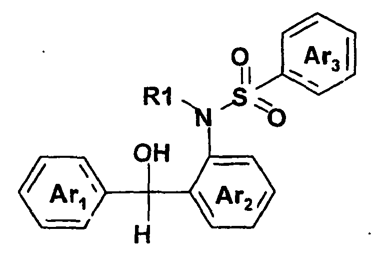

**Tableau I**

| **N° -composé** | **Nature et position des substituants** | | | | **F(°C)** | **MH⁺/ temps de rétention** |
|---|---|---|---|---|---|---|
| | **Ar₁** | **Ar₂** | **R₁** | **Ar₃** | | |
| 8 | H | H | CH₂CO₂Et | 3,4-diOMe | 58,8 | 486/8,15 |
| 7 | H | 4-Cl | CH₂CO₂Et | 3,4-diOMe | 152 | 521/8,78 |
| 9 | 2-Cl | 4-Cl | CH₂CO₂Et | 2,4-diOMe | 155 | 555/9,34 |
| 10 | 2-Cl | 4-Cl | CH₂CO₂Et | H | 109,8 | 495/9,40 |
| 13 | 2-Cl | 4-Cl | CH₂CO₂Et | 4-OMe | 98,2 | 52419,47 |
| 18 | 2-Cl | 4-Cl | CH₂CO₂Et | 3,4-diCl | 140 | 562/10,46 |
| 19. | 2-CI | 4-Cl | CH₂CONH2. | 3,4-diOMe | 154,7 | 526/7,52 |
| 28 | 2-Cl | 4-Cl | CH₂CONH₂ | 3,4-diMe | 205 | n.d. |
| 31 | 2-Cl | 4-Cl | CH₂CONH₂ | 3-Cl | 187 | 499/8,32 |
| 32 | 2-Cl | 4-Cl | CH₂CONH₂ | 4-OMe | 207 | 495/7,86 |

### Exemple 2: N-{4-chloro-2-[(2-chlorophényl)(hydroxy)méthyl]phényl}-N-éthyl-3,4-diméthoxybenzènesulfonamide (composé 20)

A 1g de N-{4-chloro-2-[(2-chlorophényl)(hydroxy)méthyl]phényl}-3,4-diméthoxy benzènesulfonamide, obtenu à l'exemple 1.2 en solution dans 43 ml d'acétonitrile on additionne successivement, 1,2 ml de triéthylamine , 0,68 ml de iodoéthane et porte à reflux 8 heures . Le milieu réactionnel est repris à l'acétate d'éthyle et lavé à l'eau. La phase organique est séchée sur sulfate de sodium anhydre et concentrée. Le résidu est chromatographié sur une colonne de gel de silice en éluant avec un mélange de solvant cyclohexane / acétate d'éthyle 4 / 6 (v/v) pour obtenir après cristallisation dans le cyclohexane 0,418 g de produit attendu.
RMN ¹H δ en ppm (DMSO d6): 0,12(t, 3H); 2,94(m, 1H); 3,21 (m, 1H); 3,75(s, 3H); 3,88(s, 3H); 6,02(d, 1H); 6,51 (d, 1H); 6,80(d, 1H); 6,96-7,49(massif, 8H); 7,91 (d, 1H). F = 153,9 °C

Le tableau II illustre les structures chimiques et les propriétés physiques de quelques composés de l'invention obtenus selon cet exemple

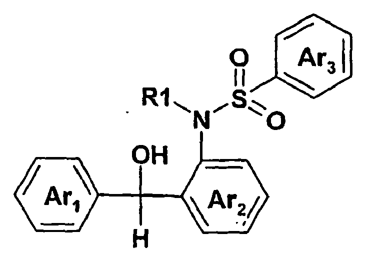

**Tableau II**

| **N° composé** | **Nature et position des substituants** | | | | | **MH⁺/ temps de rétention** |
|---|---|---|---|---|---|---|
| | **Ar₁** | **Ar₂** | **R₁** | **Ar₃** | **F(°C)** | |
| 21 | 2-Cl | 4-Cl | CH₂COCH₃ | 3,4-diOMe | 158,5 | 540/8,72 |
| 23 | 2-Cl | 4-Cl | (CH₂)₂OMe | 3,4-diOMe | 112,2 | 526/8,77 |
| 29 | 2-Cl | 4-Cl | CH₂CONH₂ | naphtyle | 218 | 515/8,52 |
| 30 | 2-Cl | 4-Cl | CH₂CONH₂ | 3-OMe | 114,8 | 495/7,87 |

### Exemple 3 : N²-{4-chloro-2-[(2-chlorophényl)(hydroxy)méthyl]phényl}-N²-[(2,4-diméthoxyphényl)sulfonyl]glycinamide (composé 33)

A 1,5g de N-{4-chloro-2-[(2-chlorophényl)(hydroxy)méthyl]phényl}-3,4-diméthoxybenzènesulfonamide, obtenu à l'exemple 1.2 en solution dans 15 ml de DMF on additionne à température ambiante 1,25g de carbonate de césium, 0,68g de 2-bromoacétamide et porte 2 heures à 100°C. Le milieu réactionnel est repris à l'acétate d'éthyle et lavé à l'eau. La phase organique est séchée sur sulfate de sodium anhydre et concentrée. Le résidu est chromatographié sur une colonne de gel de silice en éluant avec un gradient de solvant dichlorométhane / méthanol pour obtenir 0,601 g de produit attendu.
RMN 1H δ en ppm (DMSO d6): 3,85(s, 6H); 4,40(d, 1H); 4,66(d, 1H); 6,45-7,62(massif, 14H).
F=158 °C

Le tableau III illustre les structures chimiques et les propriétés physiques de quelques composés de l'invention obtenus selon cet exemple.

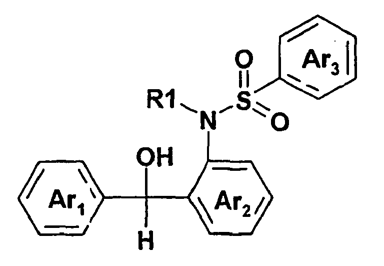

**Tableau III**

| **N° composé** | **Nature et position des substituants** | | | | **F(°C)** | **MH⁺/temps de rétention** |
|---|---|---|---|---|---|---|
| | **Ar₁** | **Ar₂** | **R₁** | **Ar₃** | | |
| 36 | 2-Cl | 4-Cl | CH₂CONH₂ | 2,5-diOMe | 109 | 525/7,57 |
| 38 | 2-Cl | 4-Cl | CH₂CONH₂ | 2,5-diMe, 4-Cl | 126,3 | 527/9,16 |

### Exemple 4 : N²-{4-chloro-2-[(2-chlorophényl)(hydroxy)méthyl]phényl}-N²-[(3,4-dichlorophényl)sulfonyl]glycinamide (composé 34)

### • Exemple 4.1 : (2-amino-5-chlorophényl)(2-chlorophényl)méthanol

A 20g (2-amino-5-chlorophényl)(2-chlorophényl)méthanone en solution dans 80ml d'éthanol on introduit par portions 8,6g de borohydrure de sodium et laisse 18 heures à température ambiante. Le milieu réactionnel est repris à l'acétate d'éthyle et lavé à l'eau. La phase organique est séchée sur sulfate de sodium anhydre et concentrée, pour obtenir 21,63g de produit attendu
RMN ¹H δ en ppm (DMSO d6): 5,16(s, 2H); 5,70(d, 1H); 5,98(d, 1H); 6,63(d, 1H); 6,85-7,29(massif, 6H).

### • Exemple 4.2 : N-{4-chloro-2-[(2-chlorophényl)(hydoxy)méthyl]phényl}-3,4-dichloro benzènesuifonamide

A 0,9g de (2-amino-5-chlorophényl)(2-chlorophényl)méthanol en solution dans 4ml de pyridine on additionne 0,822g de 3,4-dichlorobenzènesulfonylchlorure et laisse 30minutes à température ambiante. Le milieu réactionnel est repris à l'acétate d'éthyle et lavé à l'eau, puis avec une solution M d'acide chlorhydrique. La phase organique est séchée sur sulfate de sodium anhydre et concentrée. Le résidu est chromatographié sur une colonne de gel de silice en éluant au dichlorométhane pour obtenir 0,601 g de produit attendu
RMN ¹H δ en ppm (DMSO d 6): 6,07(s, 1H); 6,25(s, 1H); 7,06-7,69(massif, 11H).

### • Exemple 4.3 : N²-{4-chloro-2-[(2-chlorophényl)(hydroxy)méthyl]phényl}-N²-[(3,4-dichloro phényl)sulfonyl]glycinamide

A 1,315g de N-{4-chloro-2-[(2-chlorophényl)(hydroxy)méthyl]phényl}-3,4-diméthoxy benzènesulfonamide obtenu à l'exemple 1.2 en solution dans 10 ml de tétrahydrofurane on additionne à température ambiante 0,37g de tert-butylate de potassium, 0,46g de 2-bromoacétamide et porte 3 heures à reflux. Après 18 heures à température ambiante, le milieu réactionnel est repris à l'acétate d'éthyle et lavé à l'eau. La phase organique est séchée sur sulfate de sodium anhydre et concentrée. Le résidu est chromatographié sur une colonne de gel de silice en éluant avec un gradient de solvant dichlorométhane / méthanol pour obtenir 1,06g de produit attendu.
RMN ¹H δ en ppm (DMSO d6): 4,41(q, 2H); 6,42(d, 1H); 6,67(s, 2H); 7,02(m, 1H); 7,18-7,92(massif, 10H).
F=135 °C

Le tableau IV illustre les structures chimiques et les propriétés physiques de quelques composés de l'invention obtenus selon ce procédé

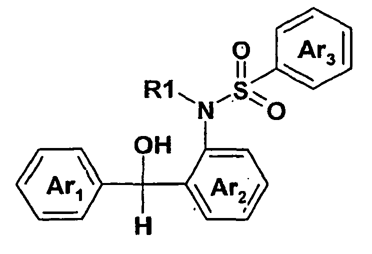

**Tableau IV**

| **N° composé** | **Nature et position des substituants** | | | | **F(°C)** | **MH*/ temps de rétention** |
|---|---|---|---|---|---|---|
| | **Ar₁** | **Ar₂** | **R₁** | **Ar₃** | | |
| 35 | 2-Cl | 4-Cl | CH₂CONH₂ | 4-Cl | 130 | 499/8,41 |
| 37 | 2-Cl | 4-Cl | CH₂CONH₂ | 2-Cl | 137 | 4997,75 |
| 41 | 2-Cl | 4-Cl | CH₂CONH₂ | 2,3-diOMe | 222 | 526/8,76 |
| 64 | 2-Cl | 4-Cl | CH₂CONH₂ | 3,4-éthylènedioxy | 102,1 | 523/7,64 |
| 79 | 2-Cl | 4-Cl | CH₂CONH₂ | 2-Me | 134 | 479/8,24 |
| 106 | 2-Cl | 4-Cl | CH₂CONH₂ | 2,4,5 -triMe | 150,2 | 507/8,95 |
| 110 | 2-Cl | 4-Cl | CH₂CONHMe | 2,4,5 -triMe | 171,7 | 521/9,25 |
| 113 | 2-Cl | 4-Cl | CH₂CONHMe | 2,4,5 -triOMe | 119,6 | 569/7,67 |
| 129 | 2-Cl | 4-Cl | CH₂CON(Me)₂ | 2,4-diCl-5-Me | 204,8 | 557/10,35 |
| 132 | 2-Cl | 4-Cl | CH₂CONHMe | 3,5-diMe | 109 | 489*/9,48 |
| 133 | 2-Cl | 4-Cl | CH₂CON(Me)₂ | 3,5-diMe | 142 | 520/9,85 |
| 52 | 2-Cl | 4-Cl | CH₂CONH₂ | H | 170,6 | 465/7,75 |
| 85 | 2-Cl | 4-Cl | CH₂CONH₂ | 2,6-diCl | 149 | 533/8,01 |
| 72 | 2-Cl | 4-Cl | CH₂CONHMe | 3.4-diOMe | 88 | 539/7,89 |
| 86 | 2-Cl | 4-Cl | CH₂CONH₂ | 3,5-diMe | 137 | 493/8,60 |
| 94 | 2-Cl | 4-Cl | CH₂CONH₂ | 2,5-diOMe,4-Me | 180,8 | 539/8,25 |
| 102 | 2-Cl | 4-Cl | CH₂CONH₂ | 2,4,5-triOMe | 123,5 | 55517,36 |
| 83 | 2-Cl | 4-Cl | (3-méthyl-1,2,4-oxadiazol-5-yl) méthyle | 3,4-diOMe | 90 | 564/9,15 |
| 22 | 2-Cl | 4-Cl | (1-méthyl-1H-imidazol-2-yl) méthyle | 3,4-diOMe | 199 | 562/5,30 |
| 148 | 2-Cl | 4-Cl | CH₂CONH₂ | 3-NH2 | 189,6 | 462*/7,86 |

| | | | | | | |
|---|---|---|---|---|---|---|
| *: l'ion observé est le M-H₂O + H⁺ | | | | | | |

Le composé 83 est obtenu par alkylation avec le dérivé 5-bromométhyl-3-méthyl-[1,2,4]oxadiazole, qui est synthétisé comme suit :
- Synthèse de (1,3-dioxo-1,3-dihydro-2H-isoindol-2-yl)acétylchloride

A 10,25g d'acide(1,3-dioxo-1,3-dihydro-2H-isoindol-2-yl)acétique en solution dans 50ml de dichlorométhane on additionne à température ambiante 4,6ml de chlorure d'oxalyle et 2 gouttes de DMF, on laisse 18 heures à température ambiante. On évapore à sec pour obtenir 11g de produit attendu.
• Synthèse de 2-(3-méthyl-[1,2,4]oxadiazol-5-yl)-isoindol-1,3-dione

A 4,4g de (1,3-dioxo-1,3-dihydro-2H-isoindol-2-yl)acétylchloride dans 20ml de pyridine on additionne 1,48g d'acétamidoxime et porte à reflux 1 heure. Le milieu réactionnel est concentré et repris par de l'acétate d'éthyle et de l'eau.La phase organique est séchée sur sulfate de sodium anhydre et concentrée. Le résidu est chromatographié sur une colonne de gel de silice en éluant avec un mélange toluéne / acétate d'éthyle 7/3 (v/v) pour obtenir 1,2g de produit attendu.
RMN¹H δ en ppm (DMSO d6): 2,26(s, 2H); 2,34(s, 3H); 3,92(s, 2H).
• Synthèse de 1-(3-méthyl-[1,2,4]oxadiazol-5-yl)-méthanamine

A 5g de 2-(3-méthyl-[1,2,4]oxadiazol-5-yl)-isoindol-1,3-dione en solution dans 100ml d'éthanol on additionne 2ml d'hydrate d'hydrazine et porte à reflux 2 heures. On filtre l'insoluble et concentre le filtrat. Le résidu est repris à l'éther diéthylique, l'insoluble est filté et le filtrat concentré pour obtenir 2g de produit attendu.
RMN¹H δ en ppm (DMSO d6): 2,39(s, 3H); 4,88(s, 2H).
• Synthèse de 5-bromométhyl-3-méthyl-[1,2,4]oxadiazole

A 2,26g de 1-(3-méthyl-[1,2,4]oxadiazol-5-yl)-méthanamine en solution dans 10 ml d'eau et 20 ml d'acide bromhydrique 6M on ajoute goutte àgoutte à 70° C, 2,76g de nitrite de sodium en solution dans 10ml d'eau. Après 1 heure à 80°C, on revient à température ambiante, reprend le milieu à l'acétate d'éthyle et lave à l'eau. La phase organique est séchée sur sulfate de sodium anhydre et concentrée, pour obtenir 2,6g de produit attendu.
RMN¹H δ en ppm (DMSO d6): 2,39(s, 3H); 4,88(s, 2H).

Le composé 22 est obtenu par alkylation avec le dérivé 2-chlorométhyl-1-méthylimidazole, qui est synthétisé comme suit :
- Synthèse de 1-méthyl-2-imidazolméthanol

A 10 g de 1-méthyl-2-imidazole carboxaldéhyde en solution dans 200 ml de méthanol on additionne 5,2g de borohydrure de sodium et laisse 48 heures à température ambiante. On évapore les solvants, reprend le résidu à l'acétate d'éthyle et lave à l'eau. La phase organique est séchée sur sulfate de sodium anhydre et concentrée. pour obtenir 6,5g de produit attendu.
RMN ¹H δ en ppm (DMSO d6): 3,67(s, 3H); 4,49(d, 2H); 5,36(t, 1H); 6,78(d, 1H); 7,08(d,1H).
F=108,2 °C
• Synthèse de 2-chlorométhyl-1-méthylimidazole

A 1,12g de 1-méthyl-2-imidazolméthanol on additionne goute à goutte 1,8ml de chlorure de thionyle à 0°C. Après 18 heures à 20°C, on chauffe 2 heures à 70°C. On concentre le milieu réactionnel pour obtenir quantitativement le produit attendu.
RMN ¹H δ en ppm (DMSO d6): 3,90(s, 3H); 5,22(s, 2H); 5,36(t, 1H); 7,77(d, 1H); 7,80(d, 1H).
Le composé 148 est obtenu à partir du dérivé nitro correspondant par une réaction de réduction par de l'hydrogène naissant.

### Exemple 5 : N-(4-chloro-2-[(2-chlorophényl)(hydroxy)méthyl]phényl)-3,4-diméthoxy-N-méthylbenzènesulfonamide (composé 25)

### • Exemple 5.1 : N-[4-chloro-2-(2-chlorobenzoyl)phényl]-3,4-diméthoxy-N-méthylbenzènesulfonamide

A 1g de N-[4-chloro-2-(2-chlorobenzoyl)phényl]-3,4-diméthoxybenzènesulfonamide obtenu à l'exemple 1.1 en solution dans 20 ml de diméthylformamide on additionne à 0°C 0,094g d'hydrure de sodium; après une heure à cette température, on introduit 0,16ml d'iodométhane et laisse à température ambiante 18 heures. On filtre le précipité, le reprend à l'acétate d'éthyle, séche sur sulfate de sodium anhydre et concentre. Le résidu est lavé à l'éther diéthylique et filtré pour donner après séchage 0,735 g de produit attendu.
RMN ¹H δ en ppm (DMSO d6): 2,75(s, 3H); 3,69(s, 3H); 3,86(s, 3H); 6,76-7,15(massif, 4H); 7,51-7,75(massif, 6H).
F=136,6 °C

### • Exemple 5.2 : N-{4-chloro-2-[(2-chlorophényl)(hydroxy)méthyl]phényl}-3,4-diméthoxy-N-méthylbenzènesuifonamide

A 0,152g du composé obtenu en 5.1 en solution dans 5ml d'éthanol on additionne 0,036g borohydrure de sodium et laisse 18 heures à température ambiante. Le milieu réactionnel est repris à l'acétate d'éthyle et lavé à l'eau. La phase organique est séchée sur sulfate de sodium anhydre et concentrée. Le résidu est repris par un minimum d'acétate d'éthyle, on filtre le précipité formé et le sèche pour obtenir 0,067g de produit attendu. RMN ¹H δ en ppm (DMSO d6) : 2,33(s, 3H); 3,75(s, 3H); 3,88(s, 3H); 6,06 (d, 1H);
6,45 (d, 1H); 6,68 (m, 1H); 6,96-7,81 (massif, 9H).
F = 173,2 °C

Le tableau V illustre les structures chimiques et les propriétés physiques de quelques composés de l'invention obtenus selon ce procédé.

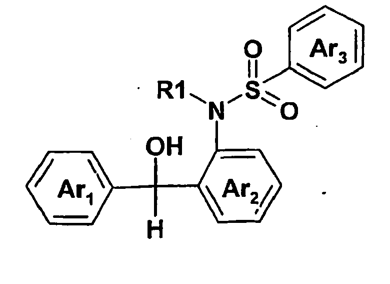

**Tableau V**

| **N° composé** | **Nature et position des substituants** | | | | | **MH⁺/temps de rétention** |
|---|---|---|---|---|---|---|
| | **Ar₁** | **Ar₂** | **R₁** | **Ar₃ F(°C)** | | |
| 66 | H | 4-Cl | CH₂CONH₂ | 3,4-diOMe | 201,6 | 491/7,39 |
| 137 | H | 4-Cl | CH₂CONHMe | 3,4-diOMe | 111,5 | 487*/8.58 |
| 57 | 2-F | 4-Cl | CH₂CONH₂ | 3,4-diOMe | 192,1 | 509/7,10 |

| | | | | | | |
|---|---|---|---|---|---|---|
| * : l'ion observé est le M-H₂O + H⁺ | | | | | | |

### Exemple 6 : Synthèse de N²-{4-chloro-2-[hydroxy(3-méthoxyphényl)méthyl]phényl}-N²-[(3,4-diméthoxyphényl)sulfonyl]glycinamide (composé 51)

### • Exemple 6.1 : (2-amino-5-chlorophényl)(3-méthoxyphényl)méthanone

A 5g de 2-amino-5-chlorobenzonitrile en solution dans 100 ml d'éther diéthylique, on additionne goutte à goutte à -5°C 100ml d'une solution molaire dans le tétrahydrofurane du magnésien du 3-bromobenzène et abandonne 18 Heures à température ambiante. Le milieu réactionnel est hydrolysé par de la glace additionnée d'acide chlorhydrique 6M. On extrait à l'acétate d'éthyle. La phase organique est séchée sur sulfate de sodium anhydre et concentrée. Le résidu est chromatographié sur une colonne de gel de silice en éluant avec un gradient cyclohexane 100 à cyclohexane/dichlorométhane 60/40 (v/v) pour obtenir 7,26g de produit attendu.
RMN¹H δ en ppm (DMSO d6) : 3,83(s, 3H); 6,92(d, 1H); 7,10-7,50(massif, 8H).

### • Exemple 6.2 : (2-amino-5-chlorophényl)(3-méthoxyphényl)méthanol

A 3,66g de (2-amino-5-chlorophényf)(3-méthoxyphényl)méthanone en solution dans 15ml d'éthanol, on additionne à 20°C 1,59g de borohydrure de sodium. Après 24 heures à 20°C, le milieu réactionnel est concentré, repris à l'acétate d'éthyle et lavé 2 fois à l'eau. La phase organique est séchée sur sulfate de sodium anhydre et concentrée. Le résidu est concrété au dichlorométhane pour obtenir 2,37g de produit attendu.
RMN ¹H δ en ppm (DMSO d6): 3,74(s, 3H); 5,14(s, 2H); 5,70(d, 1H); 5,94(d, 1H); 6,61 (d, 1H); 6,81-7,27(massif, 6H).

### • Exemple 6.3 : N-{4-chloro-2-[(3-méthoxyphényl)(hydroxy)méthyl]phényl}-3,4-diméthoxybenzènesulfonamide

A 1g de (2-amino-5-chlorophényl)(3-méthoxyphényl)méthanol en solution dans 6ml de pyridine à température ambiante on additionne 1,08g de 3,4-diméthoxybenzène sulfonylchlorure. Après 18 heures le milieu réactionnel est repris à l'acétate d'éthyle et lavé à l'eau. La phase organique est séchée sur sulfate de sodium anhydre et concentrée. Le résidu est chromatographié sur une colonne de gel de silice en éluant au dichlorométhane pour obtenir 1,65g de produit attendu.
RMN ¹H δ en ppm (DMSO d6): 3,74(s, 3H); 3,75(s, 3H); 3,85(s, 3H); 6,07(s, 1H); 6,25(s, 1H); 6,76-7,33(massif, 10H); 9,43(s, 1H).

### • Exemple 6.4 : N²-{4-chloro-2-[hydroxy(3-méthoxyphényl)méthyl]phényl}- N²-[(3,4-diméthoxyphényl)sulfonyl]glycinamide

A 0,7 g de N-{4-chloro-2-[(3-méthoxyphényl)(hydroxy)méthyl]phényl}-3,4-diméthoxybenzènesulfonamide en solution dans 5ml de THF on additionne à température ambiante 0,2g de tert-butylate de potassium, 0,25g de 2-bromoacétamide. Après 48 heures à 20°C, le milieu réactionnel est concentré, repris à l'acétate d'éthyle et lavé à l'eau. La phase organique est séchée sur sulfate de sodium anhydre et concentrée. Le résidu est chromatographié sur une colonne de gel de silice en éluant au dichlorométhane pour obtenir 0,4g de produit attendu.
RMN ¹H δ en ppm (DMSO d6): 3,76(s, 3H); 3,78(s, 3H); 3,90(s, 3H); 4,12-4,53(massif, 2H); 6,16(d, 1H); 6,47(d, 1H); 6,81-7,42(massif, 11H); 7,76(s, 1H).
F=100 °C

Le tableau VI illustre les structures chimiques et les propriétés physiques de quelques composés de invention obtenus selon ce procédé.

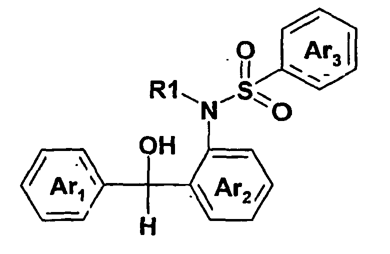

**Tableau VI**

| **N^{o} composé** | **Nature et position des substituants** | | | | **F(°C)** | **MH⁺/temps de rétention** |
|---|---|---|---|---|---|---|
| | **Ar₁** | **Ar₂** | **R₁** | **Ar₃** | | |
| 16 | 3-Cl | 4-Cl | CH₂CO₂Et | 3,4-diOMe | 149 | 554/9,48 |
| 17 | 2-OMe | 4-Cl | CH₂CO₂Et | 3,4-diOMe | 119,6 | 550/8,98 |
| 26 | 2-Me | 4-Cl | CH₂CO₂Et | 3,4-diOMe | 85,4 | 534/9,04 |
| 44 | 3-Cl | 4-Cl | CH₂CONH₂ | 3.4-diOMe | 120,1 | 525/8,95 |
| 59 | 4-OMe | 4-Cl | CH₂CONH₂ | 3,4-diOMe | 181 | 521/7,30 |
| 68 | 2-OMe | 4-Cl | CH₂CONH₂ | 3.4-diOMe | 104,6 | 521/7,26 |
| 131 | 2-Me | 4-Cl | CH₂CONH₂ | 3,4-diOMe | 144,6 | 487/8,42 |
| 76 | 3-Cl | 4-Cl | CH₂CONH₂ | 3,4-méthylènedioxy | 229 | 509/7,73 |

### Exemple 7 : N²-{4-chloro-2-{hydroxy[2-(trifluorométhyl)phényl]méthyl}phényl)-N²-[(3,4-diméthoxyphényl)sulfonyl]glycinamide (composé 125)

### • Exemple 7.1 : 6-chloro-2-méthyl-benzo[d][1,3]oxazin-4-one

A 15g d'acide 2-amino-5-chlorobenzoïque on additionne 80 ml d'anhydride acétique et porte 2 heures à reflux. On concentre le milieu réactionnel et recristallise le résidu dans de l'éthanol. Après filtration et rinçage du précipité on obtient 10,25g de produit attendu.
RMN¹H δ en ppm (DMSO d6): 2,40(s, 3H); 7,58(d,1H); 7,97(m, 2H).

### • Exemple 7.2 : (2-amino-5-chlorophényl)-(2-trifluorométhylphényl)méthanone

A 1,1g de magnésium dans 22ml de diéthyléther on additionne goutte à goutte 6,05 ml de 2-trifluorométhylbromobenzène. Au magnésien formé on additionne en 15 minutes 8g de 6-chloro-2-méthyl-benzo[d][1,3]oxazin-4-one en solution dans 60ml de dichlorométhane et laisse 18 heures à température ambiante. On hydrolyse avec une solution de chlorure d'ammonium saturée et extrait le milieu réactionnel à l'éther diéthylique. Après concentration de la phase organique on additionne au résidu 7 ml d'éthanol, 7ml d'une solution 3M d'hydroxyde de sodium et porte à reflux 1 heure 30,
A température ambiante le milieu est extrait à l'éther diéthylique, la phase éthérée est séchée sur sulfate de sodium anhydre et concentée. Le résidu est chromatographié pour obtenir 3,3g de produit attendu.
RMN ¹H δ en ppm (DMSO d6) : 6,98(d, 1H); 7,08-7,58(massif, 8H).

### • Exemple 7.3 : N-[4-chloro-2-(2-trifluorométhylbenzoyl)phényl]-3,4-diméthoxy benzènesulfonamide

A partir de 3,295g de (2-amino-5-chlorophényl)(2-trifluorométhyl-phényl)méthanone selon le procédé décrit à l'exemple 1.1 on obtient 1,22g de produit attendu.

### • Exemple 7.4 : N-{4-chloro-2-[(2-trifluorométhylphényl)(hydroxy)méthyl]phényl}-3,4-diméthoxybenzènesulfonamide

A partir de 3 g de N-[4-chloro-2-(2-trifluorométhylbenzoyl)phényl]-3,4-diméthoxybenzènesulfonamide selon le procédé décrit à l'exemple 1.2, on obtient 1,566g de produit attendu.
RMN ¹H δ en ppm (DMSO d6) : 3,76(s, 3H); 3,84(s, 3H); 6,27(s, 1H); 6,96-7,44(massif, 12H).

### • Exemple 7.5 : N²-{4-chloro-2-{hydroxy[2-(trifluorométhyl)phényl]méthyl}phényl)-N²-[(3,4-diméthoxyphényl)sulfonyl]glycinamide

A partir de 0,7g de N-{4-chloro-2-[(2-triffuorométhylphényl)(hydroxy)méthyl]phényl}-3,4-diméthoxybenzènesulfonamide selon le procédé décrit à l'exemple 1.3, on obtient 0,265g de produit attendu.
RMN ¹H δ en ppm (DMSO d6): 3,77(s, 3H); 3,94(s,3H); 4,14-4,41(massif,2H); 6,50(s,1H);6,80(d, 1H); 6,80-7,88(massif,12H).
F=128,8°C

### Exemple 8: N²-{2-[(2-chlorophényl)(hydroxy)méthyl]-4-fluorophényl}-N²-[(3,4-diméthoxyphényl)sulfonyl]glycinamide (composé 61)

### • Exemple 8.1 : (2-nitro-5-fluorophényl)-(2-chlorophényl)méthanone

A 2,6g de triclorure de chrome en suspension dans 50ml de THF, on additionne successivement 30ml d'une solution 0,5M dans le THF de 2-chlorophényliodozincique ; 2,54g de 2-nitro-5-fluorobenzaldéhyde, 5,7ml de chlorure de triméthylsilane. On porte à 65°C 1 heure puis 18 heures à température ambiante. Au milieu réactionnel on additionne une solution 1 M d'acide chlorhydrique, filtre le précipité formé et concentre le filtrat. Le résidu est repris à l'acétate d'éthyle et lavé à l'eau. La phase organique est séchée sur sulfate de sodium anhydre et concentrée. Le résidu est chromatographié sur une colonne de gel de silice en éluant au cyclohexane pour obtenir 1g de produit attendu.
RMN¹H δ en ppm (DMSO d6) : 6,95(d, 1H); 7,05-7,57(massif, 8H)

### • Exemple 8.2 : (2-amino-5-fluorophényl)-(2-chlorophényl)méthanol

A 0,5 g de (2-nitro-5-fluorophényl)-(2-chlorophényl)méthanone en solution dans 10 ml d'éthanol on additionne 0,44g d'étain, 1,5ml d'acide chlorhydrique 12M et laisse 3 heures à température ambiante. Le milieu réactionnel est concentré, le résidu est repris à l'acétate d'éthyle et lavé avec une solution 2M d'hydroxyde de sodium puis avec une solution saturée de chlorure d'ammonium. La phase organique est séchée sur sulfate de sodium anhydre et concentrée, pour obtenir 0,453g de produit attendu. RMN ¹H δ en ppm (DMSO d6) : 5,16(s, 2H); 5,77(d, 1H); 5,98(d, 1H); 6,83-7,34(massif, 7H).

### • Exemple 8.3 : N-{4-fluoro-2-[(2-chlorophényl)(hydroxy)méthyl]phényl}-3,4-diméthoxybenzènesulfonamide

A partir de 0,45 g de (2-amino-5-fluorophényl)-(2-chlorophényl)méthanol selon le procédé décrit à l'exemple 1.1 on obtient 0,56g de produit attendu.
RMN¹H δ en ppm (DMSO d6) : n.d.

### • Exemple 8.4 : N²-{2-[(2-chlorophényl)(hydroxy)méthyl]-4-fluorophényl}-N²-[(3,4-diméthoxyphényl)sulfonyl]glycinamide

A partir de 0,56g de N-{4-fluoro-2-[(2-chlorophényl)(hydroxy)méthyl]phényl}-3,4-diméthoxybenzènesulfonamide, selon le procédé décrit à l'exemple 4.3 on obtient 0,3g de produit attendu.
RMN ¹H δ en ppm (DMSO d6): 3,76(s,3H); 3,86(s,3H); 4,05-4,46(massif,2H); 6,31 (d,1H); 6,63(m,1H); 6,95-7,80(massif, 1 2H).
F= 232°C

Le tableau VII illustre les structures chimiques et les propriétés physiques de quelques composés de l'invention obtenus selon l'exemple 8 (l'activation de la réaction par addition de triméthylsilane et de trichlorure de chrome décrite dans l'exemple 8.1 n'a pas été utilisée pour les composés 69, 136, 175).

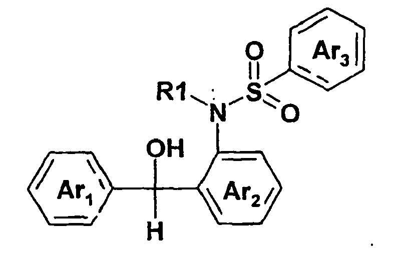

**Tableau VII**

| **N° composé** | **Nature et position des substituants** | | | | **F(°C)** | **MH⁺/temps de rétention** |
|---|---|---|---|---|---|---|
| | **Ar₁** | **Ar₂** | **R₁** | **Ar₃** | | |
| 69 | 2-Cl | 4-OMe | CH₂CONH₂ | 3,4-diOMe | 235,3 | 521/6,88 |
| 136 | 2-Cl | 4-Me | CH₂CONH₂ | 3,4-diOMe | 189,2 | 487/7,90 |
| 175 | 2-Cl | 5-Cl | CH₂CONH₂ | 3,4-diOMe | 197,1 | 507*/8,15 |

| | | | | | | |
|---|---|---|---|---|---|---|
| * : l'ion observé est le M-H₂O + H⁺ | | | | | | |

### Exemple 9 : N²-{4-chloro-2-[(2,6-dichlorophényl)(hydroxy)méthyl]phényl}-N²-[(3,4-diméthoxyphényl)sulfonyl]glycinamide (composé 117)

### • Exemple 9.1 : (2-nitro-5-chlorophényl)(2,6-dichlorophényl)méthanol

A 16,75g de 1,3-dichlorobenzène en solution dans 250ml de THF on additionne goutte à goutte à -70°C, 68ml de solution 1,6M dans l'hexane de n-butyllithium. Après une heure à -70°C, on introduit 10g de 5-chloro-2-nitrobenzaldéhyde en solution dans du THF et laisse 3 heures à cette température. On hydrolyse par addition de 5ml d'acide acétique et revient à température ambiante. On concentre et chromatographie le résidu sur une colonne de gel de silice en éluant avec un mélange cyclohexane/acétate d'éthyle 90/10 (v/v) pour obtenir 7,975g de produit attendu.
RMN ¹H δ en ppm (DMSO d6) : 6,82(s, 2H); 7,32-7,46(massif, 3H); 7,65(d, 1H); 7,90(m, 1H).

### • Exemple 9.2 : (2-amino-5-chlorophényl)(2,6-dichlorophényl)méthanol

A 3,9g de (2-nitro-5-chlorophényl)(2,6-dichlorophényl)méthanol en solution dans 37 ml de méthanol on additionne 5,25g de formiate d'ammonium, 0,374g d'oxyde de platine. On laisse 5 heures à température ambiante, puis chauffe 18 heures à 50°C. On filtre sur célite, concentre le filtrat. Le résidu est repris à l'acétate d'éthyle et lavé à l'eau. La phase organique est séchée sur sulfate de sodium anhydre et concentrée. Le résidu est chromatographié sur une colonne de gel de silice en éluant avec un mélange cyclohexane/acétate d'éthyle 90/10 (v/v) pour obtenir 1,378g de produit attendu.
RMN¹H δ en ppm (DMSO d6) : 5,16(s, 2H); 6,17(d, 1H); 6,35(d, 1H); 6,64(d, 1H); 6,72(s, 1H); 7,01 (m, 1H); 7,38-7,55(massif, 3H).

### • Exemple 9.3 : N-{4-chloro-2-[(2,6-dichlorophényl)(hydroxy)méthyl]phényl}-3,4-diméthoxybenzènesulfonamide

A partir de 1,9g de (2-amino-5-chlorophényl)(2,6-dichlorophényl)méthanol selon le procédé décrit à l'exemple 4.2 on obtient 1,47g de produit attendu.
RMN ¹H δ en ppm (DMSO d6) : 3,76(s, 3H); 3,84(s, 3H); 6,53(s, 1H); 6,83-7,48(massif, 10H); 9,29(s, 1H).

### • Exemple 9.4 : N²-{4-chloro-2-[(2,6-dichlorophényl)(hydroxy)méthyl]phényl}-N²-[(3,4-diméthoxyphényl)sulfonyl]glycinamide

A partir de 0,7g de N-(4-chloro-2-[(2,6-dichlorophényi)(hydroxy)méthyl]phényl)-3,4-diméthoxybenzènesulfonamide selon le procédé décrit à l'exemple 4.3 on obtient 0,23g de produit attendu.
RMN ¹H δ en ppm (DMSO d6): 3,76(s, 3H); 3,86(s, 3H); 4,27(q, 2H); 6,42(s, 1H); 6,91-7,89(massif, 9H); 8,02(s, 1H).
F=128,3 °C

Le tableau VIII illustré les structures chimiques et les propriétés physiques de quelques composés de l'invention obtenus selon l'exemple 9 (pour tous les composés, la réduction décrite dans l'exemple 9.2 est réalisée en utilisant de l'étain métallique et de l'acide chlorhydrique 12M à la place du formiate d'ammonium et de l'oxyde de platine)

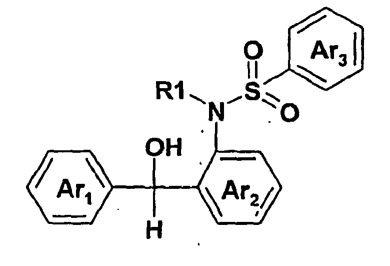

**Tableau VIII**

| **N° composé** | **Nature et position des substituants** | | | | **F(°C)** | **MH⁺/temps de rétention** |
|---|---|---|---|---|---|---|
| | **Ar₁** | **Ar₂** | **R₁** | **Ar₃** | | |
| 147 | 2,6-diF | 4-Cl | CH₂CONH₂ | 3,5-diMe | 108,7 | 477/8,59 |
| 198 | 2,6-diF | 4-Me | CH₂CONH₂ | 3,4-diOMe | 175,7 | 489*/7,40 |
| 206 | 2,6-diF | 4-F | CH₂CONH₂ | 3,4-diOMe | 178,8 | 511/6,65 |
| 209 | 2,6-diF- | 4-OMe | CH₂CONH₂ | 3.4-diOMe | 114,2 | 523/7,14 |
| 221 | 2,6-diF | 4.5-diCl | CH₂CONH₂ | 3,4-diOMe | 149,9 | 543/8,15 |
| 222 | 2-Cl,6-F | 4-Cl | CH₂CONH₂ | 3,4-diOMe | 147 | 565**/8,20 |
| 225# | 2,6-diF | 6-OMe | CH₂CONH₂ | 3,4-diOMe | 228,7 | 523*/6,81 |
| 226# | 2,6-diF | 6-OMe | CH₂CONH₂ | 3,4-diOMe | 210,1 | 523*/7,25 |
| 101 | 2.6-diF | 4-Cl | CH₂CONH₂ | 3,4-diOMe | 134,1 | 527/7,02 |

| | | | | | | |
|---|---|---|---|---|---|---|
| • * : l'ion observé est le M-H₂O + H⁺, •** : l'ion observé est le MNa⁺, • # : rotamères | | | | | | |

| | **Enantiomères du composé 101** | |
|---|---|---|
| | **Enantiomère lévogyre** | **Enantiomère dextrogyre** composé 251 |
| **[Pouvoir rotatoire [α]_{D} *** | -64,4 | +62,4 |

| | | |
|---|---|---|
| • C = 0,5g/100ml dans le méthanol, à 25°C, à 589nm. | | |

### Exemple 10 : N²-{4-chloro-2-[(2,5-dichlorophényl)(hydroxy)méthyl]phényl}-N²-[(3,4-diméthoxyphényl)sulfonyl]glycinamide (composé 74)

### • Exemple 10.1 : (2-amino-5-chlorophényl)(2,5-dichlorophényl)méthanone

A 28ml d'une solution 1 M de trichloroborane à -5°c on additionne 5,6g de 4-chloroaniline en solution dans 30ml de 1,2-dichloroéthane. Après 45 minutes à +15°c, on introduit 3,8g de 2,5-dichlorobenzonitrile et 5g de gallium trichloride et porte à reflux 3 heures. A température ambiante, on hydrolyse avec une solution 2M d'acide chlorhydrique et porte 2heures à 80°C. Le milieu réactionnel est repris à l'acétate d'éthyle et lavé à l'eau. La phase organique est séchée sur sulfate de sodium anhydre et concentrée. Le résidu est chromatographié sur une colonne de gel de silice en éluant avec un gradient cyclohexane/dichlorométhane pour obtenir 6,38g de produit attendu.
RMN¹H δ en ppm (DMSO d6) : 6,90-6,96(m, 2H); 7,37(d, 1H); 7,60-7,72(massif, 5H).

### • Exemple 10.2 : (2-amino-5-chlorophényl)(2,5-dichlorophényl)méthanol

A 6,38g de (2-amino-5-chlorophényi)(2,5-dichidrophényl)méthanone en solution dans 50ml d'éthanol on additionne 2,4g de borohydrure de sodium et laisse 18 heures à température ambiante. On concentre, reprend le résidu à l'acétate d'éthyle et lave à l'eau. La phase organique est séchée sur sulfate de sodium anhydre et concentrée pour obtenir 2,016g de produit attendu.
RMN¹H δ en ppm (DMSO d6) : 5,27(s, 2H); 5,89(d, 1H); 6,15(d, 1H); 6,51(d, 1H); 6,73(d, 1H); 7,03(d, 1H); 7,42-7,51 (m, 2H); 7,66(s, 1H).

### • Exemple 10.3 : N-{4-chloro-2-[(2,5-dichlorophényl)(hydroxy)méthyl]phényl}-3,4-diméthoxybenzènesulfonamide

A partir de 2g de (2-amino-5-chlorophényl)(2,5-dichlorophényl)méthanol selon le procédé décrit à l'exemple 4.2 on obtient 1,389g de produit attendu.
RMN¹H δ en ppm (DMSO d6) : 3,77(s, 3H); 3,84(s, 3H); 6,29(s, 2H); 6,91(d, 1H); 7,06-7,50(massif, 8H); 9,25(s, 1H).

### • Exemple 10.4 : N²-{4-chloro-2-[(2,5-dichlorophényl)(hydroxy)méthyl]phényl}-N²-[(3,4-diméthoxyphényl)sulfonyl]glycinamide

A partir de 2,42g de N-{4-chloro-2-[(2,5-dichlorophényl)(hydroxy)méthyl]phényl}-3,4-diméthoxybenzènesulfonamide, selon le procédé décrit à l'exemple 4.3 on obtient 1,5g de produit attendu.
RMN ¹H δ en ppm (DMSO d6): 3,77(s, 3H); 3,88(s, 3H); 4,30(q, 2H); 6,57-6,63(m, 2H); 6,94-7,78(massif, 11H).
F=132 °C

Le tableau IX illustre les structures chimiques et les propriétés physiques de quelques composés de l'invention obtenus selon l'exemple 10 (pour tout ces composés à l'exemple 10.1 on remplace le trichlorure de gallium par le trichlorure d'aluminium).

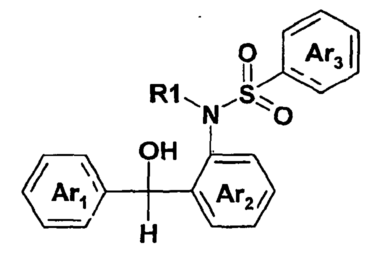

**Tableau IX**

| **N° composé** | **Nature et position des substituants** | | | | **F(°C)** | **MH⁺/temps de rétention** |
|---|---|---|---|---|---|---|
| | **Ar₁** | **Ar₂** | **R₁** | **Ar₃** | | |
| 77 | 2-Cl | 4-OCF₃ | CH₂CONH₂ | 3,4-diOMe | 116 | n.d. |
| 120 | 2-thiazolyl | 4-Cl | CH₂CONH₂ | 3,4-diOMe | 93 | 49716,42 |
| 121 | 2-thiazolyl | 4-Cl | CH₂CONHMe | 3,4-diOMe | 225 | 511/6,72 |
| 126 | 2,3-diF | 4-Cl | CH₂CONH₂ | 3,4-diOMe | 184,9 | 531**/8,16 |
| 127 | 2,3-diF | 4-Cl | CH₂CONHEt | 3,4-diOMe | 93,4 | 555/8,86 |
| 128 | 2,3-diF | 4-Cl | CH₂CON(Me)₂ | 3,4-diOMe | 100,5 | 537**/8,88 |
| 149 | 2-F, 6-OMe | 4-Cl | CH₂CONH₂ | 3,4-diOMe | 197,3 | 521*/7,66 |
| 146 | 2-thiényle | 4-Cl | CH₂CONHMe | 3,4-diOMe | 121,2 | 493**/8,20 |
| 155 | 2-thiényle | 4-Cl | CH₂CONH₂ | 3,4-diOMe | 119 | 479**17,87 |
| 162 | 2,4,6-triF | 4-Cl | CH₂CONH₂ | 3,4-diOMe | 118,8 | 527*/7,96 |
| 163 | 2,4-diF | 4-Cl | CH₂CONH₂ | 3,4-diOMe | 161,9 | 509*/8.28 |
| 164 | 2-Cl | 4,5-diCl | CH₂CONH₂ | 3,4-diOMe | 146,7 | 541*/8,79 |
| 167 | 2-F,4-Cl | 4-Cl | CH₂CONH₂ | 3,4-diOMe | 205,1 | 525*/8,79 |
| 176 | 2-F,5-Cl | 4-Cl | CH₂CONH₂ | 3,4-diOMe | 195,2 | 525*/8,71 |
| 178 | 3,5-diF | 4-Cl | CH₂CONH₂ | 3,4-diOMe | 205,7 | 509*/8,93 |
| 184 | 4-pyridyle | 4-Cl | CH₂CONH₂ | 3,4-diOMe | 122 | 492/5,14 |
| 191 | 2-Cl,6-Ome | 4-Cl | CH₂CONH₂ | 3,4-diOMe | 143,6 | 537*/8,10 |
| 194 | 2-F,5-Me | 4-Cl | CH₂CONH₂ | 3,4-diOMe | 120,5 | 505*/8,40 |

| | | | | | | |
|---|---|---|---|---|---|---|
| • * : l'ion observé est le M-H₂O + H⁺, • ** : l'ion observé est le MNa⁺ | | | | | | |

### Exemple 11 : N²-{2-[(2,6-difluorophényl)(hydroxy)méthyl]-5-méthoxy-4-méthylphényl}-N²-[(3,4-diméthoxyphényl)sulfonyl]glycinamide (composé 227)

### • Exemple 11.1: (2-amino-4-méthoxy-5-méthylphényl) (2,6-difluorophényl) méthanol

A 6,05g de dichlorophénylborane en solution dans 40ml de dichlorométhane à - 20°C, on additionne successivement 5,226g de 3-méthoxy-4-méthylaniline en solution dans 50ml de dichlorométhane ; 13,4ml de triéthylamine en solution dans 25ml de dichlorométhane. Après 30 minutes à -20°C on introduit 5,412g de 2,6-difluorobenzaldéhyde en solution dans 60ml de dichlorométhane. Après 24 heures à température ambiante on hydrolyse à l'eau. La phase organique est séchée sur sulfate de sodium anhydre et concentrée. A l'huile obtenue, en solution dans 80ml d'éther diéthylique, on additionne 80ml d'une solution 2M d'hydroxyde de sodium et abandonne sous agitation 18 heures. On décante, sèche la phase organique sur sulfate de sodium anhydre et concentré. Le résidu est concrété au n-pentane, pour obtenir 5,069g de produit attendu.
RMN¹H δ en ppm (DMSO d6): 1,98 (s, 3H); 3,69(s, 3H); 4,74(s, 2H); 5,79(d, 1H); 5,98 (d, 1H); 6,30(s, 1H); 6,75(s, 1H);7,06(m, 2H);7,41 (m, 1H).

### • Exemple 11.2: N-{2-[(2,6-difluorophényl)(hydroxy)méthyl]-5-méthoxy-4-méthylphényl}-3,4-diméthoxybenzènesulfonamide

A partir de 5,06g de (2-amino-4-méthoxy-5-méthylphényl)(2,6-difluorophényl) méthanol selon le procédé décrit à l'exemple 4.2 on obtient 8,5g de produit attendu. RMN ¹H δ en ppm (DMSO d6): 2,03(s, 3H); 3,38(s, 3H); 3,78(s, 3H); 3,84(s, 3H); 5,99(s, 1H); 6,17(s, 1H); 6,42(s, 1H); 6,98-7,40(massif, 7H); 8,98(s, 1H).

### • Exemple 11.3 : N²-{2-[(2,6-difluorophényl)(hydroxy)méthyl]-5-méthoxy-4-méthylphényl}-N²-[(3,4-diméthoxyphényl)sulfonyl]glycinamide

A partir de 4,5g de N-{2-[(2,6-difluorophényl)(hydroxy)méthyl]-5-méthoxy-4-méthylphényl}-3,4-diméthoxybenzènesulfonamide, selon le procédé décrit à l'exemple 4.3, on obtient 3,8g de produit attendu.
RMN ¹H δ en ppm (DMSO d6): 2,18(s, 3H); 3,43(s, 3H); 3,79(s, 3H); 3,87(s, 3H); 4,09-4,37(massif, 2H); 6,60(d, 1H); 6,77-7,75(massif, 11H).
F= 203,8 °C

Le tableau X illustre les structures chimiques et les propriétés physiques de quelques composés de l'invention obtenus selon l'exemple 11.

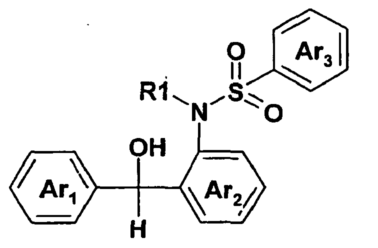

**Tableau X**

| **N^{o} composé** | **Nature et position des substituants** | | | | **F(°C)** | **MH⁺/temps de rétention** |
|---|---|---|---|---|---|---|
| | **Ar₁** | **Ar₂** | **R₁** | **Ar₃** | | |
| 213 | 2-Cl,6-Me | 4-Cl | CH₂CONH₂ | 3,4-diOMe | 130 | 521/8,67 |
| 214 # | 2,6-diF | 2,4-diCl | CH₂CONH₂ | 3,4-diOMe | 160,6 | 583**/7,80 |
| 216 # | 2,6-diF | 2,4-diCl | CH₂CONH₂ | 3,4-diOMe | 235,1 | 583**/8,24 |

| | | | | | | |
|---|---|---|---|---|---|---|
| ** : l'ion observé est le MNa⁺, #: rotamères | | | | | | |

Caractétistiques physiques des rotamères 214 et 216

| | | |
|---|---|---|
| 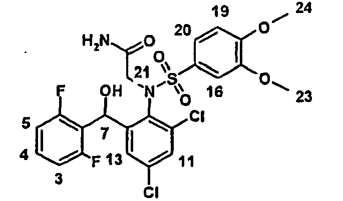 | | |
| Composé 214 | t_{R} = 7,80 min 543 uma (M-H₂O)H⁺ 583 uma (MNa+) | δ = 3,78, s, 3H, OCH₃23; δ = 3,85, s, 3H, OCH₃24; δ = 3,82, q, 2H, CH₂21; δ = 6,21, *d,* 1H, J_{H7OH} = 5.2; OH; δ =6,57, *d*, 1H, J _{H7OH} = 5,2, H7; δ = 6,62, s, 1H, NH₂; δ = 6,90, t, 2H, J_{H3H4} = J_{H5H4} = J_{h3F} = J_{H5F} = 8,4 Hz, H3 et H5; δ = 7,11, s, 1H, NH₂; δ = 7,13, *d,* 1H, J_{H19H2O} = 8,4, H19; δ = 7,29, *m*, 3H, H₄, H16 et H2O, δ = 7,51, s, 1H, JH_{11H13}= 2,8, H11; δ = 7,88, s, 1H, H13 |
| Composé 216 | t_{R} = 8,24 min 543 uma (M-H₂O)H⁺ 583 uma (MNa+) | δ = 3,76, s, 3H, OCH₃23; δ = 3,86, s, 3H, OCH₃24; δ = 4,04 et 4,53, *d*, 2H, CH₂21; δ = 7,04, *d,* 1H, J_{H7OH} = 5,6, H7; δ =7,07, s, 1H, H13; δ = 7,13, *m*, 4H, H3, H5, H16, H19; δ = 7,30, *dd,* 1H, J_{H2OH19} = 8,8, J_{H2OH16} = 1,6, H2O; δ = 7,47, *m*, 1H, H4; δ = 7,58, s, 1H, NH₂; δ = 7,63, *d,* 1H, J_{H11H13} = 2,4, H11; δ = 7,70, *d*, 1H, J_{OHH7} = 5,6, OH; δ = 8,05, s, 1H, NH₂. |
| Spectres RMN¹H réalisés dans le DMSO, à la fréquence de 400 Mz, les déplacements chimiques δ sont exprimés en ppm, les constantes de couplage J en Hertz. | | |
| Echantillon en solution 1 mg/ml de MeOH, 2µL injecté. Spectrométrie de masse : électrospray positif, balayage de 120 à 1500 uma, | | |

### Exemple 12 : Ethyl N-[4-chloro-2-(2-chlorobenzyl)phényl]-N-[(3-méthoxyphényl) sulfonyl]glycinate (composé 3)

### • Exemple 12.1 : [4-chloro-2-(2-chlorobenzyl)phényl]amine

A 10g de (2-amino-5-chlorophényl)(2-chlorophényl)méthanone en solution dans 100ml de dichlorométhane on additionne à température ambiante 18,7ml de triéthylsilane, 14,4ml d'éthérate de trifluoroborane et porte 18 heures à reflux, puis 72 heures à température ambiante. On hydrolyse avec une solution 2M d'hydroxyde de sodium, décante. La phase organique est séchée sur sulfate de sodium anhydre et concentrée. Le résidu est chromatographié sur une colonne de gel de silice en éluant avec un mélange de solvant cyclohexane/acétate d'éthyle pour obtenir 4,46g de produit attendu.
RMN¹H δ en ppm (DMSO d6) : 3,86(s, 2H); 5,17(s, 2H); 6,50(d, 1H); 6,70(d, 1H); 7,00-7,53(massif, 5H).

### δ Exemple 12.2 : N-[4-chloro-2-(2-chlorobenzyl)phényl]-3-méthoxybenzène sulfonamide

A partir de 0,71g de [4-chloro-2-(2-chlorobenzyl)phényl]amine en solution dans 5ml de THF on additionne 0,4ml de pyridine, 0,8g de 3-méthoxybenzènesulfonylchlorure et laisse 18 heures à température ambiante. Le milieu réactionnel est repris à l'acétate d'éthyle, lavé à l'eau. La phase organique est séchée sur sulfate de sodium anhydre et concentrée. Le résidu est chromatographié sur une colonne de gel de silice en éluant avec un mélange de solvant cyclohexane/acétate d'éthyle 90 / 10 (v/v) pour obtenir 0,666g de produit attendu.
RMN ¹H δ en ppm (DMSO d6) : 3,78(s, 3H); 3,97(s, 2H); 6,71(d, 1H); 6,96(m, 2H); 7,23-7,34(massif, 6H); 7,46-7,54(massif, 2H); 9,89(s, 1H).

### • Exemple 12.3 : EthylN-[4-chloro-2-(2-chlorobenzyl)phényl]-N-[(3-méthoxy phényl)sulfonyl]glycinate

A 0,525g de N-[4-chloro-2-(2-chlorobenzyl)phényl]-2-méthoxybenzènesulfonamide en solution dans 6ml de DMF on additionne à 0°C, 0,065g d'hydrure de sodium, après 40 minutes à cette température on introduit 0,14ml de 2-bromoacétate d'éthyle et laisse 18 heures à température ambiante. Le milieu réactionnel est repris à l'acétate d'éthyle, lavé à l'eau. La phase organique est séchée sur sulfate de sodium anhydre et concentrée. Le résidu est chromatographié sur une colonne de gel de silice en éluant avec un mélange de solvant cyclohexane/acétate d'éthyle 90/10 (v/v) pour obtenir 0,272g de produit attendu, sous forme d'huile.
RMN ¹H δ en ppm (DMSO d6) : 1,18(t, 3H); 3,82(s, 3H); 3,98-4,12(massif, 3H); 4,36(s, 1H); 4,40(q, 2H); 6,73(d, 1H); 7,10-7,55(massif, 10H).

Le tableau XI illustre les structures chimiques et les propriétés physiques de quelques composés de l'invention obtenus selon l'exemple 12.

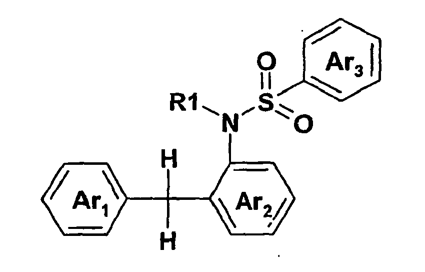

**Tableau XI**

| **N° composé** | **Nature et position des substituants** | | | | **F(°C)** | **MH⁺/temps de rétention** |
|---|---|---|---|---|---|---|
| | **Ar₁** | **Ar₂** | **R₁** | **Ar₃** | | |
| 2 | H | 4-Cl | CH₂CO₂Et | 3,4-diOMe | 139 | 504/9,06 |
| 4 | 2-Cl | 4-Cl | CH₂CO₂Et | 2-OMe | huile | 508/10,03 |
| 5 | 2-Cl | 4-Cl | CH₂CO₂Et | 2,5-diOMe | 80 | 538/9,91 |
| 6 | 2-Cl | 4-Cl | CH₂CO₂Et | 2,4-diOMe | 80 | 538/9,98 |

### Exemple 13 : N²-[4-chloro-2-(2-chlorobenzyl)phényl]-N²-[(3-méthoxyphényl)sulfonyl] glycinamide (composé 27)

A 1g de N-[4-chlpro-2-(2-chlorobenzyl)phényl]-3-méthoxybenzènesulfonamide obtenu à l'exemple 12.2, en solution dans 40ml d'acétonitrile, on additionne à température ambiante 1,34ml de triéthylamine puis 0,89g de 2-bromoacétamide et porte à reflux 18 heures. Le milieu réactionnel est repris à l'acétate d'éthyle, lavé à l'eau. La phase organique est séchée sur sulfate de sodium anhydre et concentrée. Le résidu est chromatographié sur une colonne de gel de silice en éluant avec un mélange de solvant cyclohexane/acétate d'éthyle 90 / 10 (v/v) pour obtenir 0,58g de produit attendu.
RMN ¹H δ en ppm (DMSO d6) : 3,82(s, 3H); 4,08-4,58(massif, 4H); 6,65(s, 1H); 6,91 (d, 1H); 7,08-7,57(massif, 11H).
F=65,7 °C

### Exemple 14: N-{4-chloro-2-[(2-chlorophényl)(hydroxy)méthyl]phényl}-3,4-diméthoxy-N-[(2-méthyl-2H-tétrazol-5-yl)méthyl]benzènesulfonamide (composé 14) et N-{4-chloro-2-[(2-chlorophényl)(hydroxy)méthyl]phényl}-3,4-diméthoxy-N-[(1-méthyl-1H-tétrazol-5-yl)méthyl]benzènesulfonamide (composé15)

### • Exemple 14.1 : N-[4-chloro-2-(2-chlorobenzoyl)phényl]-N-(cyanométhyl)3,4-diméthoxybenzènesulfonamide

A 5,4 g de N-[4-chloro-2-(2-chlorobenzoyl)phényl]-3,4-diméthoxybenzènesulfonamide obtenu à l'exemple 1.1 en solution dans 60ml de DMF, on additionne à 0°C, 0,57g d'hydrure de sodium; après 40 minutes à cette température, on introduit 0,9ml de 2-bromoacétonitrile et laisse 18 heures à température ambiante. Le milieu réactionnel est repris à l'acétate d'éthyle, lavé à l'eau. La phase organique est séchée sur sulfate de sodium anhydre et concentrée, pour obtenir 5,3g de produit attendu.
RMN ¹H δ en ppm (DMSO d6) : 3,76(s, 3H); 3,85(s, 3H); 4,71(m, 2H); 7,08-7,63 (massif, 9H); 7,81 (m, 1H).
F=164 °C

### • Exemple 14.2: N-[4-chloro-2-(2-chlorobenzoyl)phényl]-3,4-diméthoxy-N-(1H-tétrazol-5-ylméthyl)benzènesulfonamide

A 1g de N-[4-chloro-2-(2-chlorobenzoyl)phényl]-N-(cyanométhyl)-3,4-diméthoxy benzènesulfonamide en solution dans 20ml de THF on additionne successivement, 0,3g de d'oxyde de dibutylétain, 2,6ml d'azidotriméthylsilane et porte à reflux 18 heures. Le milieu réactionnel est repris à l'acétate d'éthyle, lavé à l'eau. La phase organique est séchée sur sulfate de sodium anhydre et concentrée. On purifie par filtration sur silice H, en éluant au dichlorométhane pour obtenir 0,95g de produit attendu.
RMN ¹H δ en ppm (DMSO d6) : 3,87(s, 3H); 5,00(s, 2H); 6,88-7,76(massif, 10H).

### • Exemple 14.3: N-{4-chloro-2-[(2-chlorophényl)(hydroxy)méthyl]phényl}-3,4-diméthoxy -N-(1H-tétrazol-5-ylméthyl)benzènesulfonamide

A 0,95g de N-[4-chloro-2-(2-chlorobenzoyl)phényl]-3,4-diméthoxy-N-(1H-tétrazol-5-ylméthyl)benzènesulfonamide en solution dans 40ml d'éthanol on additionne 0,38g de borohydrure de sodium et porte 18 heures à reflux. On évapore les solvants, reprend le résidu à l'acétate d'éthyle et le lave à l'eau. La phase organique est séchée sur sulfate de sodium anhydre et concentrée, pour obtenir 0,93g de produit attendu.

### • Exemple 14.4: N-{4-chloro-2-[(2-chlorophényl)(hydroxy)méthyl]phényl}-3,4-diméthoxy-N-[(2-méthyl-2H-tétrazol-5-yl)méthyl]benzènesulfonamide (composé 14) et N-{4-chloro-2-[(2-chlorophényl)(hydroxy)méthylphényl}-3,4-diméthoxy-N-[(1-méthyl-1H-tétrazol-5-yl)méthyl]benzènesulfonamide (composé15)

A 0,93g de N-{4-chloro-2-[(2-chlorophényl)(hydroxy)méthyl]phényl}-3,4-diméthoxy-N-(1H-tétrazol-5-ylméthyl)benzènesulfonamide en solution dans 25ml de DMF , on additionne successivement 0,2ml de iodométhane, 0,32g de carbonate de potassium et laisse 18 heures à température ambiante. Le milieu réactionnel est repris à l'acétate d'éthyle, lavé à l'eau. La phase organique est séchée sur sulfate de sodium anhydre et concentrée. Le résidu est chromatographié sur une colonne de gel de silice en éluant avec un mélange de solvant dichlorométhane / méthanol 99 / 1 (v/v) pour obtenir 0,34 g de composé 14.
RMN ¹H δ en ppm (DMSO d6): 3,77(s, 3H); 3,87(s, 3H); 4,15(s, 3H); 4,70(d, 1H); 5,15(d, 1H); 6,01(d, 1H); 6,60-7,83(massif, 11H).
F=94,8 °C

On obtient 0,19 g de composé 15 dont les caractériqtiques sont les suivantes : RMN ¹H δ en ppm (DMSO d6): 3,60(s, 3H); 3,79(s, 3H); 3,88(s, 3H); 4,25(d, 1H); 4,77(d, 1H); 5,96(d, 1H); 6,36(d, 1H); 6,65-7,79(massif, 10H).
F=118,3 °C

Les composés suivants ont été synthétisés selon ce procédé :
- Composé 11 : N-{4-chloro-2-[(2-chlorophényl)(hydroxy)méthyl]phényl}-N-[(2-éthyl-2H-tétrazol-5-yl)méthyl]-3,4-diméthoxybenzènesulfonamide. F= 113°C
- Composé 12 : N-{4-chloro-2-[(2-chlorophényl)(hydroxy)méthyl]phényl}-N-[(1-éthyl-1H-tétrazol-5-yl)méthyl]-3,4-diméthoxybenzènesulfonamide. F= 75°C

### Exemple 15 : N²-[4-chloro-2-(2-chlorobenzyl)phényl]-N²-[(4-méthoxyphényl)sulfonyl] glycinamide (composé 39)

A 0,766g de N-[4-chloro-2-(2-chlorobenzyl)phényl]-4-méthoxybenzènesulfonamide obtenu selon l'exemple 12,2 en solution dans 5ml de THF on additionne successivement 0,214g de *tert*-butylate de potassium, 0,263g de 2-bromoacétamide et laisse 18 heures à température ambiante. Le milieu réactionnel est repris à l'acétate d'éthyle, lavé à l'eau. La phase organique est séchée sur sulfate de sodium anhydre et concentrée. Le résidu est chromatographié sur une colonne de gel de silice en éluant avec gradient dichlorométhane / méthanol pour obtenir 0,50g de produit attendu.
RMN ¹H δ en ppm (DMSO d6) : 3,89(s, 3H); 4,02-4,29(m, 3H); 4,58(d, 1H); 6,66(d, 1H); 6,89(d, 1H); 7,14-7,65(massif, 11H).
F=150 °C

Le tableau XII illustre les structures chimiques et les propriétés physiques de quelques composés de l'invention obtenus selon une adaptation de l'exemple 15.

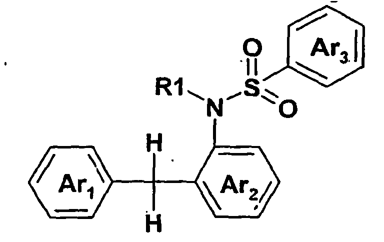

**Tableau XII**

| **N° composé** | **Nature et position des substituants** | | | | **F(°C)** | **MH⁺/temps de rétention** |
|---|---|---|---|---|---|---|
| | **Ar₁** | **Ar₂** | **R₁** | **Ar₃** | | |
| 40 | 2-Cl | 4-Cl | CH₂CONH₂ | 2,5-diOMe | 125 | 509/8,93 |
| 42 | 2-Cl | 4-Cl | CH₂CONH₂ | 3-CF3 | 119 | 517/9,25 |
| 43 | 2-Cl | 4-Cl | CH₂CONH₂ | 3-OCF3 | 129 | 533/9,38 |
| 45 | 2-Cl | 4-Cl | CH₂CONH₂ | 2-OMe | 114 | 479/8,18 |
| 46 | 2-Cl | 4-Cl | CH₂CONHMe | 3,4-diOMe | 146 | 523/8,42 |
| 47 | 2-Cl | 4-Cl | CH₂CONH₂ | 4-Cl | 205 | 483/9,06 |
| 48 | 2-Cl | 4-Cl | CH₂CONH₂ | 3,4-diCl | 166 | 517/9,64 |
| 49 | 2-Cl | 4-Cl | CH₂CONH₂ | 2,5-diMe ,4-Cl | 214,5 | 511/9,76 |
| 50 | 2-Cl | 4-Cl | CH₂CONH₂ | 2-Cl | 97 | 483/8,44 |
| 53 | 2-Cl | 4-Cl | CH₂CONH₂ | H | 150 | 449/8,40 |
| 55 | 2-F | 4-Cl | CH₂CONH₂ | 3,4-diOMe | 183,3 | 49317,80 |
| 62 | 2-Cl | 4-Cl | CH₂CONH₂ | 3-Me | 95 | 463/8,66 |
| 63 | 2-Cl | 4-Cl | CH₂CONH₂ | 3,4-éthylènedioxy | 111,6 | 507/8,19 |
| 71 | 2-Cl | 4-Cl | CH₂CONH₂ | 3,4-méthylènedioxy | 89 | 493/8,29 |
| 75 | 2-Cl | 4-Cl | CH₂CONHEt | 3,4-diOMe | 101 | 537/8,79 |
| 81 | 2-Cl | 4-Cl | CH₂CONH₂ | 2-Me | 160 | 463/9,32 |
| 82 | 2-Cl | 4-Cl | CH₂CONH₂ | 3,4-diMe | 166 | 477/9,64 |
| 89 | 2-Cl | 4-Cl | CH₂CONH₂ | 3,5-diMe | 84 | 477/9,24 |
| 105 | 2-Cl | 4-Cl | CH₂CONHMe | 2,5-diMe ,4-Cl | 172,6 | 525/10,24 |
| 109 | 2-Cl | 4-Cl | CH₂CONHMe | 2,4,5-triOMe | 160,4 | 553/8,17 |
| 104 | 2-Cl | 4-Cl | CH₂CONH₂ | 2,4,5-triOMe | 221,2 | 539/7,80 |
| 119 | 2-Cl | 4-Cl | CH₂CONH₂ | 3,5-diOMe | 110 | 509/8,71 |
| 118 | 2-Cl | 4-Cl | CH₂CON(Me)₂ | 2,5-diMe ,4-Cl | 166,4 | 539/10,71 |
| 134 | 2-Cl | 4-Cl | CH₂CONH₂ | 3-NO₂ | 91 | 494/8,72 |
| 173 | 2-Cl | 4-Cl | CH₂CONHMe | 2,4-diCl, 5-Me | 108 | 545/10,74 |
| 174 | 2-Cl | 4-Cl | CH₂CON(Me)₂ | 2,4-diCl, 5-Me | 204 | 559/11,27 |
| 54 | 2-Cl | 4-Cl | Me | 3,4-diOMe | 106,6 | 466/9,73 |
| 65 | H | 4-Cl | CH₂CONH₂ | 3,4-diOMe | 184 | 475/7,75 |
| 70 | 2-Cl | 4-Cl | CH₂CON(Me)₂ | 3,4-diOMe | 157,6 | 537/8,88 |
| 87 | 2-Cl | 4-Cl | CH₂CONH₂ | 2,6-diCl | 163 | 517/8,70 |
| 95 | H | 4-Cl | CH₂CON(Me)₂ | 3,4-diOMe | 144,2 | 503/8,67 |
| 96 | H | 4-Cl | CH₂CONHMe | 3,4-diOMe | 89,6 | 489/8,25 |
| 97 | 2-F | 4-Cl | CH₂CON(Me)₂ | 3,4-diOMe | huile | 521/8,63 |
| 98 | 2-F | 4-Cl | CH₂CONHMe | 3,4-diOMe | 107,5 | 507/8,23 |
| 99 | 2-Cl | 4-Cl | CH₂CONH₂ | 2,5-diOMe,4-Me | 172,5 | 523/8,70 |
| 114 | 2-Cl | 4-Cl | CH₂CONH₂ | 2,4,5-triMe | 184,9 | 491/9,48 |
| 115 | 2-Cl | 4-Cl | CH₂CONHMe | 2,4,5-triMe | 188,5 | 505/9,82 |
| 84 | 2-Cl | 4-Cl | (3-méthyl-1,2,4-oxadiazol-5-yl) méthyle | 3,4-diOMe | 111,4 | 548/10,20 |
| 107 | 2-Cl | 4-Cl | (1-méthyl-1H-imidazol-2-yl)méthyle | 3,4-diOMe | 105 | 546/6,32 |
| 112 | 2-Cl | 4-Cl | (1-méthyl-1H-imidazol-2-yl)méthyle | 3,5-diMe | 112 | 514/6,98 |
| 135 | 2-Cl | 4-Cl | CH₂CONH₂ | 3-NH₂ | 103 | 464/8,44 |
| 188 | 2,6-diF | 4-Cl | CH₂CONH₂ | 3-NMe₂ | 190 | 494/9,17 |
| 189 | 2,6-diF | 4-Cl | CH₂CONH₂ | 3-NHMe | 195 | 480/8,65 |
| 249 | 2,6-diF | 4-Cl | CH₂CONH₂ | 2,5-diMe,4-Cl | 196,8 | 513/9,50 |
| 263 | 2,6-diF | 4-Cl | CH₂CONH₂ | 4-tBut | 161,4 | 507/10,74 |
| 279 | 2,6-diF | 4-Cl | CH₂CONH₂ | 4-NH₂ | 111 | n.d. |
| 307 | 2,6-diF | 4-Cl | CH₂CONH₂ | 3,5-diOMe | 170,2 | 511/9,48 |
| 319 | 2,6-diF | 6-OMe | CH₂CONH₂ | 4-tBut | 173 | 503/10.1 |
| 320 | 2,6-diF | 6-OMe | CH₂CONH₂ | 3,4-diF | 259 | 483/9,13 |

Le composé 84 est obtenu par alkylation avec le dérivé 5-bromométhyl-3-méthyl-[1,2,4]oxadiazole (synthèse décrite d'après l'exemple 4.3).

Le composé 135 est obtenu à partir de 1,25g de composé 134 par réduction de la fonction nitro comme décrit à l'exemple 8.2.

Les composés 188 et 189 sont obtenus par réaction d'alkylation par l'iodométhane en présence de carbonate de césium dans le THF du *N²*-[3-aminophényl)sulfonyl]-*N²*-[4-chloro-2-(2,6-difluorobenzyl)phényl]glycinamide.

Pour les composés 54, 65, 95, 96, 97, 98 le tert-butylate de potassium est remplacé par l'hydrure de sodium.

Le composé 312 est obtenu selon ce procédé en partant du 3-méthoxybiphényl-2-amine
MH⁺=457; le temps de rétention est de 8,04 minutes
F= 86,8°C

### Exemple 16 : N²-[4-chloro-2-(2-méthoxybenzyl)phényl]-N²-[(3,4-diméthoxyphényl) sulfonyl] glycinamide (composé 56)

### • Exemple 16.1 : (2-amino-5-chlorophényl)(2-méthoxyphényl)méthane

A 1,438g de (2-amino-5-chlorophényl)-(2-méthoxyphényl)méthanone obtenu selon le procédé décrit à l'exemple 10,1 en solution dans 16ml de dichlorométhane on additionne successivement 2,61 ml de triéthylsilane, 4ml d'étherate de trifluorure de bore et porte à reflux 18 heures. Le milieu réactionnel refroidi est versé sur de la glace additionée d'une solution 2M d'hydroxyde de sodium, après décantation, la phase organique est séchée sur sulfate de sodium anhydre et concentrée. Le résidu est chromatographié sur une colonne de gel de silice pour obtenir 0,571g de produit attendu.
RMN ¹H δ en ppm (DMSO d6) : 3,70(s, 2H); 3,80(s, 3H); 5,05(s, 2H); 6,65(m, 2H); 6,88-7,29(massif, 5H).

### • Exemple 16.2 : N-[4-chloro-2-(2-méthoxybenzyl)phényl]-3,4-diméthoxybenzène sulfonamide

A 0,57g de (2-amino-5-chlorophényl)(2-méthoxyphényl)méthane en solution dans 5ml de THF on additionne successivement 0,2ml de pyridine, 0,547g de 3,4-diméthoxybenzènesulfonylchlorure et laisse 18 heures à température ambiante. Le milieu réactionnel est repris à l'acétate d'éthyle, lavé à l'eau. La phase organique est séchée sur sulfate de sodium anhydre et concentrée. Le résidu est chromatographié sur une colonne de gel de silice en éluant au dichlorométhane pour obtenir 0,832g de produit attendu.
RMN ¹H δ en ppm (DMSO d6)) : 3,70(s, 2H); 3,76(s, 3H); 3,85(s, 3H); 6,77-7,28(massif, 10H) 9,52(s, 1H).

### • Exemple 16.3 : N²-[4-chloro-2-(2-méthoxybenzyl)phényl]-N²-[(3,4-diméthoxy phényl)sulfonyl]glycinamide

A 0,83g de N-[4-chloro-2-(2-méthoxybenzyl)phényl]-3,4-méthoxybenzène sulfonamide en solution dans 10ml de DMF on additionne à 0°C 0,081g d'hydrure de sodium. Après 40 minutes à cette température on introduit 0,28g de 2-bromo acétamide et laisse 18 heures à température ambiante. Le milieu réactionnel est repris à l'acétate d'éthyle, lavé à l'eau. La phase organique est séchée sur sulfate de sodium anhydre et concentrée. Le résidu est chromatographié sur une colonne de gel de silice en éluant au dichlorométhane pour obtenir 0,77g de produit attendu.
RMN ¹H δ en ppm (DMSO d6) : 3,80(s, 6H); 3,88(s, 3H); 3,91(d, 1H);4,18(s, 2H); 4,30(d, 1H); 6,75(d, 1H); 6,91-7,28(s, 11H).

Le tableau XIII illustre les structures chimiques et les propriétés physiques de quelques composés de l'invention obtenus selon l'exemple 16.

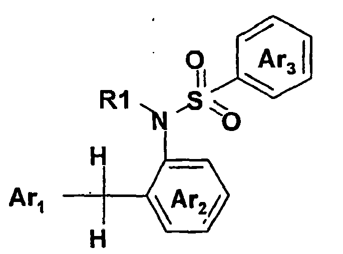

**Tableau XIII**

| **N° composé** | **Nature et position des substituants** | | | | **F(°C)** | **MH⁺/temps de rétention** |
|---|---|---|---|---|---|---|
| | **Ar₁** | **Ar₂** | **R₁** | **Ar₃** | | |
| 58 | 3-OMe phényle | 4-Cl | CH₂CONH₂ | 3,4-diOMe | 109 | 50517,79 |
| 60 | 4-OMe phényle | 4-Cl | CH₂CONH₂ | 3,4-diOMe | 214 | 50517,73 |
| 73 | 2-Me phényle | 4-Cl | CH₂CONH₂ | 3,4-diOMe | 88 | 489/8,12 |
| 116 | 3-Cl phényle | 4-Cl | CH₂CONH₂ | 3,4-diOMe | 181,8 | 509/8,39 |
| 212 | 2-Me thiényle | 4-Cl | CH₂CONH₂ | 3,4-diOMe | 100,1 | 495/8,86 |

### Exemple 17 : N²-[4-chloro-2-(2,6-dichlorobenzyl)phényl]-N²-[(3,4-diméthoxyphényl) sulfonyl] glycinamide (composé 78)

### • Exemple 17.1 : (2-amino-5-chlorophényl)(2,6-dichlorophényl)méthane

A 4,85g de (2-nitro-5-chlorophényl)(2,6-dichlorophényl)méthanol obtenu à l'exemple 9,1 en solution dans 58ml d'éthanol on additionne 5,042g d'étain, 19ml d'acide chlorhydrique 12M et porte à reflux la nuit. On concentre le milieu réactionnel, le reprend par de l'acétate d'éthyle et lave à l'eau additionée d'une solution 2M d'hydroxyde de sodium. La phase organique est séchée sur sulfate de sodium anhydre et concentrée, pour obtenir 3,71g de produit attendu.
RMN ¹H δ en ppm (DMSO d6) : 3,99(s, 2H); 6,00(d, 1H); 6,73(d, 1H); 6,98(d, 1H) ; 7,39-7,61(massif, 3H).

### • Exemple 17.2 : N-[4-chloro-2-(2,6-dichlorobenzyl)phényl]-3,4-méthoxy benzènesulfonamide

A partir de 2g de (2-amino-5-chlorophényl)(2,6-dichlorophényl)méthane selon le procédé décrit à l'exemple 12,2 on obtient 0,389g de produit attendu.

### • Exemple 17.3 : N²-[4-chloro-2-(2,6-dichlorobenzyl)phényl]-N²-[(3,4-diméthoxy phényl)sulfonyl]glycinamide

A 0,38g de N-[4-chloro-2-(2,6-dichlorobenzyl)phényl]-3,4-méthoxybenzène sulfonamide en solution dans 4ml de DMF on additionne à 0°C 0,035g d'hydrure de sodium; après 40 minutes à cette température on introduit 0,121g de 2-bromoacétamide et laisse 18 heures à température ambiante. Le milieu réactionnel est repris à l'acétate d'éthyle, lavé à l'eau. La phase organique est séchée sur sulfate de sodium anhydre et concentrée. Le résidu est chromatographié sur une colonne de gel de silice en éluant avec un mélange dichlorométhane / méthanol 98/2 (v/v) pour obtenir 0,225g de produit attendu.
RMN ¹H δ en ppm (DMSO d6) : 3,80(s, 3H); 3,88(s, 3H); 4,10-4,70(massif, 4H); 6,30(d, 1H); 7,03-7,61 (massif, 10H).
F=195,3 °C

Le tableau XIV illustre les structures chimiques et les propriétés physiques de quelques composés de l'invention obtenus selon l'exemple 17

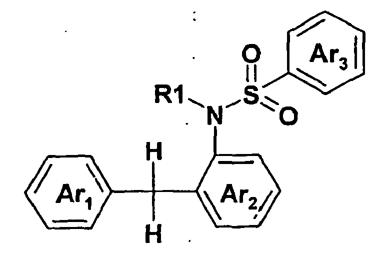

**Tableau XIV**

| **N° composé** | **Nature et position des substituants** | | | | **F(°C)** | **MH⁺/temps de rétention** |
|---|---|---|---|---|---|---|
| | **Ar₁** | **Ar₂** | **R₁** | **Ar₃** | | |
| 130 | 2,6-diCl | 4-Cl | CH₂CONHMe | 3,4-diOMe | 149,3 | 557/9,13 |
| 166 | 2,6-diCl | 4-Cl | CH₂CON(Me)₂ | 3,4-diOMe | 102,7 | 571/9,63 |
| 67 | 2-Cl | H | CH₂CONH₂ | 3,4-diOMe | 240,2 | 475/7.45 |
| 91 | 2,6-diF | 4-Cl | CH₂CONH₂ | 3,4-diOMe | 231,6 | 511/8,48 |
| 242 | 2,6-diF | 4-Br | CH₂CONH₂ | 3,4-diOMe | 209,7 | 55518,31 |
| 280 | 2,6-diF | 4-Me | CH₂CONH₂ | 3,4-diOMe | 180,4 | 490/88,80 |
| 286 | 2,6-diF | 4-Cl | CH₂CONH₂ | 3,4-diOMe | 165,3 | 511/8,89 |
| 297 | 2,6-diF | 6-OMe | CH₃ | 3,4-diOMe | 116 | 46419,92 |
| 308 | 2,6-diF | 4-Cl | CH₂CONH₂ | 3,4-diOMe | 169 | 495/9,84 |
| 314 | 2,6-diF | 4-Cl | CH₂CONH₂ | 2-F;4,5-diOMe | 212.3 | 525/8,96 |

### Exemple 18 : N²-[4-chloro-2-(2,6-difluorobenzyl)phényl]-N²-[(3,4-diméthoxyphényl) sulfonyl]-N¹-méthylglycinamide (composé 139)

### • Exemple 18:1 : (2-amino-5-chlorophényl)(2,6-fluorophényl)méthane

A 1g de (2-amino-5-chlorophényf)(2,6-fluorophényl)méthanol obtenu selon l'exemple 9,2 en solution dans 10ml de dichlorométhane, on additionne 1,8ml de triéthylsilane, 0,86ml d'acide trifluoroacétique et porte 6 heures à 50°C. Le milieu réactionnel est additionée sur de la glace, repris par du dichlorométhane et 100ml d'une solution 2M d'hydroxyde de sodium. La phase organique est séchée sur sulfate de sodium anhydre et concentrée. Le résidu est chromatographié sur une colonne de gel de silice en éluant avec un mélange dichlorométhane / cyclohexane, pour obtenir 0,997g de produit attendu.
RMN ¹H δ en ppm (DMSO d6): 3,77(s, 2H); 5,26(s, 2H); 6,36(s, 1H); 6,70(d, 1H); 6,99(m, 1H) ; 7,18(t, 2H); 7,48(m, 1H).

### • Exemple 18.2 : N-[4-chloro-2-(2,6-difluorobenzyl)phényl]-3,4-diméthoxy benzènesulfonamide

A partir de 0,97g de (2-amino-5-chlorophényl)(2,6-ftuorophényl)méthane selon une adaptation du procédé décrit a l'exemple 12.2 on obtient 1,58g de produit attendu. RMN ¹H δ en ppm (DMSO d6): 3,76(s, 3H); 3,85(s, 3H); 3,96(s, 2H); 6,64(s, 1H); 6,90(d, 1H); 7,10-7,50(massif, 7H); 9,69(s, 1H).
F=144 °C

• Exemple 18.3 : *N²*-[4-chloro-2-(2,6-difluorobenzyl)phényl]-*N²*-[(3,4-diméthoxy phényl)sulfonyl]-*N¹*-méthylglycinamide

A partir de 0,7g de N-[4-chloro-2-(2,6-difluorobenzyl)phényl]-3,4-diméthoxybenzène sulfonamide selon une adaptation du procédé 15 (présence de co solvant telque le DMF et d'iodure de sodium), on obtient 0,669g de produit attendu.
RMN ¹H δ en ppm (DMSO d6): 2,53(s, 3H); 3,81 (s, 3H); 3,89(s, 3H); 4,02-4,61 (massif, 4H); 6,62(s, 1H); 6,86(d, 1H); 7,15-7,31 (massif, 6H); 7,47(m, 1H); 7,90(m, 1H).
F = 92,9°C

Le tableau XV illustre les structures chimiques et les propriétés physiques de quelques composés de l'invention obtenus selon l'exemple 18.

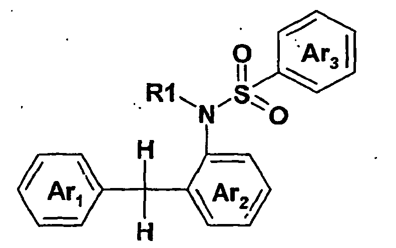

**Tableau XV**

| **N° composé** | **Nature et position des substituants** | | | | **F(°C)** | **MH⁺/ temps de rétention** |
|---|---|---|---|---|---|---|
| | **Ar₁** | **Ar₂** | **R₁** | **Ar₃** | | |
| 140 | 2,6-diF | 4-Cl | propyle | -3,4-diOMe | 135,1 | 496/10,82 |
| 150 | 2,6-diF | 4-Cl | CH₂CON(Me)₂ | 3,4-diOMe | 130,2 | 539/9,2 |
| 145 | 2,6-diF | 4-Cl | (CH₂)₂N(Me)₂ | 3,4-diOMe | 121,2 | 525/6,47 |
| 144 | 2,6-diF | 4-CI | Me | 3,4-diOMe | 191,8 | 468/10,05 |
| 156 | 2,6-diF | 4-Cl | 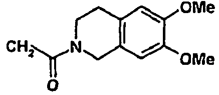 | 3,4-diOMe | 128,2 | 687/9,78 |
| 169 | 2,6-diF | 4-Cl | CH₂CONH₂ | 3,5-diMe | 146 | 479/9,49 |
| 171 | 2,6-diF | 4-Cl | CH₂CONHMe | 3,5-diMe | 196 | 493/9,80 |
| 185 | 2,6-diF | 4-Cl | prop-2-yn-1-yle | 3,4-diOMe | 172 | 492/10,13 |
| 193 | 2,6-diF | 4-OMe | CH₂CONH₂ | 3,4-diOMe | 189,7 | 507/7,93 |
| 195 | 2,6-diF | 3-Cl | CH₂CONH₂ | 3,4-diOMe | 193,5 | 511/8,33 |
| 285 | 2.5-diF | 6-OMe | CH₂CONH₂ | 3,4-diOMe | 193 | 507/8,58 |
| 200 | 2,6-diF | 4-Me | CH₂CONH₂ | 3.4-diOMe | 196 | 491/8,20 |
| 202 | 2,6-diF | 5-N(Me)₂ | CH₂CONH₂ | 3,4-diOMe | 141 | 520/7,84 |
| 205 | 2,6-diF | 4-Me | CH(Et)CONH₂ | 3,4-diOMe | 197,6 | 519/8,84 |
| 211 | 2,6-diF | 4-Cl | CH₂CN | 3,4-diOMe | 61,7 | 493/9,84 |
| 220 | 2,6-diF | 4-F | CH₂CONH₂ | 3,4-diOMe | 184,7 | 495/8,16 |
| 224 | 2-Cl,6-F | 4-Cl | CH₂CONH₂ | 3,4-diOme | 230 | 527/8,77 |
| 181 | 2,6-diF | 4-Cl | (1-méthyl-1H-imidazol-2yl)méthyle | 3,4-diOMe | 87 | 547/6,65 |
| 210 | 2,6-diF | 3,6- diOMe | CH₂CONH₂ | 3,4-diOMe | 242,9 | 537/7,88 |
| 246 | 2,6-diF | 4-Cl | CH(CH3)CN | 3,4-diOMe | 143,4 | 507/10,82 |
| 288 | 2,6-diF | 6-Me | CH₂CONH₂ | 3,4-diOMe | 225,6 | 491/8,61 |
| 293 | 2,6-diF | 6-Cl | CH₂CONH₂ | 3,4-diOMe | 227,4 | 511/8,63 |

Pour les composés 193,195 et 220 on remplace le tert-butylate de potassium par de l'hydrure de sodium.

### Exemple 19 : N²-[4-chloro-2-(2,5-dichlorobenzyl)phényl]-N²-[(3,4-diméthoxyphényl) sulfonyl]glycinamide (composé 80)

### • Exemple 19.1: (2-amino-5-chlorophényl)(2,5-dichlorophényl)méthane

A partir de 1,72g de (2-amino-5-chlorophényl)(2,5-dichlorophényl)méthanol obtenu à l'exemple 10,2 selon le procédé décrit à l'exemple 18.1, on obtient 1,41g de produit attendu.

### • Exemple 19.2 : N-[4-chloro-2-(2,5-chlororobenzyl)phényl]-3,4-méthoxy benzènesulfonamide

A partir de 1,41g de (2-amino-5-chlorophényl)(2,5-dichlorophényl)méthane selon une adaptation du procédé décrit à l'exemple 12.2 on obtient 2,3g de produit attendu.

### • Exemple 19.3 : N²-[4-chloro-2-(2,5-dichlorobenzyl)phényl]-N²-[(3,4-diméthoxy phényl)sulfonyl]glycinamide

A partir de 2,3g N-[4-chloro-2-(2,5-chlororobenzyl)phényl]-3,4-méthoxy benzène sulfonamide selon une adaptation du procédé décrit à l'exemple 15 on obtient 1g de produit attendu.
RMN ¹H δ en ppm (DMSO d6): 3,80(s, 3H); 3,89(s, 3H); 4,01-4,69(massif, 4H); 6,72(s, 1H); 6,89(d, 1H); 7,15-7,61 (massif, 9H).
F=200 °C

Le tableau XVI illustre les structures chimiques et les propriétés physiques de quelques composés de l'invention obtenus selon l'exemple 19.

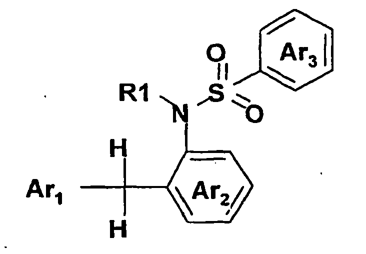

**Tableau XVI**

| **N° composé** | **Nature et position des substituants** | | | | **F(°C)** | **MH⁺/temps de rétention** |
|---|---|---|---|---|---|---|
| | **Ar₁** | **Ar₂** | **R₁** | **Ar₃** | | |
| 142 | 2,3-diFphényle | 4-Cl | CH₂CONH₂ | 3,4-diOMe | 172,2 | 511/8,70 |
| 143 | 2,3-diFphénylé | 4-CI | CH₂CON(Me)₂ | 3,4-diOMe | 88,2 | 539/9,43 |
| 141 | 2,3-diFphényle | 4-Cl | CH₂CONHMe | 3,4-diOMe | 150,9 | 525/9,01 |
| 170 | 2-F,6-OMe phényle | 4-Cl | CH₂CONH₂ | 3,4-diOMe | 160 | 523/8,56 |
| 180 | 2-thiényle | 4-Cl | CH₂CONH₂ | 3,4-diOMe | 183,9 | 481/8,41 |
| 192 | 2-Cl,6-OMe phényle | 4-Cl | CH₂CONH₂ | 3,4-diOMe | 180,6 | 539/8,82 |

### Exemple 20: N²-[4,5-dichloro-2-(2-chlorobenzyl)phényl]-N²-[(3,4-diméthoxyphényl) sulfonyl] glycinamide (composé 161)

A 0,9g de (N²-{4,5-dichloro-2-[(2-chlorophényl)(hydroxy)méthyl]phényl}-N²[(phényl)sulfonyl]glycinamide obtenu selon l'exemple 10,4, en solution dans 10ml de dichlorométhane on additionne 0,77ml de triéthylsilane, 1,2ml d'étherate de tri fluoroborane et porte 3 heures à 40°C. Le milieu réactionnel est repris du dichlorométhane et 100ml d'une solution 2M d'hydroxyde de sodium. Après décantation la phase organique est séchée sur sulfate de sodium anhydre et concentrée. Le résidu est chromatographié sur une colonne de gel de silice en éluant avec un mélange dichlorométhane / acétate d'éthyle, pour obtenir 0,622g de produit attendu.
RMN ¹H δ en ppm (DMSO d6): 3,81(s, 3H); 3,90(s, 3H); 4,07-4,19(massif, 3H); 4,51 (d, 1H); 6,87(s, 1H); 7,14-7,57(massif, 10H).
F=163 °C

Le tableau XVII illustre les structures chimiques et les propriétés physiques de quelques composés de l'invention obtenus selon l'exemple 20.

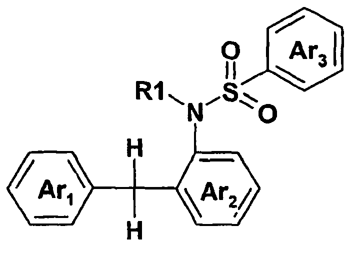

**Tableau XVII**

| **N° composé** | **Nature et position des substituants** | | | | **F(°C)** | **MH⁺/ temps de rétention** |
|---|---|---|---|---|---|---|
| | **Ar₁** | **Ar₂** | **R₁** | **Ar₃** | | |
| 177 | 2,5-diF | 4-Cl | CH₂CONH₂ | 3,4-diOMe | 216,5 | 511/8,75 |
| 182 | 2-F,5-Cl | 4-Cl | CH₂CONH₂ | 3,4-diOMe | 210,3 | 527/9,09 |
| 183 | 3,5-diF | 4-Cl | CH₂CONH₂ | 3,4-diOMe | 184 | 511/8,96 |
| 196 | 2-F,5-Me | 4-Cl | CH₂CONH₂ | 3,4-diOMe | 231,6 | 507/8,98 |
| 100 | 2-Cl | 4-Cl | CH₂CONH₂ | 2,4-diCl,5-Me | 228,3 | 531/9,72 |
| 168 | 2-F,4-Cl | 4-Cl | CH₂CONH₂ | 3,4-diOMe | 195,2 | 527/9,32 |
| 208 | 2,6-diF | 4,6-diOMe | CH₂CONH₂ | 3,4-diOMe | 191 | 537/7,98 |
| 228 | 2,6-diF | 4,5-diCl | CH₂CONH₂ | 3,4-diOMe | 181,1 | 545/8,98 |
| 103 | 2-Cl | 4-Et | CH₂CONMe₂ | 3,4-diOMe | 142,7 | 531/8,99 |
| 93 | 2-Cl | 4-Et | CH₂CONH₂ | 3,4-diOMe | 86 | 503/8,21 |

Le composé 247 est préparé à partir de (N²-{2-[(2,6-difluorophényl)(hydroxy)méthyl]-6-méthoxyphényl}-N²-[(3,4-diméthoxyphényl)sulfonyl]glycinamide obtenu selon l'exemple 9,4.
F= 244,9 ;MH⁺= 507; le temps de rétention est de 8,37 minutes.

Exemple 21 : N-[4-chloro-2-(2,6-difluorobenzyl)phényl]-3,4-diméthoxy-N-[(1-méthyl-1H-tétrazol-5-yl)méthyl]benzènesulfonamide (composé 152) et N-[4-chloro-2-(2,6-difluorobenzyl)phényl]-3,4-diméthoxy-N-[(2-méthyl-2H-tétrazol-5-yl)méthyl]benzène sulfonamide (composé 151)

### • Exemple 21.1 : N-[4-chloro-2-(2,6-difluorobenzyl)phényl]-3,4-diméthoxy-N-[(-1H-tétrazol-5-yl)méthyl]benzènesulfonamide

A 8,7g de N-[4-chloro-2-(2,6-difluorobenzyl)phényl]-N-(cyanométhyl)-3,4-diméthoxybenzène sulfonamide (composé 211) obtenu à l'exemple 18 en solution dans 100ml de THF on additionne à température ambiante, 10g d'azidotriméthylsilane, 2,29g d'oxyde de dibutylétain et porte à reflux 8 heures. Le milieu réactionnel est concentré et le résidu chromatographié sur une colonne de gel de silice en éluant avec un mélange dichlorométhane / méthanol 99/1 (v/v) pour obtenir 7g de produit attendu.
RMN ¹H δ en ppm (DMSO d6): 3,82(s, 3H); 3,90(s, 3H); 4,05(q, 2H); 4,88(d, 1H); 5,29(d, 1H); 6,60(s, 1H); 6,85(d, 1H); 7,12-7,49(massif, 7H).

### • Exemple 21.2 : N-[4-chloro-2-(2,6-difluorobenzyl)phényl]-3,4-diméthoxy-N-[(1-méthyl-1H-tétrazol-5-yl)méthyl]benzènesulfonamide

A 2,7g de N-[4-chloro-2-(2,6-difluorobenzyl)phényl]-3,4-diméthoxy-N-[(-1H-tétrazol-5-yl)méthyl]benzènesulfonamide en solution dans 50ml de DMF, on additionne à température ambiante, 1,07g d'iodométhane, 1,04g de carbonate de potassium. Après 48 heures à température ambiante, le milieu est versé sur de l'eau puis extrait à l'acétate d'éthyle. La phase organique est séchée sur sulfate de sodium anhydre et concentrée. Le résidu est chromatographié sur une colonne de gel de silice en éluant avec un mélange toluéne / acétate d'éthyle 90/10 (v/v) pour obtenir 1g de N-[4-chloro-2-(2,6-difluorobenzyl)phényl]-3,4-diméthoxy-N-[(1-méthyl-1H-tétrazol-5-yl) méthyl]benzènesulfonamide (composé 152)
RMN ¹H δ en ppm (DMSO d6): 3,81(s, 3H); 3,90(s, 3H); 3,93(q, 1H); 4,05(m, 4H); 4,93(d, 1H); 5,42(d, 1H); 6,58(s, 1H); 7,00(d,1H); 7,10-7,46(massif, 7H).
F=180,8 °C
Et 0,71g de N-[4-chloro-2-(2,6-difluorobenzyl)phényl]-3,4-diméthoxy-N-[(2-méthyl-2H-tétrazol-5-yl)méthyl]benzènesulfonamide (composé 151)
RMN ¹H δ en ppm (DMSO d6) : 3,81 (s, 3H); 3,89(s, 3H); 4,13(s, 2H); 4,26(s, 3H); 4,77(d, 1H); 5,25(d, 1H); 6,57(s, 1H); 6,86(d, 1H);7,11-7,47(massif, 7H).
F=136,3°C

Le tableau XVIII illustre les structures chimiques et les propriétés physiques de quelques composés de l'invention obtenus selon l'exemple 21.

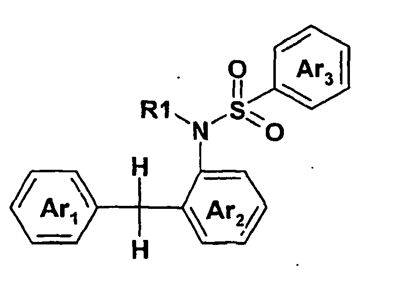

**Tableau XVIII**

| **N° composé** | **Nature et position des substituants** | | | | **F(°C)** | **MH⁺/ temps de rétention** |
|---|---|---|---|---|---|---|
| | **Ar₁ Ar₂** | | **R₁** | **Ar₃** | | |
| 153 | 2,6-diF | 4-Cl | (2-éthyl-2H-tétrazol -5-yl)méthyle | 3,4-diOMe | 139,7 | 564/9,81 |
| 154 | 2,6-diF | 4-Cl | (1-éthyl-1H-tétrazol -5-yl)méthyle | 3,4-diOMe | 150,9 | 564/9,48 |
| 159 | 2,6-diF | 4-Cl | (2-éthyl-2H- tétrazol -5-yl)méthyle | 3,5-diMe | 105,5 | 532/10,84 |
| 160 | 2,6-diF | 4-Cl | (1-éthyl-1H- tétrazol -5-yl)méthyle | 3,5-diMe | 134 | 532/10,46 |
| 157 | 2,6-diF | 4-Cl | (2-méthyl-2H-tétrazol-5-yl)méthyle | 3,5-diMe | 159 | 518/10,53 |
| 158 | 2,6-diF | 4-Cl | (1-méthyl-1H-tétrazol-5-yl)méthyle | 3,5-diMe | 240 | 518/10,20 |
| 122 | 2-Cl | 4-Cl | (2-éthyl-2H- tétrazol -5-yl)méthyle | 3,5-diMe | huile | 530/10,65 |
| 123 | 2-Cl | 4-Cl | (1-éthyl-1H- tétrazol -5-yl)méthyle | 3,5-diMe | 158 | 530/10,27 |
| 108 | 2-Cl | 4-Cl | (1-isopropyl-1H-tétrazol-5-yl)méthyle | 3,4-diOMe | 79 | 576/9,52 |
| 111 | 2-Cl | 4-Cl | (1-benzyl-1H-tétrazol-5-yl)méthyle | 3,4-diOMe | 98 | 624/9,89 |
| 90 | 2-Cl | 4-Cl | (1-éthyl-1H-tétrazol -5-yl)méthyle | 3,4-diOMe | 168,3 | 562/9,25 |
| 88 | 2-Cl | 4-Cl | (1-méthyl-1H-tétrazol-5-yl)méthyle | 3,4-diOMe | 200,6 | 548/8,96 |
| 243 | 2,6-diF | 4-Cl | 1-(2-méthyl-2H-tétrazol-5-yl) éthyle | 3,4-diOMe | 259,3 | 564/9,85 |
| 244 | 2,6-diF | 4-Cl | 1-(1-méthyl-1H-tétrazol-5-yl) éthyle | 3,4-diOMe | 178,1 | 564/10,44 |
| 310 | 2,6-diF | 6- OMe | (2-éthyl-2H- tétrazol -5-yl)méthyle | 3,4-diOMe | 136,3 | 560/9,61 |
| 311 | 2,6-diF | 6- OMe | (1-éthyl-1H-tétrazol -5-yl)méthyle | 3,4-diOMe | 161,4 | 560/9,30 |

### Exemple 22 : N²-[2-(2-chlorobenzyl)-4-méthylphényl]-N²-[(3,4-diméthoxyphényl) sulfonyl]glycinamide (composé 165)

### • Exemple 22.1 N-[4-méthyl-2-(2-chlorobenzyl)phényl]-3,4-diméthoxybenzène sulfonamide

A partir de 1g de N-{4-méthyl-2-[(2-chlorophényl)(hydroxy)méthyl]phényl}-3,4-diméthoxybenzènesulfonamide obtenu selon l'exemple 8, selon une adaptation du procédé décrit à l'exemple 18.1, on obtient 0,47g de produit attendu.
RMN ¹H δ en ppm (DMSO d6): 2,14(s, 3H); 3,73(s, 3H); 3,84(s, 3H); 4,04(s, 2H); 6,60(s, 1H); 6,81-7,47(massif, 9H); 9,43(s, 1H).

### • Exemple 22.2 : N²-[2-(2-chlorobenzyl)-4-méthylphényl]-N²-[(3,4-diméthoxy phényl)sulfonyl]glycinamide

A 0,47g de N-{4-méthyl-2-[(2-chlorophényl)(hydroxy)méthyl]phényl}-3,4-diméthoxy benzènesulfonamide en solution dans 5,7ml de DMF, on additionne à 0°C, 0,048g d'hydrure de sodium. Après 1 heure à cette température on introduit 0,166g de 2-bromoacétamide et laisse 18 heures à température ambiante. Le milieu est versé sur de l'eau puis extrait à l'acétate d'éthyle. La phase organique est séchée sur sulfate de sodium anhydre et concentrée. Le résidu est chromatographié sur une colonne de gel de silice en éluant avec un mélange cyclohexane / acétate d'éthyle 90/10 (v/v) pour obtenir 0,4g de produit attendu.
RMN ¹H δ en ppm (DMSO d6) : 2,16(s, 3H); 3,79(s, 3H); 3,88(s, 3H); 4,05-4,25(massif, 3H); 4,52(d, 1H); 6,54(s, 1H); 6,76(d, 1H); 6,94-7,53(massif, 10H). F=140,6°C
Le *N²*-[5-chloro-2-(2-chlorobenzyl)phényl]-*N²*-[(3,4-diméthoxyphényl)sulfonyl] glycinamide (composé 179) est syntétisé selon ce procédé à partir du N-{5-chloro-2-[(2-chlorophényl)(hydroxy)méthyl]phényl}-3,4-diméthoxybenzènesulfonamide obtenu selon l'exemple 9.3, F=86,4°C
Le *N²*-[2-(2-chtorobenzyl)-4-méthoxyphényl]-*N²*-[(3,4-diméthoxyphényl)sulfonyl] glycinamide (composé 138) est synthétisé selon ce procédé. F= 184,6°C

### Exemple 23 : N-[4-chloro-2-(2,6-difluorobenzyl)phényl]-N-[3-(diméthylamino)propyl]-3,4-diméthoxybenzènesulfonamide (composé 197)

A 0,45g de [4-chloro-2-(2,6-difluorobenzyl)phényl]-3,4-diméthoxybenzènesulfonamide (préparé selon 18.2) on additionne successivement, 0,207g de carbonate de potassium, 0,1g de bromure de tétrabutylammonium, 20ml de toluéne, 0,237g de 3-diméthylamino-1-bromopropane et porte 24 heures à reflux. On additionne 0, 168g de *tert*-butylate de potassium, 0,237g de 3-diméthylamino-1-bromopropane et chauffe à reflux 4 heures. Le milieu réactionnel est versé sur de l'eau et extrait à l'acétate d'éthyle. La phase organique est séchée sur sulfate de sodium anhydre et concentrée. Le résidu est chromatographié sur une colonne de gel de silice en éluant avec un mélange dichlorométhane /méthanol 99/1 (v/v). L'huile obtenue est dissoute dans du méthanol et versé sur une solution 0,5M d'hydroxyde de sodium, le précipité formé est filtré, séché sous vide pour obtenir 0,504g de produit attendu.
RMN ¹H δ en ppm (DMSO d6): 1,34-1,48(massif, 2H); 2,01 (s, 6H); 2,14(t, 2H); 3,13(m, 1H); 3,75(s, 3H); 3,78(m, 1H); 3,85(s, 3H); 4,13(d, 1H); 4,31 (d, 1H); 6,72-7,51 (massif, 9H).
F=134 °C

Le tableau XIX illustre les structures chimiques et les propriétés physiques de quelques composés de l'invention obtenus selon l'exemple 23.

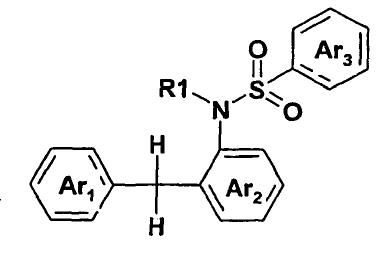

**Tableau XIX**

| | **Nature et position des substituants** | | | | **F(°C)** | **MH⁺/temps de rétention** |
|---|---|---|---|---|---|---|
| | **Ar₁** | **Ar₂** | **R₁** | **Ar₃** | | |
| 199 | 2,6-diF | 4-Cl | 2-pyrrolidin-1-yléthyle | 3,4-diOMe | 131,4 | 551/6,61 |
| 201 | 2,6-diF | 4-Cl | 2-(diméthylamino)-1-méthyléthyle | 3,4-diOMe | 128,6 | 539/6,44 |
| 203 | 2,6-diF | 4-Cl | 2-[benzyl(méthyl) amino]éthyle | 3,4-diOMe | 104,8 | 601/7,18 |
| 231 | 2,6-diF | 4-Cl | (CH₂)₂NHCONH₂ | 3,4-diOMe | 124,5 | 539/8,27 |
| 229 | 2,6-diF | 4-Cl | (CH₂)₃SO₂NEt₂ | 3,4-diOMe | 71,2 | 630/10,27 |
| 230 | 2,6-diF | 4-Cl | (CH₂)₃SO₂NMe₂ | 3,4-diOMe | 76,8 | 602/9,71 |
| 234 | 2,6-diF | 4-Cl | 2-(2-oxopyrrolidin-1-yl)éthyl | 3,4-diOMe | 138,6 | 565/8,80 |
| 236 | 2,6-diF | 4-Cl | 2-méthoxyéthyl | 3,4-diOMe | 83,8 | 512/9,38 |
| 241 | 2,6-diF | 5-Br | CH₃CONH₂ | 3,4-diOMe | 93,5 | 555/8,99 |
| 248 | 2,6-diF | 4-Cl | cyclopropylméthyl | 3,4-diOMe | 163,4 | 508/11,42 |
| 259 | 2,6-diF | 4-Cl | CH₃CONH₂ | 3-Me,4- OMe | 156,6 | 495/9,07 |
| 268 | 2,6-diF | 4-Cl | 2-(1,3-dioxolan-2-yl)éthyl | 3,4-diOMe | 288,8 | 554/10,50 |
| 283 | 2,6-diF | 4-Cl | CH₃CONH₂ | 6-OMe pyridin-3-yl | 164 | 482/9,27 |

### Exemple 24 : N-{4-chloro-2-[(2-chlorophényl)(hydroxy)méthyl]phényl}-N-(2-hydroxyéthyl)-3,4-diméthoxybenzènesulfonamide (composé 24)

### • Exemple 24.1 : N-{4-chloro-2-[(2-chlorophényl)(hydroxy)méthyl]phényl}-3,4-diméthoxy-N-[2-(tétrahydro-2H-pyran-2-yloxy)éthyl]benzènesulfonamide

A 1g de N-{4-chloro-2-[(2-chlorophényl)(hydroxy)méthyl]phényl}-3,4-diméthoxy benzènesulfonamide, obtenu à l'exemple 1.2 en solution dans 40ml d'acétonitrile on additionne successivement 1,2ml de triéthylamine, 1,29ml de 2-(2-bromo éthoxy terahydro-2H-pyrane et porte à reflux 18 heures. Le milieu réactionnel est repris à l'acétate d'éthyle et lavé à l'eau. La phase organique est séchée sur sulfate de sodium anhydre et concentrée. Le résidu est chromatographié sur une colonne de gel de silice en éluant avec un mélange de solvant cyclohexane / acétate d'éthyle, pour obtenir 0,479g de produit attendu.

### • Exemple 24.2 : N-{4-chloro-2-[(2-chlorophényl)(hydroxy)méthyl]phényl}-N-(2-hydroxyéthyl)-3,4-diméthoxybenzènesulfonamide

A 0,479g de N-{4-chloro-2-[(2-chlorophényl)(hydroxy)méthyl]phényl}-3,4-diméthoxy-N-[2-(tétrahydro-2*H*-pyran-2-yloxy)éthyl]benzènesulfonamide en solution dans 4,7ml de THF , on additionne 9,15ml d'acide acétique, 2,25ml d'eau et porte 48 heures à 40°C. Le milieu réactionnel est repris à l'acétate d'éthyle et lavé à l'eau. La phase organique est séchée sur sulfate de sodium anhydre et concentrée. Le résidu est chromatographié sur une colonne de gel de silice en éluant avec un mélange de solvant cyclohexane / acétate d'éthyle, pour obtenir 0,209g de produit attendu.
RMN ¹H δ en ppm (DMSO d6): 3,02(m, 2H); 3,40(m, 2H); 3,75(s, 3H); 3,88(s, 3H); 4,40(t, 1H); 6,01(d, 1H); 6,70-7,51 (massif, 10H); 7,86(s, 1H).
F= 86,2°C

De la même façon, on prépare le composé 237, dont les propriétés physiques sont les suivantes :
MH+=498, le temps de rétention est de 8,99 minutes
F= 165,3°C

### Exemple 25 : N²-{4-chloro-2-[1-(2-chlorophényl)-1-hydroxyéthyl]phényl}-N²-[(3,4-diméthoxyphényl)sulfonyl]glycinamide (comosé 172)

### • Exemple 25.1 : 1-(2-amino-5-chlorophényl)-1-(2-chlorophényl)éthanol

A 5g de 2-amino-2',5-dichlorobenzophénone en solution dans 100ml, de diéthyléther à -30°C, on additionne 12,5ml d'une solution 3M de bromure de méthylmagnésium et porte 18 heures à température ambiante. On additionne 6ml d'une solution 3M de bromure de méthylmagnésium et laisse 1 heure à température ambiante. On hydrolyse avec une solution 2M d'acide chlorhydrique et lave à l'eau. La phase organique est séchée sur sulfate de sodium anhydre pour obtenir 4,3g de produit attendu.
RMN ¹H δ en ppm (DMSO d6): 1,99(s, 3H); 4,91(s, 2H);6,11(s, 1H); 6,53(d, 1H);6,97-7,42(massif, 5H); 7,97(d, 1H).

### • Exemple 25.2 : N-{4-chloro-2-[1-(2-chlorophényl)-1-hydroxyéthyl]phényl}-3,4-diméthoxybenzènesulfonamide

A 1,5g de 1-(2-amino-5-chlorophényl)-1-(2-chlorophényl)éthanol selon une adaptation du procédé décrit à l'exemple 1.1 on obtient 2,9g de produit attendu. RMN ¹H δ en ppm (DMSO d6): 1,78(s, 3H); 3,75(s, 3H); 3,83(s, 3H); 4,05(q, 1H); 6,87(s, 1H); 7,04-7,54(massif, 5H); 7,82(m, 2H); 8,60(d, 2H); 10,00(s, 1H).

### • Exemple 25.3 : N-{4-chloro-2-[1-(2-chlorophényl)-1-hydroxyéthyl]phényl}N-[(3,4-diméthoxyphényl)sulfonyl]glycinamide

A 1,3g de N-{4-chloro-2-[1-(2-chlorophényl)-1-hydroxyéthyl]phényl}-3,4-diméthoxy benzènesulfonamide en solution dans du diéthyléther on additionne successivement à -5°C, 1,3ml d'une solution 2M dans l'hexane de lithium diisopropylamine, 0,45g de 2-bromoacétamide. Après 18 heures à température ambiante, on additionne successivement 0,5g de 2-bromoacétamide, 1,4g d'iodure de sodium et porte à reflux 4 heures. Le milieu réactionnel est repris à l'acétate d'éthyle et lavé à l'eau. La phase organique est séchée sur sulfate de sodium anhydre et concentré. Le résidu est chromatographié sur une colonne de gel de silice en éluant avec un mélange dichlorométhane / méthanol 95/5 (v/v), pour obtenir 0,51g de produit attendu.
RMN ¹H δ en ppm (DMSO d6): 2,11 (s, 3H); 3,74-3,98(massif, 8H); 6,25(s, 1H); 6,96-7.97(massif, 12H).

### Exemple 26 : N²-{4-chloro-2-[1-(2-chlorophényl)éthyl]phényl}-N²-[(3,4-diméthoxy phényl)sulfonyl]glycinamide (composé 186)

### • Exemple 26.1: 4-chloro-2-[1-(2-chlorophényl)éthyl]phénylamine

A 54ml d'une solution M d'hydrure mixte d'aluminium et de lithium dans le THF, on additionne par portions 7,1g de trichlorure d'aluminium. A cette solution on additionne 4g de 1-(2-amino-5-chlorophényl)-1-(2-chlorophényl)éthanol obtenu à l'exemple 25.1. en solution dans de l'éther diéthylique et agite 18 heures à température ambiante puis porte à reflux 4 heures. Le milieu réactionnel est repris à l'acétate d'éthyle et lavé à l'eau. La phase organique est séchée sur sulfate de sodium anhydre et concentré. Le résidu est chromatographié sur une colonne de gel de silice en éluant avec un mélange dichlorométhane / cyclohexane 40/60 (v/v), pour obtenir 0,9g de produit attendu.
RMN ¹H δ en ppm (DMSO d6): 1,45(d, 3H); 4,43(q, 1H); 4,96(s, 2H); 6,65(m, 2H); 6,94(d, 1H); 7,30-7,47(massif, 4H).

### • Exemple 26.2 : N-{4-chloro-2-[1-(2-chlorophényl)éthyl]-phényl}-3,4-diméthoxy benzènesulfonamide

A partir de 0,9g de 4-chloro-2-[1-(2-chlorophényl)éthyl]phénylamine selon le procédé décrit à l'exemple 1.1 on obtient 1,7g de produit attendu.

### • Exemple: 26.3 : N²-{4-chloro-2-[1-(2-chlorophényl)éthyl]phényl}-N²-[(3,4-diméthoxy phényl)sulfonyl]glycinamide

A partir de 0,9g N-{4-chloro-2-[1-(2-chlorophényl)éthyl]-phényl}-3,4-diméthoxy benzènesulfonamide selon le procédé décrit à l'exemple 12.3 on obtient 0,9g de produit attendu.
RMN ¹H δ en ppm (DMSO d6): 1,35(d, 3H); 2,75(d, 1H); 3,79(s, 3H); 3,86(s, 3H); 4,25(d, 1H); 4,74(q, 1H); 6,92-7,64(massif, 12H).
F=150 °C

Le composé 187 est obtenu suivant ce procédé. F=151 °C

### Exemple 27 : N-(2-aminoéthyl)N-[2-(2,6-difluorobenzyl)-4-méthylphényl]-3,4-diméthoxybenzènesulfonamide (composé 215)

A 1g de N-[4-méthyl-2-(2,6-difluorobenzyl)phényl]-N-[2-(phtalimido)éthyl]-3,4-diméthoxybenzènesulfonamide, obtenu selon une adaptation du procédé décrit à l'exemple 18, en solution dans 15ml d'éthanol on additionne 0,4g d'hydrate d'hydrazine et porte à reflux 3 heures. A température ambiante on filtre l'insoluble et concentre le filtrat. Le résidu est chromatographié sur une colonne de gel de silice en éluant avec un mélange dichlorométhane /méthanol 99/1 (v/v) pour obtenir 0,26g de produit attendu.
RMN ¹H δ en ppm (DMSO d6) : 1,62(s, 2H); 2,17(s, 3H); 2,40-2,67(massif, 2H); 3,12(m, 1H); 3,69(m, 1H); 3,75(s, 3H); 3,88(s, 3H); 4,09(d, 1H); 4,26(d, 1H); 6,56(m, 2H); 6,97-7,43(massif, 7H).
F= 251,8°C

Le tableau XX illustre les structures chimiques et les propriétés physiques de quelques composés de l'invention obtenus selon l'exemple 28.

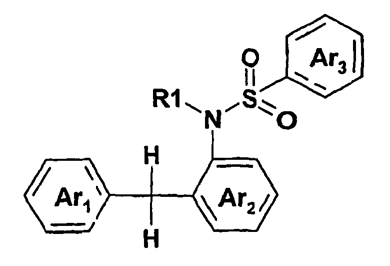

**Tableau XX**

| **N° composé** | **Nature et position des substituants** | | | | **F(°C)** | **MH⁺/temps de rétention** |
|---|---|---|---|---|---|---|
| | **Ar₁** | **Ar₂** | **R₁** | **Ar₃** | | |
| 256 | 2,6-diF | 4-Cl | 2-aminoéthyle | 3,4-diOMe | 163,4 | 497/6,82 |
| 257 | 2,5-diF | 4-Me | 2-aminoéthyle | 3,4-diOMe | 61,1 | 477/6,69 |
| 262 | 2,5-diF | 4-Cl | 2-aminoéthyle | 3,4-diOMe | 141,1 | 497/6,88 |
| 281 | 2,6-diF | 6-OMe | 2-aminoéthyle | 3,4-diOMe | 137,8 | 493/6,47 |
| 287 | 2,6-diF | 4-Cl | 3-aminopropyle | 3,4-diOMe | 149,1 | 511/6,91 |
| 294 | 2,5-diF | 6-OMe | 2-aminoéthyle | 3,4-diOMe | 114,8 | 493/6,47 |
| 300 | 2,6-diF | 4-Cl,6- OMe | 2-aminoéthyle | 3,4-diOMe | 185,4 | 527/6,82 |

### Exemple 28 : N-(2-{[(benzylamino)carbonyl]amino}éthyl)-N-[2-(2,6-difluorobenzyl)-4-méthylphényl]-3,4-diméthoxybenzènesulfonamide (composé 218)

A 1g de N-(2-aminoéthy)-N-[2-(2,6-difluorobenzyl)-4-méthylphényl]-3,4-diméthoxy benzènesulfonamide (composé 215) en solution dans 20ml de THF on additionne 0,266g de benzyl isocyanate, après 3 heures à reflux, le milieu est évaporé à sec. Le résidu est chromatographie sur une colonne de gel de silice en éluant avec un mélange toluène /acétate d'éthyle 8/2 (v/v) pour obtenir 0,65g de produit attendu.
RMN ¹H δ en ppm (DMSO d6): 2,17(s, 3H); 3,05(m, 2H); 3,24(m, 1H); 3,74(s, 3H); 3,78 (m, 1H); 3,87(s, 3H); 4,07-4,25(massif, 4H); 5,99(t, 1H); 6,50(t, 1H); 6,58(d, 2H); 6,98-7,43(massif, 12H).
F= 100,4 °C

Le tableau XXI illustre les structures chimiques et les propriétés physiques de quelques composés de l'invention obtenus selon l'exemple 28.

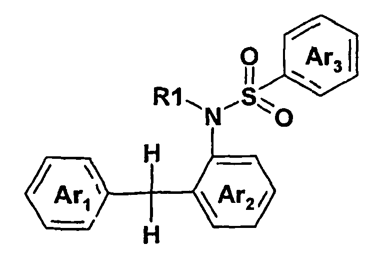

**Tableau XXI**

| **N° composé** | **Nature et position des substituants** | | | | **F(°C)** | **MH⁺/temps de rétention** |
|---|---|---|---|---|---|---|
| | **Ar₁** | **Ar₂** | **R₁** | **Ar₃** | | |
| 217 | 2,6-diF | 4-Me | 2-[(anilinocarbonyl) amino]éthyle | 3,4-diOMe | 163,4 | n.d |
| 238 | 2,6-diF | 4-Cl | 2-({[(3-chlorophényl)amino] carbonyl}amino)éthyle | 3,4-diOMe | 149,3 | 650/10,87 |
| 240 | 2,6-diF | 4-Cl | 2-({[(2-chlorophényl)amino] carbonyl}amino)éthyle | 3,4-diOMe | 136,3 | 650/10,77 |
| 252 | 2,6-diF | 4-Cl | 2-({[(3,4diméthoxyphényl) amino]carbonyl}amino) éthyle | 3,4-diOMe | 169,7 | 676/9,80 |

### Exemple 29 : N-(2-{[2-(2,6-difluorobenzyl)-4-méthylphényl][(3,4-diméthoxyphényl) sulfonyl]amino}éthyl)acétamide (composé 219)

A 1g de N-(2-aminoéthyl)N-[2-(2,6-difluorobenzyl)-4-méthylphényl]-3,4-diméthoxybenzènesulfonamide (composé 215) en solution dans 25ml de THF, on additionne 0,167ml de pyridine, 0,22g d'anhydride acétique. Après 48 heures à température ambiante, on concentre le milieu réactionnel. Le résidu est chromatographié sur une colonne de gel de silice en éluant avec un mélange dichlorométhane /méthanol 99/1 (v/v) pour obtenir 0,505g de produit attendu.
RMN ¹H 8 en ppm (DMSO d6): 1,74(s, 3H); 2,17(s, 3H); 3,05-3,28(m, 3H); 3,72(m, 1H); 3,76(s, 3H); 3,87(s, 3H); 4,05(d, 1H); 4,22(d, 1H); 6,60(d, 2H); 6,99-7.46(massif, 7H); 7,85(t, 1H).
F= 79,7 °C

### Exemple 30 : N²-{4-chloro-2-[méthyl(phényl)amino]phényl}-N²-[(3,4-diméthoxyphényl) sulfonyl]-N¹-méthylglycinamide (composé 124)

### • Exemple 30.1 : 5-chloro-2-nitro-N-diphényl-N-méthylamine

A 10g de 5-chloro-2-nitrodiphénylamine en solution dans 20ml de DMF, on additionne successivement 2,8ml de iodométhane, 14,71g de carbonate de césium et agite 18 heures à température ambiante. Le milieu réactionnel est repris à l'acétate d'éthyle et lavé à l'eau. La phase organique est séchée sur sulfate de sodium anhydre et concentrée. Le résidu est chromatographié sur une colonne de gel de silice en éluant avec un mélange cyclohexane / acétate d'éthyle 97/3 (v/v), pour obtenir 10,51g de produit attendu.
RMN ¹H δ en ppm (DMSO d6) : 3,32(s, 3H); 6,78-6,93(massif, 3H); 7,19-7,26 (m, 2H); 7,40(d, 1H); 7,65 (d, 1H); 7,93(d, 1H).

### • Exemple 30.2 : 2-amino-5-chloro-N-diphényl-N-méthylamine

A 10,49g de 5-chloro-2-nitro-N-diphényl-N-méthylamine en solution dans 100ml d'éthanol, on additionne successivement 14,22g d'étain métallique, 50ml d'une solution 12M d'acide chlorhydrique et porte 1 heure à reflux. Après évaporation de l'éthanol, le milieu réactionnel est repris à l'acétate d'éthyle, basifié et lavé à l'eau. La phase organique est séchée sur sulfate de sodium anhydre et concentrée pour obtenir 9,37g de produit attendu.
RMN ¹H δ en ppm (DMSO d6) : 3,11(s, 3H); 5,07(s, 2H); 6,57-7,21 (massif, 8H).

### • Exemple 30.3 : N-{4-chloro-2-[méthyl(phényl)amino]phényl}-3,4-diméthoxy benzènesulfonamide

A partir de 1,5g de 2-amino-5-chloro-N-diphényl-N-méthylamine on procède suivant l'exemple 1.1 pour obtenir 1,122g de produit attendu.
RMN ¹H δ en ppm (DMSO d 6) : 2,93(s, 3H); 3,67(s, 3H); 3,84(s, 3H); 6,37 (d, 2H); 6,74(t, 1H); 7,05-7,37(massif, 7H); 7,54(d, 1H); 9,46(s, 1H).

### • Exemple 30.4 : N²-{4-chloro-2-[méthyl(phényl)amino]phényl}-N²-[(3,4-diméthoxyphényl) sulfonyl]-N¹-méthylglycinamide

A partir de 0,8g de N-{4-chloro-2-[méthyl(phényl)amino]phényl}-3,4diméthoxybenzènesulfonamide suivant le procédé décrit à l'exemple 4.3 on obtient 0,766g de produit attendu.
RMN ¹H δ en ppm (DMSO d6): 2,41(d, 3H); 3,16(s, 3H); 3,77(s, 3H); 3,88(s, 3H); 4,12(s, 2H); 6,66-6,84(massif, 3H); 7,10-7,43(massif, 8H); 7,72(t, 1H).
F= 148,6 °C

Le composé 92 est obtenu par ce procédé : F= 192,5 °C

### Exemple 31 : 4-{[4-chtoro-2-(2,6-difluorobenzyl)phényl][(3,4-diméthoxyphényl) sulfonyl]amino}butanamide (composé 204)

### • Exemple 31.1 : acide 4-{[4-chloro-2-{[2-(2,6-difluorobenzyl)phényl][((3,4-diméthoxyphényl) sulfonyl]amino}butanoïque

A 2,6g de éthyl4-{[4-chloro-2-{[2-(2,6-difluorobenzyl)phényl][((3,4-diméthoxyphényl) sulfonyl]amino}butanoate obtenu selon l'exemple 18, en solution dans 50ml d'éthanol, on additionne 22,8ml d'hydroxyde de sodium et laisse 18 heures à température ambiante. On évapore le solvant, reprend le résidu avec 40ml d'une solution 1 M d'acide chlorhydrique et extrait à l'acétate d'éthyle. La phase organique est séchée sur sulfate de sodium anhydre et concentrée. Le résidu est concrété à l'éther diisopropylique, pour obtenir 1,7g de produit attendu.
RMN ¹H δ en ppm (DMSO d6): 1,54(m, 2H); 2,27(t, 2H); 3,27(m, 1H); 3,76(s, 3H); 3,85(s, 3H); 4,23(m, 2H); 6,75-7,51(massif, 10H); 12,09(s, 1H).

### • Exemple 31.2 : {[4-chloro-2-(2,6-difluorobenzyl)phényl][(3,4-diméthoxyphényl) • sulfonyl]amino}butanamide

A 1,7g d'acide 4-{[4-chloro-2-{[2-(2,6-difluorobenzyl)phényl][((3,4-diméthoxyphényl) sulfonyl]amino}butanoïque en solution dans 30ml de THF, à 0°C, on additionne 0,42ml de N-éthylmorpholine, 0,42ml de chloroformiate d'éthyle, après 30 minutes à cette température, on introduit goutte à goutte une solution d'ammoniac dans le THF, et laisse 1 heure à 20°C. On verse sur une solution saturée d'hydrogénocarbonate de sodium, reprend à l'acétate d'éthyle. La phase organique est séchée sur sulfate de sodium anhydre, concentrée, le résidu est concrété à l'éther diéthylique, pour obtenir après séchage 1,41 g de produit attendu.
RMN ¹H δ en ppm (DMSO d6): 1,51(m, 2H); 2,07(t, 2H); 3,21(m, 1H); 3,74(m, 1H); 3,78(s, 3H); 3,89(s, 3H); 4,23(q, 2H); 6,75(m, 3H); 7,04-7,51 (massif, 8H).
F= 197 °C

Le tableau XXII illustre les structures chimiques et les propriétés physiques de quelques composés de l'invention obtenus selon cet exemple.

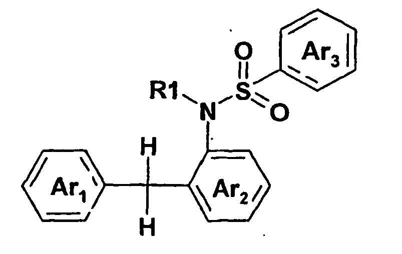

**Tableau XXII**

| **N° composé** | **Nature et position des substituants** | | | | **F(°C)** | **MH⁺/temps de rétention** |
|---|---|---|---|---|---|---|
| | **Ar₁** | **Ar₂** | **R₁** | **Ar₃** | | |
| 235 | 2,6-diF | 4-Cl | CH₂CH₂CH₂CONH₂ | 2,5-diMe, 4-Cl | n.d | 541/10,03 |
| 266 | 2,5-diF | 4-Cl | CH₂CH₂CH₂CONH₂ | 3,4-diOMe | 150 | 539/9,15 |
| 272 | 2,6-diF | 6-OMe | CH₂CH₂CH₂CONH₂ | 3,4-diOMe | 207,8 | 535/8,42 |
| 299 | 2,5-diF | 6-OMe | CH₂CH₂CH₂CONH₂ | 3,4-diOMe | 189,3 | 535/8,54 |

### Exemple 32 : N²-{4-chloro-2-[2-(trifluorométhyl)benzyl]phényl}-N²-[(3,4-diméthoxy phényl)sulfonyl]glycinamide (composé 207)

### • Exemple 32.1 : (2-amino-5-chlorophényl)(2-trifluorométhylphényl)méthanol

A 1,9g de (2-amino-5-chlorophényl)(2-trifluorométhylphényl)méthanone obtenu à l'exemple 7.2 en solution dans 87ml d'éthanol on additionne 0,775g de borohydrure de sodium et laisse 18 heures à 20°C. On évapore le solvant, reprend le résidu à l'acétate d'éthyle et lave à l'eau. La phase organique est séchée sur sulfate de sodium anhydre, et concentrée pour obtenir 1,96g de produit attendu.
RMN ¹H δ en ppm (DMSO d6): 5,15(s, 2H); 5,97(d, 1H); 6,11(d, 1H); 6,35(d, 1H); 6,72(d, 1H) ; 6,99(d, 1H); 7,53-7,81 (massif, 4H).

### • Exemple 32.2 N-{4-chloro-2-[(2-trifluorométhylphényl)(hydroxy)méthyl] phényl}-3,4-diméthoxybenzènesulfonamide

A partir de 1,28g de (2-amino-5-chlorophényl)(2-trifluorométhylphényl)méthanol selon le procédé décrit à l'exemple 1.1 on obtient 2,06g de produit attendu.
RMN ¹H δ en ppm (DMSO d6): 3,76(s, 3H); 3,85(s, 3H); 6,34(s, 2H); 6,89(d, 1H); 6,98(s, 1H); 7,08(d, 1H); 7,23-7,33(massif, 4H); 7,53-7,77(massif, 3H); 9,25(s, 1H).

### • Exemple 32.3 : N-[4-chloro-2-(2-trifluorométhylbenzyl)phényl]-3-méthoxy benzènesulfonamide

A partir de 2,06g de N-{4-chloro-2-[(2-trifluorométhylphényl)(hydroxy)méthyl]phényl}-3,4-diméthoxybenzènesulfonamide selon la méthode décrite à l'exemple 22.1 on obtient 0,94g de produit attendu.
RMN ¹H δ en ppm (DMSO d6): 3,73(s, 3H); 3,85(s, 3H); 4,06(s, 2H); 6,64(s, 1H); 6,89-7,28(massif, 6H); 7,48-7,60(m, 2H); 7,75(d, 1H); 9,72(s, 1H).

### • Exemple 32.4 : N²-{4-chloro-2-[2-(trifluorométhyl)benzyl]phényl}-N²-[(3,4-diméthoxyphényl)sulfonyl]glycinamide

A partir de 0,94g de N-[4-chloro-2-(2-trifluorométhylbenzyl)phényl]-3-méthoxybenzène sulfonamide selon l'exemple 16.3 on obtient 0,723g de produit attendu.
RMN ¹H δ en ppm (DMSO d6): 3,80(s, 3H); 3,89(s, 3H); 4,03(d, 1H); 4,27(q, 2H); 4,74(d, 1H); 6,55(s, 1H); 6,88(d, 1H); 7,12-7,63(massif, 9H); 7,82(d, 1H).
F=133°e

### Exemple 33 : N-[4-chloro-2-(2,6-difluorobenzyl)phényl]-N-[(3,4-diméthoxyphényl) sulfonyl]-2,2,2-trifluoroacétamide (composé 223)

A 2,2g de N-[4-chloro-2-(2,6-difluorobenzyl)phényl]-3,4-diméthoxybenzène sulfonamide obtenu à l'exemple 18.2 en solution dans 40ml de dichlorométhane on additionne 0,8ml de triéthylamine, 1,46g trifluoroacétyltriflate, après 10 minutes on hydrolyse le milieu et le concentre. Le résidu est chromatographié sur une colonne de gel de silice en éluant avec un mélange dichlrométhane /cyclohexane pour obtenir 1,1 g de produit attendu.
RMN ¹H δ en ppm (DMSO d6): 3,86(s, 3H); 3,93(s, 3H); 4,19(s, 2H); 6,92(s, 1H); 7,17-7,54(massif, 7H); 7,71 (d, 1H).
F= 79,7 °C

### Exemple 34 : N-[4-chloro-2-(2,6-difluorobenzyl)phényl]-3,4-diméthoxy-N-(2,2,2-trifluoroéthyl)benzènesulfonamide (composé 190)

A 1,48g de N-[4-chloro-2-(2,6-difluorobenzyl)phényl]-3,4-diméthoxybenzène sulfonamide obtenu à l'exemple 18.2 en solution dans 40ml de xylène on additionne, 2,3g de 2,2,2-trifluoroéthyltriclorométhanesulfonate, 0,44g de *tert*-butylate de potassium, 20ml de N-méthylpyrrolidone et porte à 150°C 8 heures. Le milieu réactionnel est repris à l'acétate d'éthyle et lavé à l'eau. La phase organique est séchée sur sulfate de sodium anhydre et concentrée. Le résidu est chromatographié sur une colonne de gel de silice en éluant au dichlorométhane, pour obtenir 0,455g de produit attendu.
RMN ¹H δ en ppm (DMSO d6): 3,82(s, 3H); 3,90(s, 3H); 4,14-4,35(massif, 3H); 4,82(m, 1H); 6,63(s, 1H); 6,80(d, 1H); 7,17-7,54 (massif, 7H).
F=70°C

### Exemple 35 : N-[4-chloro-2-(2,6-difluorobenzyl)phényl]-3,4-diméthoxy-N-(3-pyridin-3-ylpropyl)benzènesulfonamide (Composé 232)

A 1,5g de triphénylphospine en solution, dans 25ml de THF, on additionne 0,909g de diisopropylazodicarboxylate. Après 15 minutes à température ambiante on introduit 0,61g de 3-pyridin-3-ylpropan-1-ol et abandonne 15 minutes à 20°C. On introduit 1,36g de N-[4-chloro-2-(2,6-difluorobenzyl)phényl]-3,4-diméthoxybenzène sulfonamide et laisse 18 heures à température ambiante. Le milieu est concentré, le résidu est chromatographié sur une colonne de gel de silice en éluant avec un mélange toluène / acétate d'éthyle passant d'un ration 9/1 à 5/5 (v/v). Au produit obtenu en solution dans de l'étherdiéthylique, 5ml d'acide chlorhydrique 2M sont additionnés puis les solvants sont évaporés. Le résidu est repris dans l'éther diisopropylique pour obtenir, après filtration, 0,55g de produit attendu.
F = 102,8°C

Le tableau XXIII illustre les structures chimiques et les propriétés physiques de quelques composés de l'invention obtenus selon cet exemple.

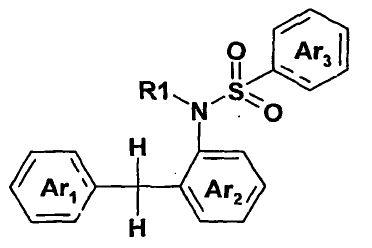

**Tableau XXIII**

| **N° composé** | **Nature et position des substituants** | | | | **F(°C),** | **MH⁺/temp side rétention** |
|---|---|---|---|---|---|---|
| | **Ar₁** | **Ar₂** | **R₁** | **Ar₃** | | |
| 245 | 2,6- diF | 4-Cl | 3-pyridin-3-ylpropyle | 3,4-diOMe | 102,8 | 573/8,10 |
| 250 | 2,6- diF | 4-Cl | 2-pyridin-2-yléthyle | 3,4-diOMe | 75,1 | 559/8,24 |
| 254 | 2,6- diF | 4-Cl | 2-pyridin-3-yléthyle | 3,4-diOMe | 90,3 | 559/7,14 |
| 255 | 2,6- diF | 4-Cl | 2-pyridin-4-yléthyle | 3,4-diOMe | 164,3 | 559/8,29 |
| 260 | 2,6- diF | 4-Cl | 2-phénoxyéthyle | 3,4-diOMe | 64,3 | 574/10,88 |
| 274 | 2,6- diF | 4-Cl | (2R)-pyrrolidin-2-ylméthyle | 3,4-diOMe | 204,8 | 537/7,07 |
| 275 | 2,6- diF | 4-Cl | (2S)-pyrrolidin-2-ylméthyle | 3,4-diOMe | 214,1 | 537/7,07 |
| 278 | 2,6- diF | 4-Cl | {(2-(diméthylamino)éthyl) amino}éthyle | 3,4-diOMe | 103 | 568/6,29 |
| 290 | 2,6- diF | 4-Cl | 1,3-thizol-2-ylméthyle | 3,4-diOMe | 184 | 551/10,26 |
| 291 | 2,6- diF | 4-Cl | Pyrimidin-2-ylméthyle | 3,4-diOMe | 104 | 546/9,89 |
| 292 | 2,6- diF | 4-Cl | 2-morpholino-4-yléthyle | 3,4-diOMe | 185,3 | 567/7,35 |
| 295 | 2,6- diF | 4-Cl | Pyridin-2-ylméthyle | 3,4-diOMe | 200,8 | 545/16,9 |
| 315 | 2,6- diF | 6- OMe | CH(CH₃)COOEt | 3,4-diOMe | 94,3 | 536/9,98 |
| 316 | 2,6- diF | 6- OMe | CH(CH₃)COOEt | 3,4-diOMe | 130,2 | 536/9,89 |
| 317 | 2,6- diF | 6- OMe | CH(CH₃)COOEt | 3,4-diOMe | 73,3 | 536/9,98 |
| 318 | 2,6- diF | 6- OMe | CH(CH₃)COOEt | 3,4-diOMe | 130,8 | 536/9,89 |

| | |
|---|---|
| 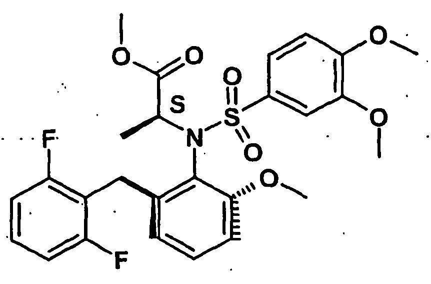 | 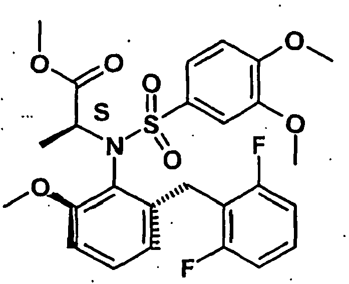 |
| **composé 315** | **composé 316** |
| **[α_{D}]²⁰= +97,59 c=0,66 (CH₂Cl₂)** | **[α_{D}]²⁰= -106,25 c=0,66 (CH₂Cl₂)** |
| 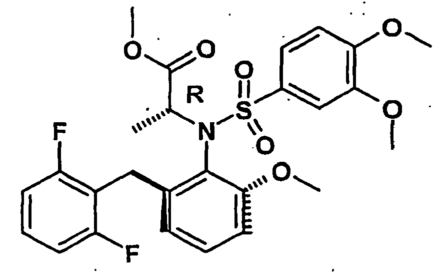 | 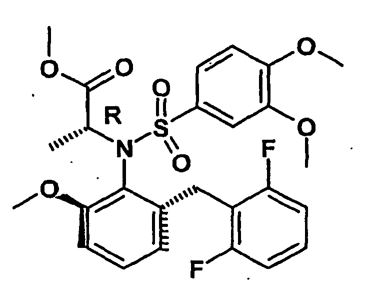 |
| **composé 317** | **composé 318** |
| **[α_{D}]²⁰= -101,53 c=0,45(CH₂Cl₂)** | **[α_{D}]²⁰= +106,66 c=0,66 (CH₂Cl₂)** |

Les composés 315 / 316 et 317 / 318 sont des couples d'atropoisoméres. Chaque atropoisomère est caractérisé par son pouvoir rotatoire (α_{D}). Le centre d'atropoisomérie est généré par l'encombrement stérique autour de la liaison N-aromatique.

### Exemple 36 : Ethyl N-[2,6-difluorobenzyl)-4-méthylphényl]-3,4-diméthoxy-N-[2-(méthylamino)éthyl] benzènesulfonamide (composé 258)

### • Exemple 36.1: Ethyl N-(2-bromoéthyl)-N-[2-(2,6-difluorobenzyl)-4-méthylphényl]-3,4-diméthoxybenzènesulfonamide

A 1g N-[2-(2,6-difluorobenzyl)-4-méthylphényl]-3,4-diméthoxybenzènesuifonamide en solution dans 1,1ml de diméthylformamide on additionne successivement 0,38g de carbonate de potassium, 0,48ml de 1,2-dibromoéthane et porte à 100° 4 heures. Après retour à température ambiante, on hydrolyse le milieu réactionnel et l'extrait à l'acétate d'éthyle. La phase organique est séchée sur sulfate de sodium anhydre et concentrée pour donner 1,24 g de produit attendu
RMN ¹H δ en ppm (DMSO d. 6): 2,17(s,3H); 3,42(m,2H);3,73(m,1H) ;3,76(s,3H);3,88(s,3H); 4,03-4,15(massif,2H); 4,45(d,1H);6,63(d,1H) ;6,98-7,48 (massif,8H).

### • Exemple 36.2 : Bromhydrate d'éthyl N-[2,6-difluorobenzyl)-4-méthylphényl]-3,4-diméthoxy-N-[2-(méthylamino)éthyl] benzènesulfonamide

A 1,24g d' éthyl N-(2-bromoéthyl)-N-[2-(2,6-difluorobenzyl)-4-méthylphényl]-3,4-diméthoxybenzènesulfonamide on additionne 10,7ml d'une solution 2M de méthylamine dans le méthanol et porte 4 heures à 105°. La réaction est complétée par addition de 10ml d'une solution 2M de méthylamine dans le méthanol et chauffage à reflux durant 8 heures. Après retour à température ambiante le milieu est concentré et le résidu chromatographié pour obtenir 0,849 g de produit attendu F= 208.5°C
RMN ¹H δ en ppm (DMSO d 6): 2,20(s,3H); 2,58(s,3H); 2,85(m,1H); 3,06(m,1H); 3,52(m,1H);3,77(s,3H);3,90(s,3H);3,99-4,07(massif,2H);4,27(d,1H);6,58-6,64(massif,2H); 7,01-7,49 (massif ,2H);8,48(s,2H).

Le tableau XXIV illustre les structures chimiques et les propriétés physiques de quelques composés de l'invention obtenus selon cet exemple.

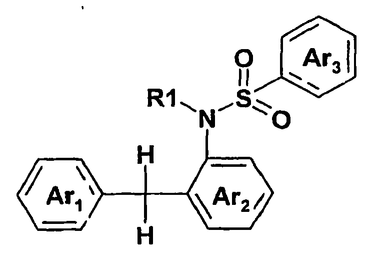

**Tableau XXIV**

| **N° composé** | **Nature et position des substituants** | | | | **F(°C)** | **MH⁺/temp s de rétention** |
|---|---|---|---|---|---|---|
| | **Ar₁** | **Ar₂** | **R₁** | **Ar₃** | | |
| 277 | 2,6-diF | 4-Cl | 2-(1H-1,2,4-triazol-1-yl)éthyle | 3,4-diOMe | 129,1 | n.d. |
| 282 | 2,6-diF | 6-OMe | méthylaminoéthyle | 3,4-diOMe | 262,9 | 507/6,54 |
| 284 | 2,6-diF | 4-Cl | méthylaminoéthyle | 3,4-diOMe | 54 | 511/6,94 |
| 306 | 2,6-diF | 6-OMe | 2-(1H-1,2,4-triazol-1-yl)éthyle | 3,4-diOMe | 132,1 | 545/8,76 |

### Exemple 37 : N-[4-chloro-2-(2,6-difluorobenzyl)phényl]-N-(3-hydroxypropyl)-3,4-diméthoxy benzènesulfonamide (composé 270)

### • Exemple 37.1 : N-(3-{[tert-butyl(dimétyl)silyl]oxy}propyl)-N-[4-chloro-2-(2,6-difluorobenzyl)phényl]-3,4-diméthoxybenzènesulfonamide

A 4,3g de triphénylphosphine dans 20 ml de tétrahydrofurane on additionne à température ambiante 3,25ml de diisopropylazodicarboxylate. Après 30 minutes on introduit 3,5ml de 3-[*tert*-butyl(diméthyl)silyloxy]propanol en solution dans 30 ml de tétrahydrofurane. On laisse 30 minutes à température ambiante puis introduit 5g de N-[4-chloro-2-(2,6-difluorobenzyl)phényl]-3,4-diméthoxybenzènesulfonamide. Après 48 heures à température ambiante le milieu réactionnel est concentré puis chromatographié sur une colonne de gel de silice pour donner 8,09 g de produit attendu.
RMN ¹H δ en ppm (DMSO d 6): -0,04(s,3H);-0,02(s,3H);0,82(s,9H);1,40-1,56 (massif,2H);3,18(m,1H);3,54(m,2H);3,76(s,3H);3,82(m,1H);3,90(s,3H);4,13(d,1H);4,2 6(d,1H);6,72-7,23(massif,9H).

### • Exemple 37.2 : N-[4-chloro-2-(2,6-difluorobenzyl)phényl]-N-(3-hydroxypropyl)-3,4-diméthoxy-benzènesulfonamide

A 8g de N-(3-{[tert-butyl(dimétyl)silyl]oxy}propyl)-N-[4-chloro-2-(2,6-difluorobenzyl) phényl]-3,4-diméthoxybenzènesulfonamide dans 80 ml de tétrahydrofurane à 0°^{c} on additionne 2,98 g de trihydrate de tétrabutylamonium fluorure. Après 40 minutes à 25°^{c}, le milieu réactionnel est concentré puis chromatographié sur une colonne de gel de silice en éluant au dichlorométhane pour obtenir 3,28 g de produit attendu.
F=138,30°^{c}
RMN ¹H δ en ppm (DMSO d 6): 1,35-1,55(massif,2H); 3,18(m,1H); 3,41(m,1H); 3,77(s,3H);3,85(m,2H);3,88(s,3H);4,13(d,1H);4,29(d,1H);4,48(t,1H);6,71-7,50(massif,9H)

### Exemple 38: N-[4-chloro-2-(2,6-difluorobenzyl)phényl]-N-(2-hydroxy-1-méthyléthyl)-3,4-diméthoxybenzénesulfonamide (composé 264)

A 1,1g de méthyl N-[4-chloro-2-(2,6-difluorobenzyl)phényl]-N-[(3,4-diméthoxyphényl) sulfonyl]alaninate dans 15 ml de tétrahydrofurane on additionne à température ambiante 158mg d'hydrure de lithium et d'aluminium. Après 5 heures à reflux, on revient à température ambiante et hydrolyse avec une solution à 15% d'hydroxyde de sodium. Le milieu est repris à l'acétate d'éthyle, lavé à l'eau, décanté et séché sur sulfate de sodium anhydre. La phase organique est concentrée, le résidu est chromatographié sur une colonne de gel de silice en éluant avec un mélange toluéne /acétate d'éthyle 90/10 (v/v) pour obtenir O,215g de produit attendu.
F=169,2°^{c}
RMN ¹H δ en ppm (DMSO d 6): 1,18(d,3H); 3,47(m,2H); 3,77(s,3H);3,88(s,3H);4,07-4,52(massif,3H);4,98(t,1H); 6,61-7,49(massif,9H)

### Exemple 39: 2-{[4-chloro-2-(2,6-difluorobenzyl)phényl][(3,4-diméthoxyphényl) sulfonyl]amino}-2-éthoxyacétamide (composé 276)

### • Exemple 39.1 : acétate d'éthyl{[4-chloro-2-(2,6-difluorobenzyl)phényl][(3,4-diméthoxyphényl) sulfonyl]amino(fluoro)

A 2g de N-[4-chloro-2-(2,6-difluorobenzyl) phényl]-3,4-diméthoxybenzènesulfonamide dans 15 ml de tétrahydrofurane on additionne successivement à température ambiante 0,742g de tert-butylate de potassium, 0,78ml d'acétate d'éthylbromo (fluoro) et laisse 18 heures à température ambiante. Pour compléter la réaction on additionne 0,742g de tert-butylate de potassium, et 0,6ml d'acétate d'éthylbromo(fluoro). Après 24 heures à température ambiante le milieu réactionnel est concentré, le résidu est repris à l'acétate d'éthyle et lavé à l'eau. La phase organique est séchée sur sulfate de sodium anhydre et concentrée. Le résidu est chromatographié sur une colonne de gel de silice en éluant avec un mélange toluène /acétate d'éthyle (100% en toluène à 80% toluène/20% acétate d'éthyle) pour donner 0,825 g de produit (pureté HPLC 77%)
RMN ¹H δ en ppm (DMSO d 6): 0,92(t,3H);3,78(s,3H);3,87(s,3H);4,12-4,38(massif,5H);6,73-7,33(massif,9H).

### • Exemple 39.2 : Acide 5-chloro-3-(2,6-difluorophényl)-1-[(3,4-diméthoxyphényl) sulfonyl]indoline-2-carboxylique

A 0,825g de éthyl{[4-chloro-2-(2,6-difluorobenzyl)phényl][(3,4-diméthoxyphényl) sulfonyl]amino(fluoro)acétate dans 10 ml de d'éthanol et 10 ml de 1,4-dioxane on additionne à température ambiante 0,062g d'hydrate d'hydroxyde de lithium, et laisse 6 heures à température ambiante. Le milieu réactionnel est concentré, le résidu est repris à l'acétate d'éthyle et avec une solutiom 1 M d'acide chlorhydrique. La phase organique est séchée sur sulfate de sodium anhydre et concentrée. Le résidu est chromatographié sur une colonne de gel de silice en éluant avec un mélange dichlorométhane/méthanol (100% en dichlorométhane à 90% dichlorométhane/10% méthanol) pour donner 0,345 g de produit (pureté HPLC 50%)
RMN ¹H δ en ppm (DMSO d 6): 3,74(s,3H);3,84(s,3H);4,17(d,1H) ;4,40(d,1H) ;5,57 (s,1H);6,49(s,1H);6,78-7,47(massif,8H).

### • Exemple 39.3 : 2-{[4-chloro-2-(2,6-difluorobenzyl)phényl][(3,4-diméthoxy phényl)sulfonyl]amino}-2-éthoxyacétamide

A 0,34g d' acide 5-chloro-3-(2,6-difluorophényl)-1-[(3,4-diméthoxyphényl) sulfonyl]indoline-2-carboxylique dans 10 ml de tétrahydrofurane on additionne successivement à 0°^{c} 90µl de N-éthylmorpholine, 68µl de chloroformate d'éthyle, laisse 30 minutes à cette température, et introduit 0,5ml d'une solution aqueuse 6M d'ammoniaque. Le milieu réactionnel est concentré, le résidu est repris à l'acétate d'éthyle et lavé à l'eau. La phase organique est séchée sur sulfate de sodium anhydre et concentrée. Le résidu est chromatographié sur une colonne de gel de silice en éluant avec un mélange dichlorométhane/méthanol (100% en dichlorométhane à 98% dichlorométhane/2% méthanol) pour donner 0,256 g de produit (pureté HPLC 99,6%)
F=153,6°C
RMN ¹H δ en ppm (DMSO d 6):1,25(t,3H); 3,57(m,2H); 3,84(s,3H); 3,87(s,3H); 4,31(q,2H); 6,53(s,1H);6,52-7,55(massif,11H)

### Exemple 40: phényl(2-{[4-chloro-2-(2,6-difluorobenzyl)phényl][(3,4-diméthoxyphényl) sulfonyl]amino} éthylcarbamate (composé 269)

A 1,4g d'éthyl N-[2,6-difluorobenzyl)-4-chlorophényl]-3,4-diméthoxy-N-[2-(méthylamino)éthyl]benzènesulfonamide dans 6 ml de tétrahydrofurane on additionne à 0°C successivement 0,29ml de pyridine, 0,37ml de phénylchloroformate et laisse 18 heures à température ambiante. Le milieu réactionnel est repris à l'acétate d'éthyle et lavé successivement avec une solution 1 M d'acide chlorhydrique, une solution d'hydrogénocarbonate de sodium, et à l'eau. La phase organique est séchée sur sulfaté de sodium anhydre et concentrée. Le résidu est chromatographié sur une colonne de gel de silice pour obtenir 1,07 g de produit attendu
F=137,5°^{c}
RMN ¹H δ en ppm (DMSO d6):3,05-3,24(massif, 4H);3,76(s,3H);3,87(s,3H);4,09(d,1H) ;4,30(d,1H);6,78-7,46(massif,14H);7,77(t,1H).

### Exemple 41: N-[4-chloro-2-(2,6-difluorobenzyl)phényl]-3,4-diméthoxy-N-(2-{[(méthyl amino)carbonyl]amino}éthyl)benzènesulfonamide (composé 253)

A 0;42g de phényl(2-{[4-chloro-2-(2,6-difluorobenzyl)phényl][(3,4-diméthoxyphényl) sulfonyl]amino}éthylcarbamate dans 1,2 ml de diméthylsulfoxyde on additionne à température ambiante 0,053ml d'une solution aqueuse de méthylamine et laisse 18 heures à température ambiante. Le milieu réactionnel est repris à l'acétate d'éthyle et lavé à l'eau. La phase organique est séchée sur sulfate de sodium anhydre et concentrée. Le résidu est chromatographié sur une colonne de gel de silice pour obtenir 0,326 g de produit attendu.
F=105,7°^{c}
RMN ¹H δ en ppm (DMSOd6):
2,48(s,3H);3,18(q,2H);3,24(m;1H);3,68(m,1H);3,76(s,3H);3,88(s,3H);4,20(q,2H) ;5,75-5,94(massif,2H);6,73-7,51 (massif,9H).

De la même façon, on prépare le composé 239, dont les propriétés physiques sont les suivantes :
MH+= 568 ; le temps de rétention est de 9,44 minutes
F=99,5°C

### Exemple 42: N- [4-chloro-2-(2,6-difluorobenzyl)phényl]-N-[2-(1H-imidazol-1-yl)éthyl]-3,4-diméthoxybenzènesulfonamide composé (271)

A 2ml de diméthylformamide oh additionne à température ambiante 51mg d'hydrure de sodium à 50% dans l'huile puis 72mg d'imidazole. Aprés 30 minutes à cette température, on introduit 0,4g d'éthyl N-(2-bromoéthyl)-N-[2-(2,6-difluorobenzyl)-4-chlorophényl]-3,4diméthoxybenzènesulfonamide en solution dans 5ml de diméthylformamide. Le milieu réactionnel est laissé 18 heures à température ambiante et repris à l'acétate d'éthyle et lavé à l'eau. La phase organique est séchée sur sulfate de sodium anhydre et concentrée. Le résidu est chromatographié sur une colonne de gel de silice en éluant avec un mélange toluène/acétate d'éthyle 97/3 (v/v) pour obtenir 0,082 g de produit attendu.
F=159,5°^{c}
RMN ¹H δ en ppm (DMSO d 6):3,66(m,1H);3,76(s,3H);3,89(s,3H);3,99-4,15(massif,5H);6,72-7,56(massif,12H).

De la même façon, on prépare le composé 301, dont les propriétés physiques sont les suivantes :
MH+= 544; le temps de rétention est de 6,62 minutes
F=153,6°C

### Exemple 43: N³-[4-chloro-2-(2,6-difluorobenzyl)phényl]-N³-[(3,4-diméthoxy phényl)sulfonyl]- β-alaninamide (composé 273)

### • Exemple 43.1 : N-[4-chloro-2-(2,6-difluorobenzyl)phényl]-N-[(3,4-diméthoxyphényl)sulfonyl]- β-alanine

A 2,63g dé N-[4-chloro-2-(2,6-difluorobenzyl)phényl]-N-(3-hydroxypropyl)-3,4-diméthoxybenzènesulfonamide (exemple 37) en solution dans 35ml d'acétonitrile on additionne à température ambiante 0,069g de trichlorure de ruthénium, 1,65g de périodate de sodium, 2,7ml d'eau. On laisse 10 heures à température ambiante. Le milieu réactionnel est filtré sur talc, concentré. Le résidu est repris au dichlorométhane et lavé avec une solution 1 M d'acide chlorhydrique. La phase organique est séchée sur sulfate de sodium anhydre est concentrée pour obtenir 3,13g de produit attendu.
RMN ¹H δ en ppm (DMSO d6):
2,3(m,2H);3,45(m,1H);3,71(s,3H);3,86(m,1H);3,90(s,3H);4,1-4,35(massif,2H);6,74-7,47(massif,9H);12,3(s,1H).

### • Exemple 43.2 : N³-[4-chloro-2-(2,6-difluorobenzyl)phényl]-N³-[(3,4-diméthoxyphényl)sulfonyl]- β-alaninamide

A 0,8g de N-[4-chloro-2-(2,6-difluorobenzyl)phényl]-N-[(3,4-diméthoxyphényl) sulfonyl]- β-alanine en solution dans 16ml de tétrahydrofurane on additionne à 0°C 0,21ml de N-éthylmorpholine, 0,16ml de chloroformate d'éthyle. On laisse 1 heure à température ambiante, puis introduit 1,36ml d'ammoniaque (20%) dans l'eau. Après 48 heures à température ambiante le milieu réactionnel est repris à l'acétate d'éthyle et lavé à l'eau. La phase organique est séchée sur sulfate de sodium anhydre et concentrée, le résidu est chromatographié sur une colonne de gel de silice en éluant avec un mélange dichlorométhane/méthanol 98/2 (v/v) pour obtenir 0,295g de produit attendu.
F=189,4°C
RMN ¹H δ en ppm (DMSO d 6): 2,08-2,25(massif,2H); 3,42(m,1H); 3,76(s,3H); 3,88(s,3H) 3,92(m,1H);;4,13(d,1H);4,27(d,1H); 6,75-7,51 (massif,11H)

De la même façon, on prépare le composé 298, dont les propriétés physiques sont les suivantes :
MH+= 539; le temps de rétention est de 9,15 minutes
F= 189,3 °C

### Exemple 44: N²-[4-chloro-2-(2,6-difluorobenzyl)phényl]-N²-[(3,4-diméthoxy phényl)sulfonyl]- β-alaninamide (composé 261)

### • Exemple 44.1: méthyl N-[4-chloro-2-(2,6-difluorobenzyl)phényl]-N-[(3,4-diméthoxy phényl)sulfonyl]alaninate

Par réaction de 2g de N-[4-chloro-2-(2,6-difluorobenzyl)phényl]-3,4-diméthoxy benzènesulfonamide avec du méthyl lactate selon le procédé 35 on obtient 1,393g de produit attendu.
RMN ¹H δ en ppm (DMSO d 6):1,12(t,3H); 1,25(d,3H); 3,75(s,3H); 3,86(s,3H); 3,98(d,1H) ;4,19 (q,2H);4,65(d, 1H); 4,87(q, 1H); 6,55(s, 1H)7,04-7,48(massif,8H).

### • Exemple 44.2: N-[4-chloro-2-(2,6-difluorobenzyl)phényl]-N-[(3,4-diméthoxy phényl)sulfonyl]alanine

A 1,373g de méthyl N-[4-chloro-2-(2,6-difluorobenzyl)phényl]-N-[(3,4-diméthoxy phényl)sulfonyl]alaninate en solution dans 20 ml d'éthanol on additionne 0,52 g d'hydroxyde de lithium monohydrate et laisse 18 heures à température ambiante. Le milieu est concentré, repris à l'acétate d'éthyle et lavé avec une solution à 5% d'hydrogénosulfate de potassium. La phase organique est séchée sur sulfate de sodium anhydre et concentrée. Le résidu est chromatographié sur une colonne de gel de silice en éluant avec un mélange dichlorométhane/méthanol 90/10 (v/v) pour obtenir 0,494g de produit attendu.
RMN ¹H δ en ppm (DMSO d 6): 1,17(d,3H); 3,76(s,3H); 3,87(s,3H); 3,92(d,1H) ;4,19 (d,1H); 4,75(q,1H); 6,53-7,52(massif,9H); 12,9(s,1H).

### • Exemple 44.3 : N²-[4-chloro-2-(2,6-difluorobenzyl)phényl]-N²-[(3,4-diméthoxy phényl)sulfonyl]- β-alaninamidé

A 0,47g de N-[4-chloro-2-(2,6-difluorobenzyl)phényl]-N-[(3,4-diméthoxyphényl) sulfonyl]alanine en solution dans 20ml de tétrahydrofurane on additionne à 0°C 0,13ml de N-éthyl morpholine, 0,094ml de chloroformate d'éthyle. On laisse 1 heure à température ambiante, puis introduit 0é75ml d'ammoniaque (20%) dans l'eau. Après 24 heures à température ambiante le milieu réactionnel est repris à l'acétate d'éthyle et lavé à l'eau. La phase organique est séchée sur sulfate de sodium anhydre et concentrée, le résidu est chromatographié sur une colonne de gel de silice en éluant avec un mélange dichlorométhane/méthanol 95/5 (v/v) pour obtenir 0,295g de produit attendu.
F=108°C
RMN ¹H δ en ppm (DMSO d 6): 1,04(d,3H); 3,77(s,3H); 3,84(s,3H); 3,93(d,1H) ;4,19 (d,1H); 4,85(q,1H); 4,89 (d,1H); 6,53-7,52(massif,9H).

Les énantioméres du composé 261 sont séparés par chromatographie chirale Enantiomére lévogyre composé 305
[α]_{D} (C=0,5g/100ml dans le méthanol) = -28,2
Enantiomére dextrogyre composé 304
[α]_{D} (C=0,5g/100ml dans le méthanol) = +25,4

Le tableau XXV illustre les structures chimiques et les propriétés physiques de quelques composés de l'invention obtenus selon cet exemple.

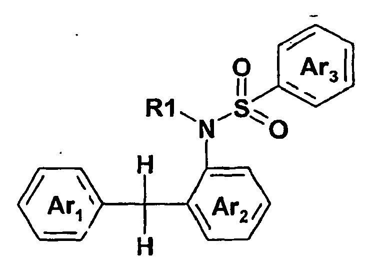

**Tableau XXV**

| **N° composé** | **Nature et position des substituants** | | | | **F(°C)** | **MH⁺/temps de rétention** |
|---|---|---|---|---|---|---|
| | **Ar₁** | **Ar₂** | **R₁** | **Ar₃** | | |
| 304 | 2,6-diF | 4-Cl | CH(CH₃)CONH₂ | 3,4-diOMe | 105,3 | 525/9,29 |
| 305 | 2,6-diF | 4-Cl | CH(CH₃)CONH₂ | 3,4-diOMe | 94,1 | 525/9,29 |

### Exemple 45: N²-[2-(2-chlorobenzyl)-4-(diméthylamino)phényl]-N²-[(3,4-diméthoxy, phényl)sulfonyl]glycinamide (composé 265)

### • Exemple 45.1: N²-[4-amino-2-(2-chlorobenzyl)phény]-N²-[(3,4-diméthoxyphényl)sulfonyl]glycinamide

A 2g de N²-[2(2-chlorobenzyl)-4-nitrophényl]-N²-[(3,4-diméthoxyphényl)sulfonyl] glycinamide obtenu selon l'exemple 15 en solution dans 150 ml d'éthanol on additionne successivement à température ambiante, 0,91g d'étain, 3,2ml d'acide chlorhydrique 12M. Après 18 heures, le milieu est concentré, repris à l'acétate d'éthyle et avec une solution d'hydroxyde de sodium (PH14). La phase organique est séchée sur sulfate de sodium anhydre et concentrée. Le résidu est chromatographié sur une colonne de gel de silice en éluant avec un mélange dichlorométhane/méthanol 95/5 (v/v) pour obtenir 0,287g de produit attendu.
RMN ¹H δ en ppm (DMSO d 6):3,79(s,3H);3,88(s,3H);3,94-4,40(massif,4H);5,15(s,2H );5,94(d,1H);6,22(d, 1H);6,50(d,1H);7,04-7,49(massif,9H).

### • Exemple 45.2 : N²-[2-(2-chlorobenzyl)-4-(diméthylamino)phényl]-N²-[(3,4-diméthoxyphényl)sulfonyl] glycinamide

A 0,6g de N²-[4-amino-2-(2-chlorobenzyl)phényl]-N²-[(3,4-diméthoxyphényl)sulfonyl] glycinamide en solution dans la N-méthylpyrrolidine on additionne 0,42 g de carbonate de caesium, 0,9ml de iodométhane et laisse 18 heures à température ambiante. Le milieu est repris à l'acétate d'éthyle et lavé avec à l'eau. La phase organique est séchée sur sulfate de sodium anhydre et concentrée. Le résidu est chromatographié sur une colonne de gel de silice en éluant avec un gradiant dichlorométhane/acétone 100 % à 90%/10% pour obtenir le produit attendu.
F=130,4°C
RMN ¹H δ en ppm (DMSO d 6):2,73(s,6H);3,78(s,3H);3,84(s,3H);3,98-4,08(massif,3H);4,40(d,1H);6,04(d, 1H);6,43(d, 1H);6,70(d, 1H);7,02-7,51 (massif,9H).

### Exemple 46: Ethyl N-[4-chloro-2-(2-chlorophényl]-3,4-diméthoxy-N-vinyl benzènesulfonamide (composé 289)

A 0,18g de 1-méthyl-2-imidazolidone en solution dans la N-méthylpyrrolidine on additionne 0,086g d'hydrure de sodium à 50% dans l'huile, on laisse 15 minutes à température ambiante et introduit 1g d'éthyl N-(2-bromoéthyl)-N-[2-(2,6-difluorobenzyl)-4-chlorophényl]-3,4-diméthoxybenzènesulfonamide. Après 18 heures le milieu réactionnel et repris à l'acétate d'éthyle et lavé à l'eau. La phase organique est séchée sur sulfate de sodium anhydre et concentrée, le résidu est chromatographié sur une colonne de gel de silice en éluant au dichlorométhane pour obtenir 0,67g de produit attendu.
F=153°C
RMN ¹H δ en ppm (DMSO d. 6):3,54(d,1H);3,79(s,3H);3,89(s,3H);3,94-4,20(massif,2H);4,22(d,1H);6,50(d,1H);7,06-7,45(massif,9H).

### Exemple 47: N²-[2-(2,6-difluorobenzyl)-5-méthoxyphényl]-N²-[(3,4-diméthoxyphényl) Sulfonyl]glycinamide (composé 296)

### • Exemple 47.1 : (2-amino-4-méthoxyphényl)(2,6-difluorophényl)méthane

A 6,22g de (2-amino-4-méthoxyphényl)(2,6-difluorophényl) méthanol obtenu selon l'exemple 11.1 en solution dans 95 ml de dichlorométhane on additionne successivement à température ambiante, 11,7ml de triéthylsilane, 10,7ml d'acide trifluoroacétique. Après 4 heures à reflux, le milieu est hydrolysé par une solution 6M d'hydroxyde de sodium. La phase organique est séchée sur sulfate de sodium anhydre et concentrée. Le résidu est chromatographié sur une colonne de gel de silice en éluant au dichlorométhane pour obtenir 1,505g de produit attendu.
RMN ¹H δ en ppm (DMSO d 6):
3,69(s,3H);4,93(s,2H);6,04(d,1H);6,21(s,1H);6,48(d,1H);7,01-7,35(massif,3H).

### • Exemple 47.2 : N-[2-(2,6-difluorobenzyl)-5-méthoxyphényl]-3,4-diméthoxy benzènesulfonamide

A partir de 1,5g de (2-amino-4-méthoxyphényl)(2,6-difluorophényl) méthane, selon l'exemple 12.2 on obtient 0,32g de produit attendu.
RMN ¹H δ en ppm (DMSO d6):
3,55(s,3H);3,74(s,3H);3,82(s,3H);3,85(d,2H);6,42(d,1H);6,56-7,42 (massif, 8H); 9, 54(s, 1H).

### • Exemple 47.3: N²-[2-(2,6-difluorobenzyl)-5-méthoxyphényl]-N²-[(3,4-diméthoxyphényl)sulfonyl]glycinamide

A partir de 0,312g de N-[2-(2,6-difluorobenzyl)-5-méthoxyphényl]-3,4-diméthoxy benzènesulfonamide, selon l'exemple 12.3 on obtient 0,219g de produit attendu.
F=188,2°C
RMN ¹H δ en ppm (DMSO d 6):3,55(s,3H);3,78(s,3H);3,88(s,3H);4,10-4,24 (massif, 3H);4,43(d,1H);6,41 (d, 1H);6,57(d, 1H);6,83(d, 1H);7,05-7,48(massif,8H).

Le tableau XXVI illustre les structures chimiques et les propriétés physiques de quelques composés de l'invention obtenus selon cet exemple.

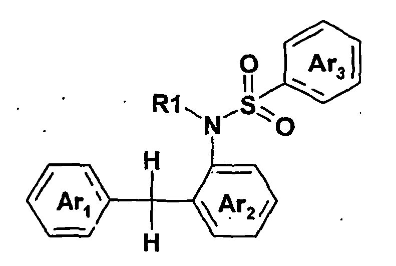

**Tableau XXVI**

| **N° composé** | **Nature et position des substituants** | | | | **F(°C)** | **MH⁺-/temps de rétention** |
|---|---|---|---|---|---|---|
| | **Ar₁** | **Ar₂** | **. R₁** | **Ar₃** | | |
| 302 | 2,6-diF | 4-Cl,6-OMe | CH₂CONH₂ | 3,4-diOMe | 238,5 | 541/8,89 |
| 303 | 2,6-diF | 4-Cl,6-Me | CH₂CONH₂ | 3,4-diOMe | 217 | 521/8,61 |

### Exemple 48: N-[4-chloro-2-(2,6-difluorobenzyl)phényl]-3,4-diméthoxy-N-{2-[(méthyl sulfonyl)amino]éthyl}benzènesulfonamide (composé 313)

A 0,543g du composé 256 en solution dans 10ml de tétrahydrofurane on additionne 106µl de pyridine, 102µl de chlorure de méthane sulfonyle et laisse 18 heures à température ambiante. Le milieu réactionnel est concentrée, le résidu est chromatographié sur une colonne de gel de silice en éluant avec un mélange dichlorométhane/méthanol pour obtenir 0,425g de produit attendu.
F=133,2°C
RMN ¹H δ en ppm (DMSO d 6):2,85(s,3H),2,90(m,1H);3,12(m,1H);3,35(m,1H); ;3,78(s,3H);3,83(m,1H);3,86 (s,3H);4,09(d,1H);4,31(d,1H);6,78-7,51(massif,10H).

### Exemple 49: N²-[2-(2,6-difluorobenzyl)-6-méthoxyphényl]-N²-[(3,4-diméthioxy phényl)sulfonyl]-N-éthylglycinamide (composé 309)

### • Exemple 49.1 : N-[2-(2,6-difluorobenzyl)-6-méthoxyphényl]-N-[(3,4-diméthoxy phényl)sulfonyl]glycine

A 1,8g de éthyl N-[2-(2,6-difluorobenzyl)-6-méthoxyphényl]-N-[(3,4-diméthoxyphényl) sulfonyl]glycinate en solution dans 50 ml d'éthanol on additionne 10ml d'une solution 2M d'hydroxyde de sodium. Après 18 heures à température ambiante, le milieu est extrait à l'éther diéthylique, la phase aqueuse est acidifiée est extraite au dichlorométhane. La phase organique est séchée sur sulfate de sodium anhydre, puis concentrée pour obtenir 1,8g de produit attendu.
RMN ¹H δ en ppm (DMSO d 6):3,18(s,3H);3,72(s,3H);3,82(d,1H);3,90(s,3H) ;4,29(d,1H);4,53(d,1H);5,01(d,1H);6,26(d,1H);6,78(d,1H);7,01-7,47(massif,7H);12,75(s,1H).

### • Exemple 49.2: N²-[2-(2,6-difluorobenzyl)-6,méthoxyphényl],N²-[(3,4-diméthoxyphényl)sulfonyl]-N-éthylglycinamide

A 1,8g de N-[2-(2,6-difluorobenzyl)-6-méthoxyphényl]-N-[(3,4-diméthoxyphényl) sulfonyl]glycine en solution dans 30ml de tétrahydrofurane à 0°C, on introduit 0,5ml de N-éthylmorpholine, 0,38ml de chloroformate d'éthyle. Après 15 minutes à 10°C, on additionne 0,8g d'éthylamine en solution dans du tétrahydrofurane et laisse 30 minutes à température ambiante. Le milieu est repris à l'acétate d'éthyle, lavé à l'eau, la phase organique est séchée sur sulfate de sodium anhydre et concentrée pour obtenir 1,4g de produit attendu.
F=180°C
RMN ¹H δ en ppm (DMSO d 6):0,98(t,3H),3,07(m,2H);3,26(s,3H);3,76(s,3H) ;3,79(m,1H);3,87(s,3H);4,26(d,1H);4,43(d,1H);4,94(d,1H);6,20(d,1H);6,81(d,1H);7,09-7,45(massif,7H);7,88(t,1H).

### Exemple 50: N²-[4-chloro-2-(2,5-difluorobenzyl)phényl]-N²-[(4-chloro-2,5-diméthyl phényl)sulfonyl]glycinamide (composé 267)

### • Exemple 50.1 : (2-nitro-5-chlorophényl)(2,5-diflulorophényl)méthanol

A 15g de 2,5-difluorobenzène en solution dans 150ml de tétrahydrofurane on additionne à ⁻70°c 50ml d'une solution 1,6M de butyllithium. Après 2 heures à ⁻70°c, on introduit 9,616g de 2-nitro,5-chlorobenzaldehyde et abandonne 3 heures à cette température, puis 18 heures à température ambiante le milieu est hydrolysé avec une solution de chlorure d'ammonium et extrait à l'acétate d'éthyle, La phase organique est séchée sur sulfate de sodium anhydre et concentrée. Le résidu est filtré sur silice en éluant au dichlorométhane pour obtenir 8,89g de produit attendu.

### • Exemple 50.2 : (2-amino-5-chlorophényl)(2,5-diflulorophényl)méthanol

A partir de 8,55g de (2-nitro-5-chlorophényl)(2,5-diflulorophényl)méthanol selon le procédé 8.2 on obtient 2,7g de produit attendu
RMN ¹H δ en ppm (DMSO d 6): 5,18 (s,2H);5,89(d,1H);6,15(d,1H);6,68-7,36(massif,6H)

### o Exemple 50.3 : (2-amino-5-chlorophényl)(2,5-diflulorophényl)méthane

A partir de 2,7g de (2-amino-5-chlorophényl)(2,5-diflulorophényl)méthanol selon le procédé 18.1 on obtient 1.947g de produit attendu
RMN⁻¹H δ en ppm (DMSO d 6): 3,80(s,2H);5,18(s,2H);6,60(massif,6H)

### o Exemple 50.4 : N-[4-chloro-2-(2,5-difluorobenzyl)phényl]-2,5-diméthyl-4-chloro benzènesulfonamide

A partir de 0,484g de (2-amino-5-chlorophényl)(2,5-diflulorophényl)méthane selon le procédé décrit à l'exemple 12.2 on obtient 0,837g de produit attendu
RMN ¹H δ en ppm (DMSO d 6):2,28(s,3H);2,45(s,3H);3,98(s,2H);6,60(m,1H);6,94-7,61 (massif,8H),9, 93(s,1H)

### o Exemple 50.5: N²-[4-chloro-2-(2,5-difluorobenzyl)phényl]-N²-[(4-chloro-2,5-diméthylphényl)sulfonyl]glycinamide

A partir de 0,83g de N-[4-chloro-2-(2,5-difluorobenzyl)phényl]-2,5-diméthyl-4-chloro benzènesulfonamide selon l'exemple 15 on obtient 0,424g de produit attendu RMN ¹H δ en ppm (DMSO d 6):
2,16(s,3H);2,36(s,3H);3,92(d,1H);4,23(d,1H);4,40(d,1H);6,87(s,1H)6,96-7,75(massif,8H)
F=169,7°C

### Exemple 51: N²-[4-chloro-2-(pyridin-2-ylméthyl)phényl]-N²-[(3,4-diméthoxy phényl)sulfonyl]glycinamide

### o Exemple 51.1 : 2-(5-chloro-2-nitroberoyl)pyridine

A 22,44g de tert-butylate de potassium dans 500ml de diméthylsulfoxyde on additionne lentement 8,66g de 4-chloronitrobenzène et 8,2g de 2clorométhylpyridine en solution dans 100ml de diméthylsulfoxyde. Après 18 heures à température ambiante on hydrolyse avec une solution saturée de chlorure d'ammonium, et extrait trois fois au dichlorométhane. La phase organique est séchée sur sulfate de sodium anhydre et concentrée. Le résidu est filtré sur silice H éluant dichlorométhane pour obtenir 10,695g de produit attendu
RMN ¹H δ en ppm (DMSO d 6): 4,49(s,2H);7,20-7,31(massif,2H);7,60-7,78(masif, 3H); 8,03(d,1H);8,41 (d,1H)
F=69°C

• Exemple 51.2 : 4-chloro-2-(pyridin-2-ylméthyl)aniline

A 5g de 2-(5-chloro-2-nitrobenzyl)pyridine selon l'exemple 8.2 on obtient 3,86g de produit attendu
RMN ¹H δ en ppm (DMSO d 6): 3,93(s,2H);5,33(s,2H);6,66(d,1H);6,93-7,06(massif,2H);7,21-7,38(masif,2H);7,76(m, 1H);8,47(d,1H)

### • Exemple 51.3: N-[4-chloro-2-(pyridin-2-ylméthyl)phényl]-3,4-diméthoxy benzènesulfonamide

A 1,86g de 4-chloro-2-(pyridin-2-ylméthyl)aniline selon l'exemple 12.2 on obtient 2,12g de produit attendu
RMN ¹H δ en ppm (DMSO d 6): 3,71(s,3H);3,83(s,3H); 3,94(s,2H); 7,07-7,32(massif,8H); 7,74(m,1H);8,54(d,1H)

### • Exemple 51.4: N²-[4-chloro-2-(pyridin-2-ylméthyl)phényl]-N²-[(3,4-di méthoxylphényl)sulfonyl]glycinamide

A 0,5g de N-[4-chloro-2-(pyridin-2-ylméthyl)phényl]-3,4-diméthoxy benzène sulfonamide selon l'exemple 12.3 on obtient 0,257g de produit attendu RMN ¹H δ en ppm (DMSO d 6): 3,79(s,3H);3,88(s,3H);4,03-4,48(massif,4H)); 6,93-7,37(massif,10H); 7,74(t,1H); 8,54(d,1H)
F=88°C

### Exemple 52: N²-[2-(2,6-difluorobenzyl)-6-méthoxyphényl]-N²-[(3,4-diméthoxy phényl)sulfonyl]-(R)-alaninamide (composé 321)

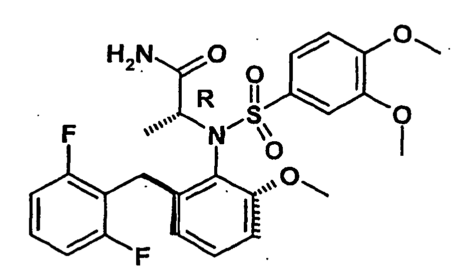

### composé 321

### [α_{D}]²⁰= +14,7 c=0,19(CH₂Cl₂)

### • Exemple 52.1 : N-[2-(2,6-difluorobenzyl)-6-méthoxyphényl]-N-[(3,4-diméthoxyphényl)sulfonyl]-(R)-alanine

A 0,164g de méthyl N-[2-(2,6-difluorobenzyl)-6-méthoxyphényl]-N-[(3,4-diméthoxyphényl)sulfonyl]-(R)-alaninate en solution dans 8ml d'un mélange1,4-dioxane/eau (4/1) on additionne à température ambiante. 0,015g d'hydroxyde de lithium monohydrate. On laisse 48 heures à température ambiante. Le milieu réactionnel est lavé avec une solution 1 M d'acide chlorhydrique, et extrait à l'acétate d'éthyle. La phase organique est séchée sur sulfate de sodium anhydre et concentrée. Le résidu est chromatographié sur une colonne de gel de silice en éluant avec un mélange dichlorométhane/éthanol pour obtenir 0,08g du produit attendu RMN ¹H δ en ppm (DMSO d6):1,64(d,3H);3,22(s,3H);3,70(s,3H);3,85(s,3H) ;4,11 (d,1H); 4,22(q,1H);4,77(d,1H);6,31 (d,1H);6,78(d,1H);7,02-7,44(massif,7H),12,5(s,1H).

### • Exemple 52.2: N²-[2-(2,6-difluorobenzyl)-6-méthoxyphényl]-N²-[(3,4-diméthoxyphényl)sulfonyl]-(R)-alaninamide

A partir de 0,098g de N-[2-(2,6-difluorobenzyl)-6-méthoxyphényl]-N-[(3,4-diméthoxyphényl)sulfonyl]-(R)-alanine selon l'exemple 43.2 on obtient 0,08g de produit attendu
RMN ¹H δ en ppm (DMSO d6):1,34(d,3H);3,32(s,3H);3,74(s,3H);3,85(s,3H) ;4,22 (d,2H);4,41 (q,1H); 6,27(d, 1H);6,88(d,1H);7,07-7,44(massif,9H).

Suivant ce procédé on synthètise le N²-[2-(2,6-difluorobenzyl)-6-méthoxyphényl]-N²-[(3,4-diméthoxyphényl)sulfonyl] -(S)-alaninamide (composé 322).
RMN ¹H δ en ppm (DMSO d6):1,34(d,3H);3,32(s,3H);3,74(s,3H);3,85(s,3H);4,22 (d,2H);4,41 (q,1H); 6,27(d,1H);6,88(d,1H);7,07-7,44(massif;9H).

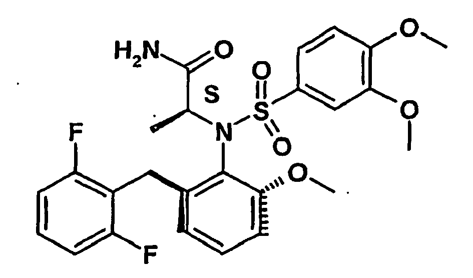

### composé 322

### [α_{D}]²⁰= -14,6 c=0,15(CH₂Cl₂)

### Exemple 53: N²-[2-méthoxy-6-(2-phényléthyl)phényl]-N²-[(3,4-diméthoxy phényl)sulfonyl]glycinamide (composé 323)

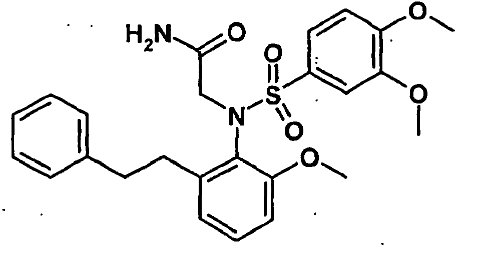

### composé 323

### Exemple 53.1 : 1-méthoxy-2-nitro-3-[(E)-2-phénylvinyl]benzène

A 10 ml d'éthanol on additionne à température ambiante 0,31g de sodium. On laisse 15 minutes à température ambiante et introduit successivement 4,05g de chlorure de benzyl triphényl phosphonium et 2g de 2-nitro 3-méthoxy benzaldéhyde Après 18 heures à température ambiante, le milieu réactionnel est repris au dichlorométhane et lavé à l'eau. La phase organique est séchée sur sulfate de sodium anhydre et concentrée. Le résidu est chromatographié sur une colonne de gel de silice pour obtenir 2,254g du produit attendu.
RMN ¹H δ en ppm (DMSO d6):3,92(s,3H) ; 6,55-7,56(massif,10H).

### • Exemple 53.2 : 2-méthoxy-6-(2-phényléthyl)aniline

2,25g de 1-méthoxy-2-nitro-3-[(E)-2-phénylvinyl]benzène sont additionnés sur 0,18g de palladium sur charbon (10%) en suspension dans 90 ml de méthanol et portés sous 4 bars d'hydrogéne. Après 18 heures on filtre le milieu réactionnel sur talc et concentre le filtrat pour obtenir 2,115g de produit attendu.
RMN ¹H δ en ppm (DMSO d6):2,74-2,88 (massif,4H);3,78(s,3H); 4,55(s,2H);6,55-7,31(massif,8H).

### • Exemple 53.3 : N-[2-méthoxy-6-(2-phényléthyl)phényl](3,4-diméthoxy phényl)sulfonamide

A partir de 2,11g de 2-méthoxy-6-(2-phényléthyl)aniline selon l'exemple 12.2 on obtient 0,5g de produit attendu.
RMN ¹H δ en ppm (DMSO d6): 2,83(m,2H),3,01 (m,2H); 3,22(s,3H); 3,73 (s,3H) ;3,84(s,3H);6,71-7,33(massif,11H);8,99(s,1H).

### • Exemple 53.4: N²-[2-méthoxy-6-(2-phényléthyl)phényl]-N'-[(3,4-diméthoxy phényl)sulfonyl]glycinamide

A partir de 0,5g de N-[2-méthoxy-6-(2-phényléthyl)phényl](3,4-diméthoxy phényl)sulfonamideselon l'exemple 12.3 on obtient 0,187 g de produit attendu.
RMN ¹H δ en ppm (DMSO d6): 2,83(m,3H),3,26(s,3H);3,40(m,1H); 3,73 (s,3H) ;3,84(s,3H);6,76(d,1H);7,03-7,36(massif,12H).
F=243, 9° C

Les composés de l'invention ont fait l'objet d'études pharmacologiques qui ont montré leur intérêt comme substances actives en thérapeutique.

Ils ont en particulier été testés quant à leurs effets. Plus particulièrement, l'affinité des composés de l'invention pour les récepteurs 2 de l'orexine a été déterminée dans un test de liaison *in vitro* selon la technique décrite ci-dessous. Cette méthode consiste à étudier le déplacement de l'orexine A radioiodée fixée aux récepteurs de l'orexine 2 humains exprimés dans des cellules CHO. Le test s'effectue sur membranes dans un tampon d'incubation Hépès 50 mM, MgCl₂ 1 mM, CaCl₂ 25 mM, NaN₃ 0,025 %, le sérum albumine bovin (BSA) 1 % et 100 pM de ligand pendant 30 minutes à 25°C. La réaction est arrêtée par filtration et lavage sur filtre Wathman GF/C. La liaison non , spécifique est mesurée en présence de 10⁻⁶M d'orexine B humaine. Les CI₅₀ (concentration inhibitrice de 50% de la liaison de l'orexine A radioiodée à ses récepteurs) sont faibles, inférieures à 300 nM, en particulier inférieures à 100 nM et plus particulièrement inférieures à 30 nM.

L'affinité des composés selon l'invention pour les récepteurs 1 de l'orexine a également été étudiée dans un test de liaison *in vitro* selon la même technique en utilisant l'orexine A radio iodée comme ligand dans une préparation membranaire de cellules CHO exprimant les récepteurs orexine 1 humains. Les composés selon l'invention sont peu ou pas affins pour les récepteurs orexine 1.

Le caractère agoniste ou antagoniste des composés est déterminé *in vitro* dans un test de mesure de calcium intracellulaire (FLIPR) sur une préparation cellulaire exprimant les récepteurs 2 de l'orexine selon la technique générale décrite dans Sullivan et al, Methods Mol. Biol., 1999, vol. 114, 125-133, en utilisant 1 µM de Fluo-4 AM en tant qu'indicateur fluorescent de calcium. Pour le test antagoniste les composés sont préincubés 30 minutes avant ajout de 0,25 nM d'orexine B. Les CI₅₀ pour les récepteurs orexine 2 mesurées dans ces études sont faibles et plus particulièrement inférieures à 100 nM.

Le tableau suivant illustre l'affinité de quelques composés selon l'invention pour les précepteurs de l'orexine dans un test de liaison *in nitro* selon la technique décrite ci-dessus ainsi que leur caractère antagoniste déterminé *in vitro* dans un test de mesure de calcium intracellulaire (FLIPR) selon la technique générale mentionnée ci-dessus.

| N° composé | Cl₅₀ OX 2 (nM) | Cl₅₀ OX 1 (nM) | Mesure de [Ca²⁺]i Cl₅₀ OX 2 (nM) - FLIPR |
|---|---|---|---|
| 1 | 13 | 446 | 5 |
| 28 | 62 | 1500 | 19 |
| 30 | 82 | 966 | 26. |
| 44 | 85 | > 10 µM | 54 |
| 107 | 20 | 899 | 11 |
| 124 | 50 | > 10 µM | 65 |
| 137 | 19 | > 10 µM | 10 |
| 138 | 8 | 4760 | 11 |
| 202 | 9 | 1870 | 7 |

Les résultats biologiques montrent que les composés selon l'invention sont bien des antagonistes spécifiques des récepteurs orexine 2.

Ainsi, les composés de la présente invention, en tant qu'antagonistes des récepteurs orexine 2, peuvent être utilisés dans la prophylaxie et le traitement de toutes maladies impliquant un dysfonctionnement lié à ces récepteurs.

Les composés de l'invention peuvent être utilisés pour la préparation d'un médicament destiné à la prophylaxie ou au traitement de toutes maladies impliquant un dysfonctionnement lié au récepteur orexine 2, et plus particulièrement dans la prophylaxie ou le traitement des pathologies dans lesquelles un antagoniste de récepteur orexine 2 apporte un bénéfice thérapeutique. De telles pathologies sont par exemple l'obésité, les perturbations de l'appétit ou du goût dont la cachexie, l'anorexie, la boulimie (Smart et al., Eur. J. Pharmacol., 2002, 440, 2-3, 199-212), le diabète (Ouedraogo et al., Diabètes, 2002, 52, 111-117), les syndromes métaboliques (Sakurai, Curr. Opin. Nutr. Metab. Care, 2003, 6, 353-360), les vomissements et la nausée (US 6, 506, 774), la dépression et l'anxiété (Salomon et al., Biol. Psychiatry, 2003, 54, 96-104 ; Jaszberenyi et al., J. Neuroendocrinol., 2000, 12, 1174-1178), les addictions (Georgescu et al., J. Neurosci., 2003, 23, 8, 3106-3111 ; Kane et al., Endocrinology, 2000, 141, 10, 3623-3629), les troubles de l'humeur et du comportement, la schizophrénie (Nishino et al., Psychiatry Res., 2002, 110, 1-7), les troubles du sommeil (Sakurai, Neuroreport, 2002, 13, 8, 987-995), la maladie des jambes sans repos (Allen et al., Neurology, 2002, 59, 4, 639-641), les troubles de l'apprentissage de la mémoire (van den Pol et al., 2002, J. Physiol., 541(1), 169-185 jauger et al., Peptides, 2003, 23, 1683-1688 ; Telegdy etc Adamik, Regul. Pept., 2002, 104, 105-110), les dysfonctions sexuelles et psychosexuelles (Gulia et al., Neuroscience, 2003, 116, 921-923), la douleur, la douleur viscérale ou neuropathique, l'hyperalgésie, l'allodynie (US 6,506,774 ; Suyama et al., in vivo, 2004, 18, 2, 119-123); les troubles digestifs (Takakashi et al., Biochem. Biophy. Res. Comm ., 1999, 254, 623-627 ; Matsuo et al., Eur. J. Pharmacol., 2002, 105-109), le syndrome des intestins irrités (US 6,506,774), la dégénérescence neuronale (van den Pol, Neuron, 2000, 27, 415-418), les attaques ischémiques ou hémorrhagiques (Irving et al., Neurosci. Lett., 2002, 324, 53-56), la maladie de Cushing, le syndrome de Guillain-Barré (Kanbayashi et al., Psychiatry Clin. Neurosci., 2002, 56, 3, 273-274), la dystrophie myotonique (Martinez-Rodriguez et al., Sleep, 2003, 26, 3, 287-290), l'incontinence urinaire (Blackstone et al., AGS Annual Meeting, poster P491,2002), l'hyperthyroïdie (Malendowicz et al., Biomed. Res., 2001, 22, 5, 229-233), les troubles de la fonction hypophysaire (Voisin et al., Cell. Mol. Life Sci., 2003, 60, 72-78), l'hypertension ou l'hypotension (Samson et al, Brain Res., 1999, 831, 1-2, 248-253).

L'utilisation des composés selon l'invention pour la préparation d'un médicament destiné à prévenir ou à traiter les pathologies ci-dessus mentionnées, fait partie intégrante de l'invention.

L'invention a également pour objet des médicaments qui comprennent un composé de formule (I). Ces médicaments trouvent leur emploi en thérapeutique, notamment dans la prophylaxie ou le traitement des pathologies ci-dessus mentionnées.

Selon un autre de ses aspects, la présente invention concerne des compositions pharmaceutiques renfermant en tant que principe actif, au moins un composé selon l'invention. Ces compositions pharmaceutiques contiennent une dose efficace d'un composé selon l'invention et éventuellement un ou plusieurs excipients pharmaceutiquement acceptables.
Lesdits excipients sont choisis selon la forme pharmaceutique et le mode d'administration souhaité, parmi les excipients habituels qui sont connus de l'homme du métier.

Dans les compositions pharmaceutiques de la présente invention pour l'administration orale, sublinguale, sous-cutanée, intramusculaire, intra-veineuse, topique, locale, intratrachéale, intranasale, transdermique ou rectale, le principe actif de formule (I) ci-dessus, ou son sel, solvat ou hydrate éventuel, peut être administré sous forme unitaire d'administration, en mélange avec des excipients pharmaceutiques classiques, aux animaux et aux êtres humains pour la prophylaxie ou le traitement des troubles ou des maladies ci-dessus.

Les formes unitaires d'administration appropriées comprennent les formes par voie orale telles que les comprimés, les gélules molles ou dures, les poudres, les granules, les chewing-gums et les solution ou suspensions orales, les formes d'administration sublinguale, buccale, intratrachéale, intraoculaire, intranasale, par inhalation, les formes d'administration sous-cutanée, intramusculaire ou intraveineuse et les formes d'administration rectale ou vaginale. Pour l'application topique, on peut utiliser les composés selon l'invention dans des crèmes, pommades ou lotions.

Par exemple, lorsqu'on prépare une composition solide sous forme de comprimés, on mélange l'ingrédient actif principal avec un excipient pharmaceutique, tel que la gélatine, l'amidon, le lactose, le stéarate de magnésium, le talc, la gomme arabique ou analogues. On peut enrober les comprimés de saccharose, d'un dérivé cellulosique, ou d'autres matières. Les comprimés peuvent être réalisés par différentes techniques, compression directe, granulation sèche, granulation humide ou fusion à chaud.

Afin d'obtenir l'effet prophylactique ou thérapeutique désiré, la dose de principe actif peut varier entre 0,1 mg et 200 mg par kg de poids du corps et par jour. Bien que ces dosages soient des exemples de situation moyenne, il peut y avoir des cas particuliers où des dosages plus élevés ou plus faibles sont appropriés, de tels dosages appartiennent également à l'invention. Selon la pratique habituelle, le dosage approprié à chaque patient est déterminé par le médecin selon le mode d'administration, le poids et la réponse dudit patient.

Chaque dose unitaire peut contenir de 0,1 à 1000 mg, de préférence de 0,1 à 500 mg, de principe actif en combinaison avec un ou plusieurs excipients pharmaceutiques. Cette dose unitaire peut être administrée 1 à 5 fois par jour de façon à administrer un dosage journalier de 0,5 à 5000 mg, de préférence de 0,5 à 2500 mg.

## Revendications

1. Composé répondant à la formule générale (I) : 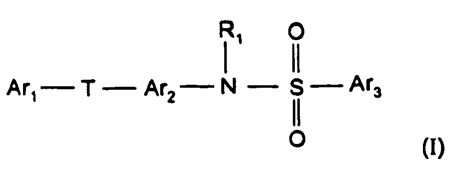 dans laquelle :
• **Ar₁** représente :
- un groupe aryle tel qu'un groupe phényle éventuellement substitué par un ou plusieurs groupes choisis indépendamment les uns des autres parmi les groupes suivants : un atome d'halogène, un groupe (C₁-C₄) alkyle, un groupe (C₁-C₄) alcoxy, un groupe fluoro(C₁-C₄)alkyle, un groupe cyano, un groupe -CO-NRₐR_{b}, un groupe - NRₐR_{b,}
avec Rₐ et R_{b} étant indépendamment l'un de l'autre un atome d'hydrogène, un groupe (C₁-C₄) alkyle,
- un groupe hétérocyclyle choisi parmi le pyridinyle, le pyrimidinyle, le thiazolyle, le thiényle, lesdits groupes hétérocyclyles étant éventuellement substitués par un atome d'halogène, un groupe (C₁-C₄) alkyle, un groupe (C₁-C₄) alcoxy,
- un groupe (C₃-C₆) cycloalkyle ;
• **T** représente :
- un groupe -(CH₂)ₙ- avec n = 0,1,2,
- un groupe 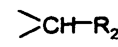 avec R₂ étant un groupe hydroxyle, un groupe (C₁-C₄) alkyle,
- un groupe 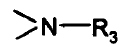 avec R₃ étant un groupe (C₁-C₄) alkyle,
- un groupe 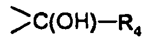 avec R₄ étant un groupe (C₁-C₄) alkyle ;
• **Ar₂** représente :
- un groupe aryle tel que le phényle éventuellement substitué par un ou plusieurs groupes choisis indépendamment les uns des autres parmi les groupes suivants : un atome d'halogène, un groupe (C₁-C₄) alkyle, un groupe (C₁-C₄) alcoxy, un groupe fluoro(C₁-C₄)alkyle, un groupe fluoro(C₁-C₄)alcoxy,
ou bien par un groupe -NR_{c}R_{d} avec R_{c} et R_{d} étant indépendamment l'un de l'autre un atome d'hydrogène, un groupe (C₁-C₄) alkyle,
- un groupe hétérocyclyle tel que le pyridinyle éventuellement substitué par un groupe (C₁-C₄)alkyle, un groupe (C₁-C₄) alcoxy ;
• **Ar₃** représente :
- un groupe aryle choisi parmi le phényle et le naphtyle, lesdits groupes aryles étant éventuellement substitués par un ou plusieurs groupes choisis indépendamment les uns des autres parmi les groupes suivants : un atome d'halogène, un groupe (C₁-C₄) alkyle, un groupe (C₁-C₄) alcoxy, un groupe fluoro(C₁-C₄)alkyle, un groupe fluoro(C₁-C₄)alcoxy, un groupe nitro, groupe hydroxy,
ou bien par un groupe -NR₅R₆ avec R₅ et R₆ étant, indépendamment l'un de l'autre, un atome d'hydrogène, un groupe (C₁-C₄) alkyle,
- un groupe hétérocyclyle tel que le pyridinyle éventuellement substitué par un groupe (C₁-C₄) alkyle ou un (C₁-C₄) alcoxy,
ou bien par un groupe -NRₓR_{y} avec Rₓ et R_{y} étant indépendamment l'un de l'autre un atome d'hydrogène, un groupe (C₁-C₄) alkyle, un groupe (C₁-C₄) alcoxy,
- un groupe choisi parmi : 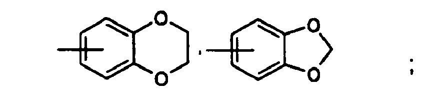
• **R₁** représente :
- un groupe -C(O)-CF₃,
- un groupe de formule : 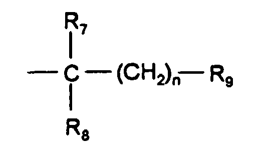 dans laquelle :
n = 0, 1, 2, 3,
o R₇ représente :
- un atome d'hydrogène, un groupe (C₁-C₄) alkyle, un atome de fluor, un groupe (C₁-C₄) alcoxy,
- un groupe -(CH₂)ₘ-aryle, avec m=1, 2, et le groupe aryle étant un groupe phényle éventuellement substitué par un atome d'halogène, un groupe (C₁-C₄) alkyle, un groupe (C₁-C₄)alcoxy,
o R₈ représente :
- un atome d'hydrogène, un atome de fluor, un groupe (C₁-C₄) alkyle,
o R₉ représente: .
- un atome d'hydrogène, un groupe (C₁-C₄) alkyle, un groupe hydroxyle, un groupe (C₃-C₆) cycloalkyle, un groupe (C₁-C₄) alcoxy, un groupe fluoro(C₁-C₄)alkyle, un groupe -C=CH, un groupe -C=N, un groupe phénoxy, un groupe (C₁-C₄)alcényle,
- un groupe -NR₁₀R₁₁ avec R₁₀ et R₁₁ étant indépendamment l'un de l'autre un atome d'hydrogène, un groupe (C₁-C₄) alkyle, un groupe (C₁-C₄) alkylcarbonyle, un groupe (C₁-C₄) alkylsulfonyle, un benzyle ou un groupe -(CH₂)₂-N(CH₃)₂,
ou bien R₁₀ et R₁₁ forment ensemble avec l'atome d'azote qui les porte une pyrrolidine, une pyrrolidinone, une morpholine ou une pipéridine,
- un groupe -CO-NR₁₂R₁₃ avec R₁₂ et R₁₃ étant indépendamment l'un de l'autre :
o un atome d'hydrogène,
o un groupe (C₁-C₄) alkyle éventuellement substitué par un groupe -C≡N, un groupe aryle tel que le phényle, un groupe hétérocyclyle tel que le pyridinyle, lesdits groupes aryle et hétérocyclyle étant éventuellement substitués par un groupe (C₁-C₄) alkyle, un groupe hydroxyle,
ou bien R₁₂ et R₁₃ forment ensemble avec l'atome d'azote qui les porte un groupe 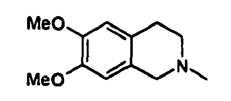
- un groupe -COOR₁₄ avec R₁₄ étant un groupe (C₁-C₄) alkyle,
- un groupe -NH-CO-NR₁₅R₁₆ avec R₁₅ et R₁₆ étant indépendamment l'un de l'autre un atome d'hydrogène, un groupe (C₁-C₄) alkyle, un groupe phényle, un groupe benzyle, lesdits groupes phényle et benzyle étant éventuellement substitués par un ou plusieurs substituants choisis parmi un atome d'halogène, un groupe (C₁-C₄)alkyle, un groupe (C₁-C₄) alcoxy,
- un groupe -SO₂-NR₁₇R₁₈ avec R₁₇ et R₁₈ étant indépendamment l'un de l'autre un atome d'hydrogène, un groupe (C₁-C₄) alkyle,
- un groupe -SO₂-R₁₉ avec R₁₉ étant un groupe (C₁-C₄) alkyle, un groupe aryle tel que le phényle,
- un groupe hétérocyclyle choisi parmi, 1,3-dioxolanyle, l'imidazolyle, le tétrazolyle, le triazolyle éventuellement substitué par un groupe (C₁-C₄) alkyle, le thiazolyle, le pyrimidinyle, l'oxadiazolyle, le pyridinyle, l'imidazolyle étant éventuellement substitué sur l'atome d'azote par un groupe (C₁-C₄) alkyle ou un benzyle, le tétrazolyle étant éventuellement substitué par un groupe (C₁-C₄) alkyle ou un benzyle, l'oxadiazolyle étant éventuellement substitué par un (C₁-C₄) alkyle,
- un groupe -NH-CO-OR₁₅, R₁₅ répondant à la définition ci-dessus,
le composé suivant étant exclu : N-méthyl benzyl-2' chloro-4' tosanilide, à l'état de base, de sel d'addition à un acide, d'hydrate ou de solvat, sous forme d'énantiomères, de diastéréoisomères, de rotamères, d'atropoisoméres ou de leurs mélanges.

2. Composé de formule générale (I) selon la revendication 1, dans laquelle
• **Ar₁** représente :
- un groupe aryle tel qu'un groupe phényle éventuellement substitué par un ou plusieurs groupes choisis indépendamment les uns des autres parmi les groupes suivants : un atome d'halogène, un groupe (C₁-C₄) alkyle, un groupe (C₁-C₄) alcoxy, un groupe fluoro(C₁-C₄)alkyle,
- un groupe hétérocyclyle choisi parmi le pyridinyle, le thiazolyle, le thiényle, le thiényle étant éventuellement substitué par un groupe (C₁-C₄) alkyle,
- un groupe (C₃-C₆) cycloalkyle :
• **T** représente :
- un groupe -(CH₂)ₙ- avec n = 1
- un groupe 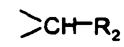 avec R₂ étant un groupe hydroxyle, un groupe (C₁-C₄) alkyle,
- un groupe 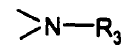 avec R₃ étant un groupe (C₁-C₄) alkyle,
- un groupe 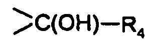 avec R₄ étant un groupe (C₁-C₄) alkyle ;
• **Ar₂** représente :
- un groupe aryle tel que le phényle éventuellement substitué par un ou plusieurs groupes choisis indépendamment les uns des autres parmi les groupes suivants : un atome d'halogène, un groupe (C₁-C₄) alkyle, un groupe (C₁-C₄) alcoxy, un groupe fluoro(C₁-C₄)alcoxy, un groupe -NR_{c}R_{d} avec R_{c} et R_{d} étant, indépendamment l'un de l'autre, un atome d'hydrogène, un groupe (C₁-C₄) alkyle ;
• **Ar₃** représente :
- un groupe aryle choisi parmi le phényle et le naphtyle, le groupe phényle étant éventuellement substitué par un ou plusieurs groupes choisis indépendamment les uns des autres parmi les groupes suivants : un atome d'halogène, un groupe (C₁-C₄) alkyle, un groupe (C₁-C₄) alcoxy, un groupe fluoro(C₁-C₄)alkyle, groupe fluoro(C₁-C₄)alcoxy, un groupe nitro,
ou bien un groupe -NR₅R₆ avec R₅ et R₆ étant, indépendamment l'un de l'autre, un atome d'hydrogène, un groupe (C₁-C₄) alkyle,
- un groupe choisi parmi : 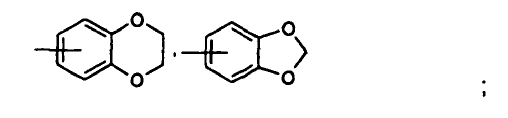
• **R₁** représente :
- un groupe -C(O)-CF₃,
- un groupe de formule 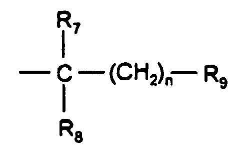 dans laquelle :
n = 0, 1, 2,
o R₇ représente :
- un atome d'hydrogène, un groupe (C₁-C₄) alkyle,
o R₈ repésente :
- un atome d'hydrogène,
o R₉ représente :
- un atome d'hydrogène, un groupe (C₁-C₄) alkyle, un groupe hydroxyle, un groupe (C₁-C₄) alcoxy, un groupe fluoro(C₁-C₄) alkyle, un groupe -C≡CH, un groupe -C≡N,
- un groupe -NR₁₀R₁₁ avec R₁₀ et R₁₁ étant indépendamment l'un de l'autre un atome d'hydrogène, un groupe (C₁-C₄) alkyle, un groupe (C₁-C₄) alkylcarbonyle, un benzyle,
ou bien R₁₀ et R₁₁ forment ensemble avec l'atome d'azote qui les porte une pyrrolidine, une pipéridine ou une pyrrolidinone,
- un groupe -CO-NR₁₂R₁₃ avec R₁₂ et R₁₃ étant indépendamment l'un de l'autre un atome d'hydrogène, un groupe (C₁-C₄) alkyle,
ou bien R₁₂ et R₁₃ forment ensemble avec l'atome d'azote qui les porte un groupe : 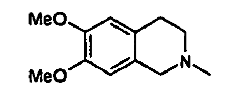
- un groupe -COOR₁₄ avec R₁₄ étant un groupe (C₁-C₄) alkyle,
- un groupe -NH-CO-NR₁₅R₁₆ avec R₁₅ et R₁₆ étant indépendamment l'un de l'autre un atome d'hydrogène, un groupe (C₁-C₄) alkyle, un phényle, un benzyle,
- un groupe hétérocyclyle choisi parmi l'imidazolyle éventuellement substitué sur l'atome d'azote par un groupe (C₁-C₄) alkyle, le tétrazolyle éventuellement substitué par un groupe (C₁-C₄) alkyle, le triazolyle,
à l'état de base, de sel d'addition à un acide, d'hydrate ou de solvat, sous forme d'énantiomères, de diastéréoisomères, de rotamères, d'atropoisoméres ou de leurs mélanges.

3. Composé de formule générale (I) selon l'une des revendications 1 ou 2 dans laquelle :
• **Ar₁** représente :
- un groupe aryle tel que le phényle éventuellement substitué par un ou plusieurs groupes choisis indépendamment les uns des autres parmi les groupes suivants : un atome d'halogène, un groupe (C₁-C₄) alkyle, un groupe (C₁-C₄) alcoxy, un groupe fluoro(C₁-C₄)alkyle,
- un groupe hétérocyclyle choisi parmi le pyridinyle, le thiazolyle, le thiényle, le thiényle étant éventuellement substitué par un groupe (C₁-C₄) alkyle ;
• **T** représente :
- un groupe -(CH₂)ₙ- avec n = 1
- un groupe 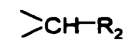 avec R₂ étant un groupe hydroxyle ;
• **Ar₂** représente :
- un groupe aryle tel que le phényle éventuellement substitué par un ou plusieurs groupes choisis indépendamment les uns des autres parmi les groupes suivants : un atome d'halogène, un groupe (C₁-C₄) alkyle, un groupe (C₁-C₄) alcoxy ; un groupe - NR_{c}R_{d} avec R_{c} et R_{d} étant, indépendamment l'un de l'autre, un atome d'hydrogène, un groupe (C₁-C₄) alkyle ;
• **Ar₃** représente :
- un groupe aryle tel que le phényle, le groupe phényle étant éventuellement substitué par un ou plusieurs groupes choisis indépendamment les uns des autres parmi les groupes suivants : un atome d'halogène, un groupe (C₁-C₄) alkyle, un groupe (C₁-C₄) alcoxy,
ou bien un groupe -NR₅R₆ avec R₅ et R₆ étant, indépendamment l'un de l'autre, un atome d'hydrogène, un groupe (C₁-C₄) alkyle ;
• **R₁** représente :
- un groupe de formule : 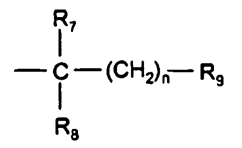 dans laquelle :
n =0, 1, 2,
o R₇ représente :
- un atome d'hydrogène, un groupe (C₁-C₄) alkyle,
o R₈ représente :
- un atome d'hydrogène,
o R₉ représente :
- un atome d'hydrogène, un groupe -C≡CH, un groupe -C≡N,
- un groupe -CO-NR₁₂R₁₃ avec R₁₂ et R₁₃ étant indépendamment l'un de l'autre un atome d'hydrogène, un groupe (C₁-C₄) alkyle,
- un hétérocyclyle choisi parmi l'imidazolyle éventuellement substitué sur l'atome d'azote par un groupe (C₁-C₄) alkyle, le tétrazolyle éventuellement substitué par un groupe (C₁-C₄) alkyle, le triazolyle
à l'état de base, de sel d'addition à un acide, d'hydrate ou de solvat, sous forme d'énantiomères, de diastéréoisomères, de rotamères , d'atropoisoméres ou de leurs mélanges.

4. Composé de formule générale (I) selon l'une quelconque des revendications 1 à 3 dans laquelle :
• **Ar₁** représente :
- un groupe aryle tel que le phényle éventuellement substitué par un ou plusieurs groupes choisis indépendamment les uns des autres parmi les groupes suivants : un atome d'halogène, un groupe (C₁-C₄) alkyle, un groupe (C₁-C₄) alcoxy,
- un groupe hétérocyclyle tel que le thiényle éventuellement substitué par un groupe (C₁-C₄) alkyle,
• T représente :
- un groupe -(CH₂)ₙ- avec n = 1,
• **Ar₂** représente :
- un groupe phényle éventuellement substitué par un ou plusieurs groupes choisis indépendamment les uns des autres parmi les groupes suivants : un atome d'halogène, un groupe (C₁-C₄) alkyle, un groupe (C₁-C₄) alcoxy, un groupe -NR_{c}R_{d} avec R_{c} et R_{d} étant, indépendamment l'un de l'autre, un atome d'hydrogène, un groupe (C₁-C₄) alkyle ;
• **Ar₃** représente :
- un groupe phényle éventuellement substitué par un ou plusieurs groupes choisis indépendamment les uns des autres parmi les groupes suivants : un atome d'halogène, un groupe (C₁-C₄) alkyle, un groupe (C₁-C₄) alcoxy,
ou bien un groupe -NR₅R₆ avec R₅ et R₆ étant, indépendamment l'un de l'autre, un atome d'hydrogène, un groupe (C₁-C₄) alkyle;
• **R₁** représente :
- un groupe de formule : 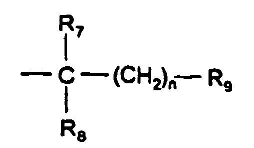 dans lequel :
n = 0, 1, 2,
o R₇ représente:
- un atome d'hydrogène, un groupe (C₁-C₄) alkyle ;
o R₈ représente :
- un atome d'hydrogène ;
o R₉ représente :
- un groupe -CO-NR₁₂R₁₃ avec R₁₂ et R₁₃ étant indépendamment l'un de l'autre un atome d'hydrogène, un groupe (C₁-C₄) alkyle,
- un hétérocycle choisi parmi l'imidazolyle substitué sur l'atome d'azote par un groupe (C₁-C₄) alkyle, le tétrazolyle substitué par un groupe (C₁-C₄) alkyle, le triazolyle,
à l'état de base, de sel d'addition à un acide, d'hydrate ou de solvat, sous forme d'énantiomères, de diastéréoisomères, de rotamères, d'atropoisoméres ou de leurs mélanges.

5. Procédé de préparation d'un composé de formule (I) selon l'une quelconque des revendications 1 à 4, **caractérisé en ce que** :
- soit l'on alkyle le composé de formule générale (II) avec le composé de formule générale (IV), en présence d'une base ;
- soit l'on effectue une réaction de Mitsunobu entre un alcool de formule (X) et un composé de formule générale (II) : 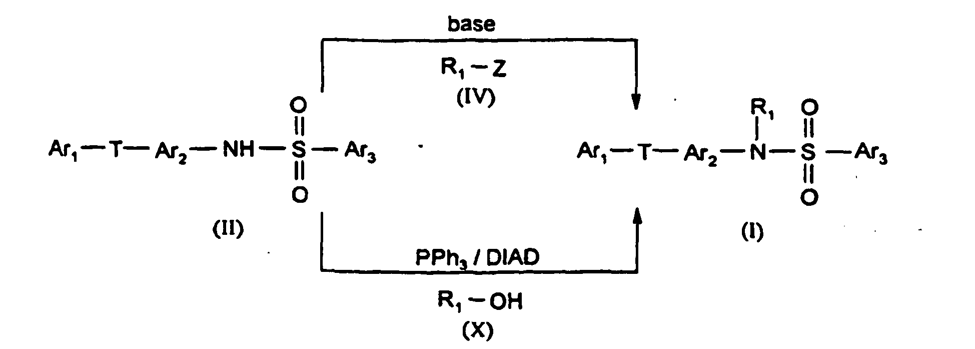 dans les composés de formule (II), (IV) et (X), Ar₁, Ar₂, Ar₃, T et R₁ sont tels que définis dans la formule (I) et Z est un groupe partant.

6. Procédé de préparation selon la revendication 5, **caractérisé en ce que** l'on obtient le composé de formule (II) par la sulfonylation de composé de formule (III) avec des chlorures de sulfonyle de formule (V), en présence d'une base : 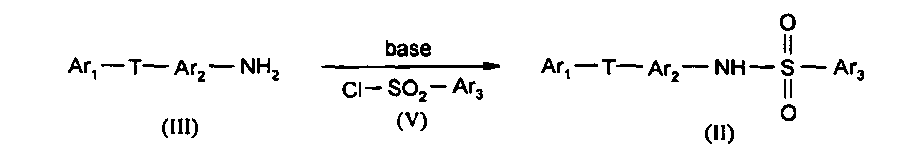 dans les composés de formule (III) et (V), Ar₁, Ar₂, Ar₃ et T sont tels que définis dans la formule (I) de la revendication 1 et Z est un groupe partant.

7. Utilisation d'un composé de formule (I) selon l'une quelconque des revendications 1 à 4 pour la préparation d'un médicament destiné à la prophylaxie ou au traitement des pathologies telles que l'obésité, les perturbations de l'appétit ou du goût dont la cachexie, l'anorexie, la boulimie, le diabète, les syndromes métaboliques, les vomissements et la nausée, la dépression et l'anxiété, les addictions, les troubles de l'humeur et du comportement, la schizophrénie, les troubles du sommeil, la maladie des jambes sans repos, les troubles de l'apprentissage de la mémoire, les dysfonctions sexuelles et psychosexuelles, la douleur, la douleur viscérale ou neuropathique, l'hyperalgésie, l'allodynie, les troubles digestifs, le syndrome des intestins irrités, la dégénérescence neuronale, les attaques ischémiques ou hémorrhagiques, la maladie de Cushing, le syndrome de Guillain-Barré, la dystrophie myotonique, l'incontinence urinaire, l'hyperthyroïdie, les troubles de la fonction hypophysaire, l'hypertension ou l'hypotension.

8. Médicament **caractérisé en ce qu'**il comprend un composé de formule (I) selon l'une quelconque des revendications 1 à 4.

9. Composition pharmaceutique contenant au moins un composé de formule (I) selon l'une quelconque des revendications 1 à 4 et éventuellement un ou plusieurs excipients pharmaceutiquement acceptables.

## Claims

1. Compound corresponding to the general formula (I): 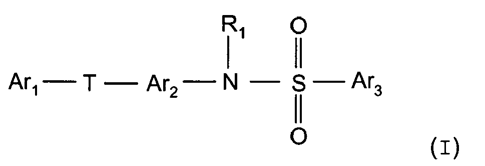 in which:
• **Ar₁** represents:
- an aryl group such as a phenyl group optionally substituted with one or more groups chosen, independently of each other, from the following groups: a halogen atom, a (C₁-C₄) alkyl group, a (C₁-C₄) alkoxy group, a fluoro (C₁-C₄) alkyl group, a cyano group, a group -CO-NRₐR_{b}, a group -NRₐR_{b},
with Rₐ and R_{b} being, independently of each other, a hydrogen atom or a (C₁-C₄) alkyl group,
- a heterocyclyl group chosen from pyridinyl, pyrimidinyl, thiazolyl and thienyl, the said heterocyclyl groups being optionally substituted with a halogen atom, a (C₁-C₄) alkyl group or a (C₁-C₄) alkoxy group,
- a (C₃-C₆) cycloalkyl group;
• **T** represents:
- a group -(CH₂)ₙ- with n = 0, 1 or 2,
- a group 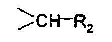 with R₂ being a hydroxyl group or a (C₁-C₄) alkyl group,
- a group 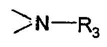 with R₃ being a (C₁-C₄) alkyl group,
- a group 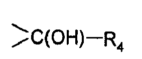 with R₄ being a (C₁-C₄) alkyl group;
• **Ar₂** represents:
- an aryl group such as phenyl optionally substituted with one or more groups chosen, independently of each other, from the following groups: a halogen atom, a (C₁-C₄) alkyl group, a (C₁-C₄) alkoxy group, a fluoro (C₁-C₄) alkyl group, a fluoro (C₁-C₄) alkoxy group, or alternatively with a group -NR_{c}R_{d} with R_{c} and R_{d} being, independently of each other, a hydrogen atom or a (C₁-C₄) alkyl group,
- a heterocyclyl group such as pyridinyl optionally substituted with a (C₁-C₄) alkyl group or a (C₁-C₄) alkoxy group;
• **Ar₃** represents:
- an aryl group chosen from phenyl and naphthyl, the said aryl groups being optionally substituted with one or more groups chosen, independently of each other, from the following groups: a halogen atom, a (C₁-C₄) alkyl group, a (C₁-C₄) alkoxy group, a fluoro (C₁-C₄) alkyl group, a fluoro (C₁-C₄) alkoxy group, a nitro group, a hydroxyl group,
or alternatively with a group -NR₅R₆ with R₅ and R₆ being, independently of each other, a hydrogen atom or a (C₁-C₄) alkyl group,
- a heterocyclyl group such as pyridinyl optionally substituted with a (C₁-C₄) alkyl group or a (C₁-C₄) alkoxy group,
or alternatively with a group -NRₓR_{y} with Rₓ and R_{y} being, independently of each other, a hydrogen atom, a (C₁-C₄) alkyl group or a (C₁-C₄) alkoxy group,
- a group chosen from: 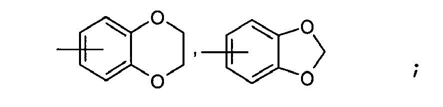
• **R₁** represents:
- a -C(O)-CF₃ group,
- a group of formula: 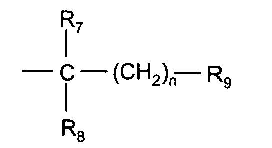 in which:
n = 0, 1, 2 or 3
. R₇ represents:
- a hydrogen atom, a (C₁-C₄) alkyl group, a fluorine atom or a (C₁-C₄) alkoxy group,
- a group -(CH₂)ₘ-aryl, with m = 1 or 2 and the aryl group being a phenyl group optionally substituted with a halogen atom, a (C₁-C₄) alkyl group or a (C₁-C₄) alkoxy group,
. R₈ represents:
- a hydrogen atom, a fluorine atom or a (C₁-C₄) alkyl group,
. R₉ represents:
- a hydrogen atom, a (C₁-C₄) alkyl group, a hydroxyl group, a (C₃-C₆) cycloalkyl group, a (C₁-C₄) alkoxy group, a fluoro (C₁-C₄) alkyl group, a -C≡CH group, a -C≡N group, a phenoxy group or a (C₁-C₄) alkenyl group,
- a group -NR₁₀R₁₁ with R₁₀ and R₁₁ being, independently of each other, a hydrogen atom, a (C₁-C₄) alkyl group, a (C₁-C₄) alkylcarbonyl group, a (C₁-C₄) alkylsulfonyl group, a benzyl group or a-(CH₂)₂-N(CH₃) group,
or alternatively R₁₀ and R₁₁ form, together with the nitrogen atom that bears them, a pyrrolidine, a pyrrolidinone, a morpholine or a piperidine,
- a group -CO-NR₁₂R₁₃ with R₁₂ and R₁₃ being, independently of each other:
. a hydrogen atom,
. a (C₁-C₄) alkyl group optionally substituted with a -C≡N group, an aryl group such as phenyl or a heterocyclyl group such as pyridinyl, the said aryl and heterocyclyl groups being optionally substituted with a (C₁-C₄) alkyl group or a hydroxyl group, or alternatively R₁₂ and R₁₃ form, together with the nitrogen atom that bears them, a group 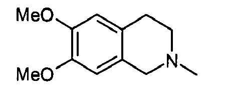
- a group -COOR₁₄ with R₁₄ being a (C₁-C₄) alkyl group,
- a group -NH-CO-NR₁₅R₁₆ with R₁₅ and R₁₆ being, independently of each other, a hydrogen atom, a (C₁-C₄) alkyl group, a phenyl group or a benzyl group, the said phenyl and benzyl groups being optionally substituted with one or more substituents chosen from a halogen atom, a (C₁-C₄) alkyl group and a (C₁-C₄) alkoxy group,
- a group -SO₂-NR₁₇R₁₈ with R₁₇ and R₁₈ being, independently of each other, a hydrogen atom or a (C₁-C₄) alkyl group,
- a group -SO₂R₁₉ with R₁₉ being a (C₁-C₄) alkyl group or an aryl group such as phenyl,
- a heterocyclyl group chosen from 1,3-dioxolanyl, imidazolyl, tetrazolyl, triazolyl optionally substituted with a (C₁-C₄) alkyl group, thiazolyl, pyrimidinyl, oxadiazolyl or pyridinyl, the imidazolyl being optionally substituted on the nitrogen atom with a (C₁-C₄) alkyl group or a benzyl, the tetrazolyl being optionally substituted with a (C₁-C₄) alkyl group or a benzyl, and the oxadiazolyl being optionally substituted with a (C₁-C₄) alkyl,
- a group -NH-CO-OR₁₅, R₁₅ corresponding to the above definition,
the following compound being excluded: N-methylbenzyl-2'-chloro-4'-tosanilide,
in the form of base, of acid-addition salt, of hydrate or of solvate, in the form of enantiomers, diastereoisomers, rotamers or atropoisomers, or mixtures thereof.

2. Compound of general formula (I) according to Claim 1, in which
• **Ar₁** represents:
- an aryl group such as a phenyl group optionally substituted with one or more groups chosen, independently of each other, from the following groups: a halogen atom, a (C₁-C₄) alkyl group, a (C₁-C₄) alkoxy group, a fluoro (C₁-C₄) alkyl group,
- a heterocyclyl group chosen from pyridinyl, thiazolyl and thienyl, the thienyl being optionally substituted with a (C₁-C₄) alkyl group,
- a (C₃-C₆) cycloalkyl group;
• **T** represents:
- a group -(CH₂)ₙ- with n = 1,
- a group 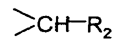 with R₂ being a hydroxyl group or a (C₁-C₄) alkyl group,
- a group 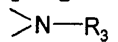 with R₃ being a (C₁-C₄) alkyl group,
- a group 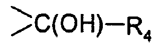 with R₄ being a (C₁-C₄) alkyl group;
• **Ar₂** represents:
- an aryl group such as phenyl optionally substituted with one or more groups chosen, independently of each other, from the following groups: a halogen atom, a (C₁-C₄) alkyl group, a (C₁-C₄) alkoxy group, a fluoro (C₁-C₄) alkoxy group, a group -NR_{c}R_{d} with R_{c} and R_{d} being, independently of each other, a hydrogen atom or a (C₁-C₄) alkyl group;
• **Ar₃** represents:
- an aryl group chosen from phenyl and naphthyl, the phenyl group being optionally substituted with one or more groups chosen, independently of each other, from the following groups: a halogen atom, a (C₁-C₄) alkyl group, a (C₁-C₄) alkoxy group, a fluoro (C₁-C₄) alkyl group, a fluoro(C₁-C₄)alkoxy group, a nitro group,
or alternatively a group -NR₅R₆ with R₅ and R₆ being, independently of each other, a hydrogen atom or a (C₁-C₄) alkyl group,
- a group chosen from: 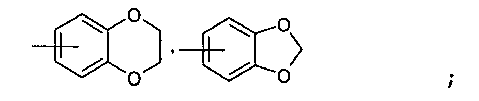
• R₁ represents:
- a -C(O)-CF₃ group,
- a group of formula 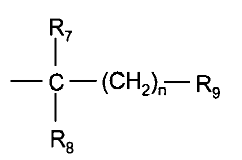 in which:
n = 0, 1 or 2,
. R₇ represents:
- a hydrogen atom or a (C₁-C₄) alkyl group
. R₈ represents:
- a hydrogen atom,
. R₉ represents:
- a hydrogen atom, a (C₁-C₄) alkyl group, a hydroxyl group, a (C₁-C₄) alkoxy group, a fluoro (C₁-C₄) alkyl group, a -C≡CH group or a -C≡N group,
- a group -NR₁₀R₁₁ with R₁₀ and R₁₁ being, independently of each other, a hydrogen atom, a (C₁-C₄) alkyl group, a (C₁-C₄) alkylcarbonyl group or a benzyl,
or alternatively R₁₀ and R₁₁ form, together with the nitrogen atom that bears them, a pyrrolidine, a piperidine or a pyrrolidinone,
- a group -CO-NR₁₂R₁₃ with R₁₂ and R₁₃ being, independently of each other, a hydrogen atom or a (C₁-C₄) alkyl group,
or alternatively R₁₂ and R₁₃ form, together with the nitrogen atom that bears them, a group: 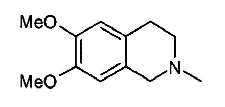
- or a group -COOR₁₄ with R₁₄ being a (C₁-C₄) alkyl group,
- a group -NH-CO-NR₁₅R₁₆ with R₁₅ and R₁₆ being, independently of each other, a hydrogen atom, a (C₁-C₄) alkyl group, a phenyl or a benzyl,
- a heterocyclyl group chosen from imidazolyl optionally substituted on the nitrogen atom with a (C₁-C₄) alkyl group, tetrazolyl optionally substituted with a (C₁-C₄) alkyl group, and triazolyl,
in the form of base, of acid-addition salt, of hydrate or of solvate, in the form of enantiomers, diastereoisomers, rotamers or atropoisomers, or mixtures thereof.

3. Compound of general formula (I) according to either of Claims 1 and 2, in which:
• **Ar₁** represents:
- an aryl group such as phenyl optionally substituted with one or more groups chosen, independently of each other, from the following groups: a halogen atom, a (C₁-C₄) alkyl group, a (C₁-C₄) alkoxy group, a fluoro (C₁-C₄) alkyl group,
- a heterocyclyl group chosen from pyridinyl, thiazolyl and thienyl, the thienyl being optionally substituted with a (C₁-C₄) alkyl group;
• **T** represents:
- a group- (CH₂)ₙ- with n = 1,
- a group 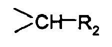 with R₂ being a hydroxyl group;
• **Ar₂** represents:
- an aryl group such as phenyl optionally substituted with one or more groups chosen, independently of each other, from the following groups: a halogen atom, a (C₁-C₄) alkyl group, a (C₁-C₄) alkoxy group; a group -NR_{c}R_{d} with R_{c} and R_{d} being, independently of each other, a hydrogen atom or a (C₁-C₄) alkyl group;
• **Ar₃** represents:
- an aryl group such as phenyl, the phenyl group being optionally substituted with one or more groups chosen, independently of each other, from the following groups: a halogen atom, a (C₁-C₄) alkyl group, a (C₁-C₄) alkoxy group,
or alternatively a group -NR₅R₆ with R₅ and R₆ being, independently of each other, a hydrogen atom or a (C₁-C₄) alkyl group;
• **R₁** represents:
- a group of formula: 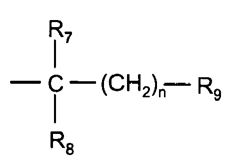 in which:
n = 0, 1 or 2,
. R₇ represents:
- a hydrogen atom or a (C₁-C₄) alkyl group,
. R₈ represents:
- a hydrogen atom,
. R₉ represents:
- a hydrogen atom, a -C≡CH group or a -C≡N group,
- a group -CO-NR₁₂R₁₃ with R₁₂ and R₁₃ being, independently of each other, a hydrogen atom or a (C₁-C₄) alkyl group,
- a heterocyclyl chosen from imidazolyl optionally substituted on the nitrogen atom with a (C₁-C₄) alkyl group, tetrazolyl optionally substituted with a (C₁-C₄) alkyl group, and triazolyl,
in the form of base, of acid-addition salt, of hydrate or of solvate, in the form of enantiomers, diastereoisomers, rotamers or atropoisomers, or mixtures thereof.

4. Compound of general formula (I) according to any one of Claims 1 to 3, in which:
• **Ar₁** represents:
- an aryl group such as phenyl optionally substituted with one or more groups chosen, independently of each other, from the following groups: a halogen atom, a (C₁-C₄) alkyl group, a (C₁-C₄) alkoxy group,
- a heterocyclyl group such as thienyl, optionally substituted with a (C₁-C₄) alkyl group,
• **T** represents:
- a group - (CH₂)ₙ- with n = 1,
• **Ar₂** represents:
- a phenyl group optionally substituted with one or more groups chosen, independently of each other, from the following groups: a halogen atom, a (C₁-C₄) alkyl group, a (C₁-C₄) alkoxy group, a group -NR_{c}R_{d} with R_{c} and R_{d} being, independently of each other, a hydrogen atom or a (C₁-C₄) alkyl group;
• **Ar₃** represents:
- a phenyl group optionally substituted with one or more groups chosen, independently of each other, from the following groups: a halogen atom, a (C₁-C₄) alkyl group, a (C₁-C₄) alkoxy group,
or alternatively a group -NR₅R₆ with R₅ and R₆ being, independently of each other, a hydrogen atom or a (C₁-C₄) alkyl group;
• **R₁** represents:
- a group of formula: 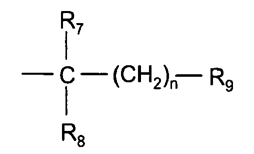 in which:
n = 0, 1 or 2,
. R₇ represents:
- a hydrogen atom or a (C₁-C₄) alkyl group;
. R₈ represents:
- a hydrogen atom;
. R₉ represents:
- a group -CO-NR₁₂R₁₃ with R₁₂ and R₁₃ being, independently of each other, a hydrogen atom or a (C₁-C₄) alkyl group,
- a heterocycle chosen from imidazolyl substituted on the nitrogen atom with a (C₁-C₄) alkyl group, tetrazolyl substituted with a (C₁-C₄) alkyl group, and triazolyl,
in the form of base, of acid-addition salt, of hydrate or of solvate, in the form of enantiomers, diastereoisomers, rotamers or atropoisomers, or mixtures thereof.

5. Process for preparing a compound of formula (I) according to any one of Claims 1 to 4, **characterized in that**:
- either the compound of general formula (II) is alkylated with the compound of general formula (IV), in the presence of a base;
- or a Mitsunobu reaction is performed between an alcohol of formula (X) and a compound of general formula (II): 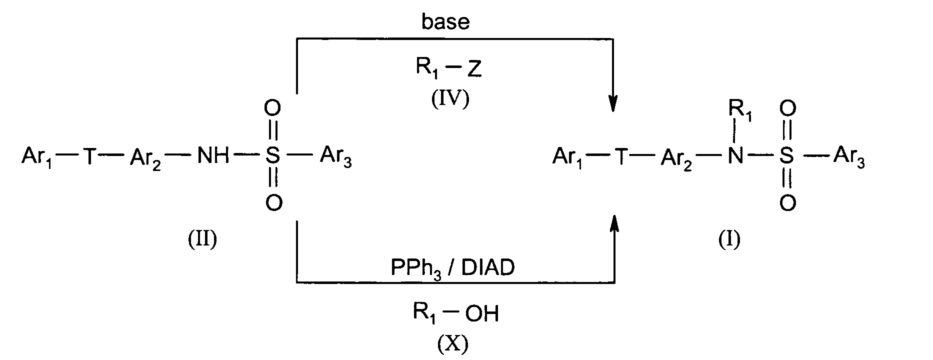
in the compounds of formulae (II), (IV) and (X) , Ar₁, Ar₂, Ar₃, T and R₁ are as defined in formula (I) and Z is a leaving group.

6. Preparation process according to Claim 5, **characterized in that** the compound of formula (II) is obtained by sulfonylation of a compound of formula (III) with sulfonyl chlorides of formula (V), in the presence of a base: 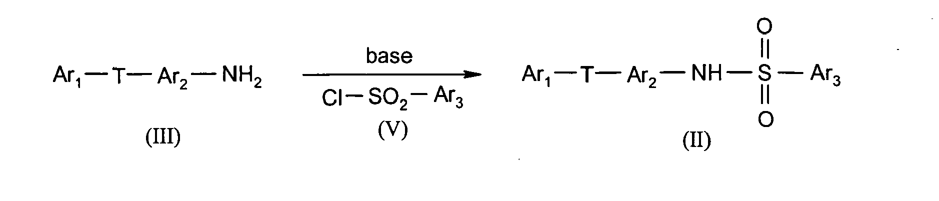 in the compounds of formulae (III) and (V), Ar₁, Ar₂, Ar₃ and T are as defined in formula (I) of Claim 1 and Z is a leaving group.

7. Use of a compound of formula (I) according to any one of Claims 1 to 4 for the preparation of a medicament for the prophylaxis or treatment of pathologies such as obesity, appetite or taste disorders including cachexia, anorexia and bulimia, diabetes, metabolic syndromes, vomiting and nausea, depression and anxiety, addictions, mood and behaviour disorders, schizophrenia, sleep disorders, restless legs syndrome, memory learning disorders, sexual and psychosexual dysfunctions, pain, visceral or neuropathic pain, hyperalgesia, allodynia, digestive disorders, irritable bowel syndrome, neuronal degenerescence, ischaemic or haemorrhagic attacks, Cushing's disease, Guillain-Barré syndrome, myotonic dystrophy, urinary incontinence, hyperthyroidism, pituitary function disorders, hypertension or hypotension.

8. Medicament, **characterized in that** it comprises a compound of formula (I) according to any one of Claims 1 to 4.

9. Pharmaceutical composition containing at least one compound of formula (I) according to any one of Claims 1 to 4 and optionally one or more pharmaceutically acceptable excipients.

## Patentansprüche

1. Verbindung der allgemeinen Formel (I): 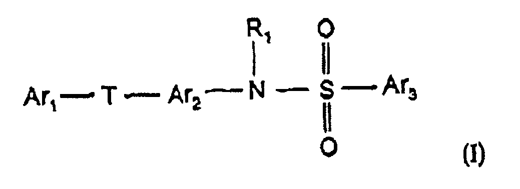 worin:
• Ar₁ für
- eine Arylgruppe wie eine Phenylgruppe, die gegebenenfalls durch eine oder mehrere unabhängig voneinander unter den folgenden Gruppen ausgewählte Gruppen substituiert ist: ein Halogenatom, eine (C₁-C₄) -Alkylgruppe, eine (C₁-C₄)-Alkoxygruppe, eine Fluor-(C₁-C₄)-alkylgruppe, eine Cyanogruppe, eine -CO-NRₐR_{b}-Gruppe, eine -NRₐR_{b}-Gruppe, wobei Rₐ und R_{b} unabhängig voneinander für ein Wasserstoffatom oder eine (C₁-C₄)-Alkylgruppe stehen,
- eine unter Pyridinyl, Pyrimidinyl, Thiazolyl und Thienyl ausgewählte Heterocyclylgruppe, wobei die Heterocyclylgruppen gegebenenfalls durch ein Halogenatom, eine (C₁-C₄)-Alkylgruppe oder eine (C₁-C₄)-Alkoxygruppe substituiert sind,
- eine (C₃-C₆) -Cycloalkylgruppe
steht,
• T für
- eine Gruppe -(CH₂)ₙ- mit n = 0, 1 oder 2,
- eine Gruppe 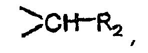 wobei R₂ für eine Hydroxylgruppe oder eine (C₁-C₄)-Alkylgruppe steht,
- eine Gruppe 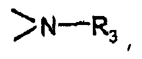 wobei R₃ für eine (C₁-C₄)-Alkylgruppe steht,
- eine Gruppe 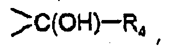 wobei R₄ für eine (C₁-C₄) - Alkylgruppe steht,
steht,
• Ar₂ für
- eine Arylgruppe wie Phenyl, die gegebenenfalls durch eine oder mehrere unabhängig voneinander unter den folgenden Gruppen ausgewählte Gruppen substituiert ist: ein Halogenatom, eine (C₁-C₄)-Alkylgruppe, eine (C₁-C₄)-Alkoxygruppe, eine Fluor-(C₁-C₄) -alkylgruppe, eine Fluor-(C₁-C₄)-alkoxygruppe,
oder auch durch eine -NR_{c}R_{d}-Gruppe, wobei R_{c} und R_{d} unabhängig voneinander für ein Wasserstoffatom oder eine (C₁-C₄)-Alkylgruppe stehen,
- eine Heterocyclylgruppe wie Pyridinyl, die gegebenenfalls durch eine (C₁-C₄)-Alkylgruppe oder eine (C₁-C₄)-Alkoxygruppe substituiert ist,
steht,
• Ar₃ für
- eine unter Phenyl und Naphthyl ausgewählte Arylgruppe, wobei die Arylgruppen gegebenenfalls durch eine oder mehrere unabhängig voneinander unter den folgenden Gruppen ausgewählte Gruppen substituiert sind: ein Halogenatom, eine (C₁-C₄)-Alkylgruppe, eine (C₁-C₄)-Alkoxygruppe, eine Fluor-(C₁-C₄)-alkylgruppe, eine Fluor-(C₁-C₄) -alkoxygruppe, eine Nitrogruppe, eine Hydroxygruppe,
oder auch durch eine -NR₅R₆-Gruppe, wobei R₅ und R₆ unabhängig voneinander für ein Wasserstoffatom oder eine (C₁-C₄)-Alkylgruppe stehen,
- eine Heterocyclylgruppe wie Pyridinyl, die gegebenenfalls durch eine (C₁-C₄)-Alkylgruppe oder eine (C₁-C₄)-Alkoxygruppe
oder auch durch eine -NRₓR_{y}-Gruppe, wobei Rₓ und R_{y} unabhängig voneinander für ein Wasserstoffatom, eine (C₁-C₄)-Alkylgruppe oder eine (C₁-C₄)-Alkoxygruppe stehen, substituiert ist,
- eine unter 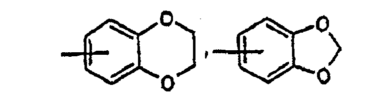 ausgewählte Gruppe
steht,
• R₁ für
- eine -C(O)-CF₃-Gruppe,
- eine Gruppe der Formel: 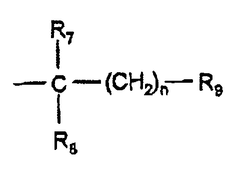 worin:
n = 0, 1, 2 oder 3,
o R₇ für
- ein Wasserstoffatom, eine (C₁-C₄)-Alkylgruppe, ein Fluoratom oder eine (C₁-C₄)-Alkoxygruppe,
- eine -(CH₂)ₘ-Arylgruppe mit m = 1 oder 2, wobei die Arylgruppe, bei der es sich um eine Phenylgruppe handelt, gegebenenfalls durch ein Halogenatom, eine (C₁-C₄)-Alkylgruppe oder eine (C₁-C₄)-Alkoxygruppe substituiert ist,
steht,
o R₈ für
- ein Wasserstoffatom, ein Fluoratom oder eine (C₁-C₄) -Alkylgruppe
steht,
o R₉ für
- ein Wasserstoffatom, eine (C₁-C₄)-Alkylgruppe, eine Hydroxylgruppe, eine (C₃-C₆)-Cycloalkylgruppe, eine (C₁-C₄)-Alkoxygruppe, eine Fluor-(C₁-C₄)-alkylgruppe, eine -C≡CH-Gruppe, eine -C≡N-Gruppe, eine Phenoxygruppe, eine (C₁-C₄)-Alkenylgruppe,
- eine -NR₁₀R₁₁-Gruppe, wobei R₁₀ und R₁₁ unabhängig voneinander für ein Wasserstoffatom, eine (C₁-C₄)-Alkylgruppe, eine (C₁-C₄)-Alkyl-carbonylgruppe, eine (C₁-C₄)-Alkylsulfonylgruppe, eine Benzylgruppe oder eine -(CH₂)₂-N(CH₃)₂-Gruppe stehen,
oder R₁₀ und R₁₁ zusammen mit dem Stickstoffatom, an das sie gebunden sind, ein Pyrrolidin, ein Pyrrolidinon, ein Morpholin oder ein Piperidin bilden,
- eine -CO-NR₁₂R₁₃-Gruppe, wobei R₁₂ und R₁₃ unabhängig voneinander für
o ein Wasserstoffatom,
o eine (C₁-C₄)-Alkylgruppe, gegebenenfalls substituiert durch eine -C≡N-Gruppe, eine Arylgruppe wie Phenyl oder eine Heterocyclylgruppe wie Pyridinyl, wobei die Aryl- und Heterocyclylgruppen gegebenenfalls durch eine (C₁-C₄)-Alkylgruppe oder eine Hydroxylgruppe substituiert sind,
stehen,
oder R₁₂ und R₁₃ zusammen mit dem Stickstoffatom, an das sie gebunden sind, eine Gruppe 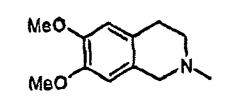 bilden,
- eine -COOR₁₄-Gruppe, wobei R₁₄ für eine (C₁-C₄)-Alkylgruppe steht,
- eine -NH-CO-NR₁₅R₁₆-Gruppe, wobei R₁₅ und R₁₆ unabhängig voneinander für ein Wasserstoffatom, eine (C₁-C₄)-Alkylgruppe, eine Phenylgruppe oder eine Benzylgruppe stehen, wobei die Phenyl- und Benzylgruppen gegebenenfalls durch einen oder mehrere unter einem Halogenatom, einer (C₁-C₄)-Alkylgruppe und einer (C₁-C₄-Alkoxygruppe ausgewählte Substituenten substituiert sind,
- eine -SO₂-NR₁₇R₁₈-Gruppe, wobei R₁₇ und R₁₈ unabhängig voneinander für ein Wasserstoffatom oder eine (C₁-C₄)-Alkylgruppe stehen,
- eine -SO₂-R₁₉-Gruppe, wobei R₁₉ für eine (C₁-C₄)-Alkylgruppe oder eine Arylgruppe wie Phenyl steht,
- eine unter 1,3-Dioxolanyl, Imidazolyl, Tetrazolyl, gegebenenfalls durch eine (C₁-C₄)-Alkylgruppe substituiertem Triazolyl, Thiazolyl, Pyrimidinyl, Oxadiazolyl oder Pyridinyl, wobei das Imidazolyl gegebenenfalls am Stickstoffatom durch eine (C₁-C₄)-Alkylgruppe oder eine Benzylgruppe substituiert ist, das Tetrazolyl gegebenenfalls durch eine (C₁-C₄)-Alkylgruppe oder eine Benzylgruppe substituiert ist und das Oxadiazolyl gegebenenfalls durch eine (C₁-C₄)-Alkylgruppe substituiert ist, ausgewählte Heterocyclylgruppe steht,
- eine -NH-CO-OR₁₅-Gruppe, wobei R₁₅ die oben angegebene Bedeutung besitzt,
steht,
wobei die folgende Verbindung ausgeschlossen ist: N-Methylbenzyl-2'-chlor-4'-tosanilid,
in Basen-, Säureadditionssalz-, Hydrat- oder Solvatform, in Form von Enantiomeren, Diastereoisomeren, Rotameren, Atropisomeren oder Gemischen davon.

2. Verbindung der allgemeinen Formel (I) nach Anspruch 1, worin
• Ar₁ für
- eine Arylgruppe wie eine Phenylgruppe, die gegebenenfalls durch eine oder mehrere unabhängig voneinander unter den folgenden Gruppen ausgewählte Gruppen substituiert ist: ein Halogenatom, eine (C₁-C₄)-Alkylgruppe, eine (C₁-C₄)-Alkoxygruppe, eine Fluor-(C₁-C₄)-alkylgruppe,
- eine unter Pyridinyl, Thiazolyl und Thienyl ausgewählte Heterocyclylgruppe, wobei das Thienyl gegebenenfalls durch eine (C₁-C₄)-Alkylgruppe substituiert ist,
- eine (C₃-C₆)-Cycloalkylgruppe
steht,
• T für
- eine Gruppe -(CH₂)ₙ- mit n = 1,
- eine Gruppe 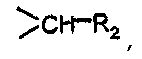 wobei R₂ für eine Hydroxylgruppe oder eine (C₁-C₄)-Alkylgruppe steht,
- eine Gruppe 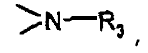 wobei R₃ für eine (C₁-C₄) - Alkylgruppe steht,
- eine Gruppe 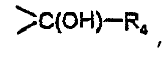 wobei R₄ für eine (C₁-C₄) - Alkylgruppe steht,
steht,
• Ar₂ für
- eine Arylgruppe wie Phenyl, die gegebenenfalls durch eine oder mehrere unabhängig voneinander unter den folgenden Gruppen ausgewählte Gruppen substituiert ist: ein Halogenatom, eine (C₁-C₄)-Alkylgruppe, eine (C₁-C₄)-Alkoxygruppe, eine Fluor-(C₁-C₄)-alkoxygruppe, eine -NR_{c}R_{d}-Gruppe, wobei R_{c} und R_{d} unabhängig voneinander für ein Wasserstoffatom oder eine (C₁-C₄)-Alkylgruppe stehen,
steht,
• Ar₃ für
- eine unter Phenyl und Naphthyl ausgewählte Arylgruppe, wobei die Phenylgruppe gegebenenfalls durch eine oder mehrere unabhängig voneinander unter den folgenden Gruppen ausgewählte Gruppen substituiert ist: ein Halogenatom, eine (C₁-C₄)-Alkylgruppe, eine (C₁-C₄) -Alkoxygruppe, eine Fluor-(C₁-C₄) -alkylgruppe, eine Fluor-(C₁-C₄)-alkoxygruppe, eine Nitrogruppe,
oder auch durch eine -NR₅R₆-Gruppe, wobei R₅ und R₆ unabhängig voneinander für ein Wasserstoffatom oder eine (C₁-C₄)-Alkylgruppe stehen,
- eine unter 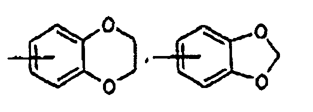 ausgewählte Gruppe
steht,
• R₁ für
- eine -C(O)-CF₃-Gruppe,
- eine Gruppe der Formel: 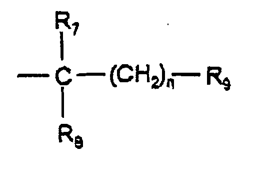 worin:
n = 0, 1 oder 2,
o R₇ für
- ein Wasserstoffatom oder eine (C₁-C₄)-Alkylgruppe
steht,
o R₈ für
- ein Wasserstoffatom
steht,
o R₉ für
- ein Wasserstoffatom, eine (C₁-C₄)-Alkylgruppe, eine Hydroxylgruppe, eine (C₁-C₄)-Alkoxygruppe, eine Fluor-(C₁-C₄)-alkylgruppe, eine -C≡CH-Gruppe, eine -C≡N-Gruppe,
- eine -NR₁₀R₁₁-Gruppe, wobei R₁₀ und R₁₁ unabhängig voneinander für ein Wasserstoffatom, eine (C₁-C₄) -Alkylgruppe, eine (C₁-C₄) -Alkylcarbonylgruppe oder eine Benzylgruppe stehen, oder R₁₀ und R₁₁ zusammen mit dem Stickstoffatom, an das sie gebunden sind, ein Pyrrolidin, ein Piperidin oder ein Pyrrolidinon bilden,
- eine -CO-NR₁₂R₁₃-Gruppe, wobei R₁₂ und R₁₃ unabhängig voneinander für ein Wasserstoffatom oder eine (C₁-C₄)-Alkylgruppe stehen,
oder R₁₂ und R₁₃ zusammen mit dem Stickstoffatom, an das sie gebunden sind, eine Gruppe 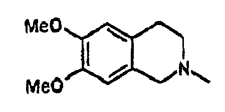 bilden,
- eine -COOR₁₄-Gruppe, wobei R₁₄ für eine (C₁-C₄)-Alkylgruppe steht,
- eine -NH-CO-NR₁₅R₁₆-Gruppe, wobei R₁₅ und R₁₆ unabhängig voneinander für ein Wasserstoffatom, eine (C₁-C₄)-Alkylgruppe, eine Phenylgruppe oder eine Benzylgruppe stehen,
- eine unter gegebenenfalls am Stickstoffatom durch eine (C₁-C₄)-Alkylgruppe substituiertem Imidazolyl, gegebenenfalls durch eine (C₁-C₄)-Alkylgruppe substituiertem Tetrazolyl und Triazolyl ausgewählte Heterocyclylgruppe steht,
steht,
in Basen-, Säureadditionssalz-, Hydrat- oder Solvatform, in Form von Enantiomeren, Diastereoisomeren, Rotameren, Atropisomeren oder Gemischen davon.

3. Verbindung der allgemeinen Formel (I) nach Anspruch 1 oder 2, worin
• Ar₁ für
- eine Arylgruppe wie Phenyl, die gegebenenfalls durch eine oder mehrere unabhängig voneinander unter den folgenden Gruppen ausgewählte Gruppen substituiert ist: ein Halogenatom, eine (C₁-C₄)-Alkylgruppe, eine (C₁-C₄)-Alkoxygruppe, eine Fluor-(C₁-C₄)-alkylgruppe,
- eine unter Pyridinyl, Thiazolyl und Thienyl ausgewählte Heterocyclylgruppe, wobei das Thienyl gegebenenfalls durch eine (C₁-C₄)-Alkylgruppe substituiert ist,
steht,
• T für
- eine Gruppe -(CH₂)ₙ- mit n = 1,
- eine Gruppe 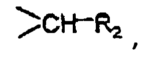 wobei R₂ für eine Hydroxylgruppe steht,
steht,
• Ar₂ für
- eine Arylgruppe wie Phenyl, die gegebenenfalls durch eine oder mehrere unabhängig voneinander unter den folgenden Gruppen ausgewählte Gruppen substituiert ist: ein Halogenatom, eine (C₁-C₄)-Alkylgruppe, eine (C₁-C₄)-Alkoxygruppe, eine -NR_{c}R_{d}-Gruppe, wobei R_{c} und R_{d} unabhängig voneinander für ein Wasserstoffatom oder eine (C₁-C₄)-Alkylgruppe stehen,
steht,
• Ar₃ für
- eine Arylgruppe wie Phenyl, wobei die Phenylgruppe gegebenenfalls durch eine oder mehrere unabhängig voneinander unter den folgenden Gruppen ausgewählte Gruppen substituiert ist: ein Halogenatom, eine (C₁-C₄)-Alkylgruppe, eine (C₁-C₄) - Alkoxygruppe,
oder auch durch eine -NR₅R₆-Gruppe, wobei R₅ und R₆ unabhängig voneinander für ein Wasserstoffatom oder eine (C₁-C₄)-Alkylgruppe stehen,
steht,
• R₁ für
- eine Gruppe der Formel: 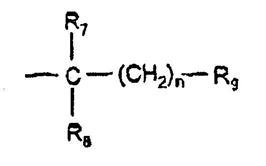 worin:
n = 0, 1 oder 2,
o R₇ für
- ein Wasserstoffatom oder eine (C₁-C₄)-Alkylgruppe
steht,
o R₈ für
- ein Wasserstoffatom
steht,
o R₉ für
- ein Wasserstoffatom, eine -C≡CH-Gruppe, eine -C≡N-Gruppe,
- eine -CO-NR₁₂R₁₃-Gruppe, wobei R₁₂ und R₁₃ unabhängig voneinander für ein Wasserstoffatom oder eine (C₁-C₄)-Alkylgruppe stehen,
- ein unter gegebenenfalls am Stickstoffatom durch eine (C₁-C₄)-Alkylgruppe substituiertem Imidazolyl, gegebenenfalls durch eine (C₁-C₄)-Alkylgruppe substituiertem Tetrazolyl und Triazolyl ausgewähltes Heterocyclyl steht,
steht,
in Basen-, Säureadditionssalz-, Hydrat- oder Solvatform, in Form von Enantiomeren, Diastereoisomeren, Rotameren, Atropisomeren oder Gemischen davon.

4. Verbindung der allgemeinen Formel (I) nach einem der Ansprüche 1 bis 3, worin
• Ar₁ für
- eine Arylgruppe wie Phenyl, die gegebenenfalls durch eine oder mehrere unabhängig voneinander unter den folgenden Gruppen ausgewählte Gruppen substituiert ist: ein Halogenatom, eine (C₁-C₄)-Alkylgruppe, eine (C₁-C₄)-Alkoxygruppe,
- eine Heterocyclylgruppe wie Thienyl, die gegebenenfalls durch eine (C₁-C₄)-Alkylgruppe substituiert ist,
steht,
• T für
- eine Gruppe - (CH₂)ₙ- mit n = 1
steht,
• Ar₂ für
- eine Phenylgruppe, die gegebenenfalls durch eine oder mehrere unabhängig voneinander unter den folgenden Gruppen ausgewählte Gruppen substituiert ist: ein Halogenatom, eine (C₁-C₄)-Alkylgruppe, eine (C₁-C₄)-Alkoxygruppe, eine -NR_{c}R_{d}-Gruppe, wobei R_{c} und R_{d} unabhängig voneinander für ein Wasserstoffatom oder eine (C₁-C₄)-Alkylgruppe stehen,
steht,
• Ar₃ für
- eine Phenylgruppe, die gegebenenfalls durch eine oder mehrere unabhängig voneinander unter den folgenden Gruppen ausgewählte Gruppen substituiert ist: ein Halogenatom, eine (C₁-C₄)-Alkylgruppe, eine (C₁-C₄)-Alkoxygruppe,
oder auch durch eine -NR₅R₆-Gruppe, wobei R₅ und R₆ unabhängig voneinander für ein Wasserstoffatom oder eine (C₁-C₄)-Alkylgruppe stehen,
steht,
• R₁ für
- eine Gruppe der Formel: 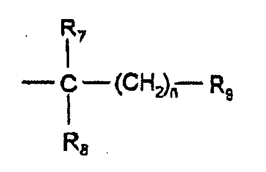 worin:
n = 0, 1 oder 2,
o R₇ für
- ein Wasserstoffatom oder eine (C₁-C₄)-Alkylgruppe
steht,
o R₈ für
- ein Wasserstoffatom
steht,
o R₉ für
- eine -CO-NR₁₂R₁₃-Gruppe, wobei R₁₂ und R₁₃ unabhängig voneinander für ein Wasserstoffatom oder eine (C₁-C₄)-Alkylgruppe stehen,
- ein unter am Stickstoffatom durch eine (C₁-C₄)-Alkylgruppe substituiertem Imidazolyl, durch eine (C₁-C₄)-Alkylgruppe substituiertem Tetrazolyl und Triazolyl ausgewähltes Heterocyclyl
steht,
steht,
in Basen-, Säureadditionssalz-, Hydrat- oder Solvatform, in Form von Enantiomeren, Diastereoisomeren, Rotameren, Atropisomeren oder Gemischen davon.

5. Verfahren zur Herstellung einer Verbindung der Formel (I) nach einem der Ansprüche 1 bis 4, **dadurch gekennzeichnet, daß** man:
- entweder die Verbindung der allgemeinen Formel (II) in Gegenwart einer Base mit der Verbindung der allgemeinen Formel (IV) alkyliert;
- oder eine Mitsunobu-Reaktion zwischen einem Alkohol der Formel (X) und einer Verbindung der allgemeinen Formel (II) durchführt: 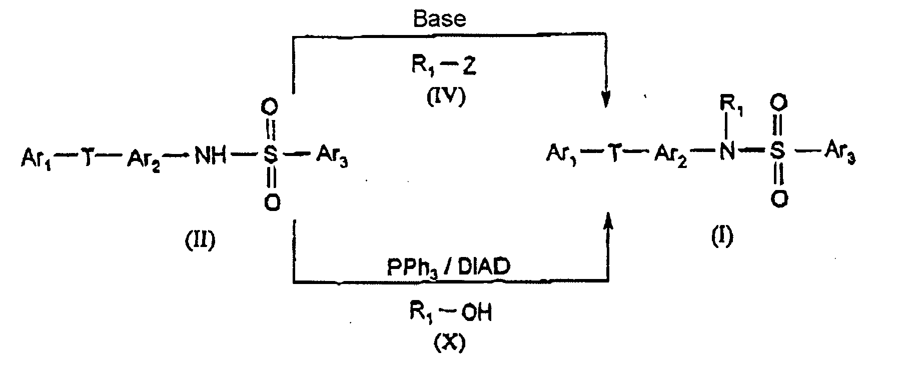 wobei in den Verbindungen der Formel (II), (IV) und (X) Ar₁, Ar₂, Ar₃, T und R₁ die in Formel (I) angegebene Bedeutung besitzen und Z für eine Abgangsgruppe steht.

6. Herstellungsverfahren nach Anspruch 5, **dadurch gekennzeichnet, daß** man die Verbindung der Formel (II) durch Sulfonylierung der Verbindung der Formel (III) mit Sulfonylchloriden der Formel (V) in Gegenwart einer Base erhält: 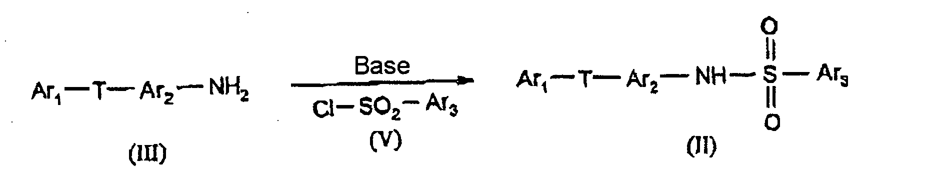 wobei in den Verbindungen der Formel (III) und (V) Ar₁, Ar₂, Ar₃ und T die in Formel (I) von Anspruch 1 angegebene Bedeutung besitzen und Z für eine Abgangsgruppe steht.

7. Verwendung einer Verbindung der Formel (I) nach einem der Ansprüche 1 bis 4 zur Herstellung eines Arzneimittels zur Prophylaxe oder Behandlung von Pathologien wie Obesitas, Appetit- oder Geschmacksstörungen einschließlich Kachexie, Anorexie, Bulimie, Diabetes, metabolischen Syndromen, Erbrechen und Übelkeit, Depression und Angst, Abhängigkeiten, Gemüts- und Verhaltensstörungen, Schizophrenie, Schlafstörungen, Syndrom der unruhigen Beine, Gedächtnislernstörungen, sexuellen und psychosexuellen Dysfunktionen, Schmerzen, viszeralen und neuropathischen Schmerzen, Hyperalgesie, Allodynie, Verdauungsstörungen, Reizkolon, neuronaler Degeneration, ischämischen und hämorrhagischen Attacken, Morbus Cushing, Guillain-Barre-Syndrom, myotonischer Dystrophie, Harninkontinenz, Hyperthyreoidismus, Hypophysenfunktionsstörungen, Hypertonie oder Hypotonie.

8. Arzneimittel, **dadurch gekennzeichnet, daß** es eine Verbindung der Formel (I) nach einem der Ansprüche 1 bis 4 enthält.

9. Pharmazeutische Zusammensetzung, enthaltend mindestens eine Verbindung der Formel (I) nach einem der Ansprüche 1 bis 4 und gegebenenfalls einen oder mehrere pharmazeutisch unbedenkliche Hilfsstoffe.
